# FASCICULE DE BREVET EUROPEEN

(11) **EP 2 941 427 B1**
(45) Date de publication et mention de la délivrance du brevet: **09.11.2016**
(21) Numéro de dépôt: 14700085.5
(22) Date de dépôt: 07.01.2014
(51) Int. Cl.: C07D 413/14, A61K 31/5355, A61P 25/00

(54) **NOUVEAUX DÉRIVÉS BENZODIOXANE-PIPERIDINE ET LEURS APPLICATIONS THÉRAPEUTIQUES POUR LE TRAITEMENT DES AFFECTIONS NEUROPSYCHIATRIQUES**
NEUARTIGE BENZODIOXANPIPERIDINDERIVATE UND THERAPEUTISCHE VERWENDUNGEN DAVON ZUR BEHANDLUNG NEUROPSYCHIATRISCHEN ERKRANKUNGEN
NOVEL BENZODIOXANE-PIPERIDINE DERIVATIVES AND THERAPEUTIC USES THEREOF FOR THE TREATMENT OF NEUROPSYCHIATRIC DISORDERS

(30) Priorité: 07.01.2013 FR 1350088
(43) Date de publication de la demande: 11.11.2015
(73) Titulaire: Pierre Fabre Médicament, 92100 Boulogne-Billancourt (FR)
(72) Inventeur: SOKOLOFF, Pierre, F-81540 Belleserre (FR); MAILLOS, Philippe, F-81500 Labastide Saint Georges (FR); CUISIAT, Florence, F-81100 Castres (FR); VIDALUC, Jean-Louis, F-81100 Castres (FR); IMBERT, Thierry, F-81290 Viviers Les Montagnes (FR)
(74) Mandataire: Regimbeau
(86) Numéro de dépôt international: PCT/EP2014/050131
(87) Numéro de publication internationale: WO 2014/106660

(56) Documents cités:
- FR-A1- 2 789 681

## Description

L'invention concerne des dérivés benzodioxane-pipéridine-oxazolidinone et benzodioxane-pipéridine-morpholinone, les compositions pharmaceutiques les contenant et leurs applications thérapeutiques, en tant qu'agonistes partiels ou antagonistes des récepteurs D2 et D3 de la dopamine et qu'antagonistes des récepteurs adrénergiques α2C pour le traitement de diverses affections neurologiques et psychiatriques. Selon l'invention, ces affections incluent, de manière non limitative, la schizophrénie, le trouble bipolaire, l'autisme et les troubles cognitifs associés à différentes affections neurologiques.

La schizophrénie est un terme employé pour décrire un groupe de pathologies d'origine mal connue, qui affecte approximativement 1 % de la population générale. Cette pathologie est caractérisée par une variété de symptômes, qui sont classifiés comme symptômes positifs (hallucinations, délires, pensée désorganisée) et symptômes négatifs (retrait social et émoussement affectif), à un âge de début dans l'adolescence ou le début de l'âge adulte et peut persister sous forme chronique avec des épisodes d'exacerbation pendant de nombreuses années. Les déficits cognitifs, tels les troubles des fonctions exécutives, de la mémoire, et de l'expression verbale font partie intégrante de la symptomatologie tout au long du décours de cette affection.

Des patients atteints de schizophrénie peuvent être traités avec des médicaments appelés neuroleptiques, également connus sous le nom d'antipsychotiques. L'effet thérapeutique des antipsychotiques est généralement reconnu comme résultant du blocage des récepteurs du neuromédiateur dopamine dans le cerveau. Il y a cinq sous-types connus de récepteurs de la dopamine, appelés D1, D2, D3, D4 et D5 (Sokoloff, P. et al., Novel dopamine receptor subtypes as targets for antipsychotic drugs. Annals New-York Academy of Sciences, 1995, 757, 278-292) et les antipsychotiques conventionnels sont des antagonistes (ou des agonistes partiels, par exemple l'aripiprazole) des récepteurs D2 et D3. L'importance du blocage du sous-type D3 pour le traitement de la schizophrénie et d'autres affections a été soulignée (Sokoloff P, Diaz J, Le Foll B, Guillin O, Leriche L, Bezard E, Gross C. The dopamine D3 receptor: a therapeutic target for the treatment of neuropsychiatric disorders. 2006, 5: 25-43).

Les antipsychotiques basés sur le blocage concomitant des récepteurs D2 et D3 sont relativement actifs pour améliorer les symptômes positifs de la schizophrénie. En revanche, ils sont peu ou pas actifs contre les symptômes négatifs et pour améliorer les déficits cognitifs. Il y a donc un besoin de trouver de nouveaux principes pour des antispychotiques à efficacité améliorée.

La littérature mentionne aussi que des produits antagonistes alpha2-adrénergiques potentialisent l'effet des antipsychotiques (Litman RE, Hong WW, Weissman EM, Su TP, Potter WZ, Pickar D. Idazoxan, an alpha 2 antagonist, augments fluphenazine in schizophrenic patients: a pilot study. J Clin Psychopharmacol. 1993, 13:264-267) et agissent favorablement sur les déficits cognitifs (Marien MR, Colpaert FC, Rosenquist AC. Noradrenergic mechanisms in neurodegenerative diseases: a theory Brain Res Brain Res Rev. 2004; 45:38-78; Svensson TH. Alpha-adrenoceptor modulation hypothesis of antipsychotic atypicality. Prog Neuropsychopharmacol Biol Psychiatry. 2003 Oct;27(7):1145-58).

Les récepteurs alpha2-adrénergiques jouent un rôle critique dans la régulation de l'activité neuronale et de la libération des neurotransmetteurs. Les récepteurs alpha2-adrénergiques sont constitués de trois sous-types génétiquement et fonctionnellement distincts : alpha2A, alpha2B et alpha2C. Des études comparatives avec des souris transgéniques ont montré que le sous-type alpha2A est le médiateur principal de certains effets induits par des agonistes alpha2 adrénergiques non sélectifs des sous-types, tels que la sédation, l'analgésie, l'hypothermie, l'inhibition sympathique et la réduction de la pression artérielle. De la même façon, des tests neuropsychopharmacologiques chez les souris KO (« knock out »), qui n'expriment pas le récepteur ou chez les souris OE (« over expressed ») qui surexpriment le récepteur montrent que le sous-type alpha2C adrénergique a un rôle distinct inhibiteur dans le développement des informations sensorielles, et dans le contrôle des activités cognitives, motrices, émotionnelles ou reliées au stress du système nerveux central (Scheinin M, Sallinen J, Haapalinna A (2001) Evaluation of the alpha2C-adrenoceptor as a neuropsychiatric drug target : studies in transgenic mouse models. Life Sci. 68: 2277-2285).

Il a été donc proposé que les composés sélectifs des récepteurs alpha2C puissent avoir une utilité thérapeutique dans des troubles ou états divers du système nerveux central, par exemple, pour améliorer les symptômes cognitifs associés avec le trouble du déficit de l'attention avec hyperactivité ou les maladies neurodégénératives et leur progression. Parmi ces maladies, on peut citer à titre d'exemples non exhaustifs la maladie de Parkinson, la maladie d'Alzheimer, la chorée d'Huntington, la sclérose latérale amyotrophique, la paralysie supranucléaire progressive, le syndrome de Down, la dégénérescence corticobasale, la démence de pugiliste, l'atrophie des systèmes multiples, la maladie à corps de Lewy, la maladie de Pick), ou encore associés à l'âge ou aux les troubles de l'humeur, la dépression, les troubles d'anxiété et des maladies mentales propagées par le stress. Sont concernés également les troubles ischémiques et post-ischémiques cérébraux, les accidents vasculaires cérébraux et leurs conséquences, la narcolepsie (MacDonald E, Kobilka BK, Scheinin M. Gene targeting: homing in on alpha 2-adrenoceptor-subtype function. Tends Pharmacol Sci. 1997, 18:211-219; Arnsten AF. Adrenergic targets for the treatment of cognitive déficits in schizophrenia. Psychopharmacology (Berl) 2004,174:25-31).

Sont aussi concernés par l'utilisation thérapeutique des produits antagonistes ou agonistes partiels des récepteurs D2 et D3 et antagonistes des récepteurs alpha2C, objet de la présente invention, les troubles et pathologies dans lesquels les patients sont généralement, ou de manière occasionnelle, traités par des antipsychotiques, tels que le trouble bipolaire, les troubles du spectre autistique et la dépression majeure.

Le trouble bipolaire est un trouble de l'humeur, historiquement connu comme trouble maniaco-dépressif, où les patients vivent alternativement dans un état où l'excitation est anormalement élevée (état maniaque ou hypomaniaque) et, dans de nombreux cas, dans un état anormalement déprimé. Ce trouble revêt plusieurs formes. Le trouble bipolaire peut apparaître comme la dépression unipolaire : état dépressif cyclique, sans la présence de manie (ou d'hypomanie). Le trouble bipolaire commence dans l'enfance ou l'âge adulte pour la plupart des patients, le plus souvent les premiers épisodes sont la dépression. Les estimations de la prévalence sur la vie du trouble bipolaire varient, avec des études donnant généralement des valeurs de l'ordre de 1%. Le DSM-IV-TR (Diagnostic and Statistical Manual of Mental Disorders, Fourth Edition, American Psychiatric Association, 2004) énumère trois sous-types spécifiques et un pour les non-spécifié :
- Le trouble bipolaire I : un ou plusieurs épisodes maniaques.
- Le trouble bipolaire II : pas d'épisodes maniaques, mais un ou plusieurs épisodes hypomaniaques et un ou plusieurs épisodes dépressifs majeurs.
- La cyclothymie : des antécédents d'épisodes hypomaniaques avec des périodes de dépression, qui ne répondent pas aux critères des épisodes dépressifs majeurs.

Les antipsychotiques sont généralement utilisés pour traiter la phase maniaque ou hypomaniaque. Certains, dont la quétiapine, sont aussi utilisés pour traiter les épisodes dépressifs de la maladie bipolaire.

Certains antipsychotiques sont aussi utilisés pour traiter les troubles autistiques, qui sont constitués de troubles de l'apprentissage et de la socialisation, ainsi que de désordres liés au stress chez l'enfant et l'adolescent, qu'ils soient associés aux Troubles Envahissants du Développement spécifiés ou non spécifiés, aux Troubles Désintégratifs de l'Enfant et de l'Adolescent, aux Psychoses Précoces Déficitaires, aux Dysharmonies Psychotiques, aux Autismes ou aux Troubles de l'Humeur à Forme Atypique, selon les classifications de la CIM-10 (10ème édition du « International Statistical Classification of Diseases and Related Health Problems », ICD-10, Classification of Mental and Behavioral Disorders : diagnostic criteria for research, World Health Organization, 1993 revised 1997), dans le DSM-IV-TR (Diagnostic and Statistical Manual of Mental Disorders, Fourth Edition, American Psychiatric Association, 2004) et la CFTMEA-R Classification Française des Troubles Mentaux de l'Enfant et de l'Adolescent (Mises, Quemada, CFTMEA R 2000. CTNERHI, Paris 2002 ; C. Bursztejn, P. Jeammet. Autisme et psychoses de l'enfant dans la CFTMEA R-2000- Autism and children psychoses in the CFTMEA R-2000, Annales médico-psychologiques, 160 : 216-219, 2002).

Enfin, des antipsychotiques sont utilisés dans les formes sévères de dépression majeure, chez des patients présentant une anxiété très importante avec des idées délirantes.

La tolérance cardiovasculaire est un facteur clé dans la sélection d'un candidat antagoniste des récepteurs alpha2 adrénergiques pour un développement clinique. Ainsi, la compréhension du rôle respectif des récepteurs alpha2 adrénergiques et des autres récepteurs adrénergiques dans la régulation de la fonction cardiovasculaire est fondamentalement importante. Quelques clarifications sur ce sujet ont été apportées par l'étude des souris KO (MacDonald et al., 1997, déjà cité) démontrant que l'activation des récepteurs alpha2B adrénergiques contrecarre les effets hypotenseurs des agonistes alpha2, faisant intervenir le sous-type alpha2A. Ces études indiquent également que les récepteurs alpha2C adrénergiques, en dépit de leur distribution dans des positions stratégiques dans le système nerveux central et en périphérie, ce qui pourrait théoriquement modifier l'homéostasie cardiovasculaire, semblent ne pas avoir d'impact fonctionnel significatif, tout au moins pas dans les modèles d'animaux transgéniques. Ainsi un antagoniste des récepteurs alpha2 adrénergiques qui serait sélectif pour le sous-type C pourrait avoir un impact cardiovasculaire réduit ou une meilleure tolérance comparativement à un alpha2 antagoniste non sélectif d'un sous-type.

L'art antérieur mentionne, des dérivés benzodioxane pipéridine portant une amide, une urée ou une imidazolidinone, comme antagonistes alpha2 non sélectifs (WO 97/28157, WO 98/02435) ; certains de ces produits possèdent aussi une faible affinité pour le récepteur dopaminergique D2 (T.Imbert, J Med. Chem, 2000, 43 : 3653-3664). Au cours de l'élaboration de la présente invention, les inventeurs ont montré que, ces produits déjà décrits agissent comme agonistes efficaces ou très efficaces (agonistes pleins) des récepteurs dopaminergiques D2 et D3. L'activité en tant qu'agoniste des récepteurs D2 et D3 a été déterminée selon les méthodes décrites (Bruins Slot LA, De Vries L, Newman-Tancredi A, Cussac D. Differential profile of antipsychotics at serotonin 5-HT1A and dopamine D2S receptors coupled to extracellular signal-regulated kinase. Eur J Pharmacol, 2006, 534 : 63-70 ; Bruins Slot LA, Palmier C, Tardif S, Cussac D. Action of novel antipsychotics at human dopamine D3 receptors coupled to G protein and ERK1/2 activation. Neuropharmacology, 2007, 53 : 232-241) pour les composés suivants, tels que décrits dans WO 97/28157 et WO 98/02435 :
- 1-benzyl-3-(2-(1-((2,3-dihydrobenzo[b][1,4]dioxin-2-yl)méthyl)pipéridin-4-yl)éthyl)imidazolidin-2-one
- 1-(2-(1-((2,3-dihydrobenzo[b][1,4]dioxin-2-yl)méthyl)pipéridin-4-yl)éthyl)-3-méthylimidazolidin-2-one
- 1-(2,6-dichlorophenyl)-3-(2-(1-((2,3-dihydrobenzo[b][1,4]dioxin-2-yl)méthyl)pipéridin-4-yl)éthyl)imidazolidin-2-one
- 1-(2-(1-((2,3-dihydrobenzo[b][1,4]dioxin-2-yl)méthyl)pipéridin-4-yl)éthyl)-3-(pyridin-4-yl)imidazolidin-2-one
- 3-(2-(1-((2,3-dihydrobenzo[b][1,4]dioxin-2-yl)méthyl)pipéridin-4-yl)éthyl)-1-phenylimidazolidine-2,4-dione
- (S)-N-(2-(1-((2,3-dihydrobenzo[b][1,4]dioxin-2-yl)méthyl)pipéridin-4-yl)éthyl)-[ 1, 1'-biphenyl]-4-carboxamide
- (S)-N-(2-(1-((2,3-dihydrobenzo[b][1,4]dioxin-2-yl)méthyl)pipéridin-4-yl)éthyl)-4-( 1H-pyrro-1-1-yl)benzamide
- (S)-N-(2-(1-((2,3-dihydrobenzo[b][1,4]dioxin-2-yl)méthyl)pipéridin-4-yl)éthyl)-4-(pyridin-4-yl)benzamide
- (S)-N-(2-(1-((2,3-dihydrobenzo[b][1,4]dioxin-2-yl)méthyl)pipéridin-4-yl)éthyl)-4-(pyridin-3-yl)benzamide
- (S)-N-(2-(1-((2,3-dihydrobenzo[b][1,4]dioxin-2-yl)méthyl)pipéridin-4-yl)éthyl)-9-oxo-9H-fluorene-1-carboxamide.

Pour ces composés, l'activité intrinsèque (en pourcentage de celle de la dopamine) variait de 20 à 88 % au récepteur D2 et de 85 à 102% au récepteur D3. La puissance des produits (concentration produisant la moitié de la réponse maximale) variait de 1,9 à 320 nanomoles/litre au récepteur D2 et de 3,3 à 43 nanomoles/litre au récepteur D3. Ces produits se comportent donc comme des agonistes efficaces à très efficaces sur les récepteurs D2 et D3, ce qui n'est pas recherché pour en faire des antipsychotiques.

De façon surprenante et inattendue l'introduction de substituants sur la partie benzodioxane ou / et le remplacement des parties amide, urée ou imidazolidinone par un hétérocycle de type oxazolidinone ou morpholinone, ont un impact pharmacologique majeur et multiple, qui rend ces produits beaucoup plus intéressants pour en faire de nouveaux antipsychotiques. En effet, ces modulations chimiques :
- augmentent l'affinité au récepteur dopaminergique D2
- rend ces nouveaux produits plus affins au récepteur D3
- diminue leur pouvoir agoniste sur les récepteurs D2 et D3
- rend ces produits plus puissants antagonistes alpha2 sur le sous-type alpha2C que sur les sous-types alpha2A et alpha2B.

Les composés de l'invention peuvent donc être employés pour la préparation de compositions et de médicaments pharmaceutiques pour le traitement de maladies, affections ou troubles neurologiques ou psychiatriques, tels que décrits ci-dessus, pour lesquels sont recherchés des produits qui soient à la fois antagonistes D2 et D3, avec une affinité préférentielle pour le récepteur D3, et antagonistes alpha2C.

Comme utilisé ici, le terme « sels » désigne les sels inorganiques d'addition d'acides et de bases des composés de la présente invention. De préférence, les sels sont pharmaceutiquement acceptables, c'est-à-dire qu'ils sont non-toxiques pour le patient auquel ils sont administrés.

Le terme « pharmaceutiquement acceptable » se réfère à des entités moléculaires et des compositions qui ne produisent aucun effet adverse, allergique ou autre réaction indésirable quand elles sont administrées à un animal ou un humain.

Quand utilisé ici, le terme « excipient pharmaceutiquement acceptable » inclut tout diluant, adjuvant ou excipient, tel que des agents préservatifs, des agents de remplissage, des agents désintégrant, mouillant, émulsifiant, dispersant, antibactérien ou antifongique, ou bien encore des agents qui permettraient de retarder l'absorption et la résorption intestinale et digestive. L'utilisation de ces milieux ou vecteurs est bien connue de l'art. Excepté si l'agent est chimiquement incompatible avec un dérivé chromone, son utilisation dans des compositions pharmaceutiques avec les composés selon l'invention est envisagée.

Dans le contexte de l'invention, le terme « traitement », comme utilisé ici, signifie empêcher ou inhiber la survenue ou la progression de l'affection à laquelle le terme s'applique, ou bien d'un ou de plusieurs symptômes de cette affection.

« Quantité thérapeutiquement active » signifie une quantité d'un dérivé benzodioxane effective pour obtenir l'effet thérapeutique désiré selon l'invention.

Selon l'invention, le terme « patient » se réfère à un mammifère humain ou non humain affecté ou bien susceptible d'être affecté par une pathologie. Préférentiellement, le patient est un humain.

Par groupe alkyle en C₁₋₄, on entend au sens de la présente invention, une chaîne hydrocarbonée linéaire ou ramifiée, comprenant de 1 à 4 atomes de carbone.

Par groupe alkoxy en C₁₋₄, on entend au sens de la présente invention, une chaîne hydrocarbonée linéaire ou ramifiée comprenant de 1 à 4 atomes de carbone et un atome d'oxygène.

Par groupe halogène, on entend au sens de la présente invention, le Fluor, le Chlore ou le Brome.

Par groupe hydroxyalkyle en C₁₋₄, on entend au sens de la présente invention un groupe alkyle tel que défini précédemment dans lequel un atome d'hydrogène est remplacé par un groupe hydroxyle.

Par groupe alkylcarbonyle en C₁₋₄,on entend au sens de la présente invention un groupe tel que C(=O)R avec R alkyle tel que défini précédemment.

Par groupe alkoxycarbonyle en C₁₋₄,on entend au sens de la présente invention un groupe tel que C(=O)OR avec R alkyle tel que défini précédement.

Par groupe phénylalkyle en C₁₋₄, on entend au sens de la présente invention un phényle relié par un atome de carbone à un groupe alkyle tel que défini précédemment.

Par groupe alkoxyphenyle en C₁₋₄ on entend au sens de la présente invention un phényle relié par un atome d'oxygène à un un groupe alkyle tel que défini précédement.

Par groupe alkoxybenzyle en C₁₋₄ on entend au sens de la présente invention un benzyle relié par un atome d'oxygène à un groupe alkyle tel que défini précédement.

Par formes stéréoisomériques, on entend au sens de la présente invention, les énantiomères ainsi que les diastéréoisomères.

La présente invention concerne des dérivés benzodioxane pipéridine ioxazolidinone, et benzodioxane pipéridine morpholinone, et leur utilisation à titre de médicament, en tant qu'agonistes partiels ou antagonistes des récepteurs de la dopamine D2 et D3 et qu'antagoniste des récepteurs adrénergiques α2C pour le traitement de diverses affections neurologiques et psychiatriques.

Ces composés répondent à la formule générale 1. 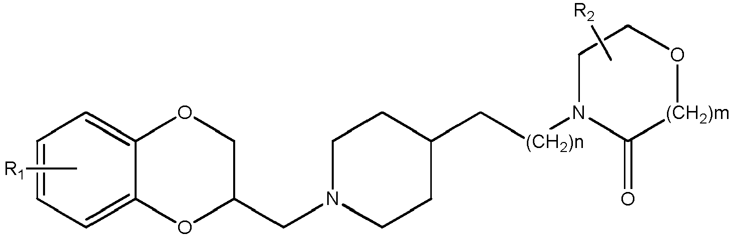 dans laquelle :
- R1 représente un ou plusieurs substituant(s) identique(s) ou différent(s) sur le noyau benzénique, chacun représentant indépendamment un atome d'hydrogène ou un halogène, ou un groupe alkyle en C₁₋₄, ou un groupe alkoxy en C₁₋₄,ou un groupe hydroxyalkyle en C₁₋₄, ou un groupe alkylcarbonyle, ou un groupe alkoxycarbonyle, ou un groupe OH, ou un groupe SO2R avec R alkyle, ou un groupe CN, ou un groupe CF3, ou un groupe OCF3 ;
- n = 1, 2 ou 3 ;
- R2 représente un ou plusieurs substituant(s) identique(s) ou différent(s) sur les noyaux oxazolidinone ou morpholinone, chacun représentant indépendamment :
   un atome d'hydrogène, ou un groupe alkyle en C1-4, ou un groupe alkoxy en C₁₋₄, ou un groupe hydroxyalkyle en C₁₋₄, ou un groupe alkylcarbonyle, ou un groupe alkoxycarbonyle, ou un groupe alkoxyphenyl - éventuellement substitué par un ou plusieurs substituant(s) identique(s) ou différent(s), chacun représentant indépendamment un groupe alkoxy en C1-4, ou un groupe alkyl en C1-4 ou un halogène, ou un hydroxy- ou un groupe alkoxybenzyle -éventuellement substitué par un ou plusieurs substituant(s) identique(s) ou différent(s), chacun représentant indépendamment un groupe alkoxy en C1-4, ou un groupe alkyl en C1-4 ou un halogène, ou un hydroxy- ou un groupe hydroxybenzyle, ou un groupe benzyle - éventuellement substitué par un ou plusieurs substituant(s) identique(s) ou différent(s), chacun représentant indépendamment un groupe alkoxy en C1-4, ou un groupe alkyl en C1-4 ou un halogène, ou un hydroxy - ou un groupe phényle -éventuellement substitué par un ou plusieurs substituant(s) identique(s) ou différent(s), chacun représentant indépendamment un groupe alkoxy en C1-4, ou un groupe alkyl en C1-4 ou un halogène, ou un hydroxy - ou alors R2 constitue un cycle fusionné avec le groupe oxazolidinone ou morpholinone qui le porte, constitué par un benzène substitué ou non par un alkoxy en C1-4, ou un hydroxy, ou un halogène ou un cyano ;
   - m = 0 ou 1.

L'invention concerne également les formes stéréisomèriques y compris énantiomériques des composés de formule générale 1, leurs sels pharmaceutiquement acceptables, ainsi que les compositions pharmaceutiques les renfermant, et leur utilisation en tant que médicament destiné au traitement des troubles du système nerveux central.

Les composés de la formule générale 1 sont préparés :
- selon le schéma 1.

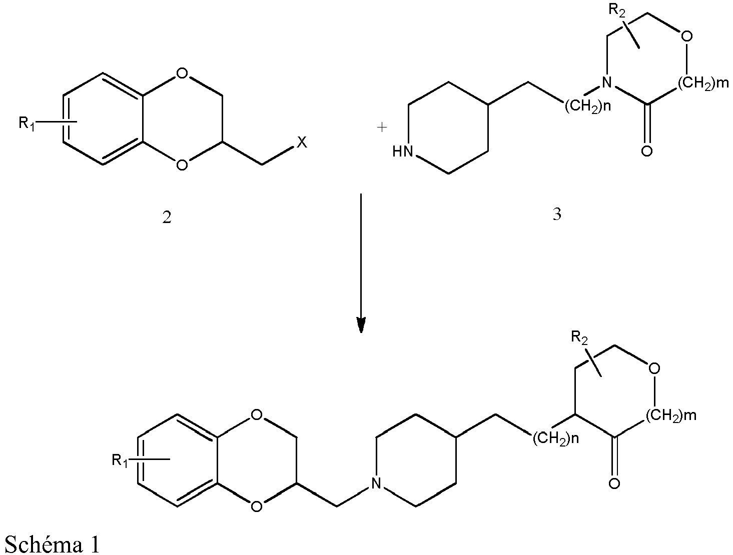

Les composés de formule générale 1 sont obtenus par substitution nucléophile ou couplage entre les dérivés pypéridinique de type 3 et les dérivés benzodioxanes de type 2 ou X est un groupe partant de type halogène, mesylate, tosylate ou brosylate.

Les dérivés de type 2 sont préparés à partir des composés hydroxylés 4 correspondants suivant le schéma 2. Les composés hydroxylés 4 sont préparés selon le procédé décrit dans J. Med. Chem, 1997, 40, 4235-4256, ou selon le procédé décrit dans J. Am. Chem. Soc, 2001, 123, 12202-12206.

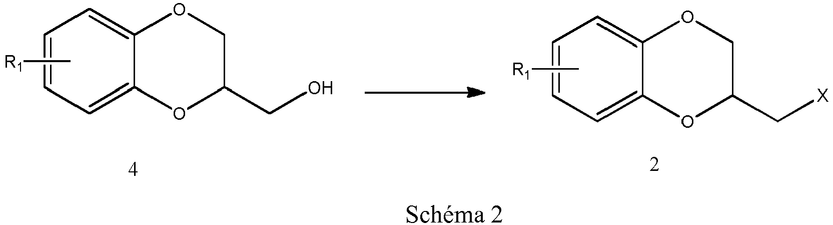

Les composés de type 3 sont préparés :
- par couplage de l'oxazolidinone ou de la morpholinone correspondante de type 5 avec un dérivé alkyle pipéridine protégé - ou P et un groupe protecteur et X un groupe partant tel que défini précédement - suivi d'une déprotection suivant le schéma 3. 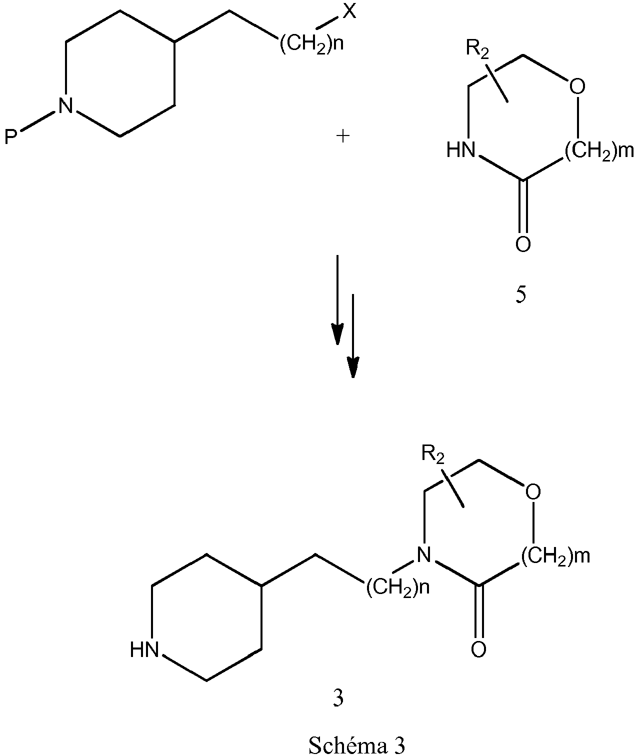
- ou pour m = 0 par construction du noyau oxazolidinone, suivant le schéma 4, à partir des dérivés pipéridinique amino alcool 6 par réaction avec le phosgène ou un analogue chimique tel que le carbonyle diimidazole, puis d'une déprotection de la fonction amine du groupement pipéridine.
- ou pour m = 1 par construction du noyau morpholinone, suivant le schéma 4, à partir des dérivés pipéridinique amino alcool 6 par réaction avec le chlorure de chloroacetyle, puis d'une déprotection de la fonction amine du groupement pipéridine.

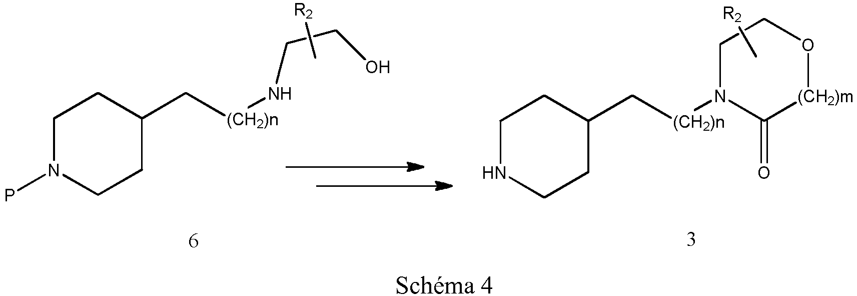

Les composés précédents de type 6 sont obtenus :
Lorsque R2 est porté par l'atome de carbone en α de l'atome d'azote de l'oxazolidinone ou de la morpholinone, à partir du produit piréridine alcool après oxydation en aldéhyde et amination réductrice avec les dérivés amino alcool 10 suivant le schéma 5 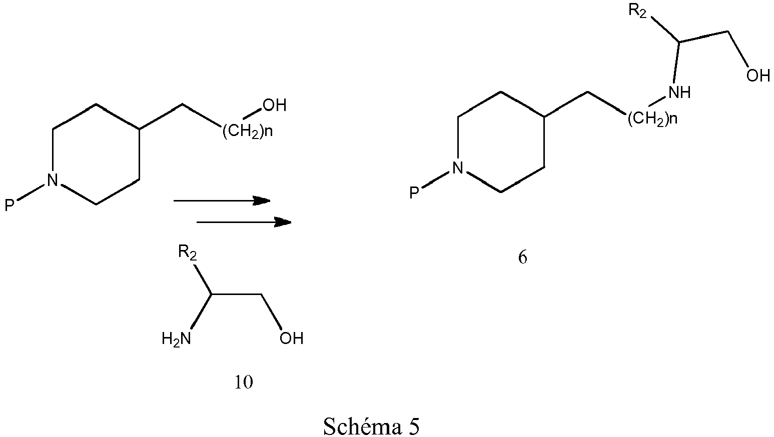 ou lorsque R2 est porté par l'atome de carbone en α de l'atome d'oxygène de l'oxazolidinone ou de la morpholinone, par ouverture des époxydes 11 par la pipéridine alkylamine elle même préparée à partir de la pipéridine alkylalcool suivant le schéma 6

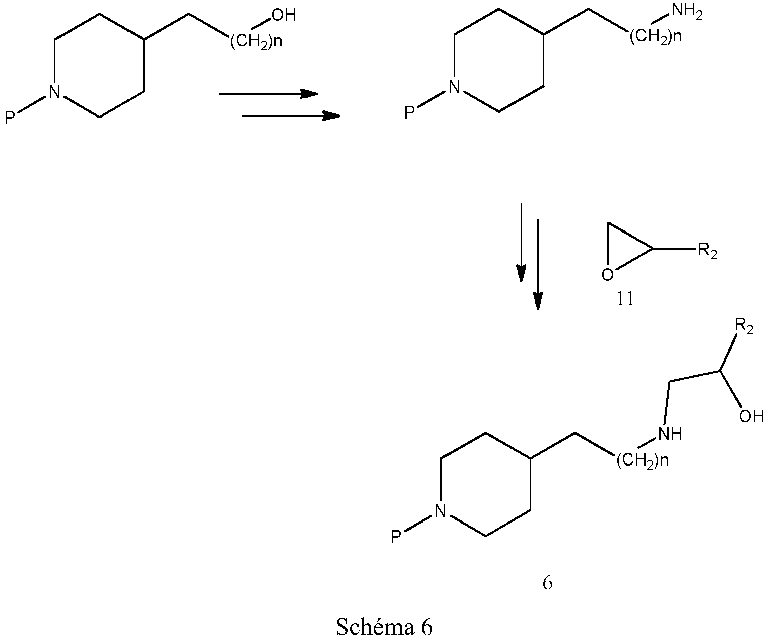

Pour les composés de type 3 ou R2 est un ester situé en α de l'azote de l'oxazolidinone ou de la morpholinone, celui-ci est soumis à des transformations chimiques telles que réduction en alcool et transformation en alkoxy 7, ou telles que réduction en aldéhyde. L'aldéhyde ou l'ester de départ soumis à l'action de nucléophiles donnent respectivement les composés hydroxylés de mono-addition 8 et de di-addition 9 ou R3 est un groupe alkyle ou phenyl ou benzyle, puis la fonction amine des composés 7,8,9 est déprotégée pour donner les composés 3 comme décrit dans le schéma 7

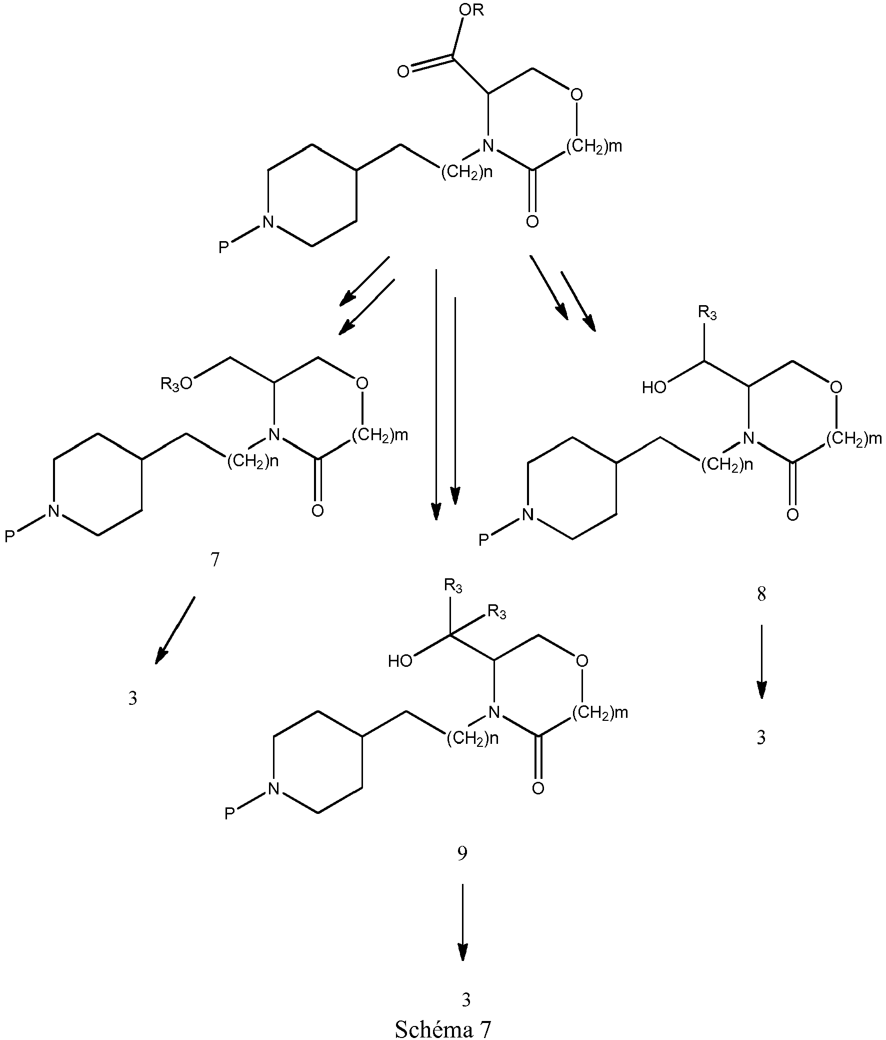

Les composés précédents de type 5 sont préparés, pour m = 0, lorqu'ils ne sont pas commerciaux :
soit lorsque R2 représente un seul substituant porté par le carbone en α de l'azote de l'oxazolidinone
   - pour R2 phényl substitué, à partir de l'aldéhyde correspondant qui par réaction de Strecker conduit aux benzylzamino cyanés de type 12 qui sont transformés en amino alcool de type 13 correspondants par transformation de la fonction nitrile en amide puis en acide qui est ensuite réduit en alcool et la fonction amine est libérée par débenzylation, l'amino alcool 10 ainsi obtenu est transformé en oxazolidinone 5 par réaction avec le phosgène ou un analogue chimique tel que la carbonyle di-imidazole suivant le schéma 8 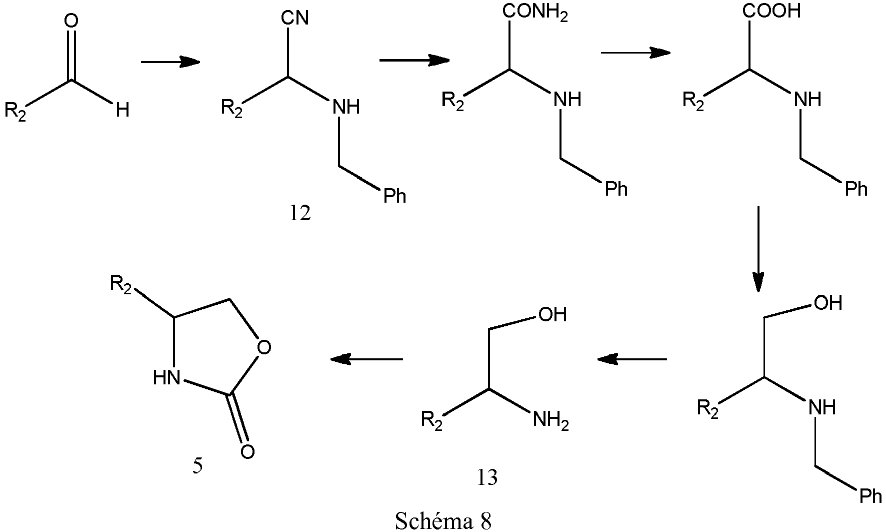
   - ou pour R2 benzyl substitué, à partir de l'amino acide ou ester correspondant 14, qui est réduit en amino alcool puis transformé en oxazolidinone correspondante par réaction avec le phosgène ou un analogue chimique tel que la carbonyle diimidazole suivant le schéma 9 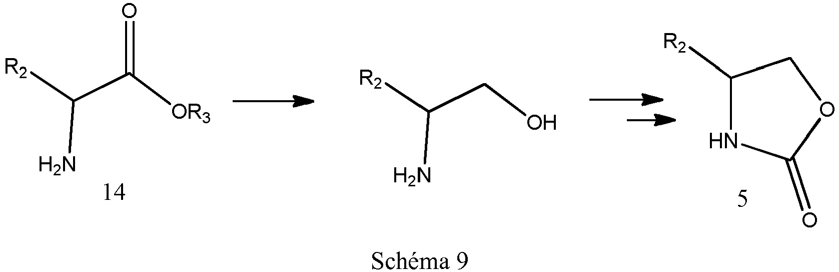
   - ou pour R2 alkoxy, ou alkoxybenzyle ou alkoxyphényle à partir de l'ester de la sérine qui est cyclisée en oxazolidinone correspondante par action du phosgène ou un analogue chimique tel que la carbonyle diimidazole, puis la fonction ester est réduite en alcool correspondant, la fonction hydroxy est transformée en groupe partant qui est ensuite substitué par action d'un alcoolate pour donner les composés 3 comme décrit dans le schéma 10 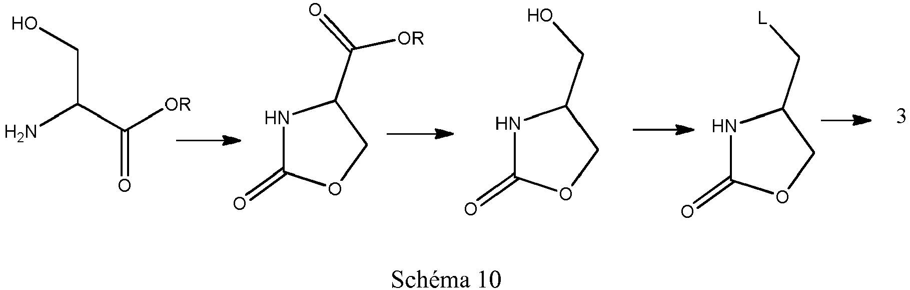
soit lorsque R2 représente plusieurs substituants identiques ou différents sur les noyaux oxazolidinone chacun représentant indépendamment :
   - un groupe alkyle en C₁₋₄
   - un groupe phenyle
   - un groupe benzyle
   - un groupe hydroxyalkyle en C₁₋₄
   à partir de l'ester de la sérine (S ou R) - ou de l'hydroxy amino acide correspondant pour des chaines plus longues- les fonctions amine et alcool sont protégées, le produit diprotégé ainsi obtenu est soit traité par un anion organique - par exemple un organomagnésien - pour donner le composé correspondant 15 disubstitué (R4 = alkyle, phenyle, benzyle), soit d'abord réduit en aldéhyde puis également traité par un anion pour donner le composé correspondant 16 monosubstitué (R4 = alkyle, phenyle, benzyle). Les composé 15 et 16 subissent ensuite les étapes de cyclisation / déprotection de l'amine ou inversement, qui sous l'action d'hydrure de sodium est cyclisé en oxazolidinone dont l'atome d'azote est ensuite déprotégé pour fournir les composés 5 correspondants après déprotection de la fonction alcool suivant le schéma 11

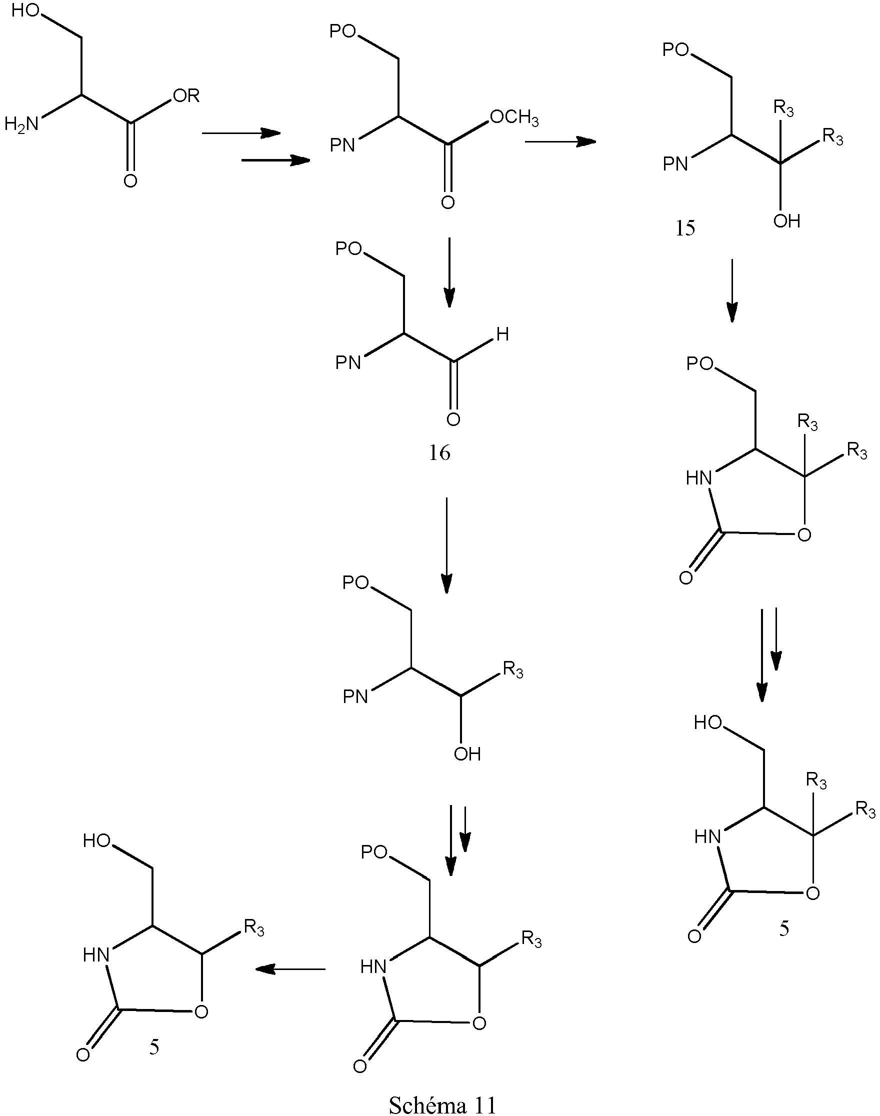

A titre d'exemple mais de façon non limitative les préparations des composés de l'invention sont illustrées dans les exemples suivants :

### Exemple 1 : (S)-3-(2-(1-((2,3-dihydrobenzo[b][1,4]dioxin-2-yl)méthyl)pipéridin-4-yl) éthyl)oxazolidin-2-one

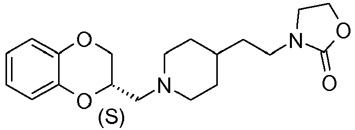

Dans un ballon monocol de 100 ml, on ajoute 0,8 g (3,58 mmol) de (R)-2-(bromométhyl)-2,3-dihydrobenzo[b][1,4]dioxine 2.1 à une solution de 0,71 g (3,58 mmol) de 3-(2-(pipéridin-4-yl)éthyl)oxazolidin-2-one 3.1 dans 15 ml d'acétonitrile. On introduit ensuite 0,65 g (4,7 mmol) de K₂CO₃ et une pointe de spatule de KI. On chauffe à 80°C pendant 8h00. Après retour à température ambiante, on concentre le milieu, puis on fait un lavage à l'eau et on extrait au dichlorométhane. La phase organique est séchée sur MgSO₄, filtrée, évaporée et purifiée par flash chromatographie sur gel de silice (éluant : dichlorométhane/acétate d'éthyle = 85/15%). On récupère 0,3 g (0,87 mmol) du composé 1 sous forme d'huile incolore.
Rendement : 24%
¹H RMN (CDCl₃) δ : 1,29 (ls, 3H) ; 1,48 (m, 2H) ; 1,71 (ls, 2H) ; 2,09 (m, 2H) ; 2,55 (m, 1H) ; 2,65 (m, 1H) ; 2,89 (m, 1H) ; 2,99 (m, 1H) ; 3,31 (t, 2H, J = 7,2 Hz) ; 3,54 (t, 2H, J = 8,4 Hz) ; 3,97 (dd, 1H, J = 7,8 et 8 Hz) ; 4,31 (m, 4H) ; 6,85 (m, 4H).

On dissout 0,3 g (0,87 mmol) du composé 1 dans un minimum de méthanol et on ajoute 0,4 ml (2 mmol) d'une solution d'HCl 5N dans l'isopropanol. On concentre le milieu et on triture avec de l'éther éthylique et de l'acétone. On filtre le précipité que l'on met à sécher à la cloche à vide. On obtient 0,19 g (0,50 mmol) du chlorhydrate du composé 1 sous la forme d'un solide blanc.
F : 120-123°C
[α] = -56,07 (c = 0,135, méthanol)
¹H RMN (DMSOd₆) δ : 1,51 (m, 5H) ; 1,9 (m, 2H) ; 3,02 (m, 2H) ; 3,19 (t, 2H, J = 6,8 Hz) ; 3,29 (m, 1H) ; 3,48 (m, 2H) ; 3,52 (t, 2H, J = 8 Hz) ; 3,66 (m, 1H) ; 4,05 (m, 1H) ; 4,25 (t, 2H, J = 8 Hz) ; 4,32 (m, 1H) ; 4,86 (m, 1H) ; 6,91 (m, 4H) ; 10,48 (ls, 1H).
MS m/z 347 (M+1).
HPLC (XBRIDGE C8, acétonitrile/eau/KH₂PO₄ (20/80/6,8G PH 4), 1 ml/min), temps de rétention = 10,08 min, pureté chimique = 99,4%
HPLC (CHIRALPACK AD-H, heptane/ethanol/diéthylamine (40/60/0,1), 0,8 ml/min) : composé 1, temps de rétention = 6,53 min, composé 2, temps de rétention = 5,92 min, rapport des AUC (1)/(2) = 99,3/0,7.

### Exemple 2 : (R)-3-(2-(1-((2,3-dihydrobenzo[b][1,4]dioxin-2-yl)méthyl)pipéridin-4-yl) éthyl)oxazolidin-2-one

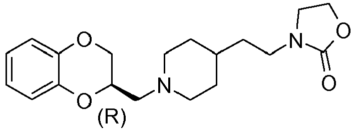

En procédant comme dans l'exemple 1, mais en remplaçant le (R)-2-(bromométhyl)-2,3-dihydrobenzo[b][1,4]dioxine 2.1 par le (S)-2-(bromométhyl)-2,3-dihydrobenzo [b][1,4]dioxine 2.2, on obtient 0,8 g (2,31 mmol) du composé 2 sous la forme d'huile jaune pâle, après purification par flash chromatographie sur gel de silice (éluant : dichlorométhane/acétate d'éthyle = 85/15%).
Rendement : 40%
¹H RMN (CDCl₃) δ : voir RMN exemple 1

On dissout 0,8 g (2,31 mmol) du composé 2 dans un minimum de méthanol et on ajoute 0,9 ml 4,52 mmol) d'une solution d'HCl 5N dans l'isopropanol. On concentre le milieu et on triture avec de l'éther éthylique et de l'acétone. On filtre le précipité que l'on met à sécher à la cloche à vide. On obtient 0,75 g (1,82 mmol) du chlorhydrate du composé 2 sous la forme d'un solide blanc.
[α] = +57,60 (c = 0,155, méthanol)
¹H RMN (DMSOd₆) δ : voir RMN Exemple 1
MS m/z 347 (M+1).
HPLC (CHIRALPACK AD-H, heptane/ethanol/diéthylamine (40/60/0,1), 0,8 ml/min) : composé 1, temps de rétention = 6,50 min, composé 2, temps de rétention = 5,88 min, rapport des AUC (1)/(2) = 1,2/98,8.

### Exemple 3 : (S)-3-((4-(2-(2-oxooxazolidin-3-yl)éthyl)pipéridin-1-yl)méthyl)-2,3-dihydrobenzo[b][1,4]dioxine-6-carbonitrile

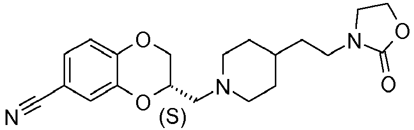

En procédant comme dans l'exemple 1, mais en remplaçant le (R)-2-(bromométhyl)-2,3-dihydrobenzo[b][1,4]dioxine 2.1 par le (R)-3-(bromométhyl)-2,3-dihydrobenzo [b][1,4]dioxine-6-carbonitrile 2.11, et le couple K₂CO₃/KI par la diisopropyl éthylamine (1,4 eq) on obtient 0,83 g (2,23 mmol) du composé 3 sous forme d'huile jaune pâle après purification par flash chromatographie sur gel de silice (éluant : dichlorométhane/méthanol = 95/5%).
Rendement : 68%
¹H RMN (CDCl₃) δ : 1,28 (ls, 3H) ; 1,49 (m, 2H) ; 1,73 (m, 2H) ; 2,54 (m, 1H) ; 2,09 (m, 2H) ; 2,65 (m, 1H) ; 2,91 (m, 2H) ; 3,31 (t, 2H, J = 7,6 Hz) ; 3,54 (t, 2H, J = 8Hz) ; 4,32 (m, 2H) ; 4,33 (t, 2H, J = 8Hz) ; 3,54 (t, 2H, J = 8Hz); 6,91 (d, 1H, J = 8,4Hz) ; 7,13 (dd, 1H, J = 8,4 Hz et 1,6 Hz) ; 7,17 (d, 1H, J = 1,6 Hz).

On dissout 0,83 g (2,23 mmol) du composé 3 dans 5 ml de méthanol, puis on ajoute 0,18 g (2,01 mmol) d'acide oxalique en solution dans 5 ml de méthanol. On concentre le milieu, puis on triture le milieu avec de l'éther éthylique et de l'acétone. Le solide qui précipite est filtré puis séché à la cloche à vide. On obtient 0,8 g (1,73 mmol) de l'oxalate du composé 3 sous la forme d'un solide blanc.
F : 182°C
[α] = -58,96 (c = 0,081, méthanol)
¹H RMN (DMSOd₆) δ : 1,37 (m, 5H) ; 1,80 (m, 2H) ; 2,60 (m, 2H) ; 3,06 (ls, 2H) ; 3,18 (t, 2H, J = 6,8 Hz) ; 3,30 (m, 2H) ; 3,51 (t, 2H, J = 8,4 Hz) ; 4,12 (dd, 1H, J = 11,6 et 6,8 Hz) ; 4,24 (t, 2H, J = 8,4 Hz) ; 4,41 (m, 1H) ; 4,68 (ls, 1H) ; 7,08 (d, 1H, J = 8,4 Hz) ; 7,35 (dd, 1H, J = 8,4 et 1,6 Hz) ; 7,44 (d, 1H, J = 1,6 Hz).
MS m/z 372 (M+1)
HPLC (Acquity BEH C8, Phase A : 100% H₂O HCOONH₄ 0.01M PH4, Phase B : 90% Acétonitrile 10% H2O.HCOONH4 0.01M PH4, gradient B : 15 à 40% sur 5 min, 0,75 ml/min), temps de rétention = 1.62 min, pureté chimique : 99,3%.
HPLC (CHIRALPACK AD-H, acétonitrile/ethanol/diéthylamine (90/10/0,1), 1 ml/min) : composé 3, temps de rétention = 6,91 min, composé 4, temps de rétention = 6,03 min, rapport des AUC (3)/(4) = 99,6/0,4.

### Exemple 4 : (R)-3-((4-(2-(2-oxooxazolidin-3-yl)éthyl)pipéridin-1-yl)méthyl)-2,3-dihydrobenzo[b][1,4]dioxine-6-carbonitrile

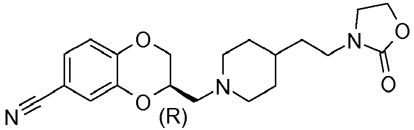

En procédant comme dans l'exemple 1, mais en remplaçant le (R)-2-(bromométhyl)-2,3-dihydrobenzo[b][1,4]dioxine 2.1 par le, (S)-3-(bromométhyl)-2,3-dihydrobenzo [b][1,4]dioxine-6-carbonitrile 2.12 et le couple K₂CO₃/KI par la diisopropyl éthylamine (1,4 eq) on obtient 1,8 g (4,85mmol) du composé 4 sous forme d'huile jaune pâle après purification par flash chromatographie sur gel de silice (éluant : dichlorométhane/méthanol = 95/5%)
Rendement : 64%
¹H RMN (CDCl₃) δ : cf RMN exemple 3

On dissout 1,8 g (4,85 mmol) du composé 4 dans 10 ml de méthanol, puis on ajoute 0,43 g (4,77 mmol) d'acide oxalique en solution dans 5 ml de méthanol. On concentre le milieu, puis on triture le milieu avec de l'éther éthylique. Le solide qui précipite est filtré puis séché à la cloche à vide. On obtient 1,8 g (3,90 mmol) de l'oxalate du composé 4 sous la forme d'un solide blanc.
F : 190°C
[α] = +51 (c = 0,114, méthanol)
¹H RMN (DMSOd₆) δ : cf RMN exemple 3
MS m/z 372 (M+1)
HPLC (Acquity BEH C8, Phase A : 100% H₂O HCOONH₄ 0.01M PH4, Phase B : 90% Acétonitrile 10% H2O.HCOONH4 0.01M PH4, gradient B : 15 à 40% sur 5 min, 0,75 ml/min), temps de rétention = 1,62 min, pureté chimique : 99,6%.
HPLC (CHIRALPACK AD-H, acétonitrile/ethanol/diéthylamine (90/10/0,1), 1 ml/min) : composé 4, temps de rétention = 6,01 min, composé 3, temps de rétention = 6,92 min, rapport des AUC (4)/(3) = 99,4/0,6.

### Exemple 5 : (S)-3-((4-(2-(2-oxooxazolidin-3-yl)éthyl)pipéridin-1-yl)méthyl)-2,3-dihydrobenzo[b][1,4]dioxine-5-carbonitrile

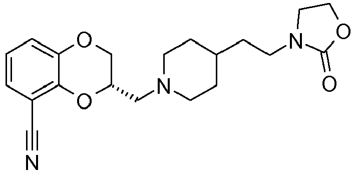

En procédant comme dans l'exemple 1, mais en remplaçant le (R)-2-(bromométhyl)-2,3-dihydrobenzo[b][1,4]dioxine 2.1 par le (R)-(8-cyano-2,3-dihydrobenzo[b][1,4] dioxin-2-yl)méthyl 4-bromobenzenesulfonate 2.13, et le couple K₂CO₃/KI par la diisopropyl éthylamine (1,4 eq) on obtient 1,04 g (2,80 mmol) du composé 5 sous forme d'huile jaune pâle après purification par flash chromatographie sur gel de silice (gradient sur 30 min dichlorométhane = 100% à dichlorométhane/méthanol/NH₄OH = 96/3,5/0,5%)
Rendement : 55%
¹H RMN (CDCl₃) δ : 1,26 (m, 3H) ; 1,49 (m, 2H) ; 1,72 (m, 2H) ; 2,12 (m, 1H) ; 2,23 (m, 1H); 2,64 (dd, 1H, J = 13.6 et 6.8 Hz) ; 2,72 (dd, 1H, J = 13.6 et 5.2 Hz) ; 2,86 (m, 1H) ; 3,01 (m, 1H) ; 3.30 (t, 2H, J = 7,2 Hz) ; 3.55 (t, 2H, J = 8 Hz) ; 4,04 (dd, 1H, J = 11.4 et 6,8 Hz) ; 4,34 (m, 4H) ; 6,87 (m, 1H) ; 7,07 (dd, 1H, J = 8,4 et 1,6 Hz) ; 7,12 (dd, 1H, J = 7,6 et 1,6 Hz).

On dissout 1,04 g (2,80 mmol) du composé 5 dans 10 ml d'acétate d'éthyle, puis on ajoute 1 ml (5 mmol) d'acide chlorhydrique 5N dans l'isopropanol. On laisse agiter le milieu, puis on filtre le solide qu'on lave avec de l'éther éthylique. Le solide est séché à la cloche à vide. On obtient 1,05 g (2.57 mmol) du chlorhydrate du composé 5 sous la forme d'un solide blanc.
F : 196°C
¹H RMN (DMSOd₆) δ : 1,53 (m, 5H) ; 1,91 (m, 2H) ; 3,11 (m, 2H) ; 3,20 (t, 2H, J = 6,8 Hz) ; 3,39 (m, 3H) ; 3,52 (t, 2H, J = 8,4 Hz) ; 3.55 (m, 1H) ; 3,75 (m, 1H) ; 4,18 (dd, 1H, J = 12 et 6,8 Hz) ; 4,25 (m, 1H) ; 4,45 (dd, 1H, 11,8 et 2 Hz) ; 5,14 (m, 1H) ; 7,05 (m, 1H) ; 7,28 (dd, 1H, J = 8,2 et 1,2 Hz) ; 7,37 (dd, 1H, J = 8 et 1,2 Hz) ; 10,98 (ls, 1H).
MS m/z 372 (M+1)
HPLC (X-BRIDGE C8, acétonitrile/H₂O.KH₂PO₄ 6,8 g PH4 (23/77), 1 ml/min), temps de rétention = 7,37 min, pureté chimique : 100%.

### Exemple 6 : (S)-2-((4-(2-(2-oxooxazolidin-3-yl)éthyl)pipéridin-1-yl)méthyl)-2,3-dihydrobenzo[b][1,4]dioxine-5-carbonitrile

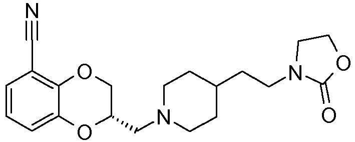

En procédant comme dans l'exemple 1, mais en remplaçant le (R)-2-(bromométhyl)-2,3-dihydrobenzo[b][1,4]dioxine 2.1 par le (R)-(5-cyano-2,3-dihydrobenzo[b][1,4] dioxin-2-yl)méthyl 4-bromobenzenesulfonate 2.14, et le couple K₂CO₃/KI par la diisopropyl éthylamine (1,4 eq) on obtient 0,14 g (0,37mmol) du composé 6 sous forme d'huile jaune pâle après purification par flash chromatographie sur gel de silice (éluant : dichlorométhane/méthanol = 95/5%).
Rendement : 50%
¹H RMN (DMSOd₆) δ : 1,17 (m, 3H) ; 1,40 (m, 2H) ; 1,64 (m, 2H) ; 1.99 (m, 2H) ; 2,55 (m, 2H) ; 2,88 (m, 2H) ; 3,17 (t, 2H, J = 7,2 Hz) ; 3,50 (t, 2H, J = 8,4 Hz) ; 4,12 (dd, 1H, J = 11,4 et 7,2 Hz) ; 4,24 (t, 2H, J = 8 Hz) ; 4,43 (m, 2H) ; 6.97 (m, 1H) ; 7,21 (dd, 1H, J = 8,2 et 1,2 Hz) ; 7,28 (dd, 1H, J = 8.4 et 1.2 Hz).

On dissout 0.14 g (0,37 mmol) du composé 6 dans 5 ml d'acétonitrile, puis on ajoute 1 ml d'eau et 0,4 ml (0,40 mmol) d'acide chlorhydrique IN. Après lyophilisation on obtient 0,15 g (0,37 mmol) du chlorhydrate du composé 6 sous la forme d'un solide blanc.
¹H RMN (DMSOd₆) δ : 1,45 (m, 5H) ; 1,90 (m, 2H) ; 3,02 (m, 2H) ; 3.19 (t, 2H) ; 3.50 (m, 4H) ; 3.52 (t, 2H, J = 7,6 Hz) ; 4,25 (m, 3H) ; 4,52 (m, 1H) ; 4,95 (m, 1H) ; 7,06 (m, 1H) ; 7,31 (m, 1H) ; 7,38 (m, 1H) ; 9,90 (ls, 1H).
MS m/z 372 (M+1)
HPLC (X-BRIDGE C18, Phase A: 100% H₂O. TFA 0.1%, Phase B: 100% Acétonitrile, gradient B : 0 à 100% sur 6 min, 1,8 ml/min), temps de rétention = 4,26 min, pureté chimique : 99,2%.

### Exemple 7 : (S)-3-(2-(1-((7-fluoro-2,3-dihydrobenzo[b][1,4]dioxin-2-yl)méthyl) pipéridin-4-yl)éthyl)oxazolidin-2-one

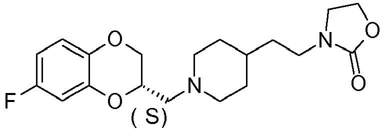

En procédant comme dans l'exemple 1, mais en remplaçant le (R)-2-(bromométhyl)-2,3-dihydrobenzo[b][1,4]dioxine 2.1 par le (R)-(7-fluoro-2,3-dihydrobenzo[b][1,4] dioxin-2-yl)méthyl 4-bromobenzenesulfonate 2.17, le couple K₂CO₃/KI par la diisopropyl éthylamine (1,4 eq) et l'acétonitrile par le diméthylsulfoxide, on obtient 0,10 g (0,27 mmol) du composé 7 sous forme d'huile jaune pâle après purification par flash chromatographie sur gel de silice (gradient sur 30 min dichlorométhane 100% à dichlorométhane/méthanol = 95/5%).
Rendement : 37%
¹H RMN (DMSOd₆) δ : 1,18 (m, 3H) ; 1,40 (m, 2H) ; 1,64 (m, 2H) ; 1,98 (m, 2H) ; 2,52 (m, 2H) ; 2,87 (m, 2H) ; 3,17 (t, 2H, J = 7,2 Hz) ; 3,50 (t, 2H, J = 8,4 Hz) ; 3,92 (dd, 1H, J = 11,6 et 7,2 Hz) ; 4.23 (t, 2H, J = 8,4 Hz) ; 4.25 (m, 1H), ; 4,35 (m, 1H) ; 6,65 (m, 1H) ; 6,76 (m, 1H) ; 6,87 (m, 1H).

On dissout 0.10 g (0,27 mmol) du composé 7 dans 5 ml d'acétonitrile, puis on ajoute 1 ml d'eau et 0,29 ml (0,29 mmol) d'acide chlorhydrique IN. Après lyophilisation on obtient 0,11 g (0,27 mmol) du chlorhydrate du composé 7 sous la forme d'un solide blanc.
¹H RMN (DMSOd₆) δ : 1,45 (m, 5H) ; 1,90 (m, 2H) ; 3,01 (m, 2H) ; 3,19 (m, 2H) ; 3,32 (m, 3H) ; 3,52 (t, 2H, J = 8,4 Hz) ; 3,65 (m, 1H) ; 4,05 (dd, 1H, J = 11,6 et 6,4 Hz) ; 4,25 (t, 2H, J = 8,4 Hz) ; 4,28 (m, 1H) ; 4.89 (m, 1H) ; 6,75 (m, 1H) ; 6,87 (m, 1H) ; 6,95 (m, 1H) ; 10,14 (ls, 1H).
MS m/z 365 (M+1)
HPLC (X-BRIDGE C18, Phase A: 100% H₂O. TFA 0.1%, Phase B: 100% Acétonitrile, gradient B : 0 à 100% sur 6 min, 1,8 ml/min), temps de rétention = 4,40 min, pureté chimique : 97,8%.

### Exemple 8 : (S)-3-(2-(1-((8-fluoro-2,3-dihydrobenzo[b][1,4]dioxin-2-yl)méthyl) pipéridin-4-yl)éthyl)oxazolidin-2-one

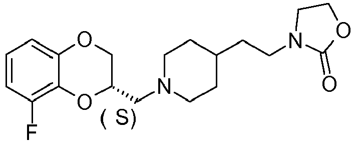

En procédant comme dans l'exemple 1, mais en remplaçant le (R)-2-(bromométhyl)-2,3-dihydrobenzo[b][1,4]dioxine 2.1 par le (R)-(8-fluoro-2,3-dihydrobenzo [b][1,4]dioxin-2-yl)méthyl 4-bromobenzenesulfonate 2.16, le couple K₂CO₃/KI par la diisopropyl éthylamine (1,4 eq) et l'acétonitrile par le diméthylsulfoxide, on obtient 0,12 g (0.33 mmol) du composé 8 sous forme d'un solide beige après purification par flash chromatographie sur gel de silice (gradient sur 30 min dichlorométhane 100% à dichlorométhane/méthanol = 95/5%).
Rendement : 44%
¹H RMN (DMSOd₆) δ : 1,16 (m, 3H) ; 1,40 (m, 2H) ; 1.65 (m, 2H) ; 2,01 (m, 2H) ; 2,56 (m, 2H) ; 2,83 (m, 1H) ; 2,94 (m, 1H) ; 3,17 (t, 2H, J = 7,2 Hz) ; 3,50 (t, 2H, J = 8 Hz) ; 4,00 (dd, 1H, J = 11,6 et 7,2 Hz) ; 4,24 (t, 2H, J = 8 Hz) ; 4,36 (m, 2H) ; 6,75 (m, 3H).

On dissout 0.12 g (0,33 mmol) du composé 8 dans 5 ml d'acétonitrile, puis on ajoute 1 ml d'eau et 0,35 ml (0,35 mmol) d'acide chlorhydrique IN. Après lyophilisation on obtient 0,12 g (0,30 mmol) du chlorhydrate du composé 8 sous la forme d'un solide jaune pâle.
¹H RMN (DMSOd₆) δ : 1,46 (m, 5H) ; 1,91 (m, 2H) ; 3,04 (m, 2H) ; 3,19 (m, 2H) ; 3.34 (m, 1H) ; 3,51 (m, 4H) ; 3,66 (m, 1H) ; 4,13 (m, 1H) ; 4,25 (m, 2H) ; 4,39 (m, 1H) ; 4,96 (m, 1H) ; 6,78 (m, 1H) ; 6.87 (m, 2H) ; 10,43 (ls, 1H).
MS m/z 365 (M+1)
HPLC (X-BRIDGE C18, Phase A: 100% H₂O. TFA 0.1%, Phase B: 100% Acétonitrile, gradient B : 0 à 100% sur 6 min, 1,8 ml/min), temps de rétention = 4,37 min, pureté chimique : 100%.

### Exemple 9 : (S)-3-(2-(1-((6-fluoro-2,3-dihydrobenzo[b][1,4]dioxin-2-yl)méthyl) pipéridin-4-yl)éthyl)oxazolidin-2-one

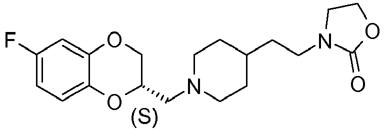

En procédant comme dans l'exemple 1, mais en remplaçant le (R)-2-(bromométhyl)-2,3-dihydrobenzo[b][1,4]dioxine 2.1 par le (R)-(6-fluoro-2,3-dihydrobenzo[b][1,4] dioxin-2-yl)méthyl 4-bromobenzenesulfonate 2.18, le couple K₂CO₃/KI par la diisopropyl éthylamine (1,4 eq) et l'acétonitrile par le diméthylsulfoxide, on obtient 0,12 g (0,33 mmol) du composé 9 sous forme d'un solide jaune pâle après purification par flash chromatographie sur gel de silice (gradient sur 30 min dichlorométhane 100% à dichlorométhane/méthanol = 95/5%).
Rendement : 52%
¹H RMN (DMSOd₆) δ : 1,17 (m, 3H) ; 1,40 (m, 2H) ; 1,64 (m, 2H) ; 1,98 (m, 2H) ; 2,50 (m, 2H) ; 2,87 (m, 2H) ; 3,17 (t, 2H, J = 7,2 Hz) ; 3,50 (t, 2H, J = 8 Hz) ; 3,95 (dd, 1H, J = 11,8 et 7,6 Hz) ; 4,26 (m, 4H) ; 6,65 (m, 1H) ; 6,76 (m, 1H) ; 6,87 (m, 1H).

On dissout 0.12 g (0,33 mmol) du composé 9 dans 5 ml d'acétonitrile, puis on ajoute 1 ml d'eau et 0,35 ml (0,35 mmol) d'acide chlorhydrique IN. Après lyophilisation on obtient 0,13 g (0,32 mmol) du chlorhydrate du composé 9 sous la forme d'un solide blanc.
¹H RMN (DMSOd₆) δ : 1,51 (m, 5H) ; 1,89 (m, 2H) ; 3,01 (m, 2H) ; 3,21 (m, 2H) ; 3,28 (m, 1H) ; 3,48 (m, 2H) ; 3,52 (t, 2H, J = 8 Hz) ; 3,66 (m, 1H) ; 4,07 (dd, 1H, J = 11,6 et 6,8 Hz) ; 4,25 (t, 2H, J = 8 Hz) ; 4,37 (m, 1H) ; 4,89 (m, 1H) ; 6,74 (m, 1H) ; 6,85 (m, 1H) ; 6,97 (m, 1H,) ; 10,70 (ls, 1H).
MS m/z 365 (M+1)
HPLC (X-BRIDGE C18, Phase A: 100% H₂O. TFA 0.1%, Phase B: 100% Acétonitrile, gradient B : 0 à 100% sur 6 min, 1,8 ml/min), temps de rétention = 4,41 min, pureté chimique : 98,1 %.

### Exemple 10 : (S)-3-(2-(1-((5,7-difluoro-2,3-dihydrobenzo[b][1,4]dioxin-2-yl)méthyl) pipéridin-4-yl)éthyl)oxazolidin-2-one

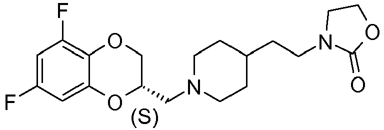

En procédant comme dans l'exemple 1, mais en remplaçant le (R)-2-(bromométhyl)-2,3-dihydrobenzo[b][1,4]dioxine 2.1 par le (R)-(5,7-difluoro-2,3-dihydrobenzo[b][1,4] dioxin-2-yl)méthyl 4-bromobenzenesulfonate 2.19, le couple K₂CO₃/KI par la diisopropyl éthylamine (1,4 eq) et l'acétonitrile par le diméthylsulfoxide, on obtient 0,11 g (0,29 mmol) du composé 10 sous forme d'huile jaune pâle après purification par flash chromatographie sur gel de silice (gradient sur 30 min dichlorométhane 100% à dichlorométhane/méthanol = 95/5%).
Rendement : 40%
¹H RMN (DMSOd₆) δ : 1,16 (m, 3H) ; 1,40 (m, 2H) ; 1,64 (m, 2H) ; 1,99 (m, 2H) ; 2,54 (m, 2H) ; 2,87 (m, 2H) ; 3,17 (t, 2H, J = 7,2 Hz) ; 3.50 (t, 2H, J = 8 Hz) ; 3,99 (dd, 1H, 11,6 Hz et 7,2 Hz) ; 4,24 (t, 2H, J = 8 Hz) ; 4,34 (m, 1H) ; 4,44 (m, 1H) ; 6,69 (m, 1H) ; 6,84 (m, 1H).

On dissout 0.11 g (0,29 mmol) du composé 10 dans 5 ml d'acétonitrile, puis on ajoute 1 ml d'eau et 0,30 ml (0,30 mmol) d'acide chlorhydrique IN. Après lyophilisation on obtient 0,11 g (0,26 mmol) du chlorhydrate du composé 10 sous la forme d'une huile jaune pâle.
¹H RMN (DMSOd₆) δ : 1,45 (m, 5H) ; 1,90 (m, 2H) ; 3,00 (m, 2H) ; 3,20 (m, 2H) ; 3,42 (m, 3H) ; 3,52 (t, 2H J =8 Hz) ; 3,65 (m, 1H) ; 4,13 (dd, 1H, J = 11,8 et 6,4 Hz) ; 4,25 (t, 2H, J = 8 Hz) ; 4,38 (m, 1H) ; 4,97 (m, 1H) ; 6,79 (m, 1H) ; 6,96 (m, 1H) ; 10,11 (ls, 1H).
MS m/z 383 (M+1)
HPLC (X-BRIDGE C18, Phase A : 100% H₂O, TFA 0.1 %, Phase B : 100% acétonitrile, gradient B : 0 à 100% sur 6 min, 1,8 ml/min), temps de rétention = 4,50 min, pureté chimique : 97,9%.

### Exemple 11 : (S)-3-(2-(1-((6,7-difluoro-2,3-dihydrobenzo[b][1,4]dioxin-2-yl)méthyl) pipéridin-4-yl)éthyl)oxazolidin-2-one

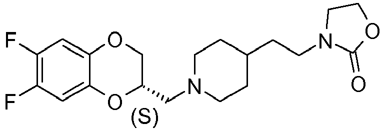

En procédant comme dans l'exemple 1, mais en remplaçant le (R)-2-(bromométhyl)-2,3-dihydrobenzo[b][1,4]dioxine 2.1 par le (R)-(6,7-difluoro-2,3-dihydrobenzo[b][1,4] dioxin-2-yl)méthyl 4-bromobenzenesulfonate 2.22, le couple K₂CO₃/KI par la diisopropyl éthylamine (1,4 eq) et l'acétonitrile par le diméthylsulfoxide, on obtient 0,11 g (0,29 mmol) du composé 11 sous forme d'huile jaune pâle après purification par flash chromatographie sur gel de silice (gradient sur 30 min dichlorométhane 100% à dichlorométhane/méthanol = 95/5%).
Rendement : 40%
¹H RMN (DMSOd₆) δ : 1,16 (m, 3H) ; 1,40 (m, 2H) ; 1,64 (m, 2H) ; 1,98 (m, 2H) ; 2,50 (m, 2H) ; 2,87 (m, 2H) ; 3,17 (t, 2H, J = 7,6 Hz) ; 3,50 (t, 2H, J = 8 Hz) ; 3,95 (dd, 1H, J = 11,2 et 7.2 Hz) ; 4,24 (t,2H, J = 8 Hz) ; 4,28 (m, 2H) ; 7,04 (m, 2H).

On dissout 0.11 g (0,29 mmol) du composé 11 dans 5 ml d'acétonitrile, puis on ajoute 1 ml d'eau et 0,30 ml (0,30 mmol) d'acide chlorhydrique IN. Après lyophilisation on obtient 0,12 g (0,28 mmol) du chlorhydrate du composé 11 sous la forme d'une gomme jaune pâle.
¹H RMN (DMSOd₆) δ : 1,46 (m, 5H) ; 1,90 (m, 2H) ; 3,02 (m, 2H) ; 3,19 (m, 2H) ; 3,38 (m, 3H) ; 3,52 (t, 2H, J = 8 Hz) ; 3,65 (m, 1H) ; 4,07 (dd, 1H, J = 11,6 et 6,4 Hz) ; 4,25 (t, 2H, J = 8 Hz) ; 4,33 (m, 1H) ; 4.87 (m, 1H) ; 7,14 (m, 2H) ; 10,1 (ls, 1H).
MS m/z 383 (M+1)
HPLC (X-BRIDGE C18, Phase A: 100% H₂O. TFA 0.1%, Phase B: 100% Acétonitrile, gradient B : 0 à 100% sur 6 min, 1,8 ml/min), temps de rétention = 4,54 min, pureté chimique : 98,1%.

### Exemple 12 : (S)-3-(2-(1-((7-chloro-5-fluoro-2,3-dihydrobenzo[b][1,4]dioxin-2-yl) méthyl)pipéridin-4-yl)éthyl)oxazolidin-2-one

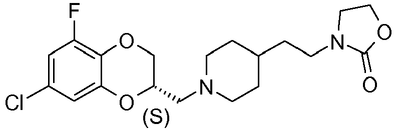

En procédant comme dans l'exemple 1, mais en remplaçant le (R)-2-(bromométhyl)-2,3-dihydrobenzo[b][1,4]dioxine 2.1 par le (R)-(7-chloro-5-fluoro-2,3-dihydrobenzo [b][1,4]dioxin-2-yl)méthyl 4-bromobenzenesulfonate 2.20, le couple K₂CO₃/KI par la diisopropyl éthylamine (1,4 eq) et l'acétonitrile par le diméthylsulfoxide, on obtient 0,10 g (0,25 mmol) du composé 12 sous forme d'un solide jaune pâle après purification par flash chromatographie sur gel de silice (gradient sur 30 min dichlorométhane 100% à dichlorométhane/méthanol = 95/5%).
Rendement : 38%
¹H RMN (DMSOd₆) δ : 1,16 (m, 3H) ; 1,40 (m, 2H) ; 1,64 (m, 2H) ; 1,99 (m, 2H) ; 2,54 (m, 2H) ; 2,87 (m, 2H) ; 3,17 (t, 2H, J = 7,2 Hz) ; 3,50 (t, 2H, J = 8 Hz) ; 4,02 (dd, 1H, J = 11,4 et 7,2 Hz) ; 4,24 (t, 2H, J = 8 Hz) ; 4,37 (m, 1H) ; 4,45 (m, 1H) ; 6,87 (m, 1H) ; 7,01 (m, 1H).

On dissout 0.10 g (0,25 mmol) du composé 12 dans 5 ml d'acétonitrile, puis on ajoute 1 ml d'eau et 0,26 ml (0,26 mmol) d'acide chlorhydrique IN. Après lyophilisation on obtient 0,11 g (0,25 mmol) du chlorhydrate du composé 12 sous la forme d'un solide jaune pâle.
¹H RMN (DMSOd₆) δ : 1,46 (m, 5H) ; 1,92 (m, 2H) ; 3.02 (m, 2H) ; 3,20 (t, 2H, J = 7,2 Hz) ; 3,40 (m, 3H) ; 3.52 (t, 2H, J = 8 Hz) ; 3,64 (m, 1H) ; 4,16 (dd, 1H, J = 11,8 et 6 Hz) ; 4,25 (t, 2H, J = 8 Hz) ; 4,40 (m, 1H) ; 4,97 (m, 1H) ; 6,97 (m, 1H) ; 7,12 (m, 1H) ; 10,01 (ls, 1H).
MS m/z 399 (M+1)
HPLC (X-BRIDGE C18, Phase A : 100% H₂O.TFA 0.1%, Phase B : 100% Acétonitrile, gradient B : 0 à 100% sur 6 min, 1,8 ml/min), temps de rétention = 4,73 min, pureté chimique : 98,6%.

### Exemple 13 : (S)-3-(2-(1-((5-chloro-7-fluoro-2,3-dihydrobenzo[b][1,4]dioxin-2-yl) méthyl)pipéridin-4-yl)éthyl)oxazolidin-2-one

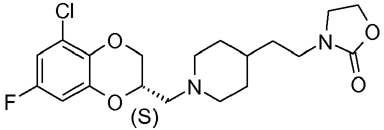

En procédant comme dans l'exemple 1, mais en remplaçant le (R)-2-(bromométhyl)-2,3-dihydrobenzo[b][1,4]dioxine 2.1 par le (R)-(5-chloro-7-fluoro-2,3-dihydrobenzo [b][1,4]dioxin-2-yl)méthyl 4-bromobenzenesulfonate 2.21, le couple K₂CO₃/KI par la diisopropyl éthylamine (1,4 eq) et l'acétonitrile par le diméthylsulfoxide, on obtient 0,10 g (0,25 mmol) du composé 13 sous forme d'une huile orange après purification par flash chromatographie sur gel de silice (gradient sur 30 min dichlorométhane 100% à dichlorométhane/méthanol = 95/5%).
Rendement : 37%
¹H RMN (DMSOd₆) δ : 1,16 (m, 3H) ; 1,40 (m, 2H) ; 1,64 (m, 2H) ; 1,98 (m, 2H) ; 2,54 (m, 2H) ; 2,88 (m, 2H) ; 3,17 (t, 2H, J = 6,8 Hz) ; 3,50 (t, 2H, J = 8 Hz) ; 4,02 (dd, 1H, J = 11,6 et 7,2 Hz) ; 4,24 (t, 2H, J = 8 Hz) ; 4,41 (m, 2H) ; 6,85 (m, 1H) ; 6,97 (m, 1H).

On dissout 0.10 g (0,25 mmol) du composé 13 dans 5 ml d'acétonitrile, puis on ajoute 1 ml d'eau et 0,26 ml (0,26 mmol) d'acide chlorhydrique IN. Après lyophilisation on obtient 0,11 g (0,25 mmol) du chlorhydrate du composé 13 sous la forme d'une gomme beige.
¹H RMN (DMSOd₆) δ : 1,46 (m, 5H) ; 1,91 (m, 2H) ; 3,01 (m, 2H) ; 3,20 (t, 2H) ; 3,37 (m, 3H) ; 3,52 (t, 2H, J = 8 Hz) ; 3,62 (m, 1H) ; 4,18 (dd, 1H, J = 12 et 5,6 Hz) ; 4,25 (t, 2H, J = 8 Hz) ; 4,41 (m, 1H) ; 4,94 (m, 1H) ; 6,94 (m, 1H) ; 7,09 (m, 1H) ; 9,9 (ls, 1H).
MS m/z 399 (M+1)
HPLC (X-BRIDGE C18, Phase A : 100% H₂O.TFA 0.1%, Phase B : 100% Acétonitrile, gradient B : 0 à 100% sur 6 min, 1,8 ml/min), temps de rétention = 4,68 min, pureté chimique : 97,4%.

### Exemple 14 : (S)-3-(2-(1-((7-chloro-2,3-dihydrobenzo[b][1,4]dioxin-2-yl)méthyl) pipéridin-4-yl)éthyl)oxazolidin-2-one

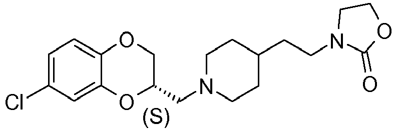

En procédant comme dans l'exemple 1, mais en remplaçant le (R)-2-(bromométhyl)-2,3-dihydrobenzo[b][1,4]dioxine 2.1 par le (R)-(7-chloro-2,3-dihydrobenzo[b][1,4] dioxin-2-yl)méthyl 4-bromobenzenesulfonate 2.7, le couple K₂CO₃/KI par la diisopropyl éthylamine (1,4 eq) et l'acétonitrile par le diméthylsulfoxide, on obtient 0,12 g (0,31 mmol) du composé 14 sous forme d'huile jaune pâle après purification par flash chromatographie sur gel de silice (gradient sur 30 min dichlorométhane 100% à dichlorométhane/méthanol = 95/5%).
Rendement : 45%
¹H RMN (DMSOd₆) δ : 1,17 (m, 3H) ; 1,40 (m, 2H) ; 1,64 (m, 2H) ; 1,98 (m, 2H) ; 2,52 (m, 2H) ; 2,87 (m, 2H) ; 3,17 (t, 2H, J = 7,2 Hz) ; 3,50 (t, 2H, J = 8 Hz) ; 3,96 (dd, 1H, J = 11,6 et 6,8 Hz) ; 4,24 (t, 2H, J = 8 Hz) ; 4,29 (m, 1H) ; 4,38 (m, 1H) ; 6,87 (m, 2H) ; 6,95 (d, 1H, J = 2,4 Hz).

On dissout 0.12 g (0,31 mmol) du composé 14 dans 5 ml d'acétonitrile, puis on ajoute 1 ml d'eau et 0,32 ml (0,32 mmol) d'acide chlorhydrique IN. Après lyophilisation on obtient 0,13 g (0,31 mmol) du chlorhydrate du composé 14 sous la forme d'une gomme jaune pâle.
¹H RMN (DMSOd₆) δ : 1,46 (m, 5H) ; 1,92 (m, 2H) ; 3,02 (m, 2H) ; 3,20 (t, 2H) ; 3,38 (m, 3H) ; 3,52 (t, 2H, J = 8 Hz) ; 3,63 (m, 1H) ; 4,08 (dd, 1H, J = 11,6 et 6 Hz) ; 4,25 (t, 2H, J = 8 Hz) ; 4,32 (m, 1H) ; 4,87 (m, 1H) ; 6,95 (ls, 2H) ; 7,07 (ls, 1H) ; 9,9 (ls, 1H).
MS m/z 381 (M+1)
HPLC (X-BRIDGE C18, Phase A : 100% H₂O.TFA 0.1%, Phase B : 100% Acétonitrile, gradient B : 0 à 100% sur 6 min, 1,8 ml/min), temps de rétention = 4,62 min, pureté chimique : 98,7%.

### Exemple 15 : (S)-3-(2-(1-((7-(méthylsulfonyl)-2,3-dihydrobenzo[b][1,4]dioxin-2-yl) méthyl)pipéridin-4-yl)éthyl)oxazolidin-2-one

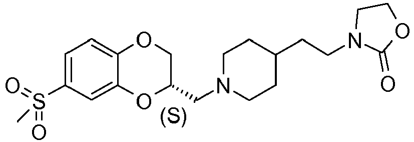

En procédant comme dans l'exemple 1, mais en remplaçant le (R)-2-(bromométhyl)-2,3-dihydrobenzo[b][1,4]dioxine 2.1 par le (R)-(7-(méthylsulfonyl)-2,3-dihydrobenzo [b][1,4]dioxin-2-yl)méthyl 4-bromobenzenesulfonate 2.23, le couple K₂CO₃/KI par la diisopropyl éthylamine (1,4 eq) et l'acétonitrile par le diméthylsulfoxide, on obtient 0,12 g (0,31 mmol) du composé 15 sous forme d'huile incolore après purification par flash chromatographie sur gel de silice (gradient sur 30 min dichlorométhane 100% à dichlorométhane/méthanol = 95/5%).
Rendement : 14%
¹H RMN (DMSOd₆) δ : 1,17 (m, 3H) ; 1,40 (m, 2H) ; 1,65 (m, 2H) ; 2,01 (m, 2H) ; 2,54 (m, 2H) ; 2,90 (m, 2H) ; 3,16 (s, 3H) ; 3,17 (t, 2H) ; 3,50 (t, 2H, J = 8 Hz) ; 4,06 (dd, 1H, J = 11,6 et 6,8 Hz) ; 4,24 (t, 2H, J = 8 Hz) ; 4,40 (m, 2H) ; 7,10 (d, 1H, J = 8,4 Hz) ; 7,36 (m, 2H).

On dissout 0.12 g (0,31 mmol) du composé 15 dans 5 ml d'acétonitrile, puis on ajoute 1 ml d'eau et 0,0028 g (0,31 mmol) d'acide oxalique. Après lyophilisation on obtient 0,13 g (0,31 mmol) de l'oxalate du composé 15 sous la forme d'un solide blanc.
¹H RMN (DMSOd₆) δ : 1,39 (m, 5H) ; 1,81 (m, 2H) ; 3,17 (s, 3H) ; 3,19 (t, 2H) ; 3,45 (lm, 6H) ; 3,51 (t, 2H, J = 8 Hz) ; 4,12 (dd, 1H) ; 4,24 (t, 2H, J = 8 Hz) ; 4,41(m, 1H) ; 4,68 (ls, 1H) ; 7,15 (d, 1H, J = 8,4 Hz) ; 7,43 (m, 2H)
MS m/z 425 (M+1)
HPLC (X-BRIDGE C18, Phase A : 100% H₂O.TFA 0,1 %, Phase B : 100% Acétonitrile, gradient B : 0 à 100% sur 6 min, 1,8 ml/min), temps de rétention = 3,93 min, pureté chimique : 100%.

### Exemple 16 : (S)-méthyl 3-((4-(2-(2-oxooxazolidin-3-yl)éthyl)pipéridin-l-yl)méthyl)-2,3-dihydrobenzo[b][1,4]dioxine-6-carboxylate

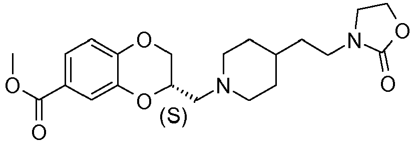

En procédant comme dans l'exemple 1, mais en remplaçant le (R)-2-(bromométhyl)-2,3-dihydrobenzo[b][1,4]dioxine 2.1 par le (R)-méthyl 3-((((4-bromophenyl) sulfonyl)oxy)méthyl)-2,3-dihydrobenzo[b][1,4]dioxine-6-carboxylate 2.24, le couple K₂CO₃/KI par la diisopropyl éthylamine (1,4 eq) et l'acétonitrile par le diméthylsulfoxide, on obtient 0,032 g (0,08 mmol) du composé 16 sous forme de solide blanc, après purification par flash chromatographie sur gel de silice (gradient sur 30 min dichlorométhane 100% à dichlorométhane/méthanol = 95/5%).
Rendement : 12%
¹H RMN (DMSOd₆) δ : 1,18 (m, 3H) ; 1,40 (m, 2H) ; 1,65 (m, 2H) ; 2,00 (m, 2H) ; 2,54 (m, 2H) ; 2,88 (m, 2H) ; 3,17 (t, 2H, J = 7,2 Hz) ; 3,50 (t, 2H, J = 8 Hz) ; 3,76 (s, 3H) ; 4,05 (m, 1H) ; 4,24 (t, 2H, J = 8 Hz) ; 4,37 (m, 2H) ; 6,98 (d, 1H, J = 8,4 Hz) ; 7,39 (d, 1H, J = 2 Hz) ; 7,46 (dd, 1H, 8,4 et 2 Hz).

On dissout 0.032 g (0,08 mmol) du composé 16 dans 5 ml d'acétonitrile, puis on ajoute 0,0072g (0,08 mmol) d'acide oxalique et 1 ml d'eau. Après lyophilisation on obtient 0,029 g (0,06 mmol) de l'oxalate du composé 16 sous la forme d'un solide blanc.
¹H RMN (DMSOd₆) δ : 1,42 (m, 5H) ; 1,85 (m, 2H) ; 2,81 (m, 2H) ; 3,20 (m, 4H) ; 3,40 (m, 2H) ; 3,52 (t, 2H J = 8 Hz) ; 3,81 (s, 3H) ; 4,11 (dd, 1H, J = 11,6 et 6,8 Hz) ; 4,23 (t, 2H, J = 8 Hz) ; 4,40 (m, 1H) ; 4,73 (ls, 1H) ; 7,03 (m, 1H) ; 7,51 (m, 2H).
MS m/z 405 (M+1)
HPLC (X-BRIDGE C18, Phase A : 100% H₂O.TFA 0,1 %, Phase B : 100% Acétonitrile, gradient B : 0 à 100% sur 6 min, 1,8 ml/min), temps de rétention = 4,36 min, pureté chimique : 99,1%.

### Exemple 17 : (S)-3-((4-(3-(2-oxooxazolidin-3-yl)propyl)pipéridin-1-yl)méthyl)-2,3-dihydrobenzo[b][1,4]dioxine-6-carbonitrile

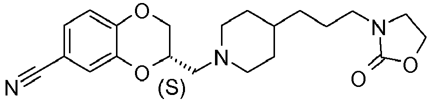

En procédant comme dans l'exemple 1, mais en remplaçant le (R)-2-(bromométhyl)-2,3-dihydrobenzo[b][1,4]dioxine 2.1 par le (R)-3-(bromométhyl)-2,3-dihydrobenzo [b][1,4]dioxine-6-carbonitrile 2.11, la 3-(2-(pipéridin-4-yl)éthyl)oxazolidin-2-one 3.1 par la 3-(3-(pipéridin-4-yl)propyl)oxazolidin-2-one 3.30 et le couple K₂CO₃/KI par la diisopropyl éthylamine (2 eq), on obtient 1,12 g (2,90 mmol) du composé 17 sous la forme d'huile incolore, après purification par flash chromatographie sur gel de silice (gradient : acétate d'éthyle = 100% à acétate d'éthyle/(méthanol/NH4OH : 10/1) = 98/2%).
Rendement : 82%
¹H RMN (CDCl₃) δ : 1,26 (m, 5H) ; 1,56 (m, 2H) ; 1,66 (m, 2H) ; 2,09 (m, 2H) ; 2,54 (dd, 1H, J = 13,2 et 6,4 Hz) ; 2,65 (dd, 1H, J = 13,2 et 5,6 Hz) ; 2,86 (m, 1H) ; 2,95 (m, 1H) ; 3,25 (t, 2H, J = 7,2 Hz) ; 3,55 (m, 2H) ; 4,02 (dd, 1H, J = 11,2 et 7,6 Hz) ; 4,29 (m, 4H) ; 6,91 (d, 1H, J = 8,4 Hz) ; 7,14 (dd, 1H, J = 8,4 et 2 Hz) ; 7,16 (d, 1H, J = 2 Hz).

On dissout 1,12 g (2,90 mmol) du composé 17 dans un minimum de méthanol et on ajoute 1,2 ml (6 mmol) d'une solution d'HCl 5N dans l'isopropanol. On concentre le milieu et on triture avec de l'éther éthylique. On filtre le précipité qui a tendance à coller et on le met à sécher à la cloche à vide. On obtient 0,99 g (2,34 mmol) du chlorhydrate du composé 17 sous la forme d'un solide blanc.
¹H RMN (DMSOd₆) δ : 1,20 (m, 2H) ; 1,52 (m, 5H) ; 1,84 (m, 2H) ; 3,03 (m, 2H) ; 3,13 (m, 2H) ; 3,40 (m, 5H) ; 3,67 (m, 1H) ; 4,22 (m, 3H) ; 4,46 (dd, 1H, J = 12 et 2,4 Hz) ; 5,02 (m, 1H) ; 7,11 (d, 1H, J = 8,4 Hz) ; 7,38 (dd, 1H, J = 8, 4 et 1,6 Hz) ; 7,49 (d, 1H, J = 1,6 Hz).
MS m/z 386 (M+1).
HPLC (Acquity BEH C8, Phase A : 100% H₂O. HCOONH₄ 0.01M PH4, Phase B : 90% Acétonitrile 10% H2O.HCOONH4 0.01M PH4, gradient B : 15 à 40% sur 5 min, 0,75 ml/min), temps de rétention = 1,88 min, pureté chimique : 98,97%.

### Exemple 18: (S)-3-(2-(1-(((S)-2,3-dihydrobenzo[b][1,4]dioxin-2-yl)méthyl)pipéridin-4-yl)éthyl)-4-isopropyloxazolidin-2-one

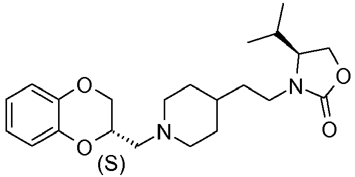

En procédant comme dans l'exemple 1, mais en remplaçant la 3-(2-(pipéridin-4-yl) éthyl)oxazolidin-2-one 3.1 par la (S)-4-isopropyl-3-(2-(pipéridin-4-yl)éthyl)oxazolidin-2-one 3.2 et l'acétonitrile par le DMSO, on obtient 0,68 g (1,75mmol) du composé 18 sous forme d'huile jaune pâle, après purification par flash chromatographie sur gel de silice (gradient sur 40 min dichlorométhane 100% à dichlorométhane/méthanol = 95/5%).
Rendement : 70%

On dissout 0,68 g (1.75 mmol) du composé 18 dans un minimum d'éthanol et on ajoute 0,5 ml (2,5 mmol) d'une solution d'HCl 5N dans l'isopropanol. On concentre le milieu et on triture avec de l'éther éthylique. On filtre le précipité que l'on met à sécher à la cloche à vide. On obtient 0,53 g (1,25 mmol) du chlorhydrate du composé 18 sous la forme d'un solide blanc.
F : 173°C
¹H RMN (DMSOd₆) δ : 0,76 (d, 3H, J = 6,8 Hz) ; 0,85 (d, 3H, J = 6,8 Hz) ; 1,48 (m, 5H) ; 1,89 (m, 2H) ; 2,03 (m, 1H) ; 2,98 (m, 3H) ; 3,41 (m, 4H) ; 3,67 (m, 1H) ; 3,80 (m, 1H) ; 4,06 (m, 2H) ; 4,18 (m, 1H) ; 4,32 (m, 1H) ; 4,91 (m, 1H) ; 6,91 (m, 4H) ; 10,53 (ls, 1H).
MS m/z 389 (M+1).
HPLC (XBRIDGE C8, acétonitrile/eau/KH₂PO₄ (30/70/6,8G PH 4), 0,4 ml/min), temps de rétention = 6.62 min, pureté chimique = 97,8%.

### Exemple 19 : (S)-3-((4-(2-((S)-4-isopropyl-2-oxooxazolidin-3-yl)éthyl)pipéridin-1-yl) méthyl)-2,3-dihydrobenzo[b][1,4]dioxine-6-carbonitrile

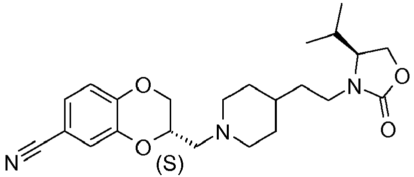

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b][1,4] dioxin-2-yl)méthyl 4-bromobenzenesulfonate 2.11, la 3-(2-(pipéridin-4-yl)éthyl) oxazolidin-2-one 3.1 par la (S)-4-isopropyl-3-(2-(pipéridin-4-yl)éthyl)oxazolidin-2-one 3.2 et le couple K₂CO₃/KI par la diisopropyl éthylamine (1,4 eq), on obtient 0,17 g (0,41 mmol) du composé 19 sous forme de solide jaune pâle, après purification par flash chromatographie sur gel de silice (éluant : dichlorométhane/acétate d'éthyle 70/30% puis dichlorométhane/méthanol = 95/5%)
Rendement : 56%
¹H RMN (CDCl₃) δ : 0,87 (d, 3H, J = 6,8 Hz) ; 0,91 (d, 3H, J = 7,2 Hz) ; 1,28 (m, 3H) ; 1,49 (m, 2H) ; 1,67 (m, 1H) ; 1,77 (m, 1H) ; 2,09 (m, 3H) ; 2,54 (m, 1H) ; 2,66 (m, 1H) ; 2,87 (m, 1H) ; 2.96 (m, 2H) ; 3,58 (m,1H) ; 3,74 (m, 1H) ; 4,04 (m, 2H) ; 4,21 (m, 1H) ; 4,28 (m, 1H) ; 4,37 (m, 1H) ; 6,91 (d, 1H, J = 8,4 Hz) ; 7,14 (m, 2H).

On dissout 0,17 g (0,41 mmol) du composé 19 dans 5 ml de méthanol, puis on ajoute 0,0.37 g (0,41 mmol) d'acide oxalique en solution dans 5 ml de méthanol. Un solide précipite ; on ajoute quelques gouttes d'éther éthylique. Le précipité est filtré puis séché à la cloche à vide. On obtient 0,14 g (0,28 mmol) de l'oxalate du composé 19 sous la forme d'un solide crème.
F : 195°C
¹H RMN (DMSOd₆) δ : 0,76 (d, 3H, J = 6,8 Hz) ; 0,84 (d, 3H, J = 7,2 Hz) ; 1,40 (m, 5H) ; 1,80 (m, 2H) ; 2,02 (m, 1H) ; 2,60 (lm, 2H) ; 2,98 (m, 3H) ; 3,30 (m, 3H) ; 3,78 (m, 1H) ; 4,11 (m, 3H) ; 4,40 (m, 1H) ; 4.67 (m, 1H) ; 7,08 (d, 1H, J = 8,4 Hz) ; 7,35 (dd, 1H, J = 8,4 et 1,8 Hz) ; 7,44 (d, 1H, J = 1,8 Hz).
MS m/z 414 (M+1).
HPLC (XBRIDGE C8, acétonitrile/eau/KH₂PO₄ (30/70/6,8G PH 4), 0,4 ml/min), temps de rétention = 6.61 min, pureté chimique = 99,05%

### Exemple 20 : (S)-3-(2-(1-((2,3-dihydrobenzo[b][1,4]dioxin-2-yl)méthyl)pipéridin-4-yl) éthyl)-6-methoxybenzo[d]oxazol-2(3H)-one

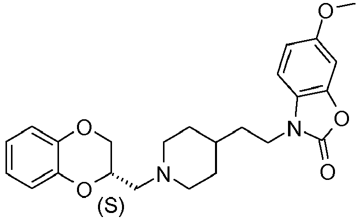

En procédant comme dans l'exemple 1, mais en remplaçant la 3-(2-(pipéridin-4-yl)éthyl)oxazolidin-2-one 3.1 par la 6-methoxy-3-(2-(pipéridin-4-yl)éthyl)benzo [d]oxazol-2(3H)-one 3.3, le K₂CO₃ par du Cs₂CO₃ et l'acétonitrile par du DMF, on obtient 0,4 g (0,94 mmol) du composé 20 sous forme de solide crème, après purification par flash chromatographie sur gel de silice (éluant : n-heptane = 100% puis dichlorométhane/acétate d'éthyle = 70/30% puis dichlorométhane/méthanol = 95/5%)
Rendement : 47%
¹H RMN (CDCl₃) δ : 1,32 (ls, 3H) ; 1,73 (m, 4H) ; 2,09 (m, 2H) ; 2,54 (m, 1H) ; 2,65 (m, 1H) ; 2,90 (m, 1H) ; 2,99 (m, 1H) ; 3,83 (m, 5H) ; 3,97 (dd, 1H J = 11,6 et7,6 Hz) ; 4,30 (m, 2H) ; 6,73 (dd, 1H, J = 8,4 et 2,4 Hz) ; 6,85 (m, 6H).

On dissout 0,4 g (0,94 mmol) du composé 20 dans 5 ml de méthanol plus 5 ml d'acétone, puis on ajoute 0,085 g (0,94 mmol) d'acide oxalique en solution dans 5 ml de méthanol. Après agitation un solide précipite; on ajoute quelques gouttes d'éther éthylique. Le précipité est filtré puis séché à la cloche à vide. On obtient 0,44 g (0,85 mmol) de l'oxalate du composé 20 sous la forme d'un solide blanc.
F : 220°C
¹H RMN (DMSOd₆) δ : 1,41 (m, 3H) ; 1,64 (m, 2H) ; 1,86 (m, 2H) ; 2,67 (m, 2H) ; 3,08 (m, 2H) ; 3,28 (m, 1H) ; 3,36 (m, 1H) ; 3,75 (s, 3H) ; 3,83 (t, 2H, J = 6,8 Hz) ; 3,99 (dd, 1H, J = 11,4 et 6,4 Hz) ; 4,29 (m, 1H) ; 4,61 (m, 1H) ; 6,86 (m, 5H) ; 7,07 (dd, 1H, J = 2 Hz) ; 7,22 (d, 1H, J = 8,8 Hz).
MS m/z 425 (M+1).
HPLC (XBRIDGE C8, acétonitrile/eau/KH₂PO₄ (35/65/6,8G PH 4), 0,4 ml/min), temps de rétention = 6,63 min, pureté chimique = 100%.

### Exemple 21 : (S)-3-((4-(2-(6-methoxy-2-oxobenzo[d]oxazol-3(2H)-yl)éthyl)pipéridin-1-yl)méthyl)-2,3-dihydrobenzo[b][1,4]dioxine-6-carbonitrile

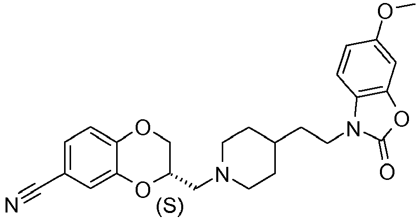

En procédant comme dans l'exemple 1, mais en remplaçant le (R)-2-(bromométhyl)-2,3-dihydrobenzo[b][1,4]dioxine 2.1 par le (S)-3-(bromométhyl)-2,3-dihydrobenzo[b] [1,4]dioxine-6-carbonitrile 2.11, la 3-(2-(pipéridin-4-yl)éthyl)oxazolidin-2-one 3.1 par la 6-methoxy-3-(2-(pipéridin-4-yl)éthyl)benzo[d]oxazol-2(3H)-one 3.3, le K₂CO₃ par le Cs₂CO₃ et l'acétonitrile par du DMF, on obtient 0,3 g (0,67 mmol) du composé 21 sous forme de solide crème, après purification par flash chromatographie sur gel de silice (éluant : n-heptane/acétate d'éthyle = 70/30% puis dichlorométhane/acétate d'éthyle = 70/30%).
Rendement : 31 %
¹H RMN (CDCl₃) δ : 1,32 (m, 3H) ; 1,70 (m, 2H) ; 1,77 (m, 2H) ; 2,10 (m, 2H) ; 2,54 (m, 1H) ; 2,65 (m, 1H) ; 2,87 (m, 1H) ; 2,95 (m, 1H) ; 3,83 (m, 5H) ; 4,02 (dd, 1H, J = 11,6 et 7,6 Hz) ; 4,28 (m, 1H) ; 4,37 (dd, 1 H, J = 11,6 et 2 Hz) ; 6,73 (m, 1H) ; 6,84 (m, 2H) ; 6,91 (d, 1H, J = 8,4 Hz) ; 7,14 (m, 2H).

On dissout 0,3 g (0,67 mmol) du composé 21 dans 5 ml de méthanol, puis on ajoute 0,060 g (0,67 mmol) d'acide oxalique en solution dans 5 ml de méthanol. Après évaporation on triture l'huile obtenue dans l'éther éthylique. Le précipité est filtré puis séché à la cloche à vide. On obtient 0,3 g (0,56 mmol) de l'oxalate du composé 21 sous la forme d'un solide blanc.
F : 105°C
¹H RMN (DMSOd₆) δ : 1,38 (m, 3H) ; 1,64 (m, 2H) ; 1,84 (m, 2H) ; 2,60 (m, 2H) ; 3,03 (m, 2H) ; 3,21 (m, 1H) ; 3,30 (m, 1H) ; 3,75 (s, 3H) ; 3,83 (t, 2H, J = 7,2 Hz) ; 4,11 (dd, 1H, J = 11,6 et 6,8 Hz) ; 4,40 (m, 1H) ; 4,67 (m, 1H) ; 6,81 (dd, 1H, J = 8,4 et 2,4 Hz) ; 7,08 (m, 2H) ; 7,21 (d, 1H, J = 8,4 Hz) ; 7,35 (dd, 1H, J = 8,4 et 2 Hz) ; 7,44 (d, 1H, J = 2 Hz).
MS m/z 450 (M+1).
HPLC (XBRIDGE C8, acétonitrile/eau/KH₂PO₄ (40/60/6,8G PH 4), 0,4 ml/min), temps de rétention = 4,35 min, pureté chimique = 97,64%.

### Exemple 22 : (S)-4-benzyl-3-(2-(1-(((S)-2,3-dihydrobenzo[b][1,4]dioxin-2-yl)méthyl) pipéridin-4-yl)éthyl)oxazolidin-2-one

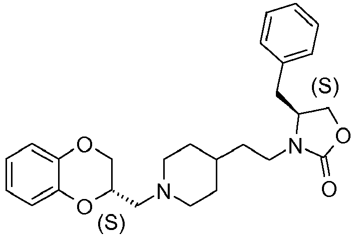

En procédant comme dans l'exemple 1, mais en remplaçant la 3-(2-(pipéridin-4-yl)éthyl)oxazolidin-2-one 3.1 par la (S)-4-benzyl-3-(2-(pipéridin-4-yl)éthyl)oxazolidin-2-one, on obtient 0,23 g (0,52 mmol) du composé 3.4 sous forme d'huile incolore, après purification par flash chromatographie sur gel de silice (éluant : dichlorométhane/méthanol = 95/5%).
Rendement : 34%
Le produit est directement salifié.

On dissout 0,23 g (0,52 mmol) du composé 22 dans 5 ml de méthanol, puis on ajoute 0,043 g (0,47 mmol) d'acide oxalique en solution dans 5 ml de méthanol. Après agitation un précipité blanc se forme; on ajoute un peu d'éther et on filtre puis on sèche le sel à la cloche à vide. On obtient 0,2 g (0,38 mmol) de l'oxalate du composé 22 sous la forme d'un solide blanc.
F : 209°C
¹H RMN (DMSOd₆) δ : 1,39 (m, 5H) ; 1,79 (m, 2H) ; 2,64 (m, 3H) ; 3,21 (m, 7 H) ; 3,98 (m, 2H) ; 4,14 (m, 2H) ; 4,29 (d, 1H) ; 4,60 (m, 1H) ; 6,88 (m, 4H) ; 7,28 (m, 5H). MS m/z 437 (M+1).
HPLC (XBRIDGE C8, acétonitrile/eau/KH₂PO₄ (35/65/6,8G PH 4), 1 ml/min), temps de rétention = 13,29 min, pureté chimique = 99,18%.
HPLC (CHIRALPACK AD-H, heptane/ethanol/diéthylamine (50/50/0,1), 1 ml/min) : composé 22, temps de rétention = 9,34 min, composé 23, temps de rétention = 8.59 min, rapport des AUC (22)/(23) = 99,5/0,5.

### Exemple 23 : (S)-4-benzyl-3-(2-(1-(((R)-2,3-dihydrobenzo[b][1,4]dioxin-2-yl)méthyl) pipéridin-4-yl)éthyl)oxazolidin-2-one

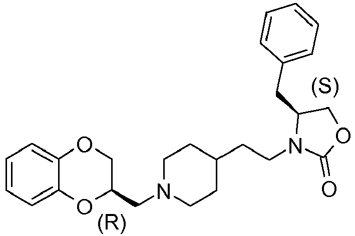

En procédant comme dans l'exemple 1, mais en remplaçant le (R)-2-(bromométhyl)-2,3-dihydrobenzo[b][1,4]dioxine 2.1 par le (S)-2-(bromométhyl)-2,3-dihydrobenzo[b] [1,4]dioxine 2.2 et la 3-(2-(pipéridin-4-yl)éthyl)oxazolidin-2-one 3.1 par la (S)-4-benzyl-3-(2-(pipéridin-4-yl)éthyl)oxazolidin-2-one 3.4, on obtient 0,35 g (0,80 mmol) du composé 23 sous forme d'huile incolore, après purification par flash chromatographie sur gel de silice (éluant : dichlorométhane/méthanol = 97/3%).
Rendement : 34%
Le produit est directement salifié.

On dissout 0,35 g (0,80 mmol) du composé 23 dans 5 ml de méthanol, puis on ajoute 0,07 g (0,77 mmol) d'acide oxalique en solution dans 5 ml de méthanol. Après évaporation on triture avec de l'éther éthylique; un précipité blanc se forme, on le filtre et on sèche le sel à la cloche à vide. On obtient 0,26 g (0,49 mmol) de l'oxalate du composé 23 sous la forme d'un solide blanc.
F : 190°C
¹H RMN (DMSOd₆) δ : 1,40 (m, 5H) ; 1,80 (m, 2H) ; 2,71 (m, 3H) ; 3,06 (m, 4H) ; 3,26 (m, 1H) ; 3,37 (m, 2H) ; 3,98 (m, 2H) ; 4,14 (m, 2H) ; 4,29 (d, 1H) ; 4,61 (m, 1H) ; 6,87 (m, 4H) ; 7,29 (m, 5H).
MS m/z 437 (M+1).
HPLC (XBRIDGE C8, acétonitrile/eau/KH₂PO₄ (35/65/6,8G PH 4), 1 ml/min), temps de rétention = 14,05 min, pureté chimique = 99,65%.
HPLC (CHIRALPACK AD-H, heptane/ethanol/diéthylamine (60/40/0,1), 1 ml/min) : composé 23, temps de rétention = 10,17 min, composé 22, temps de rétention = 11,19 min, rapport des AUC (23)/(22) = 99,6/0,4.

### Exemple 24 : (S)-3-((4-(2-((S)-4-benzyl-2-oxooxazolidin-3-yl)éthyl)pipéridin-1-yl) méthyl)-2,3-dihydrobenzo[b][1,4]dioxine-6-carbonitrile

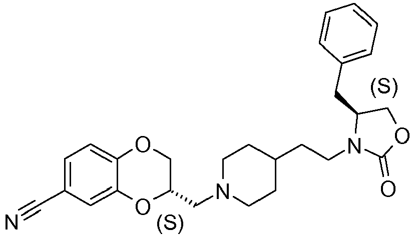

En procédant comme dans l'exemple 1, mais en remplaçant le (R)-2-(bromométhyl)-2,3-dihydrobenzo[b][1,4]dioxine 2.1 par le (R)-3-(bromométhyl)-2,3-dihydrobenzo[b] [1,4]dioxine-6-carbonitrile 2.11, la 3-(2-(pipéridin-4-yl)éthyl)oxazolidin-2-one 3.1 par la (S)-4-benzyl-3-(2-(pipéridin-4-yl)éthyl)oxazolidin-2-one 3.4, le K₂CO₃ par le Cs₂CO₃ et l'acétonitrile par le DMF, on obtient 0,6 g (1,3 mmol) du composé 24 sous forme d'huile incolore, après purification par flash chromatographie sur gel de silice (éluant : dichlorométhane/acétate d'éthyle = 70/30% puis dichlorométhane/méthanol = 95/5%).
Rendement : 40%
¹H RMN (CDCl₃) δ : 1,26 (m, 3H) ; 1,50 (m, 2H) ; 1,70 (m, 2H) ; 2,09 (m, 2H) ; 2,54 (m, 1H) ; 2,67 (m, 2H) ; 2,87 (m, 1H) ; 2,94 (m, 1H) ; 3,09 (m, 2H) ; 3,56 (m, 1H) ; 4,02 (m, 3H) ; 4,17 (m, 1H) ; 4,28 (m, 1H) ; 4,37 (dd, 1H, J = 11,4 et 2,4 Hz) ; 6,91 (d, 1H, J = 9,2 Hz) ; 7,15 (m, 4H) ; 7,31 (m, 3H).

On dissout 0,6 g (1.3 mmol) du composé 24 dans 5 ml de méthanol, puis on ajoute 0,5 ml (2,5 mmol) d'une solution d'HCl 5N dans l'isopropanol. On concentre le milieu et on triture avec de l'éther éthylique; on rajoute un minimum d'éthanol. On filtre le précipité que l'on met à sécher à la cloche à vide. On obtient 0,48 g (0,96 mmol) du chlorhydrate du composé 24 sous la forme d'un solide blanc.
F : 68°C
¹H RMN (DMSOd₆) δ : 1,47 (m, 5H) ; 1,90 (m, 2H) ; 2,50 (m, 2H) ; 2,74 (m, 1H) ; 3,08 (m, 4H) ; 3,45 (m, 2H) ; 3,66 (m, 1H) ; 3,97 (m, 1H) ; 4,14 (m, 3H) ; 4,44 (m, 1H) ; 4,97 (m, 1H) ; 7,12 (d, 1H, J = 8,4 Hz) ; 7,29 (m, 5H) ; 7,39 (dd, 1H, J = 8,4 et 2 Hz) ; 7,50 (d, 1H, J = 2 Hz) ; 10,45 (ls, 1H).
MS m/z 462 (M+1).
HPLC (XBRIDGE C8, acétonitrile/eau/KH₂PO₄ (35/65/6,8G PH 4), 0,4 ml/min), temps de rétention = 6,73 min, pureté chimique = 97,43%.

### Exemple 25 : (S)-4-benzyl-3-(2-(1-(((S)-8-bromo-2,3-dihydrobenzo[b][1,4]dioxin-2-yl)méthyl)pipéridin-4-yl)éthyl)oxazolidin-2-one

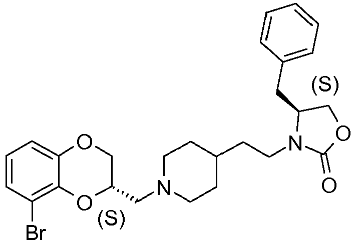

En procédant comme dans l'exemple 1, mais en remplaçant le (R)-2-(bromométhyl)-2,3-dihydrobenzo[b][1,4]dioxine 2.1 par le (R)-(8-bromo-2,3-dihydrobenzo[b][1,4] dioxin-2-yl)méthyl 4-bromobenzenesulfonate 2.4, la 3-(2-(pipéridin-4-yl)éthyl)oxazolidin-2-one 3.1 par la (S)-4-benzyl-3-(2-(pipéridin-4-yl)éthyl)oxazolidin-2-one 3.4, le K₂CO₃ par le Cs₂CO₃ et l'acétonitrile par le DMF , on obtient 0,74 g (1,43 mmol) du composé 25 sous forme d'huile incolore, après purification par flash chromatographie sur gel de silice (éluant : n-heptane/acétate d'éthyle = 70/30% puis dichlorométhane/méthanol = 95/5%).
Rendement : 54%
¹H RMN (CDCl₃) δ : 1,27 (m, 3H) ; 1,50 (m, 2H) ; 1,68 (m, 2H) ; 2,1 (m, 1H) ; 2,23 (m, 1H) ; 2,68 (m, 3H) ; 2,87 (m, 1H) ; 3,09 (m, 3H) ; 3,57 (m, 1H) ; 4,01 (m, 3H) ; 4,17 (m, 1H) ; 4,30 (dd, 1H, J = 11,4 et 2,8 Hz) ; 4,38 (m, 1H) ; 6,70 (m, 1H) ; 6,74 (m, 1H) ; 6,82 (dd, 1H, J = 8 et 1,6 Hz) ; 7,16 (m, 2H) ; 7,32 (m, 3H).

On dissout 0,74 g (1,43 mmol) du composé 25 dans 5 ml de méthanol, puis on ajoute 0,12 g (1,33 mmol) d'acide oxalique en solution dans 5 ml de méthanol. On ajoute un peu d'éther éthylique ; un précipité blanc se forme, on le filtre et on sèche le sel à la cloche à vide. On obtient 0,70 g (1,16 mmol) de l'oxalate du composé 25 sous la forme d'un solide blanc.
F : 177°C
¹H RMN (DMSOd₆) δ : 1,40 (m, 5H) ; 1,81 (m, 2H) ; 2,74 (m, 3H) ; 3,14 (m, 5H) ; 3,42 (m,2H) ; 3,96 (m, 1H) ; 4,12 (m, 3H) ; 4,33 (m, 1H) ; 4.72 (m, 1H) ; 6,82 (m, 1H) ; 6,93 (d, 1H, J = 7,6 Hz) ; 7,16 (d, 1H, J = 8 Hz) ; 7,28 (m, 5H).
MS m/z 515-517 (M+1).
HPLC (XBRIDGE C8, acétonitrile/eau/KH₂PO₄ (40/60/6,8G PH 4), 0,4 ml/min), temps de rétention = 6,25 min, pureté chimique = 94,66%.

### Exemple 26 : (S)-4-benzyl-3-(2-(1-(((S)-7-bromo-2,3-dihydrobenzo[b][1,4]dioxin-2-yl)méthyl)pipéridin-4-yl)éthyl)oxazolidin-2-one

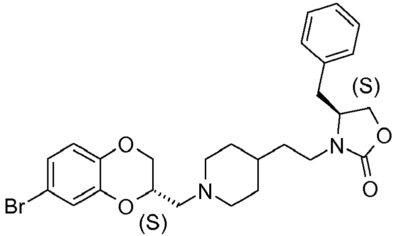

En procédant comme dans l'exemple 1, mais en remplaçant le (R)-2-(bromométhyl)-2,3-dihydrobenzo[b][1,4]dioxine 2.1 par le (R)-7-bromo-2-(bromométhyl)-2,3-dihydrobenzo[b][1,4]dioxine 2.5, la 3-(2-(pipéridin-4-yl)éthyl)oxazolidin-2-one 3.1 par la (S)-4-benzyl-3-(2-(pipéridin-4-yl)éthyl)oxazolidin-2-one 3.4 le K₂CO₃ par le Cs₂CO₃ et l'acétonitrile par le DMF, on obtient 1,1 g (2,13 mmol) du composé 26 sous forme d'huile incolore, après purification par flash chromatographie sur gel de silice (éluant : n-heptane = 100% puis dichlorométhane/méthanol = 95/5%).
Rendement : 48%
¹H RMN (CDCl₃) δ : 1,26 (m, 3H) ; 1,50 (m, 2H) ; 1,69 (m, 2H) ; 2,09 (m, 2H) ; 2,53 (m, 1H) ; 2,65 (m, 2H) ; 2,86 (m, 1H) ; 2,95 (m, 1H) ; 3,10 (m, 2H) ; 3,55 (m, 1H) ; 3,97 (m, 3H) ; 4,17 (m, 1H) ; 4,28 (m, 2H) ; 6,73 (d, 1H, J = 8,8 Hz) ; 6,92 (dd, 1H, J = 8,8 et 2,4 Hz) ; 7,03 (d, 1H, J = 2,4 Hz) ; 7,16 (m, 2H) ; 7,32 (m, 3H).

On dissout 1,1 g (2,13 mmol) du composé 26 dans 10 ml de méthanol, puis on ajoute 0,19 g (2,11 mmol) d'acide oxalique en solution dans 5 ml de méthanol. On ajoute un peu d'éther éthylique; un précipité blanc se forme, on le filtre et on sèche le sel à la cloche à vide. On obtient 0,90 g (1,48 mmol) de l'oxalate du composé 26 sous la forme d'un solide blanc.
F : 147°C
¹H RMN (DMSOd₆) δ : 1,40 (m, 5H) ; 1,79 (m, 2H) ; 2,72 (m, 3H) ; 3,07 (m, 4H) ; 3,25 (m, 1H) ; 3,38 (m, 2H) ; 3,97 (m, 1H) ; 4,04 (m, 1H) ; 4,14 (m, 2H) ; 4,30 (m, 1H) ; 4,70 (m, 1H) ; 6,88 (d, 1H, J = 8,8 Hz) ; 7,03 (dd, 1H, J = 8,8 et 2,4 Hz) ; 7,14 (d, 1H, J = 2,4 Hz) ; 7,28 (m, 5H).
MS m/z 515-517 (M+1).
HPLC (XBRIDGE C8, acétonitrile/eau/KH₂PO₄ (40/60/6,8G PH 4), 0,4 ml/min), temps de rétention = 8,68 min, pureté chimique = 100%.

### Exemple 27 : (S)-4-benzyl-3-(2-(1-(((S)-6-bromo-2,3-dihydrobenzo[b][1,4]dioxin-2-yl)méthyl)pipéridin-4-yl)éthyl)oxazolidin-2-one

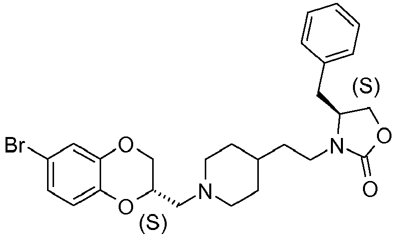

En procédant comme dans l'exemple 1, mais en remplaçant le (R)-2-(bromométhyl)-2,3-dihydrobenzo[b][1,4]dioxine 2.1 par le (R)-6-bromo-2-(bromométhyl)-2,3-dihydrobenzo[b][1,4]dioxine 2.6, la 3-(2-(pipéridin-4-yl)éthyl)oxazolidin-2-one 3.1 par la (S)-4-benzyl-3-(2-(pipéridin-4-yl)éthyl)oxazolidin-2-one 3.4 le K₂CO₃ par le Cs₂CO₃ et l'acétonitrile par le DMF, on obtient 0,65 g (1,26 mmol) du composé 27 sous forme d'huile incolore, après purification par flash chromatographie sur gel de silice (éluant : n-heptane/acétate d'éthyle = 70/30% puis dichlorométhane/méthanol = 95/5%).
Rendement : 47%
¹H RMN (CDCl₃) δ : 1,26 (m, 3H) ; 1,50 (m, 2H) ; 1,67 (m, 2H) ; 2,08 (m, 2H) ; 2,52 (m, 1H) ; 2,65 (m, 2H) ; 2,87 (m, 1H) ; 2,95 (m, 1H) ; 3,10 (m, 2H) ; 3,57 (m, 1H) ; 3,98 (m, 3H) ; 4,17 (m, 1H) ; 4,28 (m, 2H) ; 6,74 (d, 1H, J = 8,4 Hz) ; 6,93 (dd, 1H, J = 8,8 Hz et 2,4 Hz) ; 7,01 (d, 1H, J = 2,4 Hz) ; 7,16 (m, 2H) ; 7,31 (m, 3H).

On dissout 0,65 g (1,26 mmol) du composé 27 dans 5 ml de méthanol, puis on ajoute 0,11 g (1,22 mmol) d'acide oxalique en solution dans 5 ml de méthanol. On ajoute un peu d'éther éthylique; un précipité blanc se forme, on le filtre et on sèche le sel à la cloche à vide. On obtient 0,90 g (1,48 mmol) de l'oxalate du composé 27 sous la forme d'un solide blanc.
F : 191°C
¹H RMN (DMSOd₆) δ : 1,39 (m, 5H) ; 1,78 (m, 2H) ; 2,71 (m, 3H) ; 3,06 (m, 4H) ; 3,25 (m, 1H) ; 3,38 (m, 2H) ; 3,96 (dd, 1H, J = 8 et 5,2 Hz) ; 4,1 (m, 3H) ; 4,31 (m, 1H) ; 4,64 (m, 1H) ; 6,89 (d, 1H, J = 8,8) ; 7,03 (dd, 1H, J = 8,4 et 2 Hz) ; 7,12 (d, 1H, J = 2 Hz) ; 7,28 (m, 5H).
MS m/z 515-517 (M+1).
HPLC (XBRIDGE C8, acétonitrile/eau/KH₂PO₄ (40/60/6,8G PH 4), 0,4 ml/min), temps de rétention = 7,05 min, pureté chimique = 99,65%.

### Exemple 28 : (S)-4-benzyl-3-(2-(1-(((S)-5-bromo-2,3-dihydrobenzo[b][1,4]dioxin-2-yl)méthyl)pipéridin-4-yl)éthyl)oxazolidin-2-one

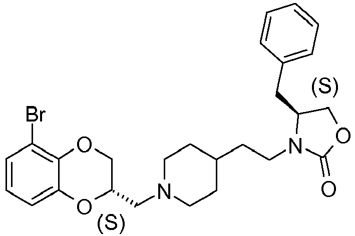

En procédant comme dans l'exemple 1, mais en remplaçant le (R)-2-(bromométhyl)-2,3-dihydrobenzo[b][1,4]dioxine 2.1 par le (R)-(5-bromo-2,3-dihydrobenzo[b][1,4] dioxin-2-yl)méthyl 4-bromobenzenesulfonate 2.15, la 3-(2-(pipéridin-4-yl)éthyl) oxazolidin-2-one 3.1 par la (S)-4-benzyl-3-(2-(pipéridin-4-yl)éthyl)oxazolidin-2-one 3.4,1e couple K₂CO₃/KI par la diisopropyl éthylamine (1,4 eq) et l'acétonitrile par le DMSO, on obtient 0,20 g (0,39 mmol) du composé 28 sous forme d'huile incolore, après purification par flash chromatographie sur gel de silice (éluant : n-heptane/acétate d'éthyle = 70/30% puis dichlorométhane/méthanol = 95/5%).
Rendement : 16%
¹H RMN (CDCl₃) δ : 1,26 (m, 3H) ; 1,49 (m, 2H) ; 1,69 (m, 2H) ; 2,10 (m, 2H) ; 2,55 (m, 1H) ; 2,66 (m, 2H) ; 2,88 (m, 1H) ; 2,96 (m, 1H) ; 3,10 (m, 2H) ; 3,57 (m, 1H) ; 4,02 (m, 3H) ; 4,17 (m, 1H) ; 4,29 (m, 1H) ; 4,43 (dd, 1H, J = 11,2 et 2,4 Hz) ; 6,17 (m, 1H) ; 6,84 (dd, 1H, J = 8,2 et 1,6 Hz) ; 7,08 (dd, 1H, J = 8 et 1,6 Hz) ; 7,16 (m, 2H) ; 7,32 (m, 3H).

On dissout 0,20 g (0,39 mmol) du composé 28 dans 5 ml de méthanol, puis on ajoute 0,035 g (0.39 mmol) d'acide oxalique en solution dans 5 ml de méthanol. On ajoute un peu d'éther éthylique; un précipité blanc se forme, on le filtre et on sèche le sel à la cloche à vide. On obtient 0,16 g (0,26 mmol) de l'oxalate du composé 28 sous la forme d'un solide blanc.
F :174°C
¹H RMN (DMSOd₆) δ : 1,39 (m, 5H) ; 1,78 (m, 2H) ; 2,62 (m, 2H) ; 2,73 (m, 1H) ; 3,06 (m, 4H) ; 3,32 (m, 3H) ; 3,96 (dd, 1H, J = 8 et 4,8 Hz) ; 4,13 (m, 3H) ; 4,42 (dd, 1H, J = 11,4 et 2 Hz) ; 4,66 (m, 1H) ; 6,82 (m, 1H) ; 6,95 (dd, 1H, J = 8 et 1,6 Hz) ; 7,16 (dd, 1H, J = 8 et 1,2 Hz) ; 7,28 (m, 5H).
MS m/z 515-517 (M+1).
HPLC (Acquity BEH C8, Phase A : 100% H₂O. HCOONH₄ 0.01M PH4, Phase B : 90% Acétonitrile 10% H2O.HCOONH4 0.01M PH4, gradient B : 45 à 70% sur 5 min, 0,75 ml/min), temps de rétention = 1,13 min, pureté chimique : 99,79%.

### Exemple 29 : (S)-4-benzyl-3-(2-(1-(((S)-7-fluoro-2,3-dihydrobenzo[b][1,4]dioxin-2-yl)méthyl)pipéridin-4-yl)éthyl)oxazolidin-2-one

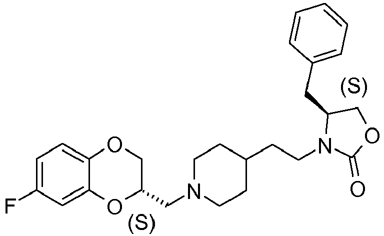

En procédant comme dans l'exemple 1, mais en remplaçant le (R)-2-(bromométhyl)-2,3-dihydrobenzo[b][1,4]dioxine 2.1 par le (R)-(7-fluoro-2,3-dihydrobenzo[b][1,4] dioxin-2-yl)méthyl 4-bromobenzenesulfonate 2.17, la 3-(2-(pipéridin-4-yl)éthyl) oxazolidin-2-one 3.1 par la (S)-4-benzyl-3-(2-(pipéridin-4-yl)éthyl)oxazolidin-2-one 3.4, le couple K₂CO₃/KI par la diisopropyl éthylamine (1,4 eq), on obtient 1 g (2,2 mmol) du composé 29 sous forme d'huile incolore, après purification par flash chromatographie sur gel de silice (éluant : dichlorométhane/acétate d'éthyle = 70/30% puis dichlorométhane/méthanol = 95/5%).
Rendement : 45%
¹H RMN (CDCl₃) δ : 1,26 (m, 3H) ; 1,50 (m, 2H) ; 1,69 (m, 2H) ; 2,08 (m, 2H) ; 2,53 (m, 1H) ; 2,66 (m, 2H) ; 2,88 (m, 1H) ; 2,96 (m, 1H) ; 3,10 (m, 2H) ; 3,57 (m, 1H) ; 3,93 (dd, 1H, J = 11,8 et 8 Hz) ; 4,00 (m, 2H) ; 4,15 (m , 1H) ; 4,27 (m, 2H) ; 6,53 (m, 1H) ; 6,61 (dd, 1H, J = 9,2 et 2,8 Hz) ; 6,78 (dd, 1H, J = 8,8 et 1,6 Hz) ; 7,16 (m, 2H) ; 7,31 (m, 3H).

On dissout 1 g (2,2 mmol) du composé 29 dans 10 ml de méthanol, puis on ajoute 0,19 g (2,11 mmol) d'acide oxalique en solution dans 10 ml de méthanol. On ajoute un peu d'éther éthylique; un précipité blanc se forme, on le filtre et on sèche le sel à la cloche à vide. On obtient 1,1 g (2,02 mmol) de l'oxalate du composé 29 sous la forme d'un solide blanc.
F : 160°C
¹H RMN (DMSOd₆) δ : 1,39 (m, 5H) ; 1,79 (m, 2H) ; 2,67 (m, 3H) ; 3,06 (m, 4H) ; 3,25 (m, 1H) ; 3,38 (m, 3H) ; 3,98 (m, 2H) ; 4,11 (m, 1H) ; 4,29 (m, 1H) ; 4,65 (m, 1H) ; 6,71 (m, 1H) ; 6,83 (dd, 1H, J = 9,6 et 3,2 Hz) ; 6,92 (dd, 1H, J = 9,2 et 1,6 Hz) ; 7,28 (m, 5H).
MS m/z 455 (M+1).
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1,82 min, pureté chimique : 99,46%.

### Exemple 30 : (S)-4-benzyl-3-(2-(1-(((S)-7-(trifluorométhyl)-2,3-dihydrobenzo[b][1,4] dioxin-2-yl)méthyl)pipéridin-4-yl)éthyl)oxazolidin-2-one

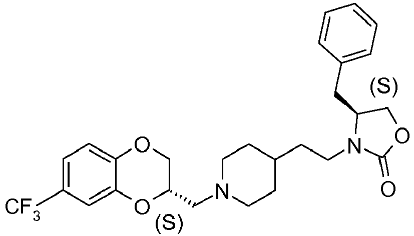

En procédant comme dans l'exemple 1, mais en remplaçant le (R)-2-(bromométhyl)-2,3-dihydrobenzo[b][1,4]dioxine 2.1 par le (R)-(7-(trifluorométhyl)-2,3-dihydrobenzo [b][1,4]dioxin-2-yl)méthyl 4-bromobenzenesulfonate 2.10, la 3-(2-(pipéridin-4-yl)éthyl) oxazolidin-2-one 3.1 par la (S)-4-benzyl-3-(2-(pipéridin-4-yl)éthyl)oxazolidin-2-one 3.4 et l'acétonitrile par le DMF , on obtient 0,24 g (0,48 mmol) du composé 30 sous forme d'huile jaune pâle, après purification par flash chromatographie sur gel de silice (éluant : gradient dichlorométhane = 100% à dichlorométhane/méthanol = 95/5% sur 30 min).
Rendement : 21%
¹H RMN (CDCl₃) δ : 1,27 (m, 3H) ; 1,50 (m, 2H) ; 1,70 (m, 2H) ; 2,09 (m, 2H) ; 2,55 (m, 1H) ; 2,67 (m, 2H) ; 2,88 (m, 1H) ; 2,96 (m, 1H) ; 3,10 (m, 2H) ; 3,56 (m, 1H) ; 4,00 (m, 3H) ; 4,17 (m, 1H) ; 4,33 (m, 2H) ; 6,93 (d, 1H, J = 8,4 Hz) ; 7,09 (m, 1H) ; 7,16 (m, 3H) ; 7,31 (m, 3H).

On dissout 0,24 g (0,48 mmol) du composé 30 dans 5 ml de méthanol, puis on ajoute 0,039 g (0,43 mmol) d'acide oxalique en solution dans 5 ml de méthanol. On concentre le milieu puis on triture avec de l'éther éthylique ; un précipité blanc se forme, on le filtre et on sèche le sel à la cloche à vide. On obtient 0,22 g (0,37 mmol) de l'oxalate du composé 30 sous la forme d'un solide blanc.
F : 139°C ¹H RMN (DMSOd₆) δ : 1,30 (m, 3H) ; 1,44 (m, 2H) ; 1,78 (m, 2H) ; 2,50 (m, 1H) ; 2,73 (m, 1H) ; 3,06 (m, 4H) ; 3,35 (m, 4H) ; 3,96 (m, 1H) ; 4,13 (m, 3H) ; 4,39 (m, 1H) ; 4,66 (m, 1H) ; 7,10 (d, 1H, J = 8,4 Hz) ; 7,28 (m, 7H).
MS m/z 505 (M+1).
HPLC (Acquity BEH C8, Phase A : 100% H₂O. HCOONH₄ 0.01M PH4, Phase B : 90% Acétonitrile 10% H2O.HCOONH4 0.01M PH4, gradient B : 55 à 100% sur 6 min, 0,75 ml/min), temps de rétention = 0,91 min, pureté chimique : 99,58%.

### Exemple 31 : (S)-4-benzyl-3-(2-(1-(((S)-7-méthyl-2,3-dihydrobenzo[b][1,4]dioxin-2-yl)méthyl)pipéridin-4-yl)éthyl)oxazolidin-2-one

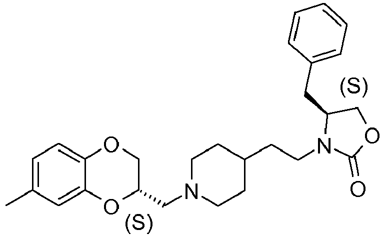

En procédant comme dans l'exemple 1, mais en remplaçant le (R)-2-(bromométhyl)-2,3-dihydrobenzo[b][1,4]dioxine 2.1 par le (R)-2-(bromométhyl)-7-méthyl-2,3-dihydrobenzo[b][1,4]dioxine 2.3, la 3-(2-(pipéridin-4-yl)éthyl)oxazolidin-2-one 3.1 par la (S)-4-benzyl-3-(2-(pipéridin-4-yl)éthyl)oxazolidin-2-one 3.4 et l'acétonitrile par le DMF, on obtient le composé 31 sous forme d'huile jaune. On solubilise cette base dans un minimum d'éthanol puis on ajoute du HCl 5N dans l'isopropanol, on concentre et on purifie ce produit salifié sur gel phase inverse C18 (éluant : HCl 5mM/acétonitrile 90/10% sur 30 min puis gradient jusqu'à HCl 5mM/acétonitrile 50/50% sur 20 min). On lyophilise les fractions récupérées pour obtenir le chlorhydrate du composé 31 sous la forme d'un solide blanc.
Rendement : 39%
F : 65°C
¹H RMN (DMSOd₆) δ : 1,45 (m, 3H) ; 1,62 (m, 2H) ; 1,91 (m, 2H) ; 2,50 (m, 1H) ; 2,74 (m, 1H) ; 3,07 (m, 4H) ; 3,4 (m, 2H) ; 3,52 (m, 1H) ; 3,62 (m, 1H) ; 4,00 (m, 2H) ; 4,12 (m, 2H) ; 4,26 (m, 1H) ; 4,79 (m, 1H) ; 6,69 (d, 1H, J = 8 Hz) ; 6,78 (m, 2H) ; 7,29 (m, 5H) ; 9,79 (ls, 1H).
MS m/z 451 (M+1).
HPLC (XBRIDGE C8, acétonitrile/eau/KH₂PO₄ (40/60/6,8G PH 4), 0,4 ml/min), temps de rétention = 5,59 min, pureté chimique = 99,92%.

### Exemple 32 : (S)-4-benzyl-3-(2-(1-(((S)-7-(trifluoromethoxy)-2,3-dihydrobenzo[b] [1,4]dioxin-2-yl)méthyl)pipéridin-4-yl)éthyl)oxazolidin-2-one

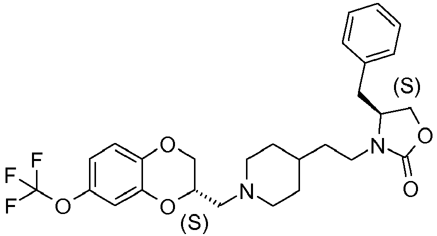

En procédant comme dans l'exemple 1, mais en remplaçant le (R)-2-(bromométhyl)-2,3-dihydrobenzo[b][1,4]dioxine 2.1 par le (R)-2-(bromométhyl)-7-(trifluoromethoxy)-2,3-dihydrobenzo[b][1,4]dioxine 2.8, la 3-(2-(pipéridin-4-yl)éthyl)oxazolidin-2-one 3.1 par la (S)-4-benzyl-3-(2-(pipéridin-4-yl)éthyl)oxazolidin-2-one 3.4 et l'acétonitrile par le DMF, on obtient 0,58 g (1,11 mmol) du composé 32 sous forme d'huile incolore, après purification sur gel de silice (gradient : dichlorométhane = 100 % à dichlorométhane/méthanol = 90/10% sur 30 min).
Rendement : 53%
¹H RMN (CDCl₃) δ : 1,27 (m, 3H) ; 1,49 (m, 2H) ; 1,72 (m, 2H) ; 2,09 (m, 2H) ; 2,53 (dd, 1H, J = 13,4 et 6 Hz) ; 2,66 (m, 2H) ; 2,88 (m, 1H) ; 2,96 (m, 1H) ; 3,10 (m, 2H) ; 3,57 (m, 1H) ; 3,98 (m, 3H) ; 4,17 (m, 1H) ; 4,30 (m, 2H) ; 6,70 (m, 1H) ; 6,78 (m, 1H) ; 6,84 (m, 1H) ; 7,16 (m, 2H) ; 7,32 (m, 3H).

On dissout 0,58 g (1,11 mmol) du composé 32 dans 10 ml de méthanol, puis on ajoute 0,095 g (1,06 mmol) d'acide oxalique en solution dans 5 ml de méthanol. On ajoute un peu d'éther éthylique; un précipité blanc se forme, on le filtre et on sèche le sel à la cloche à vide. On obtient 0,57 g (0,93 mmol) de l'oxalate du composé 32 sous la forme d'un solide blanc.
F : 194°C
¹H RMN (DMSOd₆) δ : 1,34 (m, 3H) ; 1,44 (m, 2H) ; 1,78 (m, 2H) ; 2,60 (m, 2H) ; 2,73 (m, 1H) ; 3,06 (m, 4H) ; 3,23 (m, 1H) ; 3,38 (m, 2H) ; 3,96 (dd, 1H, J = 7,8 et 5,2 Hz) ; 4,03 (dd, 1H, J = 11,6 et 6,8 Hz) ; 4,14 (m, 2H) ; 4,33 (dd, 1H, J = 11,6 et 2,4 Hz) ; 4.66 (m, 1H) ; 6,88 (m, 1H) ; 6,70 (m, 2H) ; 7,28 (m, 5H).
MS m/z 521 (M+1).
HPLC (XBRIDGE C8, acétonitrile/eau/KH₂PO₄ (40/60/6,8G PH 4), 0,4 ml/min), temps de rétention = 9,83 min, pureté chimique = 99,77%.

### Exemple 33 : (S)-4-benzyl-3-(2-(1-(((S)-6-bromo-7-methoxy-2,3-dihydrobenzo[b][1,4] dioxin-2-yl)méthyl)pipéridin-4-yl)éthyl)oxazolidin-2-one

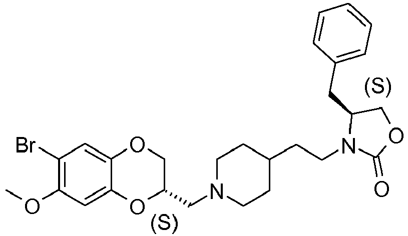

En procédant comme dans l'exemple de référenc 1, mais en remplaçant le (R)-2-(bromométhyl)-2,3-dihydrobenzo[b][1,4]dioxine 2.1 par le (R)-6-bromo-2-(bromométhyl)-7-methoxy-2,3-dihydrobenzo[b][1,4]dioxine 2.9, la 3-(2-(pipéridin-4-yl)éthyl)oxazolidin-2-one 3.1 par la (S)-4-benzyl-3-(2-(pipéridin-4-yl)éthyl)oxazolidin-2-one 3.4 et l'acétonitrile par le DMF, on obtient 0,62 g (1,33 mmol) du composé 33 sous forme d'huile incolore, après purification sur gel de silice (gradient : dichlorométhane = 100% à dichlorométhane/méthanol = 90/10%).
Rendement : 44%
¹H RMN (CDCl₃) δ : 1,29 (m, 3H) ; 1,50 (m, 2H) ; 1,70 (m, 2H) ; 2,07 (m, 2H) ; 2,51 (m, 1H) ; 2,65 (m, 2H) ; 2,88 (m, 1H) ; 2,96 (m, 1H) ; 3,11 (m, 2H) ; 3,57 (m, 1H) ; 3,80 (s, 3H) ; 3,90 (dd, 1H, J = 11,2 et 7,2 Hz) ; 4,01 (m, 2H) ; 4,17 (m, 1H) ; 4,25 (m, 2H) ; 6,50 (s, 1H) ; 7,06 (s, 1H) ; 7,16 (m, 2H) ; 7,40 (m, 3H).

On dissout 0,62 g (1,33 mmol) du composé 33 dans 5 ml de méthanol, puis on ajoute 0,114 g (1,26 mmol) d'acide oxalique en solution dans 5 ml de méthanol. On ajoute un peu d'éther éthylique; un précipité blanc se forme, on le filtre et on sèche le sel à la cloche à vide. On obtient 0,63 g (1,13 mmol) de l'oxalate du composé 33 sous la forme d'un solide blanc.
F : 204°C
¹H RMN (DMSOd₆) δ : 1,31 (m, 3H) ; 1,43 (m, 2H) ; 1,77 (m, 2H) ; 2,50 (m, 1H) ; 2,73 (m, 1H) ; 3,07 (m, 4H) ; 3,42 (m, 4H) ; 3,75 (s, 3H) ; 3,96 (m, 1H) ; 4,14 (m, 3H) ; 4,25 (m, 1H) ; 4,60 (m, 1H) ; 6,71 (s, 1H) ; 7,14 (s, 1H) ; 7,29 (m, 5H).
MS m/z 545-547 (m+1).
HPLC (XBRIDGE C8, acétonitrile/eau/KH₂PO₄ (40/60/6,8G PH 4), 0,4 ml/min), temps de rétention = 6,47 min, pureté chimique = 99,72%.

### Exemple 34 : (S)-4-benzyl-3-(2-(1-(((S)-7-methoxy-2,3-dihydrobenzo[b][1,4]dioxin-2-yl)méthyl)pipéridin-4-yl)éthyl)oxazolidin-2-one

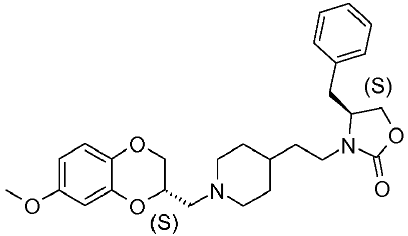

0.34 g (0,62 mmol) du composé précédent (S)-4-benzyl-3-(2-(1-(((S)-6-bromo-7-methoxy-2,3-dihydrobenzo[b][1,4]dioxin-2-yl)méthyl)pipéridin-4-yl)éthyl)oxazolidin-2-one 33 est solubilisé dans 10 ml de méthanol. On ajoute une quantité catalytique de Pd sur charbon à 10% et l'on fait une hydrogénation à TA pendant une nuit. On filtre ensuite le milieu sur célite et on concentre. On obtient 0,15 g (0,32 mmol) du composé 34 sous la forme d'une huile incolore, après purification sur gel de silice (gradient : dichlorométhane = 100% à dichlorométhane/acétate d'éthyle = 80/20% sur 30 min).
Rendement : 52%
¹H RMN (CDCl₃) δ : 1,30 (m, 3H) ; 1,50 (m, 2H) ; 1,69 (m, 2H) ; 2,09 (m, 2H) ; 2,53 (dd, 1H, J = 13,4 et 6 Hz) ; 2,66 (m, 2H) ; 2,89 (m, 1H) ; 2,97 (m, 1H) ; 3,10 (m, 2H) ; 3,58 (m, 1H) ; 3,73 (s, 3H) ; 3,92 (dd, 1H, J = 11,2 et 7,2 Hz) ; 4,01 (m, 2H) ; 4,17 (m, 1H) ; 4,26 (m, 2H) ; 6,41 (dd, 1H, J = 8,8 et 2,8 Hz) ; 6,47 (d, 1H, J = 2,8 Hz) ; 6,77 (d, 1H, J = 9,2 Hz) ; 7,16 (d, 1H, J = 6,8 Hz) ; 7,31 (m, 4H).

On dissout 0.15 g (0,32 mmol) du composé 34 dans 5 ml, puis on ajoute 0,1 ml (0,5 mmol) d'une solution d'HCl 5N dans l'isopropanol. On concentre, on rajoute un mélange 1/2 dioxanne/eau, puis on lyophilise. On obtient 0,12 g (0,24 mmol) du chlorhydrate du composé 34 sous la forme d'un solide blanc.
F : 171°C
¹H RMN (DMSOd₆) δ : 1,49 (m, 5H) ; 1,89 (m, 2H) ; 2,50 (m, 2H) ; 2,73 (m, 1H) ; 3,05 (m, 4H) ; 3,39 (m, 2H) ; 3,68 (1s, 4H) ; 3,98 (m, 2H) ; 4,14 (m, 2H) ; 4,25 (m, 1H) ; 4,90 (m, 1H) ; 6,47 (m, 1H) ; 6,53 (m, 1H) ; 6,83 (m, 1H) ; 7,28 (m, 5H).
MS m/z 467 (M+1)
HPLC (XBRIDGE C8, acétonitrile/eau/KH₂PO₄ (35/65/6,8G PH 4), 1 ml/min), temps de rétention = 8,58 min, pureté chimique = 98,96%.

### Exemple 35 : (S)-4-benzyl-3-(2-(1-(((S)-7-hydroxy-2,3-dihydrobenzo[b][1,4]dioxin-2-yl)méthyl)pipéridin-4-yl)éthyl)oxazolidin-2-one

### ° Etape 1: Préparation de (S)-4-benzyl-3-(2-(1-(((S)-7-phénoxy-2,3-dihydrobenzo[b][1,4]dioxin-2-yl)méthyl)pipéridin-4-yl)éthyl)oxazolidin-2-one

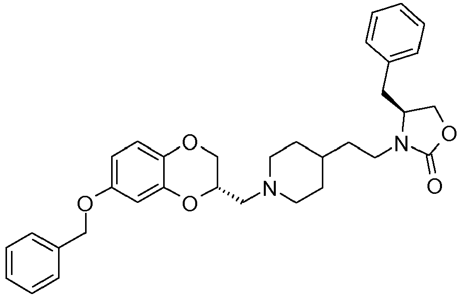

En procédant comme dans l'exemple 1, mais en remplaçant le (R)-2-(bromométhyl)-2,3-dihydrobenzo[b][1,4]dioxine 2.1 par le (R)-(7-(benzyloxy)-2,3-dihydrobenzo[b] [1,4]dioxin-2-yl)méthyl methanesulfonate 2.25, la 3-(2-(pipéridin-4-yl)éthyl)oxazolidin-2-one 3.1 par la (S)-4-benzyl-3-(2-(pipéridin-4-yl)éthyl)oxazolidin-2-one 3.4, le couple K2CO3/KI par la diisopropyl éthylamine (1,4 eq) et l'acétonitrile par le DMSO, on obtient 0,7 g (1,29 mmol) du composé 35 sous forme d'huile incolore, après purification sur gel de silice (gradient : dichlorométhane/acétate d'éthyle = 70/30% à dichlorométhane/méthanol = 95/5%).
Rendement : 24%
¹H RMN (CDCl₃) δ : 1,26 (m, 3H) ; 1,50 (m, 2H) ; 1,67 (m, 2H) ; 2,08 (m, 2H) ; 2,53 (m, 1H) ; 2,65 (m, 2H) ; 2,92 (m, 1H) ; 3,03 (m, 1H) ; 3,11 (m, 2H) ; 3,57 (m, 1H) ; 3,92 (dd, 1H, J = 11 et 6,8 Hz) ; 4,00 (m, 2H) ; 4,17 (m, 1H) ; 4,26 (m, 2H) ; 4,97 (s, 2H) ; 6,48 (dd, 1H, J =) ; 9 et 2,8 Hz) ; 6,55 (d, 1H, J = 2,8 Hz) ; 6,77 (d, 1H, J = 9 Hz) ; 7,16 (m, 2H) ; 7,35 (m, 8H)

### ° Etape 2 : Préparation de (S)-4-benzyl-3-(2-(1-(((S)-7-hydroxy-2,3-dihydrobenzo[b][1,4]dioxin-2-yl)méthyl)pipéridin-4-yl)éthyl)oxazolidin-2-one

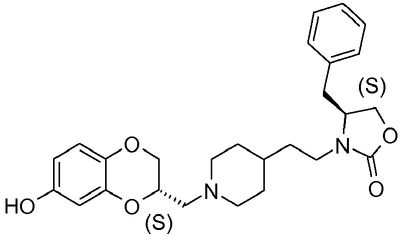

0.7 g (1,29 mmol) du composé précédent (S)-4-benzyl-3-(2-(1-(((S)-7-phénoxy -2,3-dihydrobenzo[b][1,4]dioxin-2-yl)méthyl)pipéridin-4-yl)éthyl)oxazolidin-2-one est solubilisé dans 10 ml de méthanol. On ajoute une quantité catalytique de Pd sur charbon à 5% et l'on fait une hydrogénation à TA pendant 5h00. On filtre ensuite le milieu sur célite et on concentre. Le composé 35 est obtenu sous la forme d'un solide blanc, après purification sur gel de silice (éluant : dichlorométhane/méthanol = 95/5%).
Rendement : 58%
¹H RMN (CDCl₃) δ : 1,29 (m, 3H) ; 1,49 (m, 2H) ; 1,66 (m, 2H) ; 2,1 (m, 2H) ; 2,53 (m, 1H) ; 2,65 (m, 2H) ; 2,90 (m, 1H) ; 3,08 (m, 3H) ; 3,57 (m, 1H) ; 3,90 (dd, 1H, 11,4 et 7,2 Hz) ; 4,00 (m, 2H) ; 4,18 (m, 2H) ; 4,30 (m, 1H) ; 5,30 (1s, 1H) ; 6,32 (dd, 1H, J = 8,4 et 2,8 Hz) ; 6,40 (d, 1H, J = 2,8 Hz) ; 6,71 (d, 1H, J = 8,4Hz) ; 7,16 (m, 2H) ; 7,31 (m, 3H).

On dissout 0,34 g (0,75 mmol) du composé 35 dans 5 ml de méthanol, puis on ajoute 0,068 g (0,75 mmol) d'acide oxalique en solution dans 5 ml de méthanol. On concentre à sec puis on triture dans l'éther éthylique et l'acétate d'éthyle; un précipité blanc se forme, on le filtre et on sèche le sel à la cloche à vide. On obtient 0,63 g (1,13 mmol) de l'oxalate du composé 35 sous la forme d'un solide blanc.
F : 122°C
¹H RMN (DMSOd₆) δ : 1,40 (m, 5H) ; 1,79 (m, 2H) ; 2,71 (m, 3H) ; 3,06 (m, 4H) ; 3,26 (m, 1H) ; 3,37 (m, 2H) ; 3,93 (m, 2H) ; 4,14 (m, 3H) ; 4,58 (m, 1H) ; 6,28 (m, 2H) ; 6,68 (d, 1H, J = 8,4Hz) ; 7,28 (m, 5H).
MS m/z 453 (M+1).
HPLC (Acquity BEH C8, Phase A : 100% H₂O HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1,13 min, pureté chimique : 98,47%.

### Exemple 36 : (S)-4-benzyl-3-(2-(1-(((S)-6-hydroxy-2,3-dihydrobenzo[b][1,4]dioxin-2-yl)méthyl)pipéridin-4-yl)éthyl)oxazolidin-2-one

### ° Etape 1 : Préparation de (S)-4-benzyl-3-(2-(1-(((S)-7-phénoxy-2,3-dihydrobenzo[b][1,4]dioxin-2-yl)méthyl)pipéridin-4-yl)éthyl)oxazolidin-2-one

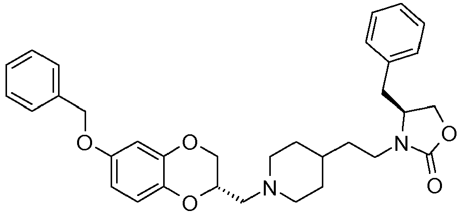

En procédant comme dans l'exemple 1, mais en remplaçant le (R)-2-(bromométhyl)-2,3-dihydrobenzo[b][1,4]dioxine 2.1 par le (R)-(6-(benzyloxy)-2,3-dihydrobenzo[b] [1,4]dioxin-2-yl)méthyl methanesulfonate 2.26 et la 3-(2-(pipéridin-4-yl) éthyl)oxazolidin-2-one 3.1 par la (S)-4-benzyl-3-(2-(pipéridin-4-yl)éthyl)oxazolidin-2-one 3.4, on obtient 0.6 g (1,11 mmol) du composé 36 sous forme d'huile incolore, après purification sur gel de silice (gradient : dichlorométhane/acétate d'éthyle = 70/30% à dichlorométhane/méthanol = 95/5%).
Rendement : 21%

### ° Etape 2: Préparation de (S)-4-benzyl-3-(2-(1-(((S)-7-hydroxy-2,3-dihydrobenzo[b][1,4]dioxin-2-yl)méthyl)pipéridin-4-yl)éthyl)oxazolidin-2-one

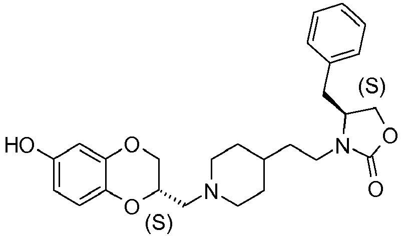

0.6 g (1,11 mmol) du composé précédent (S)-4-benzyl-3-(2-(1-(((S)-7-phénoxy-2,3-dihydrobenzo[b][1,4]dioxin-2-yl)méthyl)pipéridin-4-yl)éthyl)oxazolidin-2-one est solubilisé dans 5 ml de méthanol et 5 ml de dichlorométhane. On ajoute une quantité catalytique de Pd sur charbon à 5% et l'on fait une hydrogénation à TA pendant 5h00. On filtre ensuite le milieu sur célite et on concentre. On obtient 0.3 g (0,66 mmol) du composé 36 sous la forme d'un résidu crème, après purification sur gel de silice (gradient : dichlorométhane/méthanol = 95/5% à dichlorométhane/méthanol = 90/10% sur 30 min).
Rendement : 66%
¹H RMN (CDCl₃) δ : 1,37 (m, 5H) ; 1,72 (m, 1H) ; 1,81 (m, 1H) ; 2,27 (m, 2H) ; 2,69 (m, 3H) ; 3,09 (m, 3H) ; 3,24 (m, 1H) ; 3,54 (m, 1H) ; 3,92 (dd, 1H, J = 11,2 et 6,8 Hz) ; 4,02 (m, 2H) ; 4,18 (m, 2H) ; 4,43 (m, 1H) ; 6,35 (dd, 1H, J = 8,4 et 2,8 Hz) ; 6,41 (d, 1H, J = 2,8 Hz) ; 6,66 (d, 1H, J = 8,8 Hz) ; 7,16 (m , 2H) ; 7,31 (m, 3H).

On dissout 0,3 g (0,66 mmol) du composé 36 dans 5 ml de méthanol, puis on ajoute 0,060 g (0,66 mmol) d'acide oxalique en solution dans 5 ml de méthanol. Un précipité blanc se forme, on le filtre et on sèche le sel à la cloche à vide. On obtient 0,23 g (0.42 mmol) de l'oxalate du composé 36 sous la forme d'un solide blanc.
F : 122°C
¹H RMN (DMSOd₆) δ : 1,41 (m, 5H) ; 1,79 (m, 2H) ; 2,50 (m, 1H) ; 2,73 (m, 2H) ; 3,06 (m, 4H) ; 3,27 (m, 1H) ; 3,37 (m, 2H) ; 3,95 (m, 2H) ; 4,17 (m, 3H) ; 4,52 (m, 1H) ; 6,27 (m, 2H) ; 6,70 (d, 1H, J = 9,2 Hz) ; 7,28 (m, 5H).
MS m/z 453 (M+1).
HPLC (XBRIDGE C8, acétonitrile/eau/KH₂PO₄ (30/70/6,8G PH 4), 0,4 ml/min), temps de rétention = 7,61 min, pureté chimique = 99,91%.

### Exemple 37 : (S)-3-(2-(1-(((S)-2,3-dihydrobenzo[b][1,4]dioxin-2-yl)méthyl)pipéridin-4-yl)éthyl)-4-(4-methoxybenzyl)oxazolidin-2-one

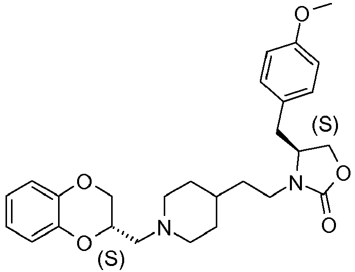

En procédant comme dans l'exemple 1, mais en remplaçant la 3-(2-(pipéridin-4-yl)éthyl)oxazolidin-2-one 3.1 par la (S)-4-(4-methoxybenzyl)-3-(2-(pipéridin-4-yl) éthyl)oxazolidin-2-one 3.9, le K₂CO₃ par le Cs₂CO₃ et l'acétonitrile par le DMF, on obtient 0,38 g (0,81 mmol) du composé 37 sous forme de solide incolore après purification sur gel de silice (éluant : gradient dichlorométhane = 100% à acétate d'éthyle = 100%).
Rendement: 41%
¹H RMN (CDCl₃) δ : 1,27 (m, 3H) ; 1,50 (m, 2H) ; 1,70 (m, 2H) ; 2,09 (m, 2H) ; 2,60 (m, 3H) ; 2,89 (m, 1H) ; 3,04 (m, 3H) ; 3,57 (m, 1H) ; 3,80 (s, 3H) ; 3,98 (m, 3H) ; 4,16 (m, 1H) ; 4,30 (m, 2H) ; 6,85 (m, 6H) ; 7,07 (m, 2H).

On dissout 0,38 g (0,81 mmol) du composé 37 dans 5 ml de méthanol, puis on ajoute 0,073 g (0,81 mmol) d'acide oxalique en solution dans 5 ml de méthanol. On ajoute un peu d'éther éthylique, un précipité blanc se forme. On le filtre et on sèche le sel à la cloche à vide. On obtient 0,37 g (0.66 mmol) de l'oxalate du composé 37 sous la forme d'un solide blanc.
F : 232°C
¹H RMN (DMSOd₆) δ : 1,40 (m, 5H) ; 1,80 (m, 2H) ; 2,66 (m, 3H) ; 2,96 (m, 1H) ; 3,09 (m, 3H) ; 3,26 (m, 1H) ; 3,36 (m, 2H) ; 3,72 (s, 3H) ; 3,99 (m, 3H) ; 4,15 (m, 1H) ; 4,30 (m, 1H) ; 4,61 (m, 1H) ; 6,88 (m, 6H) ; 7,19 (m, 2H).
MS m/z 467 (M+1).
HPLC (XBRIDGE C8, acétonitrile/eau/KH₂PO₄ (35/65/6,8G PH 4), 1 ml/min), temps de rétention = 10,73 min, pureté chimique = 99,49%.

### Exemple 38 S)-3-(2-(1-(((S)-2,3-dihydrobenzo[b][1,4]dioxin-2-yl)méthyl)pipéridin-4-yl)éthyl)-4-(4-hydroxybenzyl)oxazolidin-2-one

### ° Etape 1 : (S)-4-(4-(benzyloxy)benzyl)-3-(2-(1-(((S)-2,3-dihydrobenzo[b][1,4] dioxin-2-yl)méthyl)pipéridin-4-yl)éthyl)oxazolidin-2-one

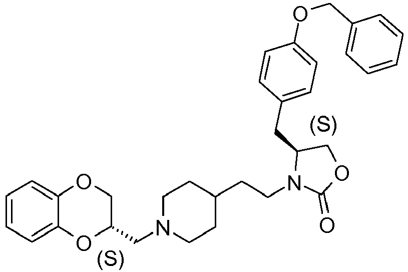

En procédant comme dans l'exemple 1, mais en remplaçant la 3-(2-(pipéridin-4-yl)éthyl)oxazolidin-2-one 3.1 par la (S)-4-(4-(benzyloxy)benzyl)-3-(2-(pipéridin-4-yl) éthyl)oxazolidin-2-one 3.10, le K₂CO₃ par le Cs₂CO₃ et l'acétonitrile par le DMF, on obtient 0,7 g (1,29 mmol) du S)-4-(4-(benzyloxy)benzyl)-3-(2-(1-(((S)-2,3-dihydrobenzo[b][1,4]dioxin-2-yl)méthyl)pipéridin-4-yl)éthyl)oxazolidin-2-one sous forme d'huile incolore, après purification sur gel de silice (éluant : dichlorométhane/méthanol = 95/5%).
Rendement : 44%
¹H RMN (CDCl₃) δ : 1,26 (m, 3H) ; 1,50 (m, 2H) ; 1,70 (m, 2H) ; 2,09 (m, 2H) ; 2,59 (m, 3H) ; 2,88 (m, 1H) ; 3,03 (m, 3H) ; 3,57 (m, 1H) ; 3,98 (m, 3H) ; 4,16 (m, 1H) ; 4,29 (m, 2H) ; 6,85 (m, 4H) ; 6,94 (m, 2H) ; 7,07 (m, 2H) ; 7,37 (m, 5H).

### ° Etape 2: (S)-3-(2-(1-(((S)-2,3-dihydrobenzo[b][1,4]dioxin-2-yl)méthyl) pipéridin-4-yl)éthyl)-4-(4-hydroxybenzyl)oxazolidin-2-one

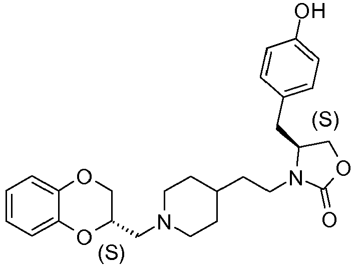

0.7 g (1,29 mmol) du composé précédent (S)-4-(4-(benzyloxy)benzyl)-3-(2-(1-(((S)-2,3-dihydrobenzo[b][1,4]dioxin-2-yl)méthyl)pipéridin-4-yl)éthyl)oxazolidin-2-one est solubilisé dans 10 ml de méthanol. On ajoute une quantité catalytique de Pd sur charbon à 10% et l'on fait une hydrogénation à TA pendant 5h30. On filtre ensuite le milieu sur célite et on concentre. On obtient 0,32 g (0,070 mmol) du composé 38 sous la forme d'un solide crème, après purification sur gel de silice (éluant : dichlorométhane/méthanol = 95/5%).
Rendement : 55%
¹H RMN (CDCl₃) δ : 1,26 (m, 3H) ; 1,47 (m, 2H) ; 1,69 (m, 2H) ; 2,11 (m, 2H) ; 2,62 (m, 3H) ; 3,00 (m, 4H) ; 3,52 (m, 1H) ; 3,97 (m, 3H) ; 4,18 (m, 1H) ; 4,30 (m, 2H) ; 6,83 (m, 6H) ; 7,00 (m, 2H).

On dissout 0,32 g (0,70 mmol) du composé 38 dans 5 ml de méthanol, puis on ajoute 0,060 g (0,70 mmol) d'acide oxalique en solution dans 5 ml de méthanol. On concentre le milieu puis on triture avec de l'éther éthylique et de l'acétate d'éthyle, un précipité blanc se forme. On le filtre et on sèche le sel à la cloche à vide. On obtient 0,32 g (0.59 mmol) de l'oxalate du composé 38 sous la forme d'un solide crème.
F : 107°C
¹H RMN (DMSOd₆) δ : 1,41 (m, 5H) ; 1,81 (m, 2H) ; 2,66 (m, 3H) ; 2,91(dd, 1H, J = 13,6 et 4 Hz) ; 3,09 (m, 3H) ; 3,26 (m, 1H) ; 3,38 (m, 2H) ; 3,94 (dd, 1H, J = 8 et 6 Hz) ; 4,00 (m, 2H) ; 4,14 (m, 1H) ; 4,29 (m, 1H) ; 4,63 (m, 1H) ; 6,69 (d, 2H, J = 8,4 Hz) ; 6,89 (m, 4H) ; 7,05 (d, 2H, J = 8,4 Hz).
MS m/z 453 (M+1).
HPLC (XBRIDGE C8, acétonitrile/eau/KH₂PO₄ (30/70/6,8G PH 4), 1 ml/min), temps de rétention = 8,11 min, pureté chimique = 98,65%.

### Exemple 39 : (S)-3-(2-(1-(((S)-2,3-dihydrobenzo[b][1,4]dioxin-2-yl)méthyl)pipéridin-4-yl)éthyl)-4-(3,4-dimethoxybenzyl)oxazolidin-2-one

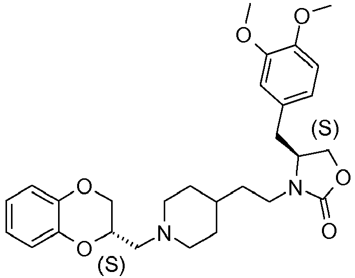

En procédant comme dans l'exemple 1, mais en remplaçant la 3-(2-(pipéridin-4-yl)éthyl)oxazolidin-2-one 3.1 par la (S)-4-(3,4-dimethoxybenzyl)-3-(2-(pipéridin-4-yl)éthyl)oxazolidin-2-one 3.11, le K₂CO₃ par le Cs₂CO₃ et l'acétonitrile par le DMF, on obtient 0,39g (0,78 mmol) du 39 sous forme d'huile incolore, après purification sur gel de silice (éluant : dichlorométhane/méthanol = 95/5%).
Rendement : 42%
¹H RMN (CDCl₃) δ : 1,29 (m, 3H) ; 1,51 (m, 2H) ; 1,70 (m, 2H) ; 2,09 (m, 2H) ; 2,54 (dd, 1H, J = 13,2 et 6 Hz) ; 2,64 (m, 2H) ; 2,90(m, 1H) ; 3,05 (m, 3H) ; 3,56 (m, 1H) ; 3,87 (s, 3H) ; 3,88 (s, 3H) ; 3,99 (m, 3H) ; 4,17 (m, 1H) ; 4,30 (m, 2H) ; 6,64 (d, 1H J = 1,4 Hz) ; 6,70 (dd, 1H, J = 5 et 1,4 Hz) ; 6,85 (m, 5H).

On dissout 0,39 g (0,78 mmol) du composé 39 dans 5 ml de méthanol, puis on ajoute 0,070 g (0,78 mmol) d'acide oxalique en solution dans 5 ml de méthanol. Un précipité blanc se forme. On le filtre et on sèche le sel à la cloche à vide. On obtient 0,34 g (0.58 mmol) de l'oxalate du composé 39 sous la forme d'un solide blanc.
F : 189°C
¹H RMN (DMSOd₆) δ : 1,38 (m, 3H) ; 1,45 (m, 2H) ; 1,81 (m, 2H) ; 2,65 (m, 3H) ; 2,95 (dd, 1H, J = 13,6 et 4,4 Hz) ; 3,10 (m, 3H) ; 3,26 (m, 1H) ; 3,37 (m, 2H) ; 3,72 (s 3H) ; 3,74 (s, 3H) ; 3,99 (m, 2H) ; 4,07 (m, 1H) ; 4,15 (m, 1H) ; 4,30 (m, 1H) ; 4,64 (m, 1H) ; 6,78 (m, 1H) ; 6,89 (m, 6H).
MS m/z 497 (M+1).
HPLC (XBRIDGE C8, acétonitrile/eau/KH₂PO₄ (30/70/6,8G PH 4), 0,4 ml/min), temps de rétention = 9,10 min, pureté chimique = 99,40%.

### Exemple 40 : (S)-3-((4-(2-((S)-4-(4-hydroxybenzyl)-2-oxooxazolidin-3-yl)éthyl) pipéridin-1-yl)méthyl)-2,3-dihydrobenzo[b][1,4]dioxine-6-carbonitrile

### ° Etape 1 : (S)-3-((4-(2-((S)-4-(4-(benzyloxy)benzyl)-2-oxooxazolidin-3-yl) éthyl)pipéridin-1-yl)méthyl)-2,3-dihydrobenzo[b][1,4]dioxine-6-carbonitrile

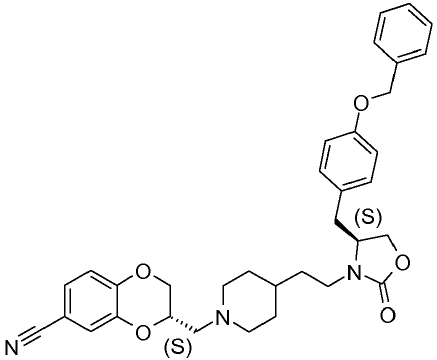

En procédant comme dans l'exemple 1, mais en remplaçant le (R)-2-(bromométhyl)-2,3-dihydrobenzo[b][1,4]dioxine 2.1 par le, (S)-3-(bromométhyl)-2,3-dihydrobenzo[b][1,4]dioxine-6-carbonitrile 2.11, la 3-(2-(pipéridin-4-yl)éthyl) oxazolidin-2-one 3.1 par la (S)-4-(4-(benzyloxy)benzyl)-3-(2-(pipéridin-4-yl)éthyl) oxazolidin-2-one 3.10, le couple K₂CO₃/KI par le DMAP (1 eq) et l'acétonitrile par le diméthylsulfoxide, on obtient 0,22 g (0,39 mmol) du (S)-3-((4-(2-((S)-4-(4-(benzyloxy)benzyl)-2-oxooxazolidin-3-yl)éthyl)pipéridin-1-yl)méthyl)-2,3-dihydrobenzo[b][1,4]dioxine-6-carbonitrile sous forme d'huile incolore, après purification sur gel de silice (éluant : gradient dichlorométhane = 100% à acétate d'éthyle = 100% sur 30 min).
Rendement : 41%
¹H RMN (CDCl₃) δ : 1,26 (m, 3H) ; 1,50 (m, 2H) ; 1,71 (m, 2H) ; 2,10 (m, 2H) ; 2,54 (dd, 1H, J = 13,4 et 6 Hz) ; 2,63 (m, 2H) ; 2,87 (m, 1H) ; 2,94 (m, 1H) ; 3,07 (m, 2H) ; 3,57 (m, 1H) ; 4,01 (m, 3H) ; 4,13 (m, 1H) ; 4,28 (m, 1H) ; 4,36 (dd, 1H, J = 11,2 et 2,4Hz) ; 5,05 (s, 2H) ; 6,92 (m, 3H) ; 7,07 (d, 2H, J = 8,4 Hz) ; 7,15 (m, 2H) ; 7,42 (m, 5H).

### ° Etape 2 : (S)-3-((4-(2-((S)-4-(4-hydroxybenzyl)-2-oxooxazolidin-3-yl)éthyl) pipéridin-1-yl)méthyl)-2,3-dihydrobenzo[b][1,4]dioxine-6-carbonitrile

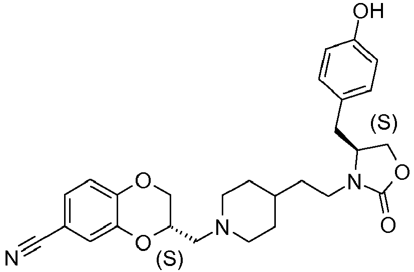

0.22 g (0.39 mmol) du composé précédent (S)-3-((4-(2-((S)-4-(4-(benzyloxy)benzyl)-2-oxooxazolidin-3-yl)éthyl)pipéridin-1-yl)méthyl)-2,3-dihydrobenzo[b][1,4]dioxine-6-carbonitrile est solubilisé dans 5 ml de méthanol et 5 ml de dichlorométhane. On ajoute une quantité catalytique de Pd sur charbon à 5% et l'on fait une hydrogénation à TA pendant 9h00. On filtre ensuite le milieu sur célite et on concentre. On obtient 0.12 g (0.25 mmol) du composé 40 sous la forme d'une mousse blanche après purification sur gel de silice (gradient : dichlorométhane = 100% à dichlorométhane/méthanol = 95/5% sur 30 min).
Rendement : 64%
¹H RMN (CDCl₃) δ : 1,27 (m, 3H) ; 1,49 (m, 2H) ; 1,71 (m, 2H) ; 2,09 (m, 2H) ; 2,54 (dd, 1H, J = 13,6 et 6 Hz) ; 2,65 (m, 2H) ; 2,89 (m, 1H) ; 2,98 (m, 2H) ; 3,09 (m, 1H) ; 3,56 (m, 1H) ; 4,00 (m, 3H) ; 4,18 (m, 1H) ; 4,31 (m, 1H) ; 4,36 (dd, 1H, J = 11,4 et 2 Hz) ; 6,79 (d, 2H, J = 6,4 Hz) ; 6,91 (m, 1H) ; 7,01 (d, 2H, J = 6,4 Hz) ; 7,13 (m, 2H).

On dissout 0.115 g (0,24 mmol) du composé 40 dans 5 ml de méthanol, puis on ajoute 0,1 ml (0,5mmol) d'une solution d'HCl 5N dans l'isopropanol. On concentre, on rajoute un mélange 1/2 dioxanne/eau, puis on lyophilise. On obtient 0,12 g (0,23 mmol) du chlorhydrate du composé 40 sous la forme d'un solide blanc.
¹H RMN (DMSOd₆) δ : 1,48 (m, 5H) ; 1,91 (m, 2H) ; 2,56 (m, 3H) ; 2,92 (dd, 1H, J = 13,8 et 4 Hz) ; 3,07 (m, 3H) ; 3,40 (m, 2H) ; 3,66 (m, 1H) ; 3,94 (dd, 1H, J = 8 et 5,2 Hz) ; 4,02 (m, 1H) ; 4,16 (m, 2H) ; 4,42 (m, 1H) ; 4,87 (m, 1H) ; 6,70 (d, 2H, J = 8,4 Hz) ; 7,05 (d, 2H, J = 8,4 Hz) ; 7,12 (d, 1H, J = 8,4 Hz) ; 7,39 (dd, 1H, J = 8,4 et 1,6 Hz) ; 7,50 (d, 1H, J = 1,6 Hz) ; 9,31 (s, 1H) ; 10,34 (1s, 1H).
MS m/z 478 (M+1)
HPLC (XBRIDGE C8, acétonitrile/eau/KH₂PO₄ (30/70/6,8G PH 4), 1 ml/min), temps de rétention = 6,95 min, pureté chimique = 97,79%.

### Exemple 41 : (S)-3-((4-(2-((S)-4-(4-fluorobenzyl)-2-oxooxazolidin-3-yl)éthyl)pipéridin-1-yl)méthyl)-2,3-dihydrobenzo[b][1,4]dioxine-6-carbonitrile

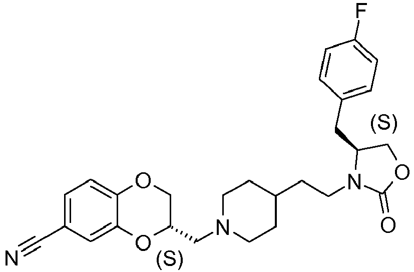

En procédant comme dans l'exemple 1, mais en remplaçant le (R)-2-(bromométhyl)-2,3-dihydrobenzo[b][1,4]dioxine 2.1 par le, (S)-3-(bromométhyl)-2,3-dihydrobenzo[b] [1,4]dioxine-6-carbonitrile 2.11, la 3-(2-(pipéridin-4-yl)éthyl)oxazolidin-2-one 3.1 par la (S)-4-(4-fluorobenzyl)-3-(2-(pipéridin-4-yl)éthyl)oxazolidin-2-one 3.12, le couple K₂CO₃/KI par le DMAP (1 eq) et l'acétonitrile par le diméthylsulfoxide, on obtient 0,26 g (0,54 mmol) du composé 41 sous forme d'huile incolore, après purification sur gel de silice (gradient : dichlorométhane = 100% à dichlorométhane/méthanol = 98/2% sur 30 min).
Rendement : 35%
¹H RMN (CDCl₃) δ : 1,26 (m, 3H) ; 1,49 (m, 2H) ; 1,71 (m, 2H) ; 2;10 (m, 2H) ; 2,54 (dd, 1H, J = 13,4 et 6,4 Hz) ; 2,67 (m, 2H) ; 2,87 (m, 1H) ; 2,94 (m, 1H) ; 3,07 (m, 2H) ; 3,59 (m, 1H) ; 4,00 (m, 3H) ; 4,18 (m, 1H) ; 4,29 (m, m, 1H) ; 4,37 (dd, 1H, J = 11,2 et 2,4 Hz) ; 6,91 (d, 1H, J = 8,4 Hz) ; 7,03 (m, 2H) ; 7,14 (m, 4H).

On dissout 0.26 g (0,54 mmol) du composé 41 dans 5 ml de méthanol, puis on ajoute 0,12 ml (0,6 mmol) d'une solution d'HCl 5N dans l'isopropanol. On concentre, on rajoute un mélange 1/2 dioxanne/eau, puis on lyophilise. On obtient 0,27 g (0,52 mmol) du chlorhydrate du composé 41 sous la forme d'un solide blanc.
¹H RMN (DMSOd₆) δ : 1,48 (m, 5H) ; 1,90 (m, 2H) ; 2,50 (m, 2H) ; 2,74 (m, 1H) ; 3,08 (m, 4H) ; 3,40 (m, 2H) ; 3,67 (m, 1H) ; 3,97 (dd, 1H, J = 7,8 et 5,2 Hz) ; 4,13 (m, 3H) ; 4,46 (m, 1H) ; 4,98 (m, 1H) ; 7,14 (m, 3H) ; 7,33 (m, 2H) ; 7,38 (dd, 1H, J = 8,4 et 2 Hz) ; 7,50 (d, 1H, J = 2 Hz) ; 10,60 (1s, 1H).
MS m/z 480 (M+1)

### HPLC (XBRIDGE C8, acétonitrile/eau/KH₂PO₄ (40/60/6,8G PH 4), 1 ml/min), temps de rétention = 7,71 min, pureté chimique = 96,89%.

### Exemple 42 : (S)-4-benzyl-3-(3-(1-(((S)-2,3-dihydrobenzo[b][1,4]dioxin-2-yl)méthyl) pipéridin-4-yl)propyl)oxazolidin-2-one

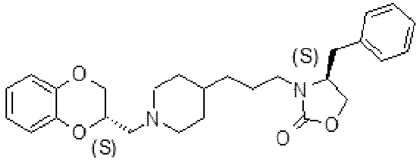

En procédant comme dans l'exemple 1, mais en remplaçant la 3-(2-(pipéridin-4-yl)éthyl)oxazolidin-2-one 3.1 par la (S)-4-benzyl-3-(3-(pipéridin-4-yl)propyl) oxazolidin-2-one 3.31 et l'acétonitrile par le diméthylformamide, on obtient 0.6 g (1,33 mmol) du composé 42 sous la forme d'huile incolore, après purification par flash chromatographie sur gel de silice (éluant : dichlorométhane/méthanol = 95/5%).
Rendement : 53%

On dissout 0,6 g (1,33 mmol) du composé 42 dans un minimum de méthanol et on ajoute 0,108 g (1,20 mmol) d'acide oxalique en solution dans 5 ml de méthanol. On concentre le milieu et on triture avec de l'éther éthylique. On filtre le précipité qui a tendance à coller et on le met à sécher à la cloche à vide. On obtient 0,56 g (1,03 mmol) de l'oxalate du composé 42 sous la forme d'un solide blanc.
¹H RMN (DMSOd₆) δ : 1,17 (m, 2H) ; 1,42 (m, 5H) ; 1,76 (m, 2H) ; 2,71 (m, 3H) ; 3,04 (m, 4H) ; 3,31 (m, 3H) ; 3,98 (m, 2H) ; 4,09 (m, 1H) ; 4,17 (m, 1H) ; 4,29 (m, 1H) ; 4,62 (m, 1H) ; 6,88 (m, 4H) ; 7,29 (m, 5H).
MS m/z 451 (M+1).
HPLC (XBRIDGE C8, acétonitrile/eau/KH₂PO₄ (40/60/6,8G PH 4), 0,4 ml/min), temps de rétention = 5,00 min, pureté chimique = 98,40%.

### Exemple 43 : (S)-3-(2-(1-(((S)-2,3-dihydrobenzo[b][1,4]dioxin-2-yl)méthyl)pipéridin-4-yl)éthyl)-4-phenyloxazolidin-2-one

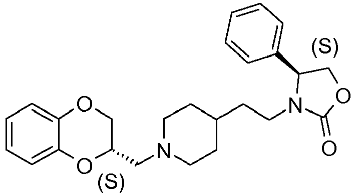

En procédant comme dans l'exemple 1, mais en remplaçant, la 3-(2-(pipéridin-4-yl)éthyl)oxazolidin-2-one 3.1 par la (S)-4-phenyl-3-(2-(pipéridin-4-yl)éthyl)oxazolidin-2-one 3.6, le K₂CO₃ par le Cs₂CO₃ et l'acétonitrile par le diméthylformamide, on obtient 0,30 g (0,71 mmol) du composé 43 sous forme d'huile incolore, après purification sur gel de silice (gradient : dichlorométhane = 100% à dichlorométhane/méthanol = 90/10% sur 30 min).
Rendement : 35%
¹H RMN (CDCl₃) δ : 1,20 (m, 3H) ; 1,36 (m, 2H) ; 1,59 (m, 2H) ; 2,04 (m, 2H) ; 2,51 (dd, 1H, J = 13,2 et 6 Hz) ; 2,62 (dd, 1H, J = 13,2 et 6 Hz) ; 2,85 (m, 3H) ; 3,48 (m, 1H) ; 3,95 (dd, 1H, 11,6 et 7,6 Hz) ; 4,13 (m, 1H) ; 4,27 (m, 2H) ; 4,61 (m, 1H) ; 4,76 (m, 1H) ; 6,84 (m, 4H) ; 7,29 (m, 2H) ; 7,40 (m, 3H).

On dissout 0.30 g (0,71 mmol) du composé 43 dans 5 ml de méthanol, puis on ajoute 0,15 ml (0,75mmol) d'une solution d'HCl 5N dans l'isopropanol. On concentre, on triture avec de l'éther éthylique. On filtre le solide et on obtient 0,30 g (0,65 mmol) du chlorhydrate du composé 43 sous la forme d'un solide blanc.
F : 68°C
¹H RMN (DMSOd₆) δ : 1,34 (m, 5H) ; 1,79 (m, 2H) ; 2,50 (m, 1H) ; 2,70 (m, 1H) ; 2,97 (m, 2H) ; 3,30 (m, 2H) ; 3,40 (m, 1H) ; 3,61 (m, 1H) ; 4,05 (m, 2H) ; 4,31 (m, 1H) ; 4,63 (m, 1H) ; 4,86 (m, 1H) ; 4,94 (m, 1H) ; 6,91 (m, 4H) ; 7,42 (m, 5H).
MS m/z 423 (M+1)
HPLC (XBRIDGE C8, acétonitrile/eau/KH₂PO₄ (35/65/6,8G PH 4), 0,4 ml/min), temps de rétention = 6,14 min, pureté chimique = 98,76%.

### Exemple 44 : (R)-3-(2-(1-(((S)-2,3-dihydrobenzo[b][1,4]dioxin-2-yl)méthyl)pipéridin-4-yl)éthyl)-4-phenyloxazolidin-2-one

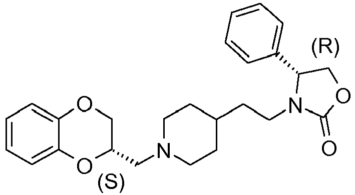

En procédant comme dans l'exemple 1, mais en remplaçant, la 3-(2-(pipéridin-4-yl)éthyl)oxazolidin-2-one 3.1 par la (R)-4-phenyl-3-(2-(pipéridin-4-yl)éthyl)oxazolidin-2-one 3.5, on obtient 0,13 g (0,31 mmol) du composé 44 sous forme d'huile incolore après purification sur gel de silice (éluant : dichlorométhane/acétate d'éthyle = 70/30% puis dichlorométhane/méthanol = 95/5%).
Rendement : 24%

On dissout 0,13 g (0,31 mmol) du composé 44 dans 5 ml de méthanol, puis on ajoute 0,027 g (0,30 mmol) d'acide oxalique en solution dans 5 ml de méthanol. On rajoute un peu d'éther éthylique ; un précipité blanc se forme. On le filtre et on sèche le sel à la cloche à vide. On obtient 0,054 g (0.11 mmol) de l'oxalate du composé 44 sous la forme d'un solide blanc.
F : 125°C
¹H RMN (DMSOd₆) δ : 1,23 (m, 5H) ; 1,67 (m, 2H) ; 2,50 (m, 1H) ; 2,69 (m, 1H) ; 2,97-3,72 (m, 6H) ; 3,97 (m, 1H) ; 4,07 (m, 1H) ; 4,28 (m, 1H) ; 4,55 (m, 1H) ; 4,63 (m, 1H) ; 4,92 (m, 1H) ; 6,87 (m, 4H) ; 7,41 (m, 5H).
MS m/z 423 (M+1).
HPLC (XBRIDGE C8, acétonitrile/eau/KH₂PO₄ (30/70/6,8G PH 4), 1 ml/min), temps de rétention = 16,79 min, pureté chimique = 100%.

### Exemple 45 : 3-(2-(1-(((S)-2,3-dihydrobenzo[b][1,4]dioxin-2-yl)méthyl)pipéridin-4-yl) éthyl)-4-(2-methoxyphenyl)oxazolidin-2-one

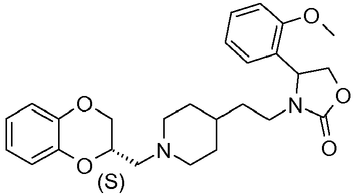

En procédant comme dans l'exemple 1, mais en remplaçant, la 3-(2-(pipéridin-4-yl)éthyl)oxazolidin-2-one 3.1 par la 4-(2-methoxyphenyl)-3-(2-(pipéridin-4-yl)éthyl) oxazolidin-2-one 3.7, on obtient 2,5 g (5,52 mmol) du composé 45 sous forme d'huile incolore, après purification sur gel de silice (éluant : dichlorométhane = 100% puis dichlorométhane/méthanol = 95/5%).
Rendement : 54%
¹H RMN (CDCl₃) δ : 1,19 (m, 3H) ; 1,39 (m, 2H) ; 1,61 (m, 2H) ; 2,04 (m, 2H) ; 2,52 (m, 1H) ; 2,62 (m, 1H) ; 2,83 (m, 2H) ; 2,94 (m, 1H) ; 3,54 (m, 1H) ; 3,85 (s, 3H) ; 3,95 (dd, 1H, J = 11,6 et 5,6 Hz) ; 4,10 (dd, 1H, J = 8,4 et 5,6 Hz) ; 4,28 (m, 2H) ; 4,59 (m, 1H) ; 5,15 (m, 1H) ; 6,83 (m, 4H) ; 6,92 (d, 1H, J = 8,2 Hz) ; 6,99 (m, 1H) ; 7,19 (dd, 1H, J = 7,5 et 1,4 Hz) ; 7,31 (m, 1H).

On dissout 0,90 g (1,99 mmol) du composé 45 dans 10 ml de méthanol, puis on ajoute 0,17 g (1,99 mmol) d'acide oxalique en solution dans 10 ml de méthanol. On rajoute un peu d'éther éthylique; un précipité blanc se forme. On le filtre et on sèche le sel à la cloche à vide. On obtient 0,65 g (1,20 mmol) de l'oxalate du composé 45 sous la forme d'un solide blanc.
F : 145°C
¹H RMN (DMSOd₆) δ : 1,27 (m, 5H) ; 1,70 (m, 2H) ; 2,67 (m, 2H) ; 3,05 (m, 2H) ; 3,30 (m, 4H) ; 3,80 (s, 3H) ; 3,98 (dd, 1H, J = 11,4 et 6,6 Hz) ; 4,06 (dd, 1H, J = 8,4 et 5,4 Hz) ; 4,28 (m, 1H) ; 4,58 (m, 2H) ; 5,12 (m, 1H) ; 6,87 (m, 4H) ; 7,00 (m, 1H) ; 7,09 (d, 1H, J = 8,2 Hz) ; 7,21 (m, 1H) ; 7,36 (m, 1H).
MS m/z 453 (M+1).
HPLC (XBRIDGE C8, acétonitrile/eau/KH₂PO₄ (35/65/6,8G PH 4), 1 ml/min), temps de rétention = 9,57 min, pureté chimique = 99,11%.

### Exemple 46 : 3-(2-(1-(((S)-2,3-dihydrobenzo[b][1,4]dioxin-2-yl)méthyl)pipéridin-4-yl) éthyl)-4-(4-methoxyphenyl)oxazolidin-2-one

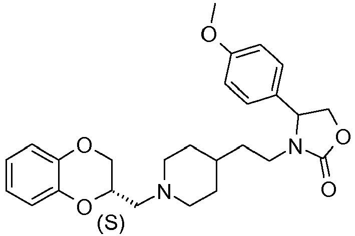

En procédant comme dans l'exemple 1, mais en remplaçant, la 3-(2-(pipéridin-4-yl)éthyl)oxazolidin-2-one 3.1 par la 4-(4-methoxyphenyl)-3-(2-(pipéridin-4-yl)éthyl) oxazolidin-2-one 3.8, on obtient 1,7 g (3,76 mmol) du composé 46 sous forme d'huile incolore, après purification sur gel de silice (éluant : dichlorométhane = 100% puis dichlorométhane/méthanol = 95/5%).
Rendement : 59%
¹H RMN (CDCl₃) δ : 1,19 (m, 3H) ; 1,36 (m, 2H) ; 1,61 (m, 2H) ; 2,06 (m, 2H) ; 2,52 (dd, 1H, J = 13,2 et 5,8 Hz) ; 2,62 (dd, 1H, J = 13,2 et 5,3 Hz) ; 2,79 (m, 2H) ; 2,93 (m, 1H) ; 3,44 (m, 1H) ; 3,83 (s, 3H) ; 3,95 (m, 1H) ; 4,10 (m, 1H) ; 4,27 (m, 2H) ; 4,58 (m, 1H) ; 4,71 (m, 1H) ; 6,84 (m, 4H) ; 6,93 (d, 2H, J = 8,1 Hz) ; 7,21 (d, 2H , J = 8,1 Hz).

On dissout 0,40 g (0,88 mmol) du composé 46 dans 5 ml de méthanol, puis on ajoute 0,079 g (0,88 mmol) d'acide oxalique en solution dans 5 ml de méthanol. On rajoute un peu d'éther éthylique; un précipité blanc se forme. On le filtre et on sèche le sel à la cloche à vide. On obtient 0,40 g (0,73 mmol) de l'oxalate du composé 46 sous la forme d'un solide crème.
F : 105°C
¹H RMN (DMSOd₆) δ : 1,29 (m, 5H) ; 1,68 (m, 2H) ; 2,67 (m, 2H) ; 3,07 (m, 2H) ; 3,32 (m, 4H) ; 3,76 (s, 3H) ; 4,01 (m, 2H) ; 4,29 (m, 1) ; 4,60 (m, 2H) ; 4,87 (m, 1H) ; 6,86 (m, 4H) ; 6,99 (d, 2H, J = 7,5 Hz) ; 7,30 (d, 2H, J = 7,5 Hz).
MS m/z 453 (M+1).
HPLC (XBRIDGE C8, acétonitrile/eau/KH₂PO₄ (35/65/6,8G PH 4), 1 ml/min), temps de rétention = 9,51 min, pureté chimique = 99,45%.

### Exemple 47 : 3-(2-(1-(((S)-2,3-dihydrobenzo[b][1,4]dioxin-2-yl)méthyl)pipéridin-4-yl) éthyl)-4-(4-hydroxyphenyl)oxazolidin-2-one

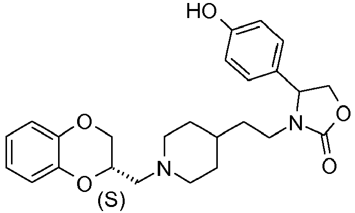

1,30 g (2,87 mmol) du composé précédent 3-(2-(1-(((S)-2,3-dihydrobenzo [b][1,4]dioxin-2-yl)méthyl)pipéridin-4-yl)éthyl)-4-(4-methoxyphenyl)oxazolidin-2-one 46 est solubilisé dans 20 ml de dichlorométhane. On se place à -78°C et on ajoute goutte à goutte 0,7 ml (7,18 mmol) de BBr₃. On laisse remonter à température ambiante. On laisse sous agitation 1h30. On refroidit à 0°C, on ajoute 50 ml d'eau et on extrait au dichlorométhane. La phase organique est séchée sur Na2SO4, filtrée, évaporée et on obtient 0.30 g (0.68 mmol) du composé 47 sous la forme d'un solide blanc, après purification sur gel de silice (éluant : dichlorométhane/méthanol = 95/5%).
Rendement : 23%
¹H RMN (CDCl₃) δ : 1,23 (m, 3H) ; 1,35 (m, 2H) ; 1,61 (m, 2H) ; 2,06 (m, 2H) ; 2,54 (m, 1H) ; 2,64 (m, 1H) ; 2,81 (m, 2H) ; 2,97 (m, 1H) ; 3,42 (m, 1H) ; 3,95 (m, 1H) ; 4,10 (m, 1H) ; 4,27 (m, 2H) ; 4,58 (m, 1H) ; 4,69 (m, 1H) ; 6,85 (m, 6H) ; 7,16 (d, 2H, J = 7,9 Hz).

On dissout 0,08 g (0,18 mmol) du composé 47 dans 5 ml de méthanol, puis on ajoute 0,016 g (0,18 mmol) d'acide oxalique en solution dans 5 ml de méthanol. On rajoute un peu d'éther éthylique; un précipité blanc se forme. On le filtre et on sèche le sel à la cloche à vide. On obtient 0,094 g (0,17 mmol) de l'oxalate du composé 47 sous la forme d'un solide blanc.
F : 113°C
¹H RMN (DMSOd₆) δ : 1,27 (m, 5H) ; 1,68 (m, 2H) ; 2,67 (m, 2H) ; 3,01 (m, 2H) ; 3,21 (m, 4H) ; 4,00 (m, 2H) ; 4,28 (m, 1H) ; 4,57 (m, 2H) ; 4.79 (m, 1H) ; 6,79 (d, 2H, J = 8 Hz) ; 6,87 (m, 4H) ; 7,17 (d, 2H , J = 8Hz).
MS m/z 439 (M+1).
HPLC (XBRIDGE C8, acétonitrile/eau/KH₂PO₄ (30/70/6,8G PH 4), 1 ml/min), temps de rétention = 7,48 min, pureté chimique = 89,56%.

### Exemple 48 : (S)-3-((4-(2-((R)-2-oxo-4-phenyloxazolidin-3-yl)éthyl)pipéridin-1-yl) méthyl)-2,3-dihydrobenzo[b][1,4]dioxine-6-carbonitrile

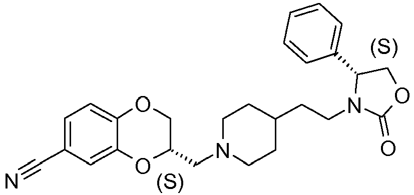

En procédant comme dans l'exemple 1, mais en remplaçant le (R)-2-(bromométhyl)-2,3-dihydrobenzo[b][1,4]dioxine 2.1 par le, (S)-3-(bromométhyl)-2,3-dihydrobenzo[b] [1,4]dioxine-6-carbonitrile 2.11, la 3-(2-(pipéridin-4-yl)éthyl)oxazolidin-2-one 3.1 par la (R)-4-phenyl-3-(2-(pipéridin-4-yl)éthyl)oxazolidin-2-one 3.5, le couple K₂CO₃/KI par la DMAP (1 eq) et l'acétonitrile par le diméthylsulfoxide, on obtient 0,2 g (0,45 mmol) du composé 48 sous forme d'huile jaune, après purification par flash chromatographie sur gel de silice (éluant : dichlorométhane/acétate d'éthyle = 70/30% puis dichlorométhane/méthanol = 95/5%).
Rendement : 70%
¹H RMN (CDCl₃) δ : 1,18 (m, 3H) ; 1,36 (m, 2H) ; 1,62 (m, 2H) ; 2,04 (m, 2H) ; 2,51 (dd, 1H, J = 13,4 et 6 Hz) ; 2,62 (dd, 1H, J = 13,2 et 5,6 Hz) ; 2,79 (m, 2H) ; 2,91 (m, 1H) ; 3,48 (m, 1H) ; 3,99 (dd, 1H, J = 11,4 et 7,2 Hz) ; 4,13 (dd, 1H, J = 8,8 et 6,8 Hz) ; 4,26 (m, 1H) ; 4,35 (dd, 1H, J = 11,4 et 2 Hz) ; 4,62 (m, 1H) ; 4,76 (dd, 1H, J = 8,8 et 6,8 Hz) ; 6,90 (d, 1H, J = 8,4 Hz) ; 7,14 (m, 2H) ; 7,29 (m, 2H) ; 7,41 (m, 3H).

On dissout 0,2 g (0,45 mmol) du composé 48 dans 5 ml de méthanol, puis on ajoute 0,040 g (0,45 mmol) d'acide oxalique en solution dans 5 ml de méthanol. On rajoute un peu d'éther éthylique; un précipité blanc se forme. On le filtre et on sèche le sel à la cloche à vide. On obtient 0,17 g (0,32 mmol) de l'oxalate du composé 48 sous la forme d'un solide blanc.
F : 200°C
¹H RMN (DMSOd₆) δ : 1,24 (m, 5H) ; 1,68 (m, 2H) ; 2,63 (m, 3H) ; 3,04 (m, 2H) ; 3,17 (m, 1H) ; 3,29 (m, 2H) ; 4,09 (m, 2H) ; 4,39 (m, 1H) ; 4,63 (m, 2H) ; 4,91 (m, 1H) ; 7,08 (d, 1H, J = 8,4 Hz) ; 7,40 (m, 7H).
MS m/z 448 (M+1).

### HPLC (XBRIDGE C8, acétonitrile/eau/KH₂PO₄ (35/65/6,8G PH 4), 0,4 ml/min), temps de rétention = 6,33 min, pureté chimique = 99,01%.

### Exemple 49 : (R)-3-(2-(1-(((S)-2,3-dihydrobenzo[b][1,4]dioxin-2-yl)méthyl)pipéridin-4-yl)éthyl)-4-(hydroxyméthyl)oxazolidin-2-one

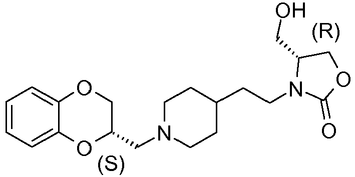

En procédant comme dans l'exemple 1, mais en remplaçant, la 3-(2-(pipéridin-4-yl)éthyl)oxazolidin-2-one 3.1 par la (R)-4-(hydroxyméthyl)-3-(2-(pipéridin-4-yl) éthyl)oxazolidin-2-one 3.21 et le K₂CO₃ par le Cs₂CO₃, on obtient 0,4 g (1,06 mmol) du composé 49 sous forme d'huile incolore, après purification sur gel de silice (gradient : dichlorométhane = 100% à dichlorométhane/méthanol = 90/10% sur 30 min).
Rendement : 36%
¹H RMN (CDCl₃) δ : 1,29 (m, 3H) ; 1,51 (m, 2H) ; 1,72 (m, 2H) ; 2,09 (m, 2H) ; 2,54 (dd, 1H, J = 13,4 et 6,4 Hz) ; 2,65 (dd, 1H, J = 13,2 et 5,6 Hz) ; 2,88 (m, 1H) ; 2,99 (m, 1H) ; 3,15 (m, 1H) ; 3,52 (m, 1H) ; 3,70 (dd, 1H, J = 11,4 et 4 Hz) ; 3,79 (dd, 1H, J = 11,4 et 4 Hz) ; 3,96 (dd, 1H, J = 11,6 et 7,6 Hz) ; 4,29 (m, 2H) ; 4,36 (m, 1H) ; 6,85 (m, 4H).

On dissout 0,28 g (0,74 mmol) du composé 49 dans 5 ml de méthanol, puis on ajoute 0,066 g (0,74 mmol) d'acide oxalique en solution dans 5 ml de méthanol. On rajoute un peu d'éther éthylique; un précipité blanc se forme. On le filtre et on sèche le sel à la cloche à vide. On obtient 0,29 g (0,62 mmol) de l'oxalate du composé 49 sous la forme d'un solide crème.
F : 76°C
¹H RMN (DMSOd₆) δ : 1,40 (m, 5H) ; 1,83 (m, 2H) ; 2,74 (m, 2H) ; 3,11 (m, 3H) ; 3,38 (m, 4H) ; 3,56 (dd, 1H, J = 11,6 et 3,6 Hz) ; 3,82 (m, 1H) ; 4,02 (m, 2H) ; 4,28 (m, 2H) ; 4,65 (m, 1H) ; 6,89 (m, 4H).
MS m/z 377 (M+1).

### HPLC (XBRIDGE C8, acétonitrile/eau/KH₂PO₄ (20/80/6,8G PH 4), 0.4 ml/min), temps de rétention = 4.95 min, pureté chimique = 97,20%.

### Exemple 50 : (S)-3-(2-(1-(((S)-2,3-dihydrobenzo[b][1,4]dioxin-2-yl)méthyl)pipéridin-4-yl)éthyl)-4-(hydroxyméthyl)oxazolidin-2-one

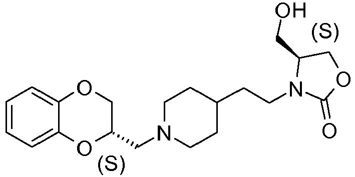

En procédant comme dans l'exemple 1, mais en remplaçant, la 3-(2-(pipéridin-4-yl)éthyl)oxazolidin-2-one 3.1 par la (S)-4-(hydroxyméthyl)-3-(2-(pipéridin-4-yl) éthyl)oxazolidin-2-one 3.22 et le K₂CO₃ par le Cs₂CO₃, on obtient 0,5 g (1,33 mmol) du composé 50 sous forme d'huile incolore, après purification sur gel de silice (éluant : dichlorométhane/méthanol = 90/10%).
Rendement : 42%
¹H RMN (CDCl₃) δ : 1,28 (m, 3H) ; 1,51 (m, 2H) ; 1,68 (m, 1H) ; 1,75 (m, 1H) ; 1,86 (1s, 1H) ; 2,08 (m, 2H) ; 2,54 (dd, 1H, 13,4 et 5,6 Hz) ; 2,65 (dd, 1H, J = 13,2 et 5,6 Hz) ; 2,89 (m, 1H) ; 2,99 (m, 1H) ; 3,15 (m, 1H) ; 3,53 (m, 1H) ; 3,69 (dd, 1H, J = 11,4 et 3,6 Hz) ; 3,79 (dd, 1H, J = 11,4 et 4 Hz) ; 3,88 (m, 1H) ; 3,96 (dd, 1H, J = 11,6 et 8 Hz) ; 4,33 (m, 3H) ; 6,85 (m, 4H).

On dissout 0,50 g (1,33 mmol) du composé 50 dans 10 ml de méthanol, puis on ajoute 0,1 g (1,32 mmol) d'acide oxalique en solution dans 5 ml de méthanol. On concentre à sec et on reprend avec un peu d'éther éthylique; un précipité blanc se forme. On le filtre et on sèche le sel à la cloche à vide. On obtient 0,51 g (1,09 mmol) de l'oxalate du composé 50 sous la forme d'un solide blanc.
F : 60°C
¹H RMN (DMSOd₆) δ : 1,39 (m, 5H) ; 1,80 (m, 2H) ; 2,66 (m, 2H) ; 3,08 (m, 3H) ; 3,23 (m, 1H) ; 3,34 (m, 2H) ; 3,42 (m, 1H) ; 3,56 (m, 1H) ; 3,81 (m, 1H) ; 4,01 (m, 2H) ; 4,28 (m, 2H) ; 4,59 (m, 1H) ; 6,87 (m, 4H).
MS m/z 377 (M+1).

### HPLC (XBRIDGE C8, acétonitrile/eau/KH₂PO₄ (20/80/6,8G PH 4), 1 ml/min), temps de rétention = 7,60 min, pureté chimique = 98,19%.

### Exemple 51 : (S)-3-((4-(2-((S)-4-(hydroxyméthyl)-2-oxooxazolidin-3-yl)éthyl) pipéridin-1-yl)méthyl)-2,3-dihydrobenzo[b][1,4]dioxine-6-carbonitrile

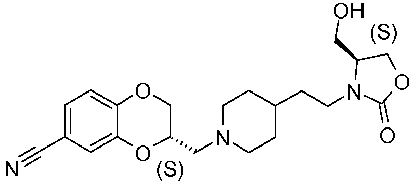

En procédant comme dans l'exemple 1, mais en remplaçant, le (R)-2-(bromométhyl)-2,3-dihydrobenzo[b][1,4]dioxine 2.1 par le (R)-(7-cyano-2,3-dihydrobenzo[b][1,4] dioxin-2-yl)méthyl 4-bromobenzenesulfonate 2.11, la 3-(2-(pipéridin-4-yl)éthyl) oxazolidin-2-one 3.1 par la (S)-4-(hydroxyméthyl)-3-(2-(pipéridin-4-yl)éthyl) oxazolidin-2-one 3.22, le couple K₂CO₃/KI par la diisopropyléthylamine et l'acétonitrile par le diméthylsulfoxide, on obtient 0,31 g (0,77 mmol) du composé 51 sous forme d'huile incolore, après purification sur gel de silice (gradient : dichlorométhane = 100% à dichlorométhane/(méthanol : NH₄OH 9 : 1) = 90/10% sur 30 min).
Rendement : 66%
¹H RMN (CDCl₃) δ : 1,29 (m, 3H) ; 1,51 (m, 2H) ; 1,72 (m, 2H) ; 1,90 (1s, 1H) ; 2,13 (m, 2H) ; 2,54 (dd, 1H, J = 13,4 et 6,4 Hz) ; 2,66 (dd, 1H, 13,4 et 6 Hz) ; 2,87 (m, 1H) ; 2,95 (m, 1H) ; 3,15 (m, 1H) ; 3,53 (m, 1H) ; 3,68 (dd, 1H, J = 11,6 et 3,6 Hz) ; 3,78 (dd, 1H, J = 11,6 et 4 Hz) ; 3,88 (m, 1H) ; 4,02 (dd, 1H, J = 11,4 et 7,2 Hz) ; 4,22 (dd, 1H, J = 8,4 et 5,6 Hz) ; 4,30 (m, 1H) ; 4,36 (m, 2H) ; 6,73 (d, 1H, J = 8,8 Hz) ; 7,15 (m, 2H).

On dissout 0.31 g (0,77 mmol) du composé 51 dans 20 ml d'acétate d'éthyle, puis on ajoute 0,20 ml (1 mmol) d'une solution d'HCl 5N dans l'isopropanol. Un précipité blanc se forme. On filtre ce précipité et on obtient 0,31 g (0,71 mmol) du chlorhydrate du composé 51 sous la forme d'un solide blanc.
F : 247°C
¹H RMN (DMSOd₆) δ : 1,49 (m, 5H) ; 1,90 (m, 2H) ; 3,07 (m, 2H) ; 3,31 (m, 4H) ; 3,45 (m, 2H) ; 3,58 (m, 1H) ; 3,67 (m, 1H) ; 3,83 (m, 1H) ; 4,04 (m, 1H) ; 4,18 (dd, 1H, J = 11,6 et 6,4 Hz) ; 4,27 (m, 1H) ; 4,45 (dd, 1H, J = 11,6 et 2,4 Hz) ; 4,99 (m, 2H) ; 7,11 (d, 1H, J = 8,6 Hz) ; 7,38 (dd, 1H, J = 8,6 et 1,6 Hz) ; 7,51 (d, 1H, J = 1,6 Hz) ; 10.70 (1s, 1H).
MS m/z 402 (M+1)
HPLC (XBRIDGE C8, acétonitrile/eau/KH₂PO₄ (20/80/6,8G PH 4), 1 ml/min), temps de rétention = 7,12 min, pureté chimique = 98,89%.
HPLC (CHIRACEL OJ-H, heptane/ethanol/diéthylamine (80/20/0,1), 1 ml/min) : composé 51, temps de rétention = 24,92 min, composé 52, temps de rétention = 19,53 min, composé 53, temps de rétention = 15,39 min, composé 54, temps de rétention = 16,87 min, rapport des AUC (51)/(52)/(53)/(54) = 96,37/0,51/3,11/0.

### Exemple 52 : (R)-3-((4-(2-((S)-4-(hydroxyméthyl)-2-oxooxazolidin-3-yl)éthyl) pipéridin-1-yl)méthyl)-2,3-dihydrobenzo[b][1,4]dioxine-6-carbonitrile

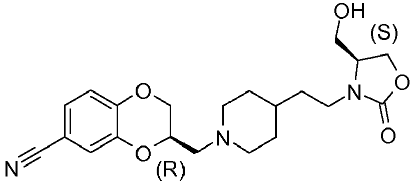

En procédant comme dans l'exemple 1, mais en remplaçant, le (R)-2-(bromométhyl)-2,3-dihydrobenzo[b][1,4]dioxine 2.1 par le (S)-(7-cyano-2,3-dihydrobenzo[b][1,4] dioxin-2-yl)méthyl 4-bromobenzenesulfonate 2.12, la 3-(2-(pipéridin-4-yl)éthyl) oxazolidin-2-one 3.1 par la (S)-4-(hydroxyméthyl)-3-(2-(pipéridin-4-yl)éthyl) oxazolidin-2-one 3.22, le couple K₂CO₃/KI par la diisopropyléthylamine (1,4 eq) et l'acétonitrile par le diméthylsulfoxide, on obtient 0,28 g (0,68 mmol) du composé 52 sous forme d'huile jaune pâle, après purification sur gel de silice (éluant : gradient dichlorométhane = 100% à dichlorométhane/(méthanol : NH₄OH 9 : 1) = 90/10% sur 30 min).
Rendement : 60%
¹H RMN (CDCl₃) δ : 1,28 (m, 3H) ; 1,52 (m, 2H) ; 1,73 (m, 2H) ; 1,98 (1s, 1H) ; 2,12 (m, 2H) ; 2,54 (dd, 1H, J = 13,4 et 6 Hz) ; 2,65 (dd, 1H, J = 13,6 et 8 Hz) ; 2,86 (m, 1H) ; 2,95 (m, 1H) ; 3,15 (m, 1H) ; 3,52 (m, 1H) ; 3,68 (m, 1H) ; 3,80 (m, 1H) ; 3,90 (m, 1H) ; 4,02 (dd, 1H, J = 11,4 et 7,6 Hz) ; 4,22 (dd, 1H, J = 8,8 et 5,6 Hz) ; 4,29 (m, 1H) ; 4,36 (m, 2H) ; 6,91 (d, 1H, J = 8,4 Hz) ; 7,19 (m, 2H).

On dissout 0.28 g (0,68 mmol) du composé 52 dans 20 ml d'acétate d'éthyle, puis on ajoute 0,20 ml (1 mmol) d'une solution d'HCl 5N dans l'isopropanol. Un précipité blanc se forme. On filtre ce précipité et on obtient 0,27 g (0,62 mmol) du chlorhydrate du composé 52 sous la forme d'un solide blanc.
F : 220°C
¹H RMN (DMSOd₆) δ : 1,53 (m, 5H) ; 1,89 (m, 2H) ; 3,06 (m, 2H) ; 3,35 (m, 4H) ; 3,45 (m, 2H) ; 3,57 (m, 1H) ; 3,67 (m, 1H) ; 3,83 (m, 1H) ; 4,04 (m, 1H) ; 4,18 (m, 1H) ; 4,27 (m, 1H) ; 4,44 (m, 1H) ; 5,00 (m, 2H) ; 7,11 (d, 1H, J = 8,4 Hz) ; 7,38 (dd, 1H, J = 8,4 et 2 Hz) ; 7,50 (d, 1H, J = 2 Hz) ; 10,65 (1s, 1H).
MS m/z 402 (M+1)
HPLC (XBRIDGE C8, acétonitrile/eau/KH₂PO₄ (20/80/6,8G PH 4), 1 ml/min), temps de rétention = 7,13 min, pureté chimique = 99,64%.
HPLC (CHIRACEL OJ-H, heptane/ethanol/diéthylamine (80/20/0,1), 1 ml/min) : rapport des AUC (51)/(52)/(53)/(54) = 0,74/96,13/0/3,13.

### Exemple 53 : (S)-3-((4-(2-((R)-4-(hydroxyméthyl)-2-oxooxazolidin-3-yl)éthyl) pipéridin-1-yl)méthyl)-2,3-dihydrobenzo[b][1,4]dioxine-6-carbonitrile

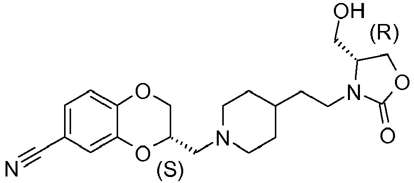

En procédant comme dans l'exemple 1, mais en remplaçant, le (R)-2-(bromométhyl)-2,3-dihydrobenzo[b][1,4]dioxine 2.1 par le (R)-(7-cyano-2,3-dihydrobenzo[b][1,4] dioxin-2-yl)méthyl 4-bromobenzenesulfonate 2.11, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, le couple K₂CO₃/KI par la diisopropyléthylamine (1,4 eq) et l'acétonitrile par le diméthylsulfoxide, on obtient 0,43 g (1,07 mmol) du composé 53 sous forme d'huile jaune pâle, après purification sur gel de silice (gradient : dichlorométhane = 100% à dichlorométhane/méthanol = 95/5% sur 40 min). Rendement : 46%
¹H RMN (CDCl₃) δ : 1,29 (m, 3H) ; 1,51 (m, 2H) ; 1,70 (m, 3H) ; 2,12 (m, 2H) ; 2,54 (dd, 1H, J = 13,4 et 6,4 Hz) ; 2,65 (dd, 1H, J = 13,2 et 5,6 Hz) ; 2,86 (m, 1H) ; 2,96 (m, 1H) ; 3,15 (m, 1H) ; 3,53 (m, 1H) ; 3,70 (dd, 1H, J = 11,4 et 4 Hz) ; 3,79 (dd, 1H, J = 11,4 et 4 Hz) ; 3,89 (m, 1H) ; 4,02 (dd, 1H, J = 11,4 et 7,2 Hz) ; 4,21 (dd, 1H, J = 8,8 et 6 Hz) ; 4,29 (m, 1H) ; 4,36 (m, 2H) ; 6,91 (d, 1H, J = 8,4 Hz) ; 7,14 (dd, 1H, J = 8,4 et 2 Hz) ; 7,16 (d, 1H, J = 2 Hz).

On dissout 0.43 g (1,07 mmol) du composé 53 dans 10 ml de méthanol, puis on ajoute 0,096 ml (1,07 mmol) d'acide oxalique en solution dans 5 ml de méthanol. On concentre le milieu et on triture avec de l'éther; un précipité blanc se forme. On filtre ce précipité et on obtient 0,44 g (0,89 mmol) de l'oxalate du composé 53 sous la forme d'un solide blanc.
F : 170°C
¹H RMN (DMSOd₆) δ : 1,35 (m, 5H) ; 1,78 (m, 2H) ; 2,57 (m, 2H) ; 3,05 (m, 3H) ; 3,27 (m, 3H) ; 3,43 (dd, 1H, J = 11,8 et 3,2 Hz) ; 3,56 (dd, 1H, J = 11,8 et 3,6 Hz) ; 3,81 (m, 1H) ; 4,04 (dd, 1H, J = 8,4 et 5,6 Hz) ; 4,12 (dd, 1H, J = 11,6 et 6,8 Hz) ; 4,27 (m, 1H) ; 4,40 (dd, 1H, J = 11,6 et 2,4 Hz) ; 4,65 (m, 1H) ; 7,08 (d, 1H, J = 8,4 Hz) ; 7,35 (dd, 1H, J = 8,4 et 2 Hz) ; 7,44 (d, 1H, J = 2Hz).
MS m/z 402 (M+1)
HPLC (Acquity BEH C8, Phase A : 100% H₂O HCOONH₄ 0.01M PH4, Phase B : 90% Acétonitrile 10% H2O.HCOONH4 0.01M PH4, gradient B : 15 à 40% sur 5 min, 0,75 ml/min), temps de rétention = 1,47 min, pureté chimique : 97,49%.
HPLC (CHIRACEL OJ-H, heptane/ethanol/diéthylamine (80/20/0,1), 1 ml/min) : rapport des AUC (51)/(52)/(53)/(54) = 2,69/0/96,87/0 ,44.

### Exemple 54 : (R)-3-((4-(2-((R)-4-(hydroxyméthyl)-2-oxooxazolidin-3-yl)éthyl) pipéridin-1-yl)méthyl)-2,3-dihydrobenzo[b][1,4]dioxine-6-carbonitrile

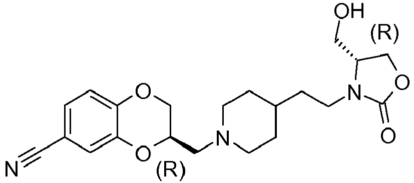

En procédant comme dans l'exemple 1, mais en remplaçant, le (R)-2-(bromométhyl)-2,3-dihydrobenzo[b][1,4]dioxine 2.1 par le (S)-(7-cyano-2,3-dihydrobenzo[b][1,4] dioxin-2-yl)méthyl 4-bromobenzenesulfonate 2.12, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, le couple K₂CO₃/KI par la diisopropyléthylamine (1,4 eq) et l'acétonitrile par le dilméthylsulfoxide, on obtient 0,7 g (1,7 mmol) du composé 54 sous forme de mousse jaune pâle, après purification sur gel de silice (gradient : dichlorométhane = 100% à dichlorométhane/(méthanol: NH₄OH 9:1) = 95/5% sur 30 min).
Rendement : 72%
¹H RMN (CDCl₃) δ : 1,29 (m, 3H) ; 1,51 (m, 2H) ; 1,73 (m, 2H) ; 2,10 (m, 3H) ; 2,54 (dd, 1H, J = 13,4 et 6 Hz) ; 2,66 (dd, 1H, J = 13,6 et 5,6 Hz); 2,87 (m, 1H) ; 2,95 (m, 1H) ; 3,16 (m, 1H) ; 3,53 (m, 1H) ; 3,69 (dd, 1H, J = 11,2 et 3,6 Hz) ; 3,79 (dd, 1H, J = 11,6 et 4,4 Hz) ; 3,89 (m, 1H) ; 4,02 (dd, 1H, J = 11,4 et 7,2 Hz) ; 4,22 (dd, 1H, J = 8,8 et 5,6 Hz) ; 4,29 (m, 1H) ; 4,36 (m, 2H) ; 6,91 (d, 1H, J = 8,4 Hz) ; 7,14 (dd, 1H, J = 8,4 et 2 Hz) ; 7,17 (d, 1H, J = 2 Hz).
On dissout 0.7 g (1,7 mmol) du composé 54 dans 20 ml d'acétate d'éthyle, puis on ajoute 0,40 ml (2 mmol) d'une solution d'HCl 5N dans l'isopropanol. Un précipité blanc se forme. On filtre ce précipité et on obtient 0,69 g (1,6 mmol) du chlorhydrate du composé 54 sous la forme d'un solide blanc.
F : 247°C
¹H RMN (DMSOd₆) δ : 1,50 (m, 5H) ; 1,91 (m, 2H) ; 3,04 (m, 2H) ; 3,39 (m, 6H) ; 3,57 (m, 1H) ; 3,68 (m, 1H) ; 3,83 (m, 1H) ; 4,04 (m, 1H) ; 4,18 (m, 1H) ; 4,27 (m, 1H) ; 4,45 (m, 1H) ; 5,00 (m, 2H) ; 7,11 (d, 1H, J = 8,4 Hz) ; 7,38 (d, 1H, J = 8,4 Hz) ; 7,51 (s, 1H).
MS m/z 402 (M+1)
HPLC (XBRIDGE C8, acétonitrile/eau/KH₂PO₄ (20/80/6,8G PH 4), 1 ml/min), temps de rétention = 7,13 min, pureté chimique = 98,81%.
HPLC (CHIRACEL OJ-H, heptane/ethanol/diéthylamine (80/20/0,1), 1 ml/min) : rapport des AUC (51)/(52)/(53)/(54) = 0/2,40/0,46/97,14.

### Exemple 55 : (S)-2-((4-(2-((R)-4-(hydroxyméthyl)-2-oxooxazolidin-3-yl)éthyl) pipéridin-1-yl)méthyl)-2,3-dihydrobenzo[b][1,4]dioxine-5-carbonitrile

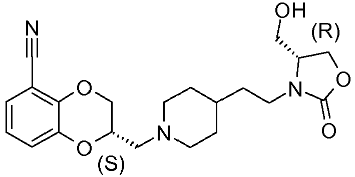

En procédant comme dans l'exemple 1, mais en remplaçant, le (R)-2-(bromométhyl)-2,3-dihydrobenzo[b][1,4]dioxine 2.1 par le (R)-(5-cyano-2,3-dihydrobenzo[b][1,4] dioxin-2-yl)méthyl 4-bromobenzenesulfonate 2.14, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, le couple K₂CO₃/KI par la diisopropyléthylamine (1,4 eq), on obtient 0,072 g (0,18 mmol) du composé 55 sous forme d'huile incolore, après purification sur gel de silice (éluant : dichlorométhane/méthanol = 90/10%).
Rendement : 37%
¹H RMN (DMSOd₆) δ : 1,17 (m, 3H) ; 1,41 (m, 2H) ; 1,64 (m, 2H) ; 2,00 (m, 2H) ; 2,55 (m, 1H) ; 2,88 (m, 2H) ; 3,05 (m, 1H) ; 3,17 (m, 1H) ; 3,30 (m, 1H) ; 3,44 (m, 1H) ; 3,55 (m, 1H) ; 3,81 (m, 1H) ; 4,03 (m, 1H) ; 4,12 (m, 1H) ; 4,27 (m, 1H) ; 4,47 (m, 2H) ; 4,98 (m, 1H) ; 6,97 (m, 1H) ; 7,22 (d, 1H, J = 8 Hz) ; 7,28 (d, 1H, J = 7,6 Hz).

On dissout 0.072 g (0,18 mmol) du composé 55 dans 5 ml d'acétonitrile, puis on ajoute 0,016 g (0,18 mmol) d'acide oxalique et 1 ml d'eau. Après lyophilisation on obtient 0,081 g (0,16 mmol) de l'oxalate du composé 55 sous la forme d'un solide blanc.
¹H RMN (DMSOd₆) : 1,42 (m, 5H) ; 1,80 (m, 2H) ; 2,67 (m, 2H) ; 3,09 (m, 3H) ; 3,30 (m, 3H) ; 3,43 (dd, 1H, J = 11,6 et 7,2 Hz) ; 3,56 (dd, 1H, J = 11,8 et 3,6 Hz) ; 3,82 (m, 1H) ; 4,04 (dd, 1H, J = 8,4 et 5,6 Hz) ; 4,19 (dd, 1H, J = 11,6 et 6,8 Hz) ; 4,27 (m, 1H) ; 4,49 (dd, 1H, J = 11,6 et 2,4 Hz) ; 4,74 (m, 1H) ; 7,02 (m, 1H) ; 7,27 (d, 1H, J = 8 Hz) ; 7,34 (dd, 1H, J = 7,8 et 1,6 Hz).
HPLC (X-BRIDGE C18, Phase A : 100% H₂O.TFA 0.1%, Phase B : 100% Acétonitrile, gradient B : 0 à 100% sur 6 min, 1,8 ml/min), temps de rétention = 4,11 min, pureté chimique : 99,4%.

### Exemple 56 : (R)-3-(2-(1-(((S)-7-chloro-2,3-dihydrobenzo[b][1,4]dioxin-2-yl)méthyl) pipéridin-4-yl)éthyl)-4-(hydroxyméthyl)oxazolidin-2-one

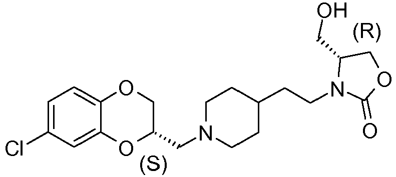

En procédant comme dans l'exemple 1, mais en remplaçant le (R)-2-(bromométhyl)-2,3-dihydrobenzo[b][1,4]dioxine 2.1 par le (R)-2-(bromométhyl)-7-chloro-2,3-dihydrobenzo[b][1,4]dioxine 2.7, la 3-(2-(pipéridin-4-yl)éthyl)oxazolidin-2-one 3.1 par la (R)-4-(hydroxyméthyl)-3-(2-(pipéridin-4-yl)éthyl)oxazolidin-2-one 3.21, le K₂CO₃ par le Cs₂CO₃ et l'acétonitrile par le diméthylformamide, on obtient 0,9 g (2,19 mmol) du composé 56 sous forme d'huile incolore, après purification sur gel de silice (éluant : dichlorométhane/méthanol = 90/10%).
Rendement : 60%
¹H RMN (CDCl₃) δ : 1,29 (m, 3H) ; 1,50 (m, 2H) ; 1,72 (m, 3H) ; 2,08 (m, 2H) ; 2,53 (dd, 1H, J = 13,4 et 6 Hz) ; 2,63 (dd, 1H, J = 13,4 et 5,6 Hz) ; 2,87 (m, 1H) ; 2,97 (m, 1H) ; 3,15 (m, 1H) ; 3,53 (m, 1H) ; 3,69 (dd, 1H, J = 11,4 et 3,6 Hz) ; 3,79 (dd, 1H, J = 11,4 et 4 Hz) ; 3,89 (m, 1H) ; 3,94 (dd, 1H, J = 12 et 7,6 Hz) ; 4,22 (dd, 1H, J = 8,8 et 5,6 Hz) ; 4,27 (m, 2H) ; 4,36 (m, 1H) ; 6,78 (m, 2H) ; 6,88 (m, 1H).

On dissout 0.9 g (2,19 mmol) du composé dans 20 ml de méthanol, puis on ajoute 0,19 g (2, 11mmol) d'acide oxalique en solution dans 10 ml de méthanol. On ajoute quelques ml d'éther éthylique et après trituration, on obtient 0,81 g (1,62 mmol) de l'oxalate du composé 56 sous la forme d'un solide blanc.
¹H RMN (DMSOd₆) δ : 1,4 (m, 5H) ; 1,82 (m, 2H) ; 2,70 (m, 2H) ; 3,09 (m, 3H) ; 3,33 (m, 3H) ; 3,43 (dd, 1H, J = 11,6 et 3,6 Hz) ; 3,56 (dd, 1H, J = 11,6 et 3,6 Hz) ; 3,82 (m, 1H) ; 4,04 (m, 2H) ; 4,28 (m, 2H) ; 4,67 (lm, 1H) ; 6,92 (m, 2H) ; 7,02 (d, 1H, J = 1,6 Hz).
MS m/z 411 (M+1)
HPLC (XBRIDGE C8, acétonitrile/eau/KH₂PO₄ (50/50/6,8G PH 4), 0,4 ml/min), temps de rétention = 6,63 min, pureté chimique = 99,75%.

### Exemple 57 : (R)-3-(2-(1-(((S)-7-fluoro-2,3-dihydrobenzo[b][1,4]dioxin-2-yl)méthyl) pipéridin-4-yl)éthyl)-4-(hydroxyméthyl)oxazolidin-2-one

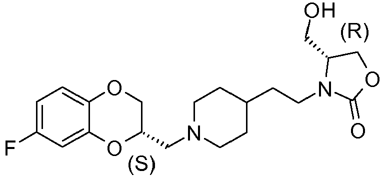

En procédant comme dans l'exemple 1, mais en remplaçant, le (R)-2-(bromométhyl)-2,3-dihydrobenzo[b][1,4]dioxine 2.1 par le (R)-(7-fluoro-2,3-dihydrobenzo[b][1,4] dioxin-2-yl)méthyl 4-bromobenzenesulfonate 2.17, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, et le couple K₂CO₃/KI par la diisopropyléthylamine (1,4 eq), on obtient 0,097 g (0,25 mmol) du composé 57 sous forme d'huile incolore, après purification sur gel de silice (éluant : dichlorométhane/méthanol = 90/10%).
Rendement : 49%
¹H RMN (DMSOd₆) δ : 1,16 (m, 3H) ; 1,41 (m, 2H) ; 1,63 (m, 2H) ; 1,99 (m, 2H) ; 2,50 (m, 2H) ; 2,83 (m, 1H) ; 2,91 (m, 1H) ; 3,05 (m, 1H) ; 3,33 (m, 1H) ; 3,43 (m, 1H) ; 3,54 (m, 1H) ; 3,81 (m, 1H) ; 3,93 (dd, 1H, J = 11,4 et 7,2 Hz) ; 4,03 (dd, 1H, J = 8,4 et 5,6 Hz) ; 4,27 (m, 2H) ; 4.35 (m, 1H) ; 4,98 (t, 1H, J = 5,2 Hz) ; 6,65 (m, 1H) ; 6,76 (m, 1H) ; 6,87 (m, 1H).

On dissout 0.097 g (0,24 mmol) du composé 57 dans 5 ml d'acétonitrile, puis on ajoute 0,022 g (0,24 mmol) d'acide oxalique et 1 ml d'eau. Après lyophilisation on obtient 0,112 g (0,23 mmol) de l'oxalate du composé 57 sous la forme d'un solide blanc.
¹H RMN (DMSOd₆) : 1,40 (m, 5H) ; 1,80 (m, 2H) ; 2,67 (m, 2H) ; 3,08 (m, 3H) ; 3,30 (m, 3H) ; 3,43 (dd, 1H, J = 11,8 et 3,6 Hz) ; 3,56 (dd, 1H, J = 11,8 et 3,6 Hz) ; 3,82 (m, 1H) ; 4,02 (m, 2H) ; 4,26 (m, 2H) ; 4,65 (m, 1H) ; 6,71 (m, 1H) ; 6,82 (m, 1H) ; 6,92 (m, 1H).
MS m/z 395 (M+1)
HPLC (X-BRIDGE C18, Phase A : 100% H₂O.TFA 0.1%, Phase B : 100% Acétonitrile, gradient B : 0 à 100% sur 6 min puis 2 min acétonitrile, 1,8 ml/min), temps de rétention = 4,24 min, pureté chimique : 100%.

### Exemple 58 : (R)-3-(2-(1-(((S)-6-fluoro-2,3-dihydrobenzo[b][1,4]dioxin-2-yl)méthyl) pipéridin-4-yl)éthyl)-4-(hydroxyméthyl)oxazolidin-2-one

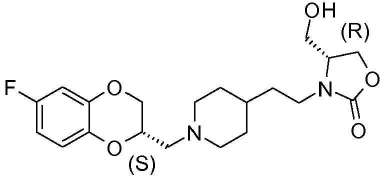

En procédant comme dans l'exemple 1, mais en remplaçant, le (R)-2-(bromométhyl)-2,3-dihydrobenzo[b][1,4]dioxine 2.1 par le (R)-(6-fluoro-2,3-dihydrobenzo[b][1,4] dioxin-2-yl)méthyl 4-bromobenzenesulfonate 2.18, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, et le couple K₂CO₃/KI par la diisopropyléthylamine (1,4 eq), on obtient 0,089 g (0,22 mmol) du composé 58 sous forme de solide blanc, après purification sur gel de silice (éluant : dichlorométhane/méthanol = 90/10%).
Rendement : 44%
¹H RMN (DMSOd₆) δ : 1,17 (m, 3H) ; 1,41 (m, 2H) ; 1,63 (m, 2H) ; 1,99 (m, 2H) ; 2,50 (m, 2H) ; 2,83 (m, 1H) ; 2,91 (m, 1H) ; 3,04 (m, 1H) ; 3,33 (m, 1H) ; 3,42 (m, 1H) ; 3,54 (m, 1H) ; 3,80 (m, 1H) ; 3,95 (dd, 1H, J = 11,8 et 7,6 Hz) ; 4,03 (dd, 1H, J = 8,4 et 5,6 Hz) ; 4,28 (m, 3H) ; 4,98 (t, 1H, J = 5,2 Hz) ; 6,65 (m, 1H) ; 6,77 (m, 1H) ; 6,86 (m, 1H).

On dissout 0.089 g (0,22 mmol) du composé 58 dans 5 ml d'acétonitrile, puis on ajoute 0,020 g (0,22 mmol) d'acide oxalique et 1 ml d'eau. Après lyophilisation on obtient 0,103 g (0,21 mmol) de l'oxalate du composé 58 sous la forme d'un solide blanc.
¹H RMN (DMSOd₆) : 1,31 (m, 3H) ; 1,43 (m, 2H) ; 1,77 (m, 2H) ; 2,52 (m, 2H) ; 2,95 (m, 2H) ; 3,07 (m, 1H) ; 3,16 (m, 1H) ; 3,24 (m, 1H) ; 3,33 (m, 1H) ; 3,42 (dd, 1H, J = 12 et 2,8 Hz) ; 3,56 (dd, 1H, J = 11,6 et 3,6 Hz) ; 3,82 (m, 1H) ; 4,02 (m, 2H) ; 4,29 (m, 2H) ; 4,52 (m, 1H) ; 6,70 (m, 1H) ; 6,81 (m, 1H) ; 6,92 (m, 1H).
MS m/z 395 (M+1)
HPLC (X-BRIDGE C18, Phase A : 100% H₂O.TF 0.1 %, Phase B : 100% Acétonitrile, gradient B : 0 à 100% sur 6 min puis 2 min acétonitrile, 1,8 ml/min), temps de rétention = 4,31 min, pureté chimique : 100%.

### Exemple 59 : (R)-3-(2-(1-(((S)-8-fluoro-2,3-dihydrobenzo[b][1,4]dioxin-2-yl)méthyl) pipéridin-4-yl)éthyl)-4-(hydroxyméthyl)oxazolidin-2-one

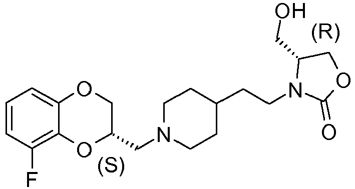

En procédant comme dans l'exemple 1, mais en remplaçant, le (R)-2-(bromométhyl)-2,3-dihydrobenzo[b][1,4]dioxine 2.1 par le (R)-(8-fluoro-2,3-dihydrobenzo[b][1,4] dioxin-2-yl)méthyl 4-bromobenzenesulfonate 2.16, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, et le couple K₂CO₃/KI par la diisopropyléthylamine (1,4 eq), on obtient 0,079 g (0,20 mmol) du composé 59 sous forme d'huile incolore, après purification sur gel de silice (éluant : dichlorométhane/méthanol = 90/10%).
Rendement : 40%
¹H RMN (DMSOd₆) δ : 1,17 (m, 3H) ; 1,41 (m, 2H) ; 1,64 (m, 2H) ; 2,01 (m, 2H) ; 2,56 (m, 2H) ; 2,83 (m, 1H) ; 2,94 (m, 1H) ; 3,04 (m, 1H) ; 3,33 (m 1H) ; 3,42 (m, 1H) ; 3,55 (m, 1H) ; 3,81 (m, 1H) ; 4,01 (m, 2H) ; 4,32 (m, 3H) ; 4,98 (t, 1H, J = 5,2 Hz) ; 6,71 (m, 1H) ; 6,78 (m, 2H).

On dissout 0.079 g (0,20 mmol) du composé 59 dans 5 ml d'acétonitrile, puis on ajoute 0,018 g (0,20 mmol) d'acide oxalique et 1 ml d'eau. Après lyophilisation on obtient 0,090 g (0,19 mmol) de l'oxalate du composé 59 sous la forme d'un solide blanc.
¹H RMN (DMSOd₆) : 1,30 (m, 3H) ; 1,44 (m, 2H) ; 1,78 (m, 2H) ; 2,57 (m, 2H) ; 3,05 (m, 3H) ; 3,17 (m, 1H) ; 3,31 (m, 2H) ; 3,42 (dd, 1H, J = 11,8 et 3,6 Hz) ; 3,56 (dd, 1H, J = 11,8 et 3,6) ; 3,81 (m, 1H) ; 4,05 (m, 2H) ; 4,27 (m, 1H) ; 4,35 (m, 1H) ; 4,62 (m, 1H) ; 6,74 (m, 1H) ; 6,83 (m, 2H).
MS m/z 395 (M+1)
HPLC (X-BRIDGE C18, Phase A : 100% H₂O.TF 0.1%, Phase B : 100% Acétonitrile, gradient B : 0 à 100% sur 6 min puis 2 min acétonitrile, 1,8 ml/min), temps de rétention = 4,23 min, pureté chimique : 100%.

### Exemple 60 : (R)-3-(2-(1-(((S)-5,7-difluoro-2,3-dihydrobenzo[b][1,4]dioxin-2-yl) méthyl)pipéridin-4-yl)éthyl)-4-(hydroxyméthyl)oxazolidin-2-one

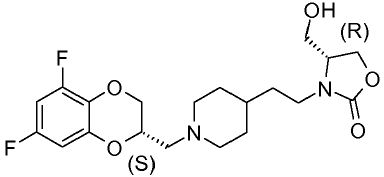

En procédant comme dans l'exemple 1, mais en remplaçant, le (R)-2-(bromométhyl)-2,3-dihydrobenzo[b][1,4]dioxine 2.1 par le (R)-(5,7-difluoro-2,3-dihydrobenzo[b][1,4] dioxin-2-yl)méthyl 4-bromobenzenesulfonate 2.19, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, et le couple K₂CO₃/KI par la diisopropyléthylamine (1,4 eq), on obtient 0,099 g (0,24 mmol) du composé 60 sous forme d'huile incolore, après purification sur gel de silice (éluant : dichlorométhane/méthanol = 90/10%).
Rendement : 49%
¹H RMN (DMSOd₆) δ : 1,17 (m, 3H) ; 1,41 (m, 2H) ; 1,63 (m, 2H) ; 1,99 (m, 2H) ; 2,54 (m, 2H) ; 2,84 (m, 1H) ; 2,91 (m, 1H) ; 3,06 (m, 1H) ; 3,34 (m, 1H) ; 3,43 (m, 1H) ; 3,54 (m, 1H) ; 3,81 (m, 1H) ; 4,00 (m, 2H) ; 4,27 (m, 1H) ; 4,35 (m, 1H) ; 4,43 (m, 1H) ; 4,98 (t, 1H, J = 5,2 Hz) ; 6,69 (m, 1H) ; 6,84 (m, 1H).
On dissout 0.099 g (0,24 mmol) du composé 60 dans 5 ml d'acétonitrile, puis on ajoute 0,022 g (0,24 mmol) d'acide oxalique et 1 ml d'eau. Après lyophilisation on obtient 0,115 g (0,23 mmol) de l'oxalate du composé 60 sous la forme d'un solide blanc.
¹H RMN (DMSOd₆) : 1,33 (m, 3H) ; 1,44 (m, 2H) ; 1,77 (m, 2H) ; 2,52 (m, 2H) ; 2,97 (m, 2H) ; 3,07 (m, 1H) ; 3,19 (m, 2H) ; 3,34 (m, 1H) ; 3,43 (dd, 1H, J = 11,8 et 3,6 Hz) ; 3,56 (dd, 1H, J = 11,8 et 3,6 Hz) ; 3,81 (m, 1H) ; 4,05 (m, 2H) ; 4,27 (m, 1H) ; 4,36 (m, 1H) ; 4,66 (m, 1H) ; 6,73 (m, 1H) ; 6,90 (m, 1H).
MS m/z 413 (M+1)
HPLC (X-BRIDGE C18, Phase A : 100% H₂O.TFA 0.1%, Phase B : 100% Acétonitrile, gradient B : 0 à 100% sur 6 min puis 2 min acétonitrile, 1,8 ml/min), temps de rétention = 4,36 min, pureté chimique : 100%.

### Exemple 61 : (R)-3-(2-(1-(((S)-6,7-difluoro-2,3-dihydrobenzo[b][1,4]dioxin-2-yl) méthyl)pipéridin-4-yl)éthyl)-4-(hydroxyméthyl)oxazolidin-2-one

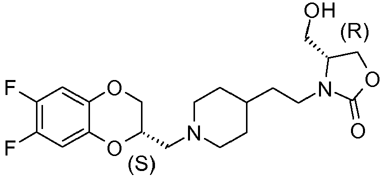

En procédant comme dans l'exemple 1, mais en remplaçant, le (R)-2-(bromométhyl)-2,3-dihydrobenzo[b][1,4]dioxine 2.1 par le (R)-(6,7-difluoro-2,3-dihydrobenzo[b][1,4] dioxin-2-yl)méthyl 4-bromobenzenesulfonate 2.22, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, et le couple K₂CO₃/KI par la diisopropyléthylamine (1,4 eq), on obtient 0,066 g (0,16 mmol) du composé 61 sous forme d'huile incolore, après purification sur gel de silice (éluant : dichlorométhane/méthanol = 90/10%).
Rendement : 33%
¹H RMN (DMSOd₆) δ : 1,15 (m, 3H) ; 1,40 (m, 2H) ; 1,64 (m, 2H) ; 1,98 (m, 2H) ; 2,50 (m, 2H) ; 2,83 (m, 1H) ; 2,90 (m, 1H) ; 3,04 (m, 1H) ; 3,32 (m, 1H) ; 3,42 (m, 1H) ; 3,55 (m, 1H) ; 3,80 (m, 1H) ; 3,95 (dd, 1H, J = 11,2 et 7,2 Hz) ; 4,03 (dd, 1H, J = 8,4 et 5,6 Hz) ; 4,29 (m, 3H) ; 4,98 (t, 1H, J = 5,6 Hz) ; 7,04 (m, 2H).

On dissout 0.066 g (0,16 mmol) du composé 61 dans 5 ml d'acétonitrile, puis on ajoute 0,014 g (0,16 mmol) d'acide oxalique et 1 ml d'eau. Après lyophilisation on obtient 0,072 g (0,14 mmol) de l'oxalate du composé 61 sous la forme d'un solide blanc.
¹H RMN (DMSOd₆) : 1,37 (m, 5H) ; 1,79 (m, 2H) ; 2,63 (m, 2H) ; 3,07 (m, 3H) ; 3,23 (m, 1H) ; 3,33 (m, 2H) ; 3,43 (dd, 1H, J = 11,8 et 3,6 Hz) ; 3,56 (dd, 1H, J = 11,6 et 4 Hz) ; 3,82 (m, 1H) ; 4,02 (m, 2H) ; 4,26 (m, 2H) ; 4,63 (m, 1H) ; 7,09 (m, 2H).
MS m/z 413 (M+1)
HPLC (X-BRIDGE C18, Phase A : 100% H₂O.TFA 0.1%, Phase B : 100% Acétonitrile, gradient B : 0 à 100% sur 6 min puis 2 min acétonitrile, 1,8 ml/min), temps de rétention = 4,40 min, pureté chimique : 98,1%.

### Exemple 62 : (R)-3-(2-(1-(((S)-7-chloro-5-fluoro-2,3-dihydrobenzo[b][1,4]dioxin-2-yl) méthyl)pipéridin-4-yl)éthyl)-4-(hydroxyméthyl)oxazolidin-2-one

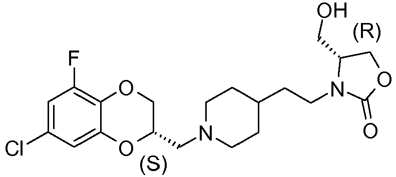

En procédant comme dans l'exemple 1, mais en remplaçant, le (R)-2-(bromométhyl)-2,3-dihydrobenzo[b][1,4]dioxine 2.1 par le (R)-(7-chloro-5-fluoro-2,3-dihydrobenzo[b] [1,4]dioxin-2-yl)méthyl 4-bromobenzenesulfonate 2.20, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, et le couple K₂CO₃/KI par la diisopropyléthylamine (1,4 eq), on obtient 0,084 g (0,19 mmol) du composé 62 sous forme d'huile incolore, après purification sur gel de silice (éluant : dichlorométhane/méthanol = 90/10%).
Rendement : 42%
¹H RMN (DMSOd₆) δ : 1,15 (m, 3H) ; 1,41 (m, 2H) ; 1,64 (m, 2H) ; 1,99 (m, 2H) ; 2,54 (m, 2H) ; 2,84 (m, 1H) ; 2,91 (m, 1H) ; 3,05 (m, 1H) ; 3,33 (m, 1H) ; 3,42 (m, 1H) ; 3,55 (m, 1H) ; 3,81 (m, 1H) ; 4,02 (m, 2H) ; 4,27 (m, 1H) ; 4,37 (m, 1H) ; 4,44 (m, 1H) ; 4,98 (t, 1H, J = 5,6 Hz) ; 6,87 (s, 1H) ; 7,00 (m, 1H).

On dissout 0.084 g (0,19 mmol) du composé 62 dans 5 ml d'acétonitrile, puis on ajoute 0,017 g (0,19 mmol) d'acide oxalique et 1 ml d'eau. Après lyophilisation on obtient 0,097 g (0,18 mmol) de l'oxalate du composé 62 sous la forme d'un solide blanc.
¹H RMN (DMSOd₆) : 1,35 (m, 3H) ; 1,44 (m, 2H) ; 1,80 (m, 2H) ; 2,63 (m, 2H) ; 3,07 (m, 3H) ; 3,22 (m, 1H) ; 3,33 (m, 2H) ; 3,43 (dd, 1H, J = 11,8 et 3,6 Hz) ; 3,56 (dd, 1H, J = 11,8 et 3,6 Hz) ; 3,82 (m, 1H) ; 4,04 (dd, 1H, J = 8,4 et 5,6 Hz) ; 4,10 (dd, 1H, J = 11,6 et 6,4 Hz) ; 4,25 (m, 1H) ; 4,38 (m, 1H) ; 4,72 (m, 1H) ; 6,92 (m, 1H) ; 7,07 (m, 1H).
MS m/z 429 (M+1)
HPLC (X-BRIDGE C18, Phase A : 100% H₂O.TFA 0.1%, Phase B : 100% Acétonitrile, gradient B : 0 à 100% sur 6 min puis 2 min acétonitrile, 1,8 ml/min), temps de rétention = 4,58 min, pureté chimique : 99,5%.

### Exemple 63 : (R)-3-(2-(1-(((S)-5-chloro-7-fluoro-2,3-dihydrobenzo[b][1,4]dioxin-2-yl) méthyl)pipéridin-4-yl)éthyl)-4-(hydroxyméthyl)oxazolidin-2-one

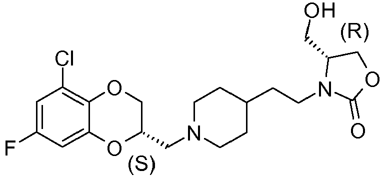

En procédant comme dans l'exemple 1, mais en remplaçant, le (R)-2-(bromométhyl)-2,3-dihydrobenzo[b][1,4]dioxine 2.1 par le (R)-(5-chloro-7-fluoro-2,3-dihydrobenzo[b] [1,4]dioxin-2-yl)méthyl 4-bromobenzenesulfonate 2.21, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, et le couple K₂CO₃/KI par la diisopropyléthylamine (1,4 eq), on obtient 0,067 g (0,15 mmol) du composé 63 sous forme d'huile incolore, après purification sur gel de silice (éluant : dichlorométhane/méthanol = 90/10%).
Rendement : 34%
¹H RMN (DMSOd₆) δ : 1,17 (m, 3H) ; 1,41 (m, 2H) ; 1,63 (m, 2H) ; 1,99 (m, 2H) ; 2,53 (m, 2H) ; 2,84 (m, 1H) ; 2,91 (m, 1H) ; 3,05 (m, 1H) ; 3,33 (m, 1H) ; 3,43 (m, 1H) ; 3,53 (m, 1H) ; 3,80 (m, 1H) ; 4,02 (m, 2H) ; 4,27 (m, 1H) ; 4,39 (m, 2H) ; 4,98 (t, 1H, J = 5,6 Hz) ; 6,84 (dd, 1H, J = 9,6 et 2,8 Hz) ; 6,97 (dd, 1H, J = 8,4 et 2,8 Hz).

On dissout 0.067 g (0,15 mmol) du composé 63 dans 5 ml d'acétonitrile, puis on ajoute 0,013 g (0,15 mmol) d'acide oxalique et 1 ml d'eau. Après lyophilisation on obtient 0,072 g (0,14 mmol) de l'oxalate du composé 63 sous la forme d'un solide blanc.
¹H RMN (DMSOd₆) : 1,33 (m, 3H) ; 1,44 (m, 2H) ; 1,79 (m, 2H) ; 2,60 (m, 2H) ; 3,07 (m, 3H) ; 3,22 (m, 1H) ; 3,33 (m, 2H) ; 3,43 (dd, 1H, J = 11,6 et 3,6 Hz) ; 3,56 (dd, 1H, J = 11,6 et 3,6 Hz) ; 3,81 (m, 1H) ; 4,04 (dd, 1H, J = 8,4 et 5,6 Hz) ; 4,10 (dd, 1H, J = 11,8 et 6,4 Hz) ; 4,27 (m, 1H) ; 4,40 (m, 1H) ; 4,69 (m, 1H) ; 6,89 (dd, 1H, J = 9,6 et 2,8 Hz) ; 7,04 (dd, 1H, J = 8,6 et 2,8 Hz).
MS m/z 429 (M+1)
HPLC (X-BRIDGE C18, Phase A : 100% H₂O.TFA 0.1%, Phase B : 100% Acétonitrile, gradient B : 0 à 100% sur 6 min puis 2 min acétonitrile, 1,8 ml/min), temps de rétention = 4,54 min, pureté chimique : 100%.

### Exemple 64 : (R)-4-(hydroxyméthyl)-3-(2-(1-(((S)-7-(méthylsulfonyl)-2,3-dihydrobenzo[b][1,4]dioxin-2-yl)méthyl)pipéridin-4-yl)éthyl)oxazo lidin-2-one

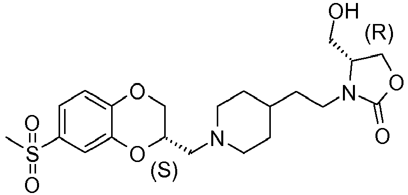

En procédant comme dans l'exemple 1, mais en remplaçant, le (R)-2-(bromométhyl)-2,3-dihydrobenzo[b][1,4]dioxine 2.1 par le (R)-(7-(méthylsulfonyl)-2,3-dihydrobenzo [b][1,4]dioxin-2-yl)méthyl 4-bromobenzenesulfonate 2.23, la 3-(2-(pipéridin-4-yl)éthyl) oxazolidin-2-one 3.1 par la (R)-4-(hydroxyméthyl)-3-(2-(pipéridin-4-yl)éthyl) oxazolidin-2-one 3.21, et le couple K₂CO₃/KI par la diisopropyléthylamine (1,4 eq), on obtient 0,068 g (0,15 mmol) du composé 64 sous forme de mousse blanche, après purification sur gel de silice (éluant : dichlorométhane/méthanol = 90/10%).
Rendement : 52%
¹H RMN (DMSOd₆) δ : 1,17 (m, 3H) ; 1,41 (m, 2H) ; 1,65 (m, 2H) ; 2,00 (m, 2H) ; 2,55 (m, 2H) ; 2,85 (m, 1H) ; 2,93 (m, 1H) ; 3,04 (m, 1H) ; 3,16 (s, 3H) ; 3,33 (m, 1H) ; 3,43 (m, 1H) ; 3,54 (m, 1H) ; 3,81 (m, 1H) ; 4,05 (m, 2H) ; 4,27 (m, 1H) ; 4,40 (m, 2H) ; 4,98 (t, 1H, J = 5,2 Hz) ; 7,10 (d, 1H, J = 8,4 Hz) ; 7,37 (m, 2H).

On dissout 0.068 g (0,15 mmol) du composé 64 dans 5 ml d'acétonitrile, puis on ajoute 0,013 g (0,15 mmol) d'acide oxalique et 1 ml d'eau. Après lyophilisation on obtient 0,075 g (0,14 mmol) de l'oxalate du composé 64 sous la forme d'un solide blanc.
¹H RMN (DMSOd₆) : 1,35 (m, 3H) ; 1,45 (m, 2H) ; 1,78 (m, 2H) ; 2,62 (m, 2H) ; 3,08 (m, 3H) ; 3,17 (s, 3H) ; 3,23 (m, 1H) ; 3,34 (m, 2H) ; 3,43 (dd, 1H, J = 11,8 et 3,2 Hz) ; 3,56 (dd, 1H, J = 11,8 et 3,2 Hz) ; 3,82 (m, 1H) ; 4,04 (dd, 1H, J = 8,4 et 5,6 Hz) ; 4,12 (dd, 1H, J = 11,6 et 6,8 Hz) ; 4,27 (m, 1H) ; 4,41 (m, 1H) ; 4,69 (m, 1H) ; 7,15 (d, 1H, J = 8,4 Hz) ; 7,43 (m, 2H).
MS m/z 455 (M+1)
HPLC (X-BRIDGE C18, Phase A : 100% H₂O.TFA 0.1%, Phase B : 100% Acétonitrile, gradient B : 0 à 100% sur 6 min puis 2 min acétonitrile, 1,8 ml/min), temps de rétention = 3,82 min, pureté chimique : 100%.

### Exemple 65 : (S)-méthyl 3-((4-(2-((R)-4-(hydroxyméthyl)-2-oxooxazolidin-3-yl)éthyl) pipéridin-1-yl)méthyl)-2,3-dihydrobenzo[b][1,4]dioxine-6-carboxylate

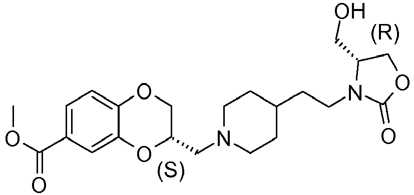

En procédant comme dans l'exemple 1, mais en remplaçant, le (R)-2-(bromométhyl)-2,3-dihydrobenzo[b][1,4]dioxine 2.1 par le (R)-méthyl 3-((((4-bromophenyl) sulfonyl)oxy)méthyl)-2,3-dihydrobenzo[b][1,4]dioxine-6-carboxylate 2.24, la 3-(2-(pipéridin-4-yl)éthyl)oxazolidin-2-one 3.1 par la (R)-4-(hydroxyméthyl)-3-(2-(pipéridin-4-yl)éthyl)oxazolidin-2-one 3.21, et le couple K₂CO₃/KI par la diisopropyléthylamine (1,4 eq), on obtient 0,046 g (0,10 mmol) du composé 65 sous forme de solide blanc, après purification sur gel de silice (éluant : dichlorométhane/méthanol = 90/10%).
Rendement : 23%
¹H RMN (DMSOd₆) δ : 1,16 (m, 3H) ; 1,40 (m, 2H) ; 1,65 (m,2H) ; 2,00 (m, 2H) ; 2,54 (m, 2H) ; 2,83 (m, 1H) ; 2,93 (m, 1H) ; 3,05 (m, 1H) ; 3,35 (m, 1H) ; 3,42 (m, 1H) ; 3,55 (m, 1H) ; 3,80 (m, 4H) ; 4,04 (m, 2H) ; 4,27 (m, 1H) ; 4,37 (m, 2H) ; 4,98 (t, 1H, J = 5,2 Hz) ; 6,98 (d, 1H, J = 8,4 Hz) ; 7,39 (d, 1H, J = 2 Hz) ; 7,46 (dd, 1H, J = 8,4 et 2 Hz).

On dissout 0.046 g (0,10 mmol) du composé 65 dans 5 ml d'acétonitrile, puis on ajoute 0,009 g (0,10 mmol) d'acide oxalique et 1 ml d'eau. Après lyophilisation on obtient 0,047 g (0,09 mmol) de l'oxalate du composé 65 sous la forme d'un solide blanc.
¹H RMN (DMSOd₆) : 1,40 (m, 5H) ; 1,82 (m, 2H) ; 2,67 (m, 2H) ; 3,08 (m, 3H) ; 3,33 (m, 3H) ; 3,43 (dd, 1H, J = 11,8 et 3,6 Hz) ; 3,56 (dd, 1H, J = 11,8 et 3,6 Hz) ; 3,81 (m, 4H) ; 4,04 (dd, 1H, J = 8,4 et 5,6 Hz) ; 4,10 (dd, 1H, J = 11,6 et 6,8 Hz) ; 4,27 (m, 1H) ; 4,39 (m, 1H) ; 4,67 (m, 1H) ; 7,02 (m, 1H) ; 7,50 (m, 2H).
MS m/z 435 (M+1)
HPLC (X-BRIDGE C18, Phase A : 100% H₂O.TFA 0.1%, Phase B : 100% Acétonitrile, gradient B : 0 à 100% sur 6 min puis 2 min acétonitrile, 1,8 ml/min), temps de rétention = 4,31 min, pureté chimique : 98,5%.

### Exemple 66 : (R)-3-(2-(1-(((S)-2,3-dihydrobenzo[b][1,4]dioxin-2-yl)méthyl)pipéridin-4-yl)éthyl)-4-(phénoxyméthyl)oxazolidin-2-one

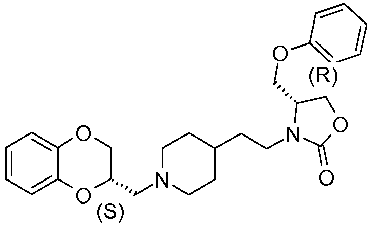

En procédant comme dans l'exemple 1, mais en remplaçant la 3-(2-(pipéridin-4-yl)éthyl)oxazolidin-2-one 3.1 par la (R)-4-(phénoxyméthyl)-3-(2-(pipéridin-4-yl) éthyl)oxazolidin-2-one 3.13, on obtient 1,6 g (3,53 mmol) du composé 66 sous forme d'huile incolore, après purification sur gel de silice (éluant : dichlorométhane = 100% puis dichlorométhane/méthanol = 95/5%).
Rendement : 67%
¹H RMN (CDCl₃) δ : 1,28 (m, 3H) ; 1,54 (m, 2H) ; 1,69 (m, 2H) ; 2,07 (m, 2H) ; 2,53 (dd, 1H, J = 13,2 et 6 Hz) ; 2,64 (dd, 1H, J = 13,2 et 5,6 Hz) ; 2,86 (m, 1H) ; 2,98 (m, 1H) ; 3,21 (m, 1H) ; 3,54 (m, 1H) ; 3,96 (dd, 1H, J = 11,7 et 7,7 Hz) ; 4,04 (m, 2H) ; 4,19 (m, 2H) ; 4,35 (m, 2H) ; 4,45 (m, 1H) ; 6,85 (m, 6H) ; 7,01 (m, 1H) ; 7,31 (m, 2H).

On dissout 1,6 g (3,53 mmol) du composé 66 dans 10 ml de méthanol, puis on ajoute 0,31 g (3,53 mmol) d'acide oxalique en solution dans le méthanol. On ajoute de l'éther éthylique et après trituration, on obtient 1,6 g (2,95 mmol) de l'oxalate du composé 66 sous la forme d'un solide blanc.
F : 164°C
¹H RMN (DMSOd₆) δ : 1,38 (m, 5H) ; 1,77 (m, 2H) ; 2,67 (m, 2H) ; 3,13 (m, 3H) ; 3,24 (m, 1H) ; 3,35 (m, 2H) ; 3,99 (dd, 1H, J = 11,5 et 6,7 Hz) ; 4,08 (m, 1H) ; 4,15 (m, 3H) ; 4,28 (m, 1H) ; 4,41 (m, 1H) ; 4,62 (m, 1H) ; 6,87 (m, 4H) ; 6,95 (m, 3H) ; 7,31 (m, 2H).
MS m/z 453 (M+1)
HPLC (XBRIDGE C8, acétonitrile/eau/KH₂PO₄ (35/65/6,8G PH 4), 1 ml/min), temps de rétention = 11,35 min, pureté chimique = 99,41%.

### Exemple 67 : (S)-3-(2-(1-(((S)-2,3-dihydrobenzo[b][1,4]dioxin-2-yl)méthyl)pipéridin-4-yl)éthyl)-4-(phénoxyméthyl)oxazolidin-2-one

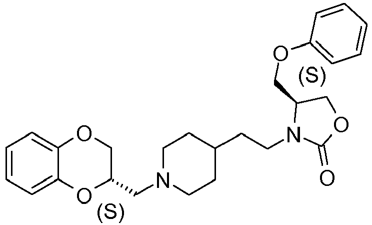

En procédant comme dans l'exemple 1, mais en remplaçant la 3-(2-(pipéridin-4-yl)éthyl)oxazolidin-2-one 3.1 par la (S)-4-(phénoxyméthyl)-3-(2-(pipéridin-4-yl)éthyl) oxazolidin-2-one 3.14, on obtient 0.5 g (1,10 mmol) du composé 67 sous forme d'huile incolore, après purification sur gel de silice (éluant : dichlorométhane = 100% puis dichlorométhane/méthanol = 95/5%).
Rendement : 45%
¹H RMN (CDCl₃) δ : 1,27 (m, 3H) ; 1,51 (m, 2H) ; 1,69 (m, 2H) ; 2,03 (m, 1H) ; 2,11 (m, 1H) ; 2,55 (m, 1H) ; 2,65 (m, 1H) ; 2,87 (m, 1H) ; 2,97 (m, 1H) ; 3,21 (m, 1H) ; 3,56 (m, 1H) ; 3,96 (m, 1H) ; 4,04 (m, 2H) ; 4,19 (m, 2H) ; 4,29 (m, 2H) ; 4,45 (m, 1H) ; 6,85 (m, 6H) ; 7,01 (m, 1H) ; 7,31 (m, 2H).

On dissout 0,5 g (1,10 mmol) du composé 67 dans 10 ml de méthanol, puis on ajoute 0,099 g (1,10 mmol) d'acide oxalique en solution dans le méthanol. On ajoute de l'éther éthylique et après trituration, on obtient 0,52 g (0.96 mmol) de l'oxalate du composé 67 sous la forme d'un solide blanc.
F : 172°C
¹H RMN (DMSOd₆) δ : 1,38 (m, 5H) ; 1,77 (m, 2H) ; 2,65 (m, 2H) ; 3,22 (m, 6H) ; 3,99 (dd, 1H, J = 11,6 et 3,2 Hz) ; 4,06 (dd, 1H, J = 10 et 6,8 Hz) ; 4,17 (m, 3H) ; 4,28 (m, 1H) ; 4,41 (m, 1H) ; 4,60 (m, 1H) ; 6,87 (m, 4H) ; 6,97 (m, 3H) ; 7,31 (m, 2H).
MS m/z 453 (M+1)
HPLC (XBRIDGE C8, acétonitrile/eau/KH₂PO₄ (35/65/6,8G PH 4), 1 ml/min), temps de rétention = 11,31 min, pureté chimique = 99,40%.

### Exemple 68 : (S)-3-((4-(2-((R)-2-oxo-4-(phénoxyméthyl)oxazolidin-3-yl)éthyl) pipéridin-1-yl)méthyl)-2,3-dihydrobenzo[b][1,4]dioxine-6-carbonitrile

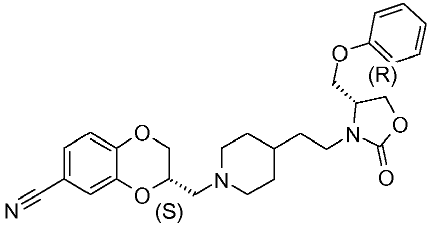

En procédant comme dans l'exemple 1, mais en remplaçant le (R)-2-(bromométhyl)-2,3-dihydrobenzo[b][1,4]dioxine 2.1 par le (R)-3-(bromométhyl)-2,3-dihydrobenzo[b] [1,4]dioxine-6-carbonitrile 2.11, la 3-(2-(pipéridin-4-yl)éthyl)oxazolidin-2-one 3.1 par la (R)-4-(phénoxyméthyl)-3-(2-(pipéridin-4-yl)éthyl)oxazolidin-2-one 3.13, le K₂CO₃ par le Cs₂CO₃ et l'acétonitrile par le diméthylformamide, on obtient 0,19 g (0,40 mmol) du composé 68 sous forme d'huile jaune pâle, après purification par flash chromatographie sur gel de silice (éluant : n-heptane = 100% puis dichlorométhane/méthanol = 95/5%).
Rendement : 42%
¹H RMN (CDCl₃) δ : 1,26 (m, 3H) ; 1,55 (m, 2H) ; 1,71 (m, 2H) ; 2,09 (m, 2H) ; 2,53 (dd, 1H, J = 13,2 et 6,4 Hz) ; 2,64 (dd, 1H, J = 13,4 et 5,6 Hz) ; 2,84 (m, 1H) ; 2,94 (m, 1H) ; 3,22 (m, 1H) ; 3,56 (m, 1H) ; 4,02 (m, 3H) ; 4,20 (m, 3H) ; 4,36 (m, 1H) ; 4,56 (m, 1H) ; 6,90 (m, 3H) ; 7,00 (m, 1H) ; 7,14 (m, 1H) ; 7,31 (m, 2H) ; 8,00 (m, 1H).

On dissout 0.19 g (0,40 mmol) du composé 68 dans 5 ml de méthanol, puis on ajoute 0,036 g (0,40 mmol) d'acide oxalique en solution dans 5 ml de méthanol. On ajoute un peu d'éther éthylique, on triture le milieu et on obtient 0,13 g (0.23 mmol) de l'oxalate du composé 68 sous la forme d'un solide blanc.
F = 139°C
¹H RMN (DMSOd₆) δ : 1,36 (5H) ; 1,75 (m, 2H) ; 2,57 (m, 2H) ; 3,18 (m, 6H) ; 4,12 (m, 5H) ; 4,41 (m, 2H) ; 4,65 (m, 1H) ; 6,97 (m, 3H) ; 7,08 (m, 1H) ; 7,33 (m, 3H) ; 7,44 (s , 1H).
MS m/z 478 (M+1)
HPLC (XBRIDGE C8, acétonitrile/eau/KH₂PO₄ (35/65/6,8G PH 4), 0,4 ml/min), temps de rétention = 6,36 min, pureté chimique = 99,12%.

### Exemple 69 : (R)-3-(2-(1-(((S)-7-bromo-2,3-dihydrobenzo[b][1,4]dioxin-2-yl)méthyl) pipéridin-4-yl)éthyl)-4-(phénoxyméthyl)oxazolidin-2-one

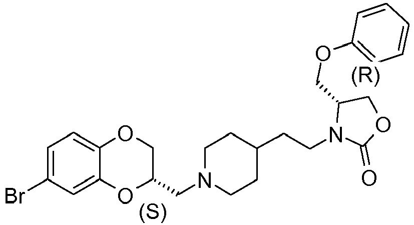

En procédant comme dans l'exemple 1, mais en remplaçant le (R)-2-(bromométhyl)-2,3-dihydrobenzo[b][1,4]dioxine 2.1 par le (R)-7-bromo-2-(bromométhyl)-2,3-dihydrobenzo[b][1,4]dioxine 2.5 et la 3-(2-(pipéridin-4-yl)éthyl)oxazolidin-2-one 3.1 par la (R)-4-(phénoxyméthyl)-3-(2-(pipéridin-4-yl)éthyl)oxazolidin-2-one 3.13, le K₂CO₃ par le Cs₂CO₃ et l'acétonitrile par le diméthylformamide, on obtient 0,40 g (0,75 mmol) du composé 69 sous forme d'huile jaune pâle, après purification par flash chromatographie sur gel de silice (éluant : n-heptane = 100% puis dichlorométhane/méthanol = 95/5%).
Rendement : 51 %
¹H RMN (CDCl₃) δ : 1,27 (m, 3H) ; 1,54 (m, 2H) ; 1,70 (m, 2H) ; 2,08 (m, 2H) ; 2,52 (dd, 1H, J = 13,4 et 6 Hz) ; 2,62 (dd, 1H, J = 13,2 et 5,6 Hz) ; 2,84 (m, 1H) ; 2,94 (m, 1H) ; 3,21 (m, 1H) ; 3,55 (m, 1H) ; 3,94 (dd, 1H, J = 11,6 et 7,6 Hz) ; 4,04 (m, 2H) ; 4,22 (m, 4H) ; 4,45 (m, 1H) ; 6,72 (m, 1H) ; 6.92 (m, 3H) ; 7,02 (m, 2H) ; 7,31 (m, 2H).

On dissout 0.40 g (0,75 mmol) du composé 69 dans 5 ml de méthanol, puis on ajoute 0,068 g (0,75 mmol) d'acide oxalique en solution dans 5 ml de méthanol. On concentre puis on ajoute un peu d'acétone, on triture le milieu et on obtient 0,31 g (0.50 mmol) de l'oxalate du composé 69 sous la forme d'un solide blanc.
F = 115°C
¹H RMN (DMSOd₆) δ : 1,38 (m, 5H) ; 1,77 (m, 2H) ; 2,67 (m, 2H) ; 3,15 (m, 4H) ; 3,37 (m, 2H) ; 4,04 (m, 2H) ; 4,17 (m, 3H) ; 4,29 (m, 1H) ; 4,41 (m, 1H) ; 4,65 (m, 1H) ; 6,87 (d, 1H, J = 8,8 Hz) ; 6,97 (m, 3H) ; 7,03 (dd, 1H, J = 8,6 et 2,4 Hz) ; 7.13 (d, 1H, J = 2,4 Hz) ; 7,31 (m, 2H).
MS m/z 531 -533 (M+1)
HPLC (XBRIDGE C8, acétonitrile/eau/KH₂PO₄ (40/60/6,8G PH 4), 0,4 ml/min), temps de rétention = 7,13 min, pureté chimique = 98,17%.

### Exemple 70 : (R)-3-(2-(1-(((S)-7-fluoro-2,3-dihydrobenzo[b][1,4]dioxin-2-yl)méthyl) pipéridin-4-yl)éthyl)-4-(phénoxyméthyl)oxazolidin-2-one

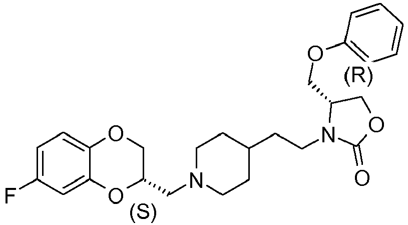

En procédant comme dans l'exemple 1, mais en remplaçant le (R)-2-(bromométhyl)-2,3-dihydrobenzo[b][1,4]dioxine 2.1 par le (R)-7-fluoro-2-(bromométhyl)-2,3-dihydrobenzo[b][1,4]dioxine 2.17 et la 3-(2-(pipéridin-4-yl)éthyl)oxazolidin-2-one 3.1 par la (R)-4-(phénoxyméthyl)-3-(2-(pipéridin-4-yl)éthyl)oxazolidin-2-one 3.13, le K₂CO₃ par le Cs₂CO₃ et l'acétonitrile par le diméthylformamide, on obtient 0,50 g (1,06 mmol) du composé 70 sous forme d'huile jaune pâle, après purification par flash chromatographie sur gel de silice (éluant : n-heptane = 100% puis dichlorométhane/méthanol = 95/5%).
Rendement : 32%
¹H RMN (CDCl₃) δ : 1,27 (m, 3H) ; 1,55 (m, 2H) ; 1,70 (m, 2H) ; 2,09 (m, 2H) ; 2,52 (dd, 1H, J = 13,4 et 6 Hz) ; 2,62 (dd, 1H, J = 13,2 et 5,6 Hz) ; 2,85 (m, 1H) ; 2,96 (m, 1H) ; 3.22 (m, 1H) ; 3,56 (m, 1H) ; 3,92 (dd, 1H, J = 11,4 et 7,2 Hz) ; 4,04 (m, 2H) ; 4,20 (m, 4H) ; 4,45 (m, 1H) ; 6,53 (m, 1H) ; 6,61 (m, 1H) ; 6,78 (m, 1H) ; 6,89 (m, 2H) ; 7,01 (m, 1H) ; 7,29 (m, 2H).

On dissout 0.25 g (0,53 mmol) du composé 70 dans 5 ml de méthanol, puis on ajoute 0,048 g (0,53 mmol) d'acide oxalique en solution dans 5 ml de méthanol. On concentre puis on ajoute un peu d'éther et d'acétone, on triture le milieu et on obtient 0,22 g (0.39 mmol) de l'oxalate du composé 70 sous la forme d'un solide blanc.
F = 103°C
¹H RMN (DMSOd₆) δ : 1,29 (m, 2H) ; 1,44 (m, 3H) ; 1,75 (m, 2H) ; 2,60 (m, 2H) ; 3,02 (m, 2H) ; 3,18 (m, 2H) ; 3,33 (m, 2H) ; 3,99 (m, 1H) ; 4,06 (m, 1H) ; 4,17 (m, 3H) ; 4,27 (m, 1H) ; 4,41 (m, 1H) ; 4,61 (m, 1H) ; 6,71 (m, 1H) ; 6,82 (m, 1H) ; 6.94 (m, 4H) ; 7,31 (m, 2H).
MS m/z 471 (M+1)
HPLC (XBRIDGE C8, acétonitrile/eau/KH₂PO₄ (40/60/6,8G PH 4), 0,4 ml/min), temps de rétention = 5,20 min, pureté chimique = 92,96%.

### Exemple 71 : (R)-3-(2-(1-(((S)-6-fluoro-2,3-dihydrobenzo[b][1,4]dioxin-2-yl)méthyl) pipéridin-4-yl)éthyl)-4-(phénoxyméthyl)oxazolidin-2-one

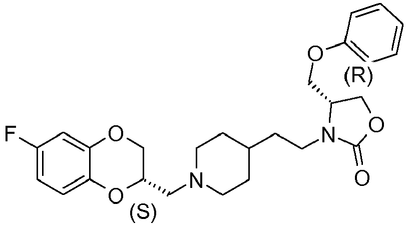

En procédant comme dans l'exemple 1, mais en remplaçant le (R)-2-(bromométhyl)-2,3-dihydrobenzo[b][1,4]dioxine 2.1 par le (R)-6-fluoro-2-(bromométhyl)-2,3-dihydrobenzo[b][1,4]dioxine 2.18, la 3-(2-(pipéridin-4-yl)éthyl)oxazolidin-2-one 3.1 par la (R)-4-(phénoxyméthyl)-3-(2-(pipéridin-4-yl)éthyl)oxazolidin-2-one 3.13, le K₂CO₃ par le Cs₂CO₃ et l'acétonitrile par le diméthylformamide, on obtient 0,40 g (0,85 mmol) du composé 71 sous forme d'huile jaune pâle, après purification par flash chromatographie sur gel de silice (éluant : n-heptane = 100% puis dichlorométhane/méthanol = 95/5%).
Rendement : 46%
¹H RMN (CDCl₃) δ : 1,28 (m, 3H) ; 1,54 (m, 2H) ; 1,70 (m, 2H) ; 2,07 (m, 2H) ; 2,51 (dd, 1H, J = 13,4 et 6 Hz) ; 2,63 (dd, 1H, J = 13,4 et 6 Hz) ; 2,85 (m, 1H) ; 2,96 (m, 1H) ; 3,21 (m, 1H) ; 3,56 (m, 1H) ; 3,94 (dd, 1H, J = 11.2 et 7,2 Hz) ; 4,04 (m, 2H) ; 4,19 (m, 3H) ; 4,29 (m, 1H) ; 4,45 (m, 1H) ; 6,55 (m, 1H) ; 6,61 (m, 1H) ; 6,79 (m, 1H) ; 6,89 (m, 2H) ; 7,01 (m, 1H) ; 7,31 (m, 2H).

On dissout 0.40 g (0,85 mmol) du composé 71 dans 5 ml de méthanol, puis on ajoute 0,077 g (0,85 mmol) d'acide oxalique en solution dans 5 ml de méthanol. On concentre puis on ajoute un peu d'éther et d'acétone, on triture le milieu et on obtient 0,31 g (0,55 mmol) de l'oxalate du composé 71 sous la forme d'un solide blanc.
F = 115°C
¹H RMN (DMSOd₆) δ : 1,38 (m, 5H) ; 1,77 (m, 2H) ; 2,67(m, 2H) ; 3,06 (m, 2H) ; 3,20 (m, 2H) ; 3,36 (m, 2H) ; 4,03 (m, 2H) ; 4,17 (m, 3H) ; 4,31 (m, 1H) ; 4,41 (m, 1H) ; 4,59 (m, 1H) ; 6,70 (m, 1H) ; 6,82 (m, 1H) ; 6,95 (m, 4H) ; 7,31 (m, 2H).
MS m/z 471 (M+1)
HPLC (XBRIDGE C8, acétonitrile/eau/KH₂PO₄ (40/60/6,8G PH 4), 0,4 ml/min), temps de rétention = 5,26 min, pureté chimique = 98,86%.

### Exemple 72 : (R)-3-(2-(1-(((S)-6-hydroxy-2,3-dihydrobenzo[b][1,4]dioxin-2-yl) méthyl)pipéridin-4-yl)éthyl)-4-(phénoxyméthyl)oxazolidin-2-one

### ° Etape 1 : (R)-3-(2-(1-(((S)-6-(benzyloxy)-2,3-dihydrobenzo[b][1,4]dioxin-2-yl)méthyl)pipéridin-4-yl)éthyl)-4-(phénoxyméthyl)oxazolidin-2-one

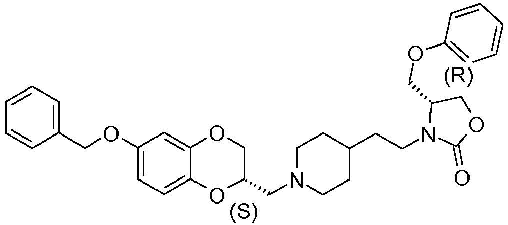

En procédant comme dans l'exemple 1, mais en remplaçant le (R)-2-(bromométhyl)-2,3-dihydrobenzo[b][1,4]dioxine 2.1 par le (R)-(6-(benzyloxy)-7-bromo-2,3-dihydrobenzo[b][1,4]dioxin-2-yl)méthyl methanesulfonate 2.26 et la 3-(2-(pipéridin-4-yl)éthyl)oxazolidin-2-one 3.1 par la (R)-4-(phénoxyméthyl)-3-(2-(pipéridin-4-yl)éthyl)oxazolidin-2-one 3.13, on obtient 0,80 g (1,25 mmol) du composé (R)-3-(2-(1-(((S)-6-(benzyloxy)-2,3-dihydrobenzo[b][1,4]dioxin-2-yl)méthyl)pipéridin-4-yl)éthyl)-4-(phénoxyméthyl)oxazolidin-2-one sous forme d'huile claire, après purification par flash chromatographie sur gel de silice (éluant : dichlorométhane/méthanol = 95/5%).
Rendement : 38%
¹H RMN (CDCl₃) δ : 1,28 (m, 3H) ; 1,55 (m, 2H) ; 1,70 (m, 2H) ; 2,06 (m, 2H) ; 2,50 (m, 1H) ; 2,60 (m, 1H) ; 2,84 (m, 1H) ; 2,95 (m, 1H) ; 3,21 (m, 1H) ; 3,55 (m, 1H) ; 3,92 (m, 1H) ; 4,04 (m, 2H) ; 4,19 (m, 4H) ; 4,46 (m, 1H) ; 5,04 (s, 2H) ; 6,51 (s, 1H) ; 6,88 (m, 2H) ; 7,01 (m, 1H) ; 7,09 (s, 1H) ; 7,31 (m, 3H) ; 7,38 (m, 2H) ; 7,46 (m, 2H).

### ° Etape 2 : (R)-3-(2-(1-(((S)-6-hydroxy-2,3-dihydrobenzo[b][1,4]dioxin-2-yl) méthyl)pipéridin-4-yl)éthyl)-4-(phénoxyméthyl)oxazolidin-2-one

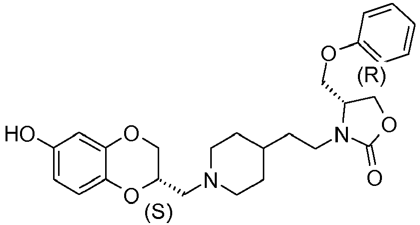

On solubilise 1 g (1,57 mmol) du composé précédent (R)-3-(2-(1-(((S)-6-(benzyloxy)-7-bromo-2,3-dihydrobenzo[b][1,4]dioxin-2-yl)méthyl)pipéridin-4-yl)éthyl)-4-(phénoxyméthyl)oxazolidin-2-one dans un mélange méthanol/dichlorométhane = 5/5 ml. On ajoute une quantité catalytique de Pd sur charbon à 5% et l'on fait une hydrogénation à TA pendant 2h00. On filtre ensuite le milieu sur célite et on concentre. On obtient 0,4 g (0.85 mmol) du produit 72 sous la forme d'un solide blanc, après purification sur gel de silice (éluant : dichlorométhane/méthanol = 95/5%).
Rendement : 54%
¹H RMN (CDCl₃) δ : 1,49 (m, 5H) ; 1,78 (m, 2H) ; 2,30 (m, 2H) ; 2,73 (1s, 2H) ; 3,03 (m, 1H) ; 3,27 (m, 2H) ; 3,52 (m, 1H) ; 3,95 (m, 1H) ; 4,04 (m, 2H) ; 4,20 (m, 3H) ; 4,46 (m, 2H) ; 6,35 (m, 1H) ; 6,40 (s, 1H) ; 6,69 (m, 1H) ; 6,88 (m, 2H) ; 7,01 (m, 1H) ; 7,31 (m, 2H).

On dissout 0.30 g (0,64 mmol) du composé 72 dans 5 ml de méthanol, puis on ajoute 0,058 g (0,64 mmol) d'acide oxalique en solution dans 5 ml de méthanol. On concentre puis on ajoute un peu d'éther et d'acétone, on triture le milieu et on obtient 0,20 g (0.36 mmol) de l'oxalate du composé 72 sous la forme d'un solide blanc.
F = 131°C
¹H RMN (DMSOd₆) δ : 1,40 (m, 5H) ; 1,79 (m, 2H) ; 2,76 (m, 2H) ; 3,16 (m, 2H) ; 3,37 (m, 4H) ; 3,94 (m, 1H) ; 4,07 (m, 1H) ; 4,18 (m, 4H) ; 4,41 (m, 1H) ; 4,54 (m, 1H) ; 6,28 (m, 2H) ; 6,71 (m, 1H) ; 6,97 (m, 3H) ; 7,31 (m, 2H).
MS m/z 469 (M+1)
HPLC (XBRIDGE C8, acétonitrile/eau/KH₂PO₄ (35/65/6,8G PH 4), 0,4 ml/min), temps de rétention = 4,01 min, pureté chimique = 98,69%.

### Exemple 73 : (R)-3-(2-(1-(((S)-2,3-dihydrobenzo[b][1,4]dioxin-2-yl)méthyl)pipéridin-4-yl)éthyl)-4-((4-fluorophénoxy)méthyl)oxazolidin-2-one

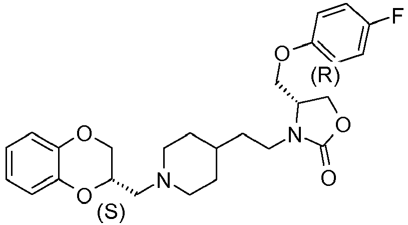

En procédant comme dans l'exemple 1, mais en remplaçant la 3-(2-(pipéridin-4-yl)éthyl)oxazolidin-2-one 3.1 par la (R)-4-((4-fluorophénoxy)méthyl)oxazolidin-2-one 3.15, le K₂CO₃ par le Cs₂CO₃ et l'acétonitrile par le diméthylformamide, on obtient 0,24 g (0,51 mmol) du composé 73 sous forme d'huile jaune pâle, après purification par flash chromatographie sur gel de silice (éluant : n-heptane = 100% puis dichlorométhane/méthanol = 95/5%).
Rendement : 51 %
¹H RMN (CDCl₃) δ : 1,29 (m, 3H) ; 1,53 (m, 2H) ; 1,70 (m, 2H) ; 2,09 (m, 2H) ; 2,54 (dd, 1H, J = 13,4 et 6 Hz) ; 2,64 (dd, 1H, J = 13,2 et 5,6 Hz) ; 2,87 (m, 1H) ; 2,98 (m, 1H) ; 3,19 (m, 1H) ; 3,55 (m, 1H) ; 3,97 (m, 3H) ; 4,18 (m, 2H) ; 4,29 (m, 2H) ; 4,45 (m, 1H) ; 6,85 (m, 6H) ; 7,00 (m, 2H).

On dissout 0.24 g (0,51 mmol) du composé 73 dans 5 ml de méthanol, puis on ajoute 0,046 g (0,51 mmol) d'acide oxalique en solution dans 5 ml de méthanol. On concentre puis on ajoute un peu d'acétone, on triture le milieu et on obtient 0,15 g (0,27 mmol) de l'oxalate du composé 73 sous la forme d'un solide blanc.
F = 114°C
¹H RMN (DMSOd₆) δ : 1,38 (m, 5H) ; 1,77 (m, 2H) ; 2,65 (m, 2H) ; 3,12 (m, 3H) ; 3,24 (m, 1H) ; 3,33 (m, 2H) ; 4,02 (m, 2H) ; 4,16 (m, 3H) ; 4,29 (m, 1H) ; 4,40 (m, 1H) ; 4,60 (m, 1H) ; 6,87 (m, 4H) ; 6,97 (m, 2H) ; 7,14 (m, 2H).
MS m/z 471 (M+1)
HPLC (XBRIDGE C8, acétonitrile/eau/KH₂PO₄ (40/60/6,8G PH 4), 0,4 ml/min), temps de rétention = 5,30 min, pureté chimique = 96,29%.

### Exemple 74 : (R)-3-(2-(1-(((S)-2,3-dihydrobenzo[b][1,4]dioxin-2-yl)méthyl)pipéridin-4-yl)éthyl)-4-((3-fluorophénoxy)méthyl)oxazolidin-2-one

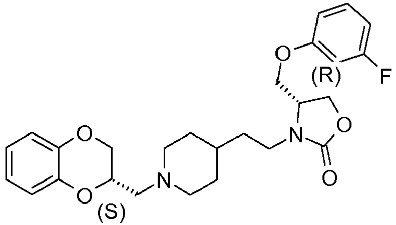

En procédant comme dans l'exemple 1, mais en remplaçant la 3-(2-(pipéridin-4-yl) éthyl)oxazolidin-2-one 3.1 par la (R)-4-((3-fluorophénoxy)méthyl)oxazolidin-2-one 3.16, le K₂CO₃ par le Cs₂CO₃ et l'acétonitrile par le diméthylformamide, on obtient 0,6 g (1.27 mmol) du composé 74 sous forme d'huile jaune pâle, après purification par flash chromatographie sur gel de silice (éluant : n-heptane = 100% puis dichlorométhane/méthanol = 95/5%).
Rendement : 43%
¹H RMN (CDCl₃) δ : 1,29 (m, 3H) ; 1,53 (m, 2H) ; 1,69 (m, 2H) ; 2,08 (m, 2H) ; 2,54 (dd, 1H, J = 13,2 et 6 Hz) ; 2,64 (dd, 1H, J = 13,4 et 6 Hz) ; 2,87 (m, 1H) ; 2,98 (m, 1H) ; 3,20 (m, 1H) ; 3,56 (m, 1H) ; 3,96 (dd, 1H, J = 11,6 et 8 Hz) ; 4,02 (m, 2H) ; 4,18 (m, 2H) ; 4,29 (m, 2H) ; 4,45 (m, 1H) ; 6,60 (m, 1H) ; 6,64 (m, 1H) ; 6,71 (m, 1H) ; 6,85 (m, 4H) ; 7,26 (m, 1H).

On dissout 0.59 g (1,25 mmol) du composé 74 dans 5 ml de méthanol, puis on ajoute 0,11 g (1,25 mmol) d'acide oxalique en solution dans 5 ml de méthanol. On concentre puis on ajoute un peu d'éther éthylique, on triture le milieu et on obtient 0,47 g (0,84 mmol) de l'oxalate du composé 74 sous la forme d'un solide blanc.
F = 136°C
¹H RMN (DMSOd₆) δ : 1,36 (m, 5H) ; 1,77 (m, 2H) ; 2,67 (m, 2H) ; 3,12 (m, 3H) ; 3,23 (m, 1H) ; 3,35 (m, 2H) ; 3,99 (dd, 1H, J = 11,6 et 6,8 Hz) ; 4,11 (m, 2H) ; 4,20 (m, 2H) ; 4,28 (m, 1H) ; 4,42 (m, 1H) ; 4,61 (m, 1H) ; 6,85 (m, 7H) ; 7,33 (m, 1H).
MS m/z 471 (M+1)
HPLC (XBRIDGE C8, acétonitrile/eau/KH₂PO₄ (35/65/6,8G PH 4), 0,4 ml/min), temps de rétention = 10,35 min, pureté chimique = 98,56%.

### Exemple 75 : (R)-3-(2-(1-(((S)-2,3-dihydrobenzo[b][1,4]dioxin-2-yl)méthyl)pipéridin-4-yl)éthyl)-4-((3,4-dimethoxyphénoxy)méthyl)oxazolidin-2-one

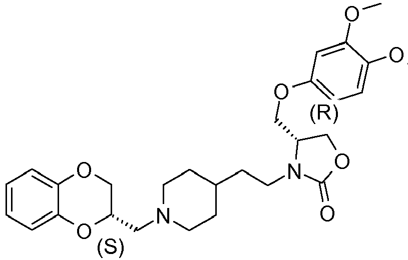

En procédant comme dans l'exemple de référence 1, mais en remplaçant la 3-(2-(pipéridin-4-yl)éthyl)oxazolidin-2-one 3.1 par la (R)-4-((3,4-dimethoxyphénoxy) méthyl)oxazolidin-2-one 3.17, le K₂CO₃ par le Cs₂CO₃ et l'acétonitrile par le diméthylformamide, on obtient 0,6 g (1,17 mmol) du composé 75 sous forme d'huile jaune pâle, après purification par flash chromatographie sur gel de silice (éluant : dichlorométhane/acétate d'éthyle = 70/30% puis dichlorométhane/méthanol = 95/5%).
Rendement : 43%
¹H RMN (CDCl₃) δ : 1,27 (m, 3H) ; 1,52 (m, 2H) ; 1,71 (m, 2H) ; 2,08 (m, 2H) ; 2,53 (dd, 1H, J = 13,4 et 6,4 Hz) ; 2,64 (dd, 1H, J = 13,6 et 5,6 Hz) ; 2,87 (m, 1H) ; 2,97 (m, 1H) ; 3,19 (m, 1H) ; 3,55 (m, 1H) ; 3,84 (s, 3H) ; 3,86 (s, 3H) ; 3,97 (m, 3H) ; 4,13 (m, 1H) ; 4,26 (m, 3H) ; 4,44 (m, 1H) ; 6,36 (dd, 1H, J = 8,8 et 2,8 Hz) ; 6,50 (d, 1H, J = 2,8 Hz) ; 6,83 (m, 5H).

On dissout 0.40 g (0,78 mmol) du composé 75 dans 5 ml de méthanol, puis on ajoute 0,07 g (0,78 mmol) d'acide oxalique en solution dans 5 ml de méthanol. On concentre puis on ajoute un peu d'éther éthylique, on triture le milieu et on obtient 0,38 g (0,63 mmol) de l'oxalate du composé 75 sous la forme d'un solide blanc.
F = 147°C
¹H RMN (DMSOd₆) δ : 1,38 (m, 5H) ; 1,78 (m, 2H) ; 2,67 (m, 2H) ; 3,12 (m, 3H) ; 3,23 (m, 1H) ; 3,34 (m, 2H) ; 3,68 (s, 3H) ; 3,74 (s, 3H) ; 3,99 (m, 2H) ; 4,14 (m, 3H) ; 4,28 (m, 1H) ; 4,40 (m, 1H) ; 4,60 (m, 1H) ; 6,46 (dd, 1H, J = 8,8 et 2,8 Hz) ; 6,56 (d, 1H, J = 2,8 Hz) ; 6,88 (m, 5H).
MS m/z 513 (M+1)
HPLC (XBRIDGE C8, acétonitrile/eau/KH₂PO₄ (35/65/6,8G PH 4), 0,4 ml/min), temps de rétention = 5,12 min, pureté chimique = 95,93%.

### Exemple 76 : (R)-3-(2-(1-(((S)-2,3-dihydrobenzo[b][1,4]dioxin-2-yl)méthyl)pipéridin-4-yl)éthyl)-4-((3-hydroxyphénoxy)méthyl)oxazolidin-2-one

### ° Etape 1 : (R)-4-((3-(benzyloxy)phénoxy)méthyl)-3-(2-(1-(((S)-2,3-dihydrobenzo[b][1,4]dioxin-2-yl)méthyl)pipéridin-4-yl)éthyl)oxazolidin-2-one

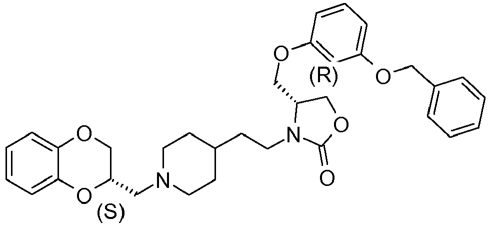

En procédant comme dans l'exemple 1, mais en remplaçant la 3-(2-(pipéridin-4-yl)éthyl)oxazolidin-2-one 3.1 par la (R)-4-((3-(benzyloxy)phénoxy)méthyl)oxazolidin-2-one 3.18, le K₂CO₃ par le Cs₂CO₃ et l'acétonitrile par le diméthylformamide, on obtient 0,85 g (1,52 mmol) du composé (R)-4-((3-(benzyloxy)phénoxy)méthyl)-3-(2-(1-((S)-2,3-dihydrobenzo[b][1,4]dioxin-2-yl)méthyl)pipéridin-4-yl)éthyl)oxazolidin-2-one sous forme d'huile jaune pâle, après purification par flash chromatographie sur gel de silice (éluant : dichlorométhane/acétate d'éthyle = 70/30% puis dichlorométhane/méthanol = 95/5%).
Rendement : 48%
¹H RMN (CDCl₃) δ : 1,29 (m, 3H) ; 1,52 (m, 2H) ; 1,69 (m, 2H) ; 2,07 (m, 2H) ; 2,53 (dd, 1H, J = 13,2 et 6 Hz) ; 2,64 (dd, 1H, J = 13,2 et 5,6 Hz) ; 2,87 (m, 1H) ; 2,97 (m, 1H) ; 3,19 (m, 1H) ; 3,54 (m, 1H) ; 3,95 (dd, 1H, J = 11,6 et 8 Hz) ; 4,01 (m, 1H) ; 4,15 (m, 3H) ; 4,28 (m, 2H) ; 4,44 (m, 1H) ; 5,04 (s, 2H) ; 6,51 (m, 2H) ; 6,63 (m, 1H) ; 6,85 (m, 4H) ; 7,20 (m, 1H) ; 7,37 (m, 5H).

### ° Etape 2 : (R)-3-(2-(1-(((S)-2,3-dihydrobenzo[b][1,4]dioxin-2-yl)méthyl) pipéridin-4-yl)éthyl)-4-((3-hydroxyphénoxy)méthyl)oxazolidin-2-one

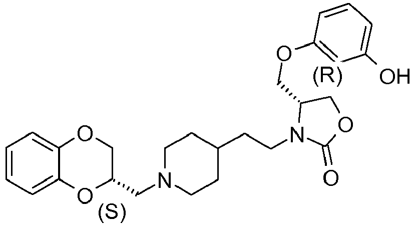

0,85 g (1,52 mmol) du composé précédent (R)-4-((3-(benzyloxy)phénoxy)méthyl)-3-(2-(1-(((S)-2,3-dihydrobenzo[b][1,4]dioxin-2-yl)méthyl)pipéridin-4-yl)éthyl)oxazolidin-2-one est solubilisé dans 10 ml d'éthanol. On ajoute une quantité catalytique de Pd sur charbon à 5% et l'on fait une hydrogénation à TA pendant 3h30. On filtre ensuite le milieu sur célite et on concentre. On obtient 0,40 g (0.85 mmol) du produit 76 sous la forme d'un solide blanc, après purification sur gel de silice (éluant : dichlorométhane/méthanol = 95/5%).
Rendement : 56%
¹H RMN (CDCl₃) δ : 1,27 (m, 3H) ; 1,52 (m, 2H) ; 1,69 (m, 2H) ; 2,07 (m, 2H) ; 2,53 (dd, 1H, J = 13,2 et 5,6 Hz) ; 2,66 (dd, 1H, J = 13,4 et 6 Hz) ; 2,90 (m, 1H) ; 3,01 (m, 1H) ; 3,20 (m, 1H) ; 3,51 (m, 1H) ; 3,95 (dd, 1H, J = 11,2 et 7,6 Hz) ; 4,00 (m, 2H) ; 4,16 (m, 2H) ; 4,28 (m, 2H) ; 4,44 (m, 1H) ; 6,42 (m, 2H) ; 6,49 (m, 1H) ; 6,84 (m, 4H) ; 7,13 (m, 1H).

On dissout 0.40 g (0,85 mmol) du composé 76 dans 5 ml de méthanol, puis on ajoute 0,077 g (0,85 mmol) d'acide oxalique en solution dans 5 ml de méthanol. On concentre puis on ajoute un peu d'éther éthylique, on triture le milieu et on obtient 0,35 g (0,63 mmol) de l'oxalate du composé 76 sous la forme d'un solide blanc.
F = 123°C
¹H RMN (DMSOd₆) δ : 1,37 (m, 5H) ; 1,76 (m, 2H) ; 2,58 (m, 2H) ; 3,00 (m, 2H) ; 3,17 (m, 2H) ; 3,35 (m, 2H) ; 3,98 (m, 2H) ; 4,11 (m, 2H) ; 4,18 (m, 1H) ; 4,28 (m, 1H) ; 4,39 (m, 1H) ; 4,56 (m, 1H) ; 6,36 (m, 3H) ; 6,87 (m, 4H) ; 7,06 (m, 1H).
MS m/z 469 (M+1)
HPLC (XBRIDGE C8, acétonitrile/eau/KH₂PO₄ (30/70/6,8G PH 4), 0,4 ml/min), temps de rétention = 7,13 min, pureté chimique = 98,95%.

### Exemple 77 : (R)-3-(2-(1-(((S)-2,3-dihydrobenzo[b][1,4]dioxin-2-yl)méthyl)pipéridin-4-yl)éthyl)-4-((4-hydroxyphénoxy)méthyl)oxazolidin-2-one

### ° Etape 1 : (R)-4-((4-(benzyloxy)phénoxy)méthyl)-3-(2-(1-(((S)-2,3-dihydrobenzo[b][1,4]dioxin-2-yl)méthyl)pipéridin-4-yl)éthyl)oxazolidin-2-one

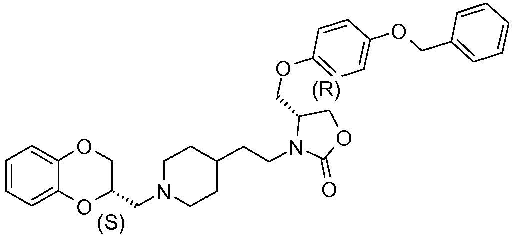

En procédant comme dans l'exemple 1, mais en remplaçant la 3-(2-(pipéridin-4-yl)éthyl)oxazolidin-2-one 3.1 par la (R)-4-((4-(benzyloxy)phénoxy)méthyl)oxazolidin-2-one 3.19, le K₂CO₃ par le Cs₂CO₃ et l'acétonitrile par le diméthylformamide, on obtient 0,80 g (1,43 mmol) du composé 77 sous forme d'huile jaune pâle, après purification par flash chromatographie sur gel de silice (éluant : dichlorométhane/acétate d'éthyle = 70/30% puis dichlorométhane/méthanol = 95/5%).
Rendement : 51 %
¹H RMN (CDCl₃) δ : 1,29 (m, 3H) ; 1,53 (m, 2H) ; 1,69 (m, 2H) ; 2,09 (m, 2H) ; 2,53 (dd, 1H, J = 13,2 et 6 Hz) ; 2,64 (dd, 1H, J = 13,2 et 5,6 Hz) ; 2,87 (m, 1H) ; 2,97 (m, 1H) ; 3,20 (m, 1H) ; 3,54 (m, 1H) ; 3,96 (m, 3H) ; 4,16 (m, 2H) ; 4,29 (m, 2H) ; 4,44 (m, 1H) ; 5,02 (s, 2H) ; 6,86 (m, 8H) ; 7,37 (m, 5H).

### ° Etape 2 : (R)-3-(2-(1-(((S)-2,3-dihydrobenzo[b][1,4]dioxin-2-yl)méthyl) pipéridin-4-yl)éthyl)-4-((4-hydroxyphénoxy)méthyl)oxazolidin-2-one

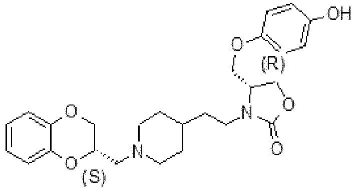

On solubilise 0,80 g (1,43 mmol) du composé précédent (R)-4-((4-(benzyloxy) phénoxy)méthyl)-3-(2-(1-(((S)-2,3-dihydrobenzo[b][1,4]dioxin-2-yl)méthyl)pipéridin-4-yl)éthyl)oxazolidin-2-one dans un mélange éthanol/dichlorométhane 10/5 ml. On ajoute une quantité catalytique de Pd sur charbon à 5% et l'on fait une hydrogénation à TA pendant 3h30. On filtre ensuite le milieu sur célite et on concentre. On obtient 0,65 g (1,39 mmol) du produit 77 sous la forme d'une huile claire.
Rendement : 97%
¹H RMN (CDCl₃) δ : 1,33 (m, 3H) ; 1,52 (m, 2H) ; 1,71 (m, 2H) ; 2,13 (m, 2H) ; 2,65 (m, 2H) ; 2,94 (m, 1H) ; 3,09 (m, 1H) ; 3,21 (m, 1H) ; 3,52 (m, 1H) ; 3,97 (m, 3H) ; 4,12 (m, 1H) ; 4,20 (m, 1H) ; 4,27 (m, 1H) ; 4,42 (m, 2H) ; 6,76 (m, 4H) ; 6,83 (m, 4H).

On dissout 0.65 g (1,39 mmol) du composé 77 dans 5 ml de méthanol, puis on ajoute 0,125 g (1,39 mmol) d'acide oxalique en solution dans 5 ml de méthanol. On concentre puis on ajoute un peu d'éther éthylique, on triture le milieu et on obtient 0,55 g (0,98 mmol) de l'oxalate du composé 77 sous la forme d'un solide blanc.
F = 93°C
¹H RMN (DMSOd₆) δ : 1,38 (m, 5H) ; 1,78 (m, 2H) ; 2,67 (m, 2H) ; 3,12 (m, 3H) ; 3,24 (m, 1H) ; 3,34 (m, 2H) ; 4,00 (m, 3H) ; 4,13 (m, 2H) ; 4,29 (m, 1H) ; 4,39 (m, 1H) ; 4,62 (m, 1H) ; 6,68 (d, 2H, J = 8,4 Hz) ; 6,77 (d, 2H, J = 8,4 Hz) ; 6,88 (m, 4H).
MS m/z 469 (M+1)
HPLC (XBRIDGE C8, acétonitrile/eau/KH₂PO₄ (25/75/6,8G PH 4), 0,4 ml/min), temps de rétention = 9,87 min, pureté chimique = 98,56%.

### Exemple 78 : (R)-3-(2-(1-(((S)-2,3-dihydrobenzo[b][1,4]dioxin-2-yl)méthyl)pipéridin-4-yl)éthyl)-4-((2,6-diméthylphénoxy)méthyl)oxazolidin-2-one

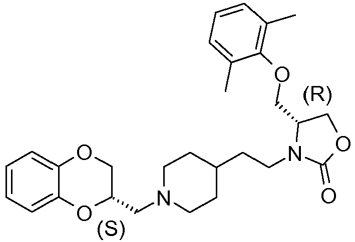

En procédant comme dans l'exemple 1, mais en remplaçant la 3-(2-(pipéridin-4-yl)éthyl)oxazolidin-2-one 3.1 par la (R)-4-((2,6-diméthylphénoxy)méthyl)-3-(2-(pipéridin-4-yl)éthyl)oxazolidin-2-one 3.20, le K₂CO₃ par le Cs₂CO₃ et l'acétonitrile par le diméthylformamide, on obtient 0,6 g (1,25 mmol) du composé 78 sous forme d'huile jaune pâle, après purification par flash chromatographie sur gel de silice (éluant : dichlorométhane/acétate d'éthyle = 70/30% puis dichlorométhane/méthanol = 95/5%). Rendement : 46%
¹H RMN (CDCl₃) δ : 1,30 (m, 3H) ; 1,55 (m, 2H) ; 1,72 (m, 2H) ; 2,08 (m, 2H) ; 2,54 (dd, 1H, J = 13,2 et 6 Hz) ; 2,64 (dd, 1H, J = 13,4 et 5,6 Hz) ; 2,87 (m, 1H) ; 2,99 (m, 1H) ; 3,25 (m, 1H) ; 3,63 (m, 1H) ; 3,80 (m, 1H) ; 3,95 (m, 2H) ; 4,17 (m, 1H) ; 4,30 (m, 3H) ; 4,48 (m, 1H) ; 6,85 (m, 4H) ; 6,96 (m, 1H) ; 7,03 (m, 2H).

On dissout 0.60 g (1,25 mmol) du composé 78 dans 5 ml de méthanol, puis on ajoute 0,112 g (1,25 mmol) d'acide oxalique en solution dans 5 ml de méthanol. On concentre puis on ajoute un peu d'éther éthylique, on triture le milieu et on obtient 0,50 g (0,88 mmol) de l'oxalate du composé 78 sous la forme d'un solide blanc.
F = 187°C
¹H RMN (DMSOd₆) δ : 1,44 (m, 5H) ; 1,84 (m, 2H) ; 2,22 (s, 6H) ; 2,74 (m, 2H) ; 3,17 (m, 3H) ; 3,31 (m, 1H) ; 3,40 (m, 1H) ; 3,47 (m, 1H) ; 3,89 (m, 2H) ; 4,00 (dd, 1H, J = 11,6 et 6,8 Hz) ; 4,19 (m, 1H) ; 4,30 (m, 2H) ; 4,42 (m, 1H) ; 4,65 (m, 1H) ; 6,90 (m, 5H) ; 7,03 (m, 2H).
MS m/z 481 (M+1)
HPLC (XBRIDGE C8, acétonitrile/eau/KH₂PO₄ (40/60/6,8G PH 4), 0,4 ml/min, temps de rétention = 7,72 min, pureté chimique = 97,97%.

### Exemple 79 : (S)-3-(2-(1-(((S)-2,3-dihydrobenzo[b][1,4]dioxin-2-yl)méthyl)pipéridin-4-yl)éthyl)-4-(2-hydroxypropan-2-yl)oxazolidin-2-one

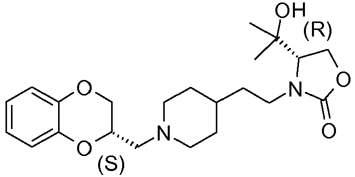

En procédant comme dans l'exemple 1, mais en remplaçant la 3-(2-(pipéridin-4-yl) éthyl)oxazolidin-2-one 3.1 par la (S)-4-(2-hydroxypropan-2-yl)-3-(2-(pipéridin-4-yl) éthyl)oxazolidin-2-one 3.24 et l'acétonitrile par le diméthylformamide, on obtient 0,63 g (1,56 mmol) du composé 79 sous forme d'huile jaune pâle, après purification par flash chromatographie sur gel de silice (gradient : dichlorométhane/méthanol = 100/0% à 95/5%).
Rendement : 61%
¹H RMN (CDCl₃) δ : 1,22 (s, 3H) ; 1,25 (s, 3H) ; 1,28 (m, 3H) ; 1,57 (m, 2H) ; 1,69 (m, 2H) ; 2,07 (m, 2H) ; 2,54 (dd, 1H, J = 13,2 et 6,8 Hz) ; 2,64 (dd, 1H, J = 13,8 et 6 Hz) ; 2,87 (m, 1H) ; 2,98 (m, 1H) ; 3,38 (m, 1H) ; 3,65 (m, 1H) ; 3,73 (dd, 1H, J = 9,2 et 5,2 Hz) ; 3,99 (m, 2H) ; 4,28 (m, 3H) ; 6,85 (m, 4H).

On dissout 0.63 g (1,56 mmol) du composé 79 dans 5 ml de méthanol, puis on ajoute 0,126 g (1,40 mmol) d'acide oxalique en solution dans 5 ml de méthanol. On concentre puis on ajoute un peu d'éther éthylique, on triture le milieu et on obtient 0,64 g (1,29 mmol) de l'oxalate du composé 79 sous la forme d'un solide blanc.
¹H RMN (DMSOd₆) δ : 1,08 (s, 6H) ; 1,37 (m, 3H) ; 1,51 (m, 2H) ; 1,80 (m, 2H) ; 2,70 (m, 2H) ; 3,07 (m, 2H) ; 3,27 (m, 1H) ; 3,40 (m, 3H) ; 3,68 (dd, 1H, J = 9,2 et 5,2 Hz) ; 3,99 (m, 2H) ; 4,21 (m, 1H) ; 4,38 (m, 1H) ; 4,63 (m, 1H) ; 6,88 (m, 4H).
MS m/z 405 (M+1)
HPLC (XBRIDGE C8, acétonitrile/eau/KH₂PO₄ (25/75/6,8G PH 4), 0,4 ml/min, temps de rétention = 4,83 min, pureté chimique = 96,98%.

### Exemple 80 : (S)-3-((4-(2-((S)-4-(2-hydroxypropan-2-yl)-2-oxooxazolidin-3-yl)éthyl) pipéridin-1-yl)méthyl)-2,3-dihydrobenzo[b][1,4]dioxine-6-carbonitrile

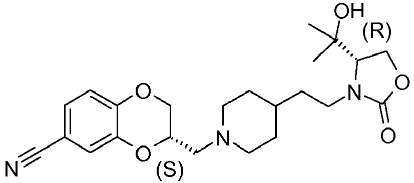

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b][1,4] dioxin-2-yl)méthyl 4-bromobenzenesulfonate 2.11, la 3-(2-(pipéridin-4-yl)éthyl) oxazolidin-2-one 3.1 par la (S)-4-(2-hydroxypropan-2-yl)-3-(2-(pipéridin-4-yl)éthyl) oxazolidin-2-one 3.24, le couple K₂CO₃/KI par la diisopropyléthylamine (1,4 eq) et l'acétonitrile par le diméthylsulfoxide, on obtient 0,65 g (1,51 mmol) du composé 80 sous forme de solide jaune pâle, après purification par flash chromatographie sur gel de silice (gradient : dichlorométhane = 100% à dichlorométhane/(méthanol :NH4OH 9:1) = 90/10% sur 30 min).
Rendement : 63%
¹H RMN (CDCl₃) δ : 1,22 (s, 3H) ; 1,25 (s, 3H) ; 1,27 (m, 3H) ; 1,57 (m, 2H) ; 1,71 (m, 2H) ; 2,08 (m, 2H) ; 2,53 (dd, 1H, J = 13,2 et 6,4 Hz) ; 2,64 (dd, 1H, J = 14,4 et 5,6 Hz) ; 2,85 (m, 1H) ; 2,95 (m, 1H) ; 3,39 (m, 1H) ; 3,65 (m, 1H) ; 3,73 (dd, 1H, J = 9,2 et 5,2 Hz) ; 4,01 (m, 2H) ; 4,27 (m, 2H) ; 4,37 (m, 1H) ; 6,91 (d, 1H, J = 8,4 Hz) ; 7,14 (dd, 1H, J = 8,4 et 1,6 Hz) ; 7,16 (d, 1H, J = 1,6 Hz).

On dissout 0.65 g (1,51 mmol) du composé 80 dans 20 ml d'acétone, puis on ajoute 0,6 ml d'une solution HCl 5N dans l'isopropanol. On concentre puis on ajoute un peu de l'acétate d'éthyle, on triture le milieu et on obtient 0,59 g (1,27 mmol) du chlorhydrate du composé 80 sous la forme d'un solide blanc.
F = 187°C
¹H RMN (DMSOd₆) δ : 1,55 (m, 5H) ; 1,87 (m, 2H) ; 3,01 (m, 2H) ; 3,37 (m, 5H) ; 3,69 (m, 2H) ; 3,99 (m, 1H) ; 4,18 (m, 2H) ; 4,46 (m, 1H) ; 4,85 (m, 1H) ; 7,11 (d, 1H, J = 8,4 Hz) ; 7,39 (dd, 1H, J = 8,4 et 2 Hz) ; 7,50 (d, 1H, J = 2 Hz) ; 10,79 (1s, 1H).
MS m/z 430 (M+1)
HPLC (XBRIDGE C8, acétonitrile/eau/KH₂PO₄ (20/80/3,4G PH 4), 0,4 ml/min, temps de rétention = 9,03 min, pureté chimique = 97,77%.

### Exemple 81 : (S)-3-((4-(2-((R)-4-(hydroxyméthyl)-5,5-diméthyl-2-oxooxazolidin-3-yl)éthyl)pipéridin-1-yl)méthyl)-2,3-dihydrobenzo[b][1,4]dioxine-6-carbonitrile

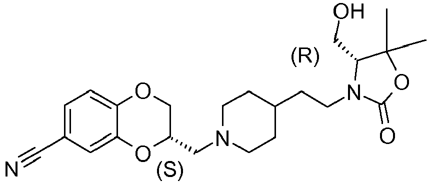

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b][1,4] dioxin-2-yl)méthyl 4-bromobenzenesulfonate 2.11, la 3-(2-(pipéridin-4-yl)éthyl) oxazolidin-2-one 3.1 par la (R)-4-(hydroxyméthyl)-5,5-diméthyl-3-(2-(pipéridin-4-yl) éthyl)oxazolidin-2-one 3.23, le couple K₂CO₃/KI par la diisopropyléthylamine (1,4 eq) et l'acétonitrile par le diméthylsulfoxide, on obtient 0,80 g (1,85 mmol) du composé 81, après purification par flash chromatographie sur gel de silice (gradient : dichlorométhane = 100% à dichlorométhane/(méthanol : NH4OH 9 : 1) = 90/10% sur 30 min).
Rendement : 85%
¹H RMN (CDCl₃) δ : 1,28 (m, 3H) ; 1,44 (s, 3H) ; 1,45 (s, 3H) ; 1,51 (m, 2H) ; 1,72 (m, 2H) ; 2,09 (m, 2H) ; 2,54 (dd, 1H, J = 13,4 et 6 Hz) ; 2,65 (dd, 1H, J = 13,4 et 6 Hz) ; 2,86 (m, 1H) ; 2,95 (m, 1H) ; 3,13 (m, 1H) ; 3,43 (t, 1H, J = 4,8 Hz) ; 3,55 (m, 1H) ; 3,81 (d, 2H, J = 4,8 Hz) ; 4,02 (dd, 1H, J = 11,4 et 7,2 Hz) ; 4,28 (m, 1H) ; 4,37 (m, 1H) ; 6,91 (d, 1H, J = 8 Hz) ; 7,14 (dd, 1H, J = 8 et 2 Hz) ; 7,16 (d, 1H, J = 2 Hz).
HPLC (XBRIDGE C8, acétonitrile/eau/HCl 5 mM (23/77), 1 ml/min, temps de rétention = 7,23 min, pureté chimique = 98,41%.

On dissout 0.80 g (1,85 mmol) du composé 81 dans 15 ml d'acétone, puis on ajoute 0,37 ml d'une solution HCl 5N dans l'isopropanol. On obtient 0,71 g (1,52 mmol) du chlorhydrate du composé 81 sous la forme d'un solide blanc.
F = 208°C
¹H RMN (DMSOd₆) δ : 1,34 (s, 3H) ; 1,35 (s, 3H) ; 1,53 (m, 5H) ; 1,89 (m, 2H) ; 3,06 (m, 2H) ; 3,34 (m, 4H) ; 3,46 (m, 2H) ; 3,60 (m, 2H) ; 3,68 (m, 1H) ; 4,19 (dd, 1H, J = 11,6 et 6,4 Hz) ; 4,45 (m, 1H) ; 4,89 (m, 1H) ; 7,12 (d, 1H, J = 8,4 Hz) ; 7,39 (dd, 1H, J = 8,4 et 2 Hz) ; 7,51 (d, 1H, J = 2 Hz) ; 10,84 (1s, 1H).
MS m/z 430 (M+1)

### Exemple 82 : (3S)-3-((4-(2-((4R)-4-(hydroxyméthyl)-2-oxo-5-phenyloxazolidin-3-yl) éthyl)pipéridin-1-yl)méthyl)-2,3-dihydrobenzo[b][1,4]dioxine-6-carbonitrile

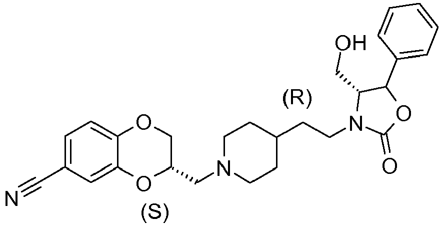

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b] [1,4]dioxin-2-yl)méthyl 4-bromobenzenesulfonate 2.11, la 3-(2-(pipéridin-4-yl)éthyl) oxazolidin-2-one 3.1 par la (4R,5R)-4-(hydroxyméthyl)-5-phenyl-3-(2-(pipéridin-4-yl) éthyl)oxazolidin-2-one 3.25, le couple K₂CO₃/KI par la diisopropyléthylamine (2,2 eq) et l'acétonitrile par le diméthylsulfoxide, on obtient 1,27 g (2,66 mmol) du composé 82 sous forme de solide blanc, après purification par flash chromatographie sur gel de silice (gradient : dichlorométhane = 100% à dichlorométhane/(méthanol : NH4OH 9 : 1)= 95/5% sur 30 min).
Rendement : 84%
¹H RMN (CDCl₃) δ : 1,23 (m, 3H) ; 1,48 (m, 2H) ; 1,61 (m, 1H) ; 1,73 (m, 1H) ; 2,04 (m, 3H) ; 2,53 (dd, 1H, J = 13,6 et 6,4 Hz) ; 2,64 (dd, 1H, J = 13,6 et 5,6 Hz) ; 2,85 (m, 1H) ; 2,92 (m, 1H) ; 3,17 (m, 1H) ; 3,59 (m, 1H) ; 3,66 (m, 1H) ; 3,79 (m, 1H) ; 3,89 (m, 1H) ; 4,00 (dd, 1H, J = 11,2 et 7,6 Hz) ; 4,27 (m, 1H) ; 4,36 (m, 1H) ; 5,35 (d, 1H, J = 5,6 Hz) ; 6,91 (d, 1H, J = 8,4 Hz) ; 7,14 (m, 2H) ; 7,38 (m, 5H).

On dissout 1,27 g (2,66 mmol) du composé 82 dans 30 ml d'acétate d'éthyle, puis on ajoute 1 ml d'une solution HCl 5N dans l'isopropanol. On concentre puis on ajoute un peu d'éther diisopropylique, on triture le milieu et on obtient 1,35 g (2,62 mmol) du chlorhydrate du composé 82 sous la forme d'un solide blanc.
F = 130°C
¹H RMN (DMSOd₆) δ : 1,53 (m, 5H) ; 1,82 (m, 1H) ; 1,92 (m, 1H) ; 2,48 (m, 2H) ; 3,16 (m, 1H) ; 3,38 (m, 5H) ; 3,57 (m, 1H) ; 3,66 (m, 2H) ; 4,18 (dd, 1H, J = 11,6 et 6,4 Hz) ; 4,45 (m, 1H) ; 5,01 (m, 1H) ; 5,30 (d, 1H, J = 5,6 Hz) ; 7,11 (d, 1H, J = 8,4 Hz) ; 7,44 (m, 7H).
MS m/z 478 (M+1)
HPLC (XBRIDGE C8, acétonitrile/eau/KH₂PO₄ (33/67/6,8G PH 4), 1 ml/min, temps de rétention = 7,07 min, pureté chimique = 98,69%.

### Exemple 83 : (S)-3-((4-(2-((S)-4-((R ou S)-hydroxy(phenyl)méthyl)-2-oxooxazolidin-3-yl)éthyl)pipéridin-1-yl)méthyl)-2,3-dihydrobenzo[b][1,4]dioxine-6-carbonitrile

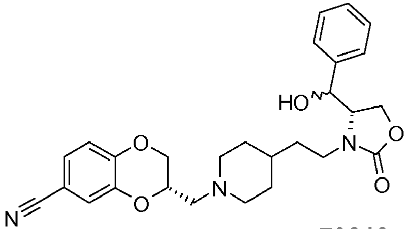

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b] [1,4]dioxin-2-yl)méthyl 4-bromobenzenesulfonate 2.11, la 3-(2-(pipéridin-4-yl)éthyl) oxazolidin-2-one 3.1 par la (S)-4-((S ou R)-hydroxy(phenyl)méthyl)-3-(2-(pipéridin-4-yl)éthyl)oxazolidin-2-one 3.26 et le couple K₂CO₃/KI par la diisopropyléthylamine (2,2 eq), on obtient 1 g (2,09 mmol) du composé 83 sous forme d'une mousse beige, après purification par flash chromatographie sur gel de silice (gradient : dichlorométhane = 100% à dichlorométhane/(méthanol : NH4OH 9 : 1)= 95/5% sur 30 min).
Rendement : 72%
¹H RMN (CDCl₃) δ : 1,26 (m, 3H) ; 1,58 (m, 2H) ; 1,70 (m, 2H) ; 2,08 (m, 2H) ; 2,24 (1s, 1H) ; 2,53 (dd, 1H, J = 13,4 et 6 Hz) ; 2,64 (dd, 1H, J = 13,4 et 6 Hz) ; 2,84 (m, 1H) ; 2,95 (m, 1H) ; 3,40 (m, 1H) ; 3,61 (m, 1H) ; 3,87 (dd, 1H, J = 9,2 et 6 Hz) ; 4,02 (m, 3H) ; 4,28 (m, 1H) ; 4,36 (m, 1H) ; 4,75 (m, 1H) ; 6,91 (d, 1H, J = 8,4 Hz) ; 7,14 (m, 2H) ; 7,38 (m, 5H).
On dissout 1 g (2,09 mmol) du composé 83 dans 30 ml d'acétate d'éthyle, puis on ajoute 0,8 ml d'une solution HCl 5N dans l'isopropanol. On concentre puis on rajoute un peu d'acétate d'éthyle, on triture le milieu et on obtient 0,91 g (1,77 mmol) du chlorhydrate du composé 83 sous la forme d'un solide blanc.
F = 120°C
¹H RMN (DMSOd₆) δ : 1,41 (m, 3H) ; 1,53 (m, 2H) ; 1,81 (m, 2H) ; 2,99 (m, 2H) ; 3,32 (m, 5H) ; 3,65 (m, 1H) ; 3,95 (m, 1H) ; 4,03 (m, 1H) ; 4,15 (m, 2H) ; 4,45 (m, 1H) ; 4,72 (m, 1H) ; 4,99 (m, 1H) ; 5,86 (m, 1H) ; 7,11 (d, 1H, J = 8,4 Hz) ; 7,29 (m, 1H) ; 7,37 (m, 5H) ; 7,49 (m, 1H).
MS m/z 478 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 25 à 60% sur 5 min, 0,75 ml/min), temps de rétention = 1,62 min, pureté chimique : 99,73%.

### Exemple 84 : (S)-3-((4-(2-((S)-4-((S ou R)-hydroxy(phenyl)méthyl)-2-oxooxazolidin-3-yl)éthyl)pipéridin-1-yl)méthyl)-2,3-dihydrobenzo[b][1,4]dioxine-6-carbonitrile

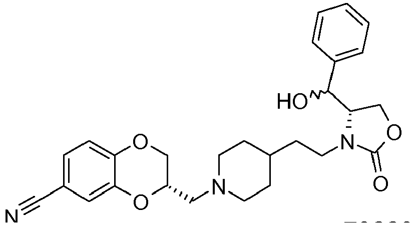

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b] [1,4]dioxin-2-yl)méthyl 4-bromobenzenesulfonate 2.11, la 3-(2-(pipéridin-4-yl)éthyl) oxazolidin-2-one 3.1 par la (S)-4-((S ou R)-hydroxy(phenyl)méthyl)-3-(2-(pipéridin-4-yl)éthyl)oxazolidin-2-one 3.27 et le couple K₂CO₃/KI par la diisopropyléthylamine (2,2 eq), on obtient 0,5 g (1,05 mmol) du composé 84 sous forme d'une mousse blanche, après purification par flash chromatographie sur gel de silice (gradient : dichlorométhane = 100% à dichlorométhane/(méthanol : NH4OH 9 : 1) = 95/5% sur 30 min).
Rendement : 64%
¹H RMN (CDCl₃) δ : 1,25 (m, 3H) ; 1,49 (m, 2H) ; 1,67 (m, 2H) ; 2,08 (m, 2H) ; 2,53 (dd, 1H, J = 13,4 et 6 Hz) ; 2,64 (dd, 1H, J = 13,4 et 6 Hz) ; 2,85 (m, 1H) ; 2,96 (m, 2H) ; 3,55 (m, 1H) ; 4,04 (m, 3H) ; 4,28 (m, 1H) ; 4,37 (m, 2H) ; 4,94 (m, 1H) ; 6,91 (d, 1H, J = 8,4 Hz) ; 7,14 (m, 2H) ; 7,37 (m, 5H).

On dissout 0,5 g (1,05 mmol) du composé 84 dans 30 ml d'acétate d'éthyle, puis on ajoute 0,4 ml d'une solution HCl 5N dans l'isopropanol. On concentre puis on ajoute un peu de l'acétate d'éthyle, on triture le milieu et on obtient 0,47 g (0,91 mmol) du chlorhydrate du composé 84 sous la forme d'un solide blanc.
F = 160°C
¹H RMN (DMSOd₆) δ : 1,58 (m, 5H) ; 1,90 (m, 2H) ; 3,06 (m, 3H) ; 3,40 (m, 4H) ; 3,68 (m, 1H) ; 3,94 (m, 1H) ; 4,04 (m,1H) ; 4,10 (m, 1H) ; 4,17 (m, 1H) ; 4,45 (m, 1H) ; 4,92 (1s, 1H) ; 5,00 (m, 1H) ; 5,84 (1s, 1H) ; 7,11 (d, 1H, J = 8,4 Hz) ; 7,28 (m, 1H) ; 7,39 (m, 5H) ; 7,49 (m, 1H).
MS m/z 478 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 25 à 60% sur 5 min, 0,75 ml/min), temps de rétention = 1,83 min, pureté chimique : 97,94%.

### Exemple 85 : (S)-3-((4-(2-(3-oxomorpholino)éthyl)pipéridin-1-yl)méthyl)-2,3-dihydrobenzo[b][1,4]dioxine-6-carbonitrile

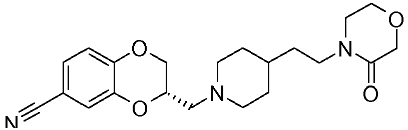

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b] [1,4]dioxin-2-yl)méthyl 4-bromobenzenesulfonate 2.11, la 3-(2-(pipéridin-4-yl)éthyl) oxazolidin-2-one 3.1 par la 4-(2-(pipéridin-4-yl)éthyl)morpholin-3-one 3.28, le couple K₂CO₃/KI par le DMAP (1 eq) et l'acétonitrile par le diméthylsulfoxide, on obtient 0,68 g (1,76 mmol) du composé 85 sous forme d'huile claire, après purification par flash chromatographie sur gel de silice (éluant : dichlorométhane/méthanol = 95/5%).
Rendement : 62%
¹H RMN (CDCl₃) δ : 1,28 (m, 3H) ; 1,51 (m, 2H) ; 1,73 (m, 2H) ; 2,08 (m,2H) ; 2,54 (dd, 1H, J = 13,6 et 6,4 Hz) ; 2,65 (dd, 1H, J = 13,2 et 5,6 Hz) ; 2,86 (m, 1H) ; 2,95 (m, 1H) ; 3,35 (m, 2H) ; 3,45 (t, 2H, J = 7,6 Hz) ; 3,88 (m, 2H) ; 4,02 (dd, 1H, J = 11,4 et 7,2 Hz) ; 4,16 (s, 2H) ; 4,29 (m, 1H) ; 4,37 (dd, 1H, J = 11,4 et 2,4) ; 6,91 (d , 1H, J = 8,2 Hz) ; 7,14 (dd, 1H, J = 8,2 et 2 Hz) ; 7,16 (d, 1H, J = 2 Hz).

On dissout 0,63 g (1,63 mmol) du composé 85 dans 10 ml de méthanol, puis on ajoute 0,147 g (1,63 mmol) d'acide oxalique en solution dans 5 ml de méthanol. On concentre puis on ajoute un peu d'éther éthylique, on triture le milieu et on obtient 0,64 g (1,34 mmol) de l'oxalate du composé 85 sous la forme d'un solide blanc.
F = 168°C
¹H RMN (DMSOd₆) δ : 1,36 (m, 3H) ; 1,44 (m, 2H) ; 1,81 (m, 2H) ; 2,65 (m, 2H) ; 3,07 (m, 2H) ; 3,23 (m, 1H) ; 3,35 (m, 5H) ; 3,81 (m, 2H) ; 4,00 (s, 2H) ; 4,12 (dd, 1H, J = 11,6 et 6,4 Hz) ; 4,40 (dd, 1H, J = 11,6 et 2,4 Hz) ; 4,69 (m, 1H) ; 7,08 (d, 1H, J = 8,4 Hz) ; 7,35 (dd, 1H, J = 8,4 et 2 Hz) ; 7,45 (d, 1H, = 2 Hz).
MS m/z 386 (M+1)
HPLC (Acquity BEH C8, Phase A : 100% H₂O. HCOONH₄ 0.01M PH4, Phase B : 90% Acétonitrile 10% H2O.HCOONH 0.01M PH4, gradient B : 10 à 40% sur 5 min, 0,75 ml/min), temps de rétention = 2,33 min, pureté chimique : 98,80%.

### Exemple 86 : (S)-2-((4-(2-(3-oxomorpholino)éthyl)pipéridin-1-yl)méthyl)-2,3-dihydrobenzo[b][1,4]dioxine-5-carbonitrile

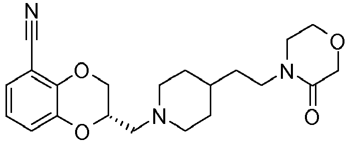

En procédant comme dans l'exemple 1, mais en remplaçant le (R)-2-(bromométhyl)-2,3-dihydrobenzo[b][1,4]dioxine 2.1 par le (R)-(5-cyano-2,3-dihydrobenzo[b][1,4] dioxin-2-yl)méthyl 4-bromobenzenesulfonate 2.14, la 3-(2-(pipéridin-4-yl)éthyl) oxazolidin-2-one 3.1 par la 4-(2-(pipéridin-4-yl)éthyl)morpholin-3-one 3.28 et le couple K₂CO₃/KI par la diisopropyl éthylamine (1,4 eq), on obtient 0,18 g (0,47 mmol) du composé 86 sous forme d'huile claire, après purification par flash chromatographie sur gel de silice (gradient : dichlorométhane = 100% à dichlorométhane/méthanol = 96/4% sur 30 min).
Rendement : 63%
¹H RMN (DMSOd₆) δ : 1,16 (m, 3H) ; 1,40 (m, 2H) ; 1,65 (m, 2H) ; 1,99 (m, 2H) ; 2,53 (m, 2H) ; 2,88 (m, 2H) ; 3,32 (m, 4H) ; 3,80 (m, 2H) ; 3,99 (s, 2H) ; 4,12 (dd, 1H, J = 11,2 et 7,2 Hz) ; 4,46 (m, 2H) ; 6,97 (m, 1H) ; 7,21 (dd, 1H, J = 8,2 et 1,6 Hz) ; 7,28 (dd, 1H, J = 7,8 et 1,6 Hz).

On dissout 0,18 g (0,47 mmol) du composé 86 dans 0,5 ml d'acétonitrile, puis on ajoute 0,042 g (0,47 mmol) d'acide oxalique et 2 ml d'eau. On lyophilise et on obtient 0,22 g (0.46 mmol) de l'oxalate du composé 86 sous la forme d'un solide blanc.
¹H RMN (DMSOd₆) δ : 1,37 (m, 3H) ; 1,44 (m, 2H) ; 1,82 (m, 2H) ; 2,68 (m, 2H) ; 3,11 (m, 2H) ; 3,31 (m, 6H) ; 3,81 (m, 2H) ; 4,00 (s, 2H) ; 4,20 (dd, 1H, J = 11,6 et 6,8 Hz) ; 4,49 (dd, 1H, J = 11,6 et 2,4 Hz) ; 4,75 (m, 1H) ; 7,02 (m, 1H) ; 7,27 (dd, 1H, J = 8 et 1,2 Hz) ; 7,34 (dd, 1H, J = 7,6 et 1,2 Hz).
MS m/z 386 (M+1)
HPLC (X-BRIDGE C18, Phase A : 100% H₂O.TFA 0.1%, Phase B : 100% Acétonitrile, gradient B : 0 à 100% sur 6 min, 1,8 ml/min), temps de rétention = 4,23 min, pureté chimique : 98,58%.

### Exemple 87 : (S)-4-(2-(1-((7-chloro-2,3-dihydrobenzo[b][1,4]dioxin-2-yl)méthyl) pipéridin-4-yl)éthyl)morpholin-3-one

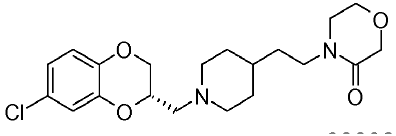

En procédant comme dans l'exemple 1, mais en remplaçant le (R)-2-(bromométhyl)-2,3-dihydrobenzo[b][1,4]dioxine 2.1 par le (R)-(7-chloro-2,3-dihydrobenzo[b][1,4] dioxin-2-yl)méthyl 4-bromobenzenesulfonate 2.7, la 3-(2-(pipéridin-4-yl)éthyl) oxazolidin-2-one 3.1 par la 4-(2-(pipéridin-4-yl)éthyl)morpholin-3-one 3.28 et le couple K₂CO₃/KI par la diisopropyl éthylamine (1,4 eq), on obtient 0,21 g (0,53 mmol) du composé 87 sous forme d'huile claire, après purification par flash chromatographie sur gel de silice (gradient : dichlorométhane = 100% à dichlorométhane/méthanol = 90/10% sur 30 min).
Rendement : 73%
¹H RMN (DMSOd₆) δ : 1,15 (m, 3H) ; 1,41 (m, 2H) ; 1,65 (m, 2H) ; 1,97 (m, 2H) ; 2,50 (m, 2H) ; 2,82 (m, 1H) ; 2,91 (m, 1H) ; 3,32 (m, 4H) ; 3,80 (m, 2H) ; 3,96 (dd, 1H, J = 11,6 et 7,2 Hz) ; 3,99 (s, 2H) ; 4,29 (dd, 1H, J = 11,6 et 2 Hz) ; 4,35 (m, 1H) ; 6,87 (m, 2H) ; 6,95 (d, 1H, J = 2,4 Hz).

On dissout 0,21 g (0,53 mmol) du composé 87 dans 0,5 ml d'acétonitrile, puis on ajoute 0,043 g (0,48 mmol) d'acide oxalique et 2 ml d'eau. On lyophilise et on obtient 0,25 g (0.51 mmol) de l'oxalate du composé 87 sous la forme d'un solide blanc.
¹H RMN (DMSOd₆) δ : 1,38 (m, 3H) ; 1,45 (m, 2H) ; 1,83 (m, 2H) ; 2,72 (m, 2H) ; 3,11 (m, 2H) ; 3,33 (m, 6H) ; 3,81 (m, 2H) ; 4,00 (s, 2H) ; 4,04 (dd, 1H, J = 11,6 et 6,4 Hz) ; 4,30 (dd, 1H, J = 11,6 et 2 Hz) ; 4,87 (m, 1H) ; 6,92 (m, 2H) ; 7,02 (d, 1H, J = 2 Hz).
MS m/z 395 (M+1)
HPLC (X-BRIDGE C18, Phase A : 100% H₂O.TFA 0.1%, Phase B : 100% Acétonitrile, gradient B : 0 à 100% sur 6 min, 1,8 ml/min), temps de rétention = 4,59 min, pureté chimique : 100%.

### Exemple 88 : (S)-4-(2-(1-((7-fluoro-2,3-dihydrobenzo[b][1,4]dioxin-2-yl)méthyl) pipéridin-4-yl)éthyl)morpholin-3-one

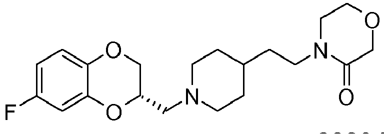

En procédant comme dans l'exemple 1, mais en remplaçant le (R)-2-(bromométhyl)-2,3-dihydrobenzo[b][1,4]dioxine 2.1 par le (R)-(7-fluoro-2,3-dihydrobenzo[b][1,4] dioxin-2-yl)méthyl 4-bromobenzenesulfonate 2.17, la 3-(2-(pipéridin-4-yl)éthyl) oxazolidin-2-one 3.1 par la 4-(2-(pipéridin-4-yl)éthyl)morpholin-3-one 3.28 et le couple K₂CO₃/KI par la diisopropyl éthylamine (1,4 eq), on obtient 0,17 g (0,45 mmol) du composé 88 sous forme d'huile claire, après purification par flash chromatographie sur gel de silice (gradient : dichlorométhane = 100% à dichlorométhane/méthanol = 96/4% sur 30 min).
Rendement : 59%
¹H RMN (DMSOd₆) δ : 1,14 (m, 3H) ; 1,41 (m, 2H) ; 1,65 (m, 2H) ; 2,48 (m, 2H) ; 2,51 (m, 2H) ; 2,83 (m, 1H) ; 2,91 (m, 1H) ; 3,32 (m, 4H) ; 3,80 (m, 2H) ; 3,93 (dd, 1H, J = 11,6 et 7,2 Hz) ; 3,99 (s, 2H) ; 4,26 (dd, 1H, J = 11,6 et 2,4 Hz) ; 4,35 (m, 1H) ; 6,65 (m, 1H) ; 6,76 (m, 1H) ; 6,87 (m, 1H).

On dissout 0,17 g (0,45 mmol) du composé 88 dans 0,5 ml d'acétonitrile, puis on ajoute 0,041 g (0,45 mmol) d'acide oxalique et 2 ml d'eau. On lyophilise et on obtient 0,21 g (0.45 mmol) de l'oxalate du composé 88 sous la forme d'un solide blanc.
¹H RMN (DMSOd₆) δ : 1,38 (m, 3H) ; 1,44 (m, 2H) ; 1,82 (m, 2H) ; 2,70 (m, 2H) ; 3,10 (m, 2H) ; 3,35 (m, 6H) ; 3,81 (m, 2H) ; 4,00 (m, 3H) ; 4,27 (dd, 1H, J = 11,4 et 2 Hz) ; 4,68 (m, 1H) ; 6,71 (m, 1H) ; 6,83 (m, 1H) ; 6,92 (m, 1H).
MS m/z 379 (M+1)
HPLC (X-BRIDGE C18, Phase A : 100% H₂O.TFA 0.1 %, Phase B : 100% Acétonitrile, gradient B : 0 à 100% sur 6 min, 1,8 ml/min), temps de rétention = 4,35 min, pureté chimique : 99,30%.

### Exemple 89 : (S)-4-(2-(1-((8-fluoro-2,3-dihydrobenzo[b][1,4]dioxin-2-yl)méthyl) pipéridin-4-yl)éthyl)morpholin-3-one

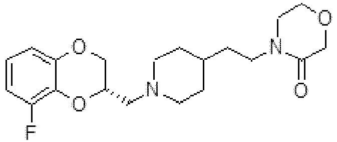

En procédant comme dans l'exemple 1, mais en remplaçant le (R)-2-(bromométhyl)-2,3-dihydrobenzo[b][1,4]dioxine 2.1 par le (R)-(8-fluoro-2,3-dihydrobenzo[b][1,4] dioxin-2-yl)méthyl 4-bromobenzenesulfonate 2.16, la 3-(2-(pipéridin-4-yl)éthyl) oxazolidin-2-one 3.1 par la 4-(2-(pipéridin-4-yl)éthyl)morpholin-3-one 3.28 et le couple K₂CO₃/KI par la diisopropyl éthylamine (1,4 eq), on obtient 0,16 g (0,42 mmol) du composé 89 sous forme d'huile claire, après purification par flash chromatographie sur gel de silice (gradient : dichlorométhane = 100% à dichlorométhane/méthanol = 96/4% sur 30 min).
Rendement : 56%
¹H RMN (DMSOd₆) δ : 1,16 (m, 3H) ; 1,42 (m, 2H) ; 1,65 (m, 2H) ; 1,99 (m, 2H) ; 2,56 (m, 2H) ; 2,83 (m, 1H) ; 2,94 (m, 1H) ; 3,32 (m, 4H) ; 3,80 (m, 2H) ; 3,99 (s, 2H) ; 4,01 (m, 1H) ; 4,36 (m, 2H) ; 6,71 (m, 1H) ; 6,79 (m, 2H).

On dissout 0,16 g (0,42 mmol) du composé 89 dans 0,5 ml d'acétonitrile, puis on ajoute 0,039 g (0,42 mmol) d'acide oxalique et 2 ml d'eau. On lyophilise et on obtient 0,18 g (0.38 mmol) de l'oxalate du composé 89 sous la forme d'un solide blanc.
¹H RMN (DMSOd₆) δ : 1,38 (m, 3H) ; 1,45 (m, 2H) ; 1,84 (m, 2H) ; 2,76 (m, 2H) ; 3,16 (m, 2H) ; 3,34 (m, 6H) ; 3,81 (m, 2H) ; 4,00 (s, 2H) ; 4,08 (dd, 1H, J = 11,6 et 6,8 Hz) ; 4,35 (dd, 1H, J = 11,6 et 2 Hz) ; 4,73 (m, 1H) ; 6,76 (m, 1H) ; 6,84 (m, 2H).
MS m/z 379 (M+1)
HPLC (X-BRIDGE C18, Phase A : 100% H₂O.TFA 0.1%, Phase B : 100% Acétonitrile, gradient B : 0 à 100% sur 6 min, 1,8 ml/min), temps de rétention = 4,32 min, pureté chimique : 99,21%.

### Exemple 90 : S)-4-(2-(1-((6-fluoro-2,3-dihydrobenzo[b][1,4]dioxin-2-yl)méthyl) pipéridin-4-yl)éthyl)morpholin-3-one

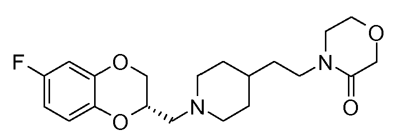

En procédant comme dans l'exemple 1, mais en remplaçant le (R)-2-(bromométhyl)-2,3-dihydrobenzo[b][1,4]dioxine 2.1 par le (R)-(6-fluoro-2,3-dihydrobenzo[b][1,4] dioxin-2-yl)méthyl 4-bromobenzenesulfonate 2.18, la 3-(2-(pipéridin-4-yl)éthyl) oxazolidin-2-one 3.1 par la 4-(2-(pipéridin-4-yl)éthyl)morpholin-3-one 3.28 et le couple K₂CO₃/KI par la diisopropyl éthylamine (1,4 eq), on obtient 0,18 g (0,47 mmol) du composé 90 sous forme d'huile claire, après purification par flash chromatographie sur gel de silice (gradient : dichlorométhane = 100% à dichlorométhane/méthanol = 96/4% sur 30 min).
Rendement : 59%
¹H RMN (DMSOd₆) δ : 1,15 (m, 3H) ; 1,40 (m, 2H) ; 1,65 (m, 2H) ; 2,48 (m, 2H) ; 2,51 (m, 2H) ; 2,82 (m, 1H) ; 2,91 (m, 1H) ; 3,32 (m, 4H) ; 3,80 (m, 2H) ; 3,95 (dd, 1H, J = 11,6 et 7,6 Hz) ; 3,99 (s, 2H) ; 4,29 (m, 2H) ; 6,65 (m, 1H) ; 6,77 (m, 1H) ; 6,87 (m, 1H).

On dissout 0,18 g (0,47 mmol) du composé 90 dans 0,5 ml d'acétonitrile, puis on ajoute 0,043 g (0,47 mmol) d'acide oxalique et 2 ml d'eau. On lyophilise et on obtient 0,21 g (0.45 mmol) de l'oxalate du composé 90 sous la forme d'un solide blanc.
¹H RMN (DMSOd₆) δ : 1,42 (m, 5H) ; 1,83 (m, 2H) ; 2,74 (m, 2H) ; 3,12 (m, 2H) ; 3,34 (m, 6H) ; 3,81 (m, 2H) ; 4,00 (s, 2H) ; 4,02 (m, 1H) ; 4,32 (dd, 1H, J = 11,6 et 2,4 Hz) ; 4,63 (m, 1H) ; 6,71 (m, 1H) ; 6,82 (m, 1H) ; 6,94 (m, 1H).
MS m/z 379 (M+1)
HPLC (X-BRIDGE C18, Phase A : 100% H₂O.TFA 0.1 %, Phase B : 100% Acétonitrile, gradient B : 0 à 100% sur 6 min, 1,8 ml/min), temps de rétention = 4,38 min, pureté chimique : 99,33%.

### Exemple 91 : (S)-4-(2-(1-((5,7-difluoro-2,3-dihydrobenzo[b][1,4]dioxin-2-yl)méthyl) pipéridin-4-yl)éthyl)morpholin-3-one

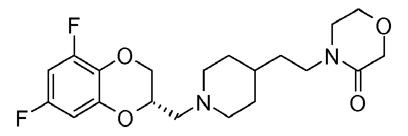

En procédant comme dans l'exemple 1, mais en remplaçant le (R)-2-(bromométhyl)-2,3-dihydrobenzo[b][1,4]dioxine 2.1 par le (R)-(5,7-difluoro-2,3-dihydrobenzo[b][1,4] dioxin-2-yl)méthyl 4-bromobenzenesulfonate 2.19, la 3-(2-(pipéridin-4-yl)éthyl) oxazolidin-2-one 3.1 par la 4-(2-(pipéridin-4-yl)éthyl)morpholin-3-one 3.28 et le couple K₂CO₃/KI par la diisopropyl éthylamine (1,4 eq), on obtient 0,15 g (0,38 mmol) du composé 91 sous forme d'huile claire, après purification par flash chromatographie sur gel de silice (gradient : dichlorométhane = 100% à dichlorométhane/méthanol = 96/4% sur 30 min).
Rendement : 54%
¹H RMN (DMSOd₆) δ : 1,14 (m, 3H) ; 1,41 (m, 2H) ; 1,65 (m, 2H) ; 1,99 (m, 2H) ; 2,54 (m, 2H) ; 2,84 (m, 1H) ; 2,90 (m, 1H) ; 3,32 (m, 4H) ; 3,80 (m, 2H) ; 3,98 (m, 1H) ; 3,99 (s, 2H) ; 4,34 (dd, 1H, J = 11,6 et 2,4 Hz) ; 4,43 (m, 1H) ; 6,69 (m, 1H) ; 6,84 (m, 1H).

On dissout 0,15 g (0,38 mmol) du composé 91 dans 0,5 ml d'acétonitrile, puis on ajoute 0,034 g (0,38 mmol) d'acide oxalique et 2 ml d'eau. On lyophilise et on obtient 0,17 g (0.35 mmol) de l'oxalate du composé 91 sous la forme d'un solide blanc.
¹H RMN (DMSOd₆) δ : 1,41 (m, 5H) ; 1,82 (m, 2H) ; 2,69 (m, 2H) ; 3,10 (m, 2H) ; 3,31 (m, 6H) ; 3,81 (m, 2H) ; 4,00 (s, 2H) ; 4,07 (dd, 1H, J = 11,6 et 6,4 Hz) ; 4,35 (dd, 1H, J = 11,6 et 2,4 Hz) ; 4,76 (m, 1H) ; 6,75 (m, 1H) ; 6,91 (m, 1H).
MS m/z 397 (M+1)
HPLC (X-BRIDGE C18, Phase A : 100% H₂O.TFA 0.1 %, Phase B : 100% Acétonitrile, gradient B : 0 à 100% sur 6 min, 1,8 ml/min), temps de rétention = 4,45 min, pureté chimique : 100%.

### Exemple 92 : (S)-4-(2-(1-((6,7-difluoro-2,3-dihydrobenzo[b][1,4]dioxin-2-yl)méthyl) pipéridin-4-yl)éthyl)morpholin-3-one

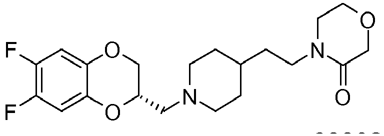

En procédant comme dans l'exemple 1, mais en remplaçant le (R)-2-(bromométhyl)-2,3-dihydrobenzo[b][1,4]dioxine 2.1 par le (R)-(6,7-difluoro-2,3-dihydrobenzo[b][1,4] dioxin-2-yl)méthyl 4-bromobenzenesulfonate 2.22, la 3-(2-(pipéridin-4-yl)éthyl) oxazolidin-2-one 3.1 par la 4-(2-(pipéridin-4-yl)éthyl)morpholin-3-one 3.28 et le couple K₂CO₃/KI par la diisopropyl éthylamine (1,4 eq), on obtient 0,13 g (0,33 mmol) du composé 92 sous forme d'huile claire, après purification par flash chromatographie sur gel de silice (gradient : dichlorométhane = 100% à dichlorométhane/méthanol = 96/4% sur 30 min).
Rendement : 46%
¹H RMN (DMSOd₆) δ : 1,14 (m, 3H) ; 1,41 (m, 2H) ; 1,65 (m, 2H) ; 1,98 (m, 2H) ; 2,51 (m, 2H) ; 2,82 (m, 1H) ; 2,90 (m, 1H) ; 3,32 (m, 4H) ; 3,80 (m, 2H) ; 3,94 (dd, 1H, J = 12 et 7,2 Hz) ; 3,99 (s, 2H) ; 4,31 (m, 2H) ; 7,04 (m, 2H).

On dissout 0,13 g (0,33 mmol) du composé 92 dans 0,5 ml d'acétonitrile, puis on ajoute 0,030 g (0,38 mmol) d'acide oxalique et 2 ml d'eau. On lyophilise et on obtient 0,16 g (0.33 mmol) de l'oxalate du composé 92 sous la forme d'un solide blanc.
¹H RMN (DMSOd₆) δ : 1,41 (m, 5H) ; 1,82 (m, 2H) ; 2,70 (m, 2H) ; 3,10 (m, 2H) ; 3,31 (m, 6H) ; 3,81 (m, 2H) ; 4,00 (s, 2H) ; 4,02 (m, 1H) ; 4,30 (dd, 1H, J = 11,6 et 2 Hz) ; 4,67 (m, 1H) ; 7,10 (m, 2H).
MS m/z 397 (M+1)
HPLC (X-BRIDGE C18, Phase A : 100% H₂O.TFA 0.1%, Phase B : 100% Acétonitrile, gradient B : 0 à 100% sur 6 min, 1,8 ml/min), temps de rétention = 4,45 min, pureté chimique : 98,74%.

### Exemple 93 : (S)-4-(2-(1-((5-chloro-7-fluoro-2,3-dihydrobenzo[b][1,4]dioxin-2-yl) méthyl)pipéridin-4-yl)éthyl)morpholin-3-one

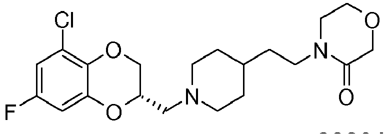

En procédant comme dans l'exemple 1, mais en remplaçant le (R)-2-(bromométhyl)-2,3-dihydrobenzo[b][1,4]dioxine 2.1 par le (R)-(5-chloro-7-fluoro-2,3-dihydrobenzo[b] [1,4]dioxin-2-yl)méthyl 4-bromobenzenesulfonate 2.21, la 3-(2-(pipéridin-4-yl)éthyl)oxazolidin-2-one 3.1 par la 4-(2-(pipéridin-4-yl)éthyl)morpholin-3-one 3.28, le couple K₂CO₃/KI par la diisopropyl éthylamine (1,4 eq), on obtient 0,6 g (1,45 mmol) du composé 93 sous forme d'huile claire, après purification par flash chromatographie sur gel de silice (éluant : dichlorométhane/méthanol = 95/5%).
Rendement : 58%
¹H RMN (CDCl₃) δ : 1,28 (m, 3H) ; 1,50 (m, 2H) ; 1,72 (m, 2H) ; 2,08 (m, 2H) ; 2,55 (dd, 1H, J = 13,4 et 6 Hz) ; 2,65 (dd, 1H, J = 13,4 et 5,6 Hz) ; 2,87 (m, 1H) ; 2,96 (m, 1H) ; 3,35 (t, 2H, J = 5,2 Hz) ; 3,44 (t, 2H, J = 7,6 Hz) ; 3,88 (t, 2H, J = 5,2 Hz) ; 4,00 (dd, 1H, J = 11,4 et 7,6 Hz) ; 4,16 (s, 2H) ; 4,30 (m, 1H) ; 4,39 (dd, 1H, J = 11,4 et 2,4 Hz) ; 6,56 (1H, J = 9,2 et 2,8 Hz) ; 6,69 (dd, 1H, J = 8,2 et 2,8 Hz).

On dissout 0,6 g (1,45 mmol) du composé 93 dans 10 ml d'éthanol, puis on ajoute 0,58 ml (2,90 mmol) d'une solution d'HCl 5N dans l'isopropanol. On concentre puis on ajoute un peu d'acétone et d'acétate d'éthyle, on triture le milieu et on obtient 0,45 g (1 mmol) du chlorhydrate du composé 93 sous la forme d'un solide blanc.
F = 180°C
¹H RMN (DMSOd₆) δ : 1,45 (m, 3H) ; 1,54 (m, 2H) ; 1,90 (m, 2H) ; 3,00 (m, 2H) ; 3,32 (m, 6H) ; 3,46 (m, 1H) ; 3,66 (m, 1H) ; 3,82 (m, 2H) ; 4,01 (s, 2H) ; 4,18 (dd, 1H, J = 11,6 et 6 Hz) ; 4,44 (dd, 1H, J = 11,6 et 2,4 Hz) ; 5,04 (m, 1H) ; 6,94 (dd, 1H, J = 9,6 et 2,8 Hz) ; 7,08 (dd, 1H, J = 8,4 et 3,2 Hz).
MS m/z 413 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 25 à 60% sur 5 min, 0,75 ml/min), temps de rétention = 1,51 min, pureté chimique : 98,94%.

### Exemple 94 : (S)-4-(2-(1-((7-chloro-5-fluoro-2,3-dihydrobenzo[b][1,4]dioxin-2-yl) méthyl)pipéridin-4-yl)éthyl)morpholin-3-one

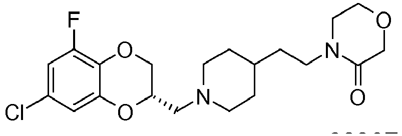

En procédant comme dans l'exemple 1, mais en remplaçant le (R)-2-(bromométhyl)-2,3-dihydrobenzo[b][1,4]dioxine 2.1 par le (R)-(5-fluoro-7-chloro-2,3-dihydrobenzo[b] [1,4]dioxin-2-yl)méthyl 4-bromobenzenesulfonate 2.20, la 3-(2-(pipéridin-4-yl)éthyl) oxazolidin-2-one 3.1 par la 4-(2-(pipéridin-4-yl)éthyl)morpholin-3-one 3.28 et le couple K₂CO₃/KI par la diisopropyl éthylamine (1,4 eq), on obtient 0,17 g (0,41 mmol) du composé 94 sous forme d'huile claire, après purification par flash chromatographie sur gel de silice (gradient : dichlorométhane = 100% à dichlorométhane/méthanol = 96/4% sur 30 min).
Rendement : 59%
¹H RMN (DMSOd₆) δ : 1,14 (m, 3H) ; 1,41 (m, 2H) ; 1,65 (m, 2H) ; 1,99 (m, 2H) ; 2,54 (m, 2H) ; 2,83 (m, 1H) ; 2,90 (m, 1H) ; 3,32 (m, 4H) ; 3,80 (m, 2H) ; 3,99 (s, 2H) ; 4,02 (dd, 1H, J = 11,6 et 7,2 Hz) ; 4,37 (dd, 1H, J = 11,6 et 2 Hz) ; 4,44 (m, 1H) ; 6,87 (m, 1H) ; 7,01 (dd, 1H, J = 10,4 et 2,4 Hz).

On dissout 0,17 g (0,41 mmol) du composé 94 dans 0,5 ml d'acétonitrile, puis on ajoute 0,037 g (0,41 mmol) d'acide oxalique et 2 ml d'eau. On lyophilise et on obtient 0,20 g (0.40 mmol) de l'oxalate du composé 94 sous la forme d'un solide blanc.
¹H RMN (DMSOd₆) δ : 1,37 (m, 3H) ; 1,44 (m, 2H) ; 1,82 (m, 2H) ; 2,68 (m, 2H) ; 3,11 (m, 2H) ; 3,31 (m, 6H) ; 3,81 (m, 2H) ; 4,00 (s, 2H) ; 4,11 (dd, 1H, J = 11,6 et 6,4 Hz) ; 4,38 (dd, 1H, J = 11,6 et 2 Hz) ; 4,75 (m, 1H) ; 6,93 (m, 1H) ; 7,07 (dd, 1H, J = 10,4 et 2,4 Hz).
MS m/z 413 (M+1)
HPLC (X-BRIDGE C18, Phase A : 100% H₂O.TFA 0.1%, Phase B : 100% Acétonitrile, gradient B : 0 à 100% sur 6 min, 1,8 ml/min), temps de rétention = 4,67 min, pureté chimique : 99,52%.

### Exemple 95 : (S)-méthyl 3-((4-(2-(3-oxomorpholino)éthyl)pipéridin-1-yl)méthyl)-2,3-dihydrobenzo[b][1,4]dioxine-6-carboxylate

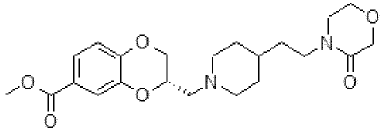

En procédant comme dans l'exemple 1, mais en remplaçant le (R)-2-(bromométhyl)-2,3-dihydrobenzo[b][1,4]dioxine 2.1 par le (R)-méthyl 3-((((4-bromophenyl)sulfonyl) oxy)méthyl)-2,3-dihydrobenzo[b][1,4]dioxine-6-carboxylate 2.24, la 3-(2-(pipéridin-4-yl)éthyl)oxazolidin-2-one 3.1 par la 4-(2-(pipéridin-4-yl)éthyl)morpholin-3-one 3.28 et le couple K₂CO₃/KI par la diisopropyl éthylamine (1,4 eq), on obtient 0,13 g (0,31 mmol) du composé 95 sous forme d'huile claire, après purification par flash chromatographie sur gel de silice (gradient : dichlorométhane = 100% à dichlorométhane/méthanol = 96/4% sur 30 min).
Rendement : 47%
¹H RMN (DMSOd₆) δ : 1,14 (m, 3H) ; 1,41 (m, 2H) ; 1,65 (m, 2H) ; 2,00 (m, 2H) ; 2,54 (m, 2H) ; 2,83 (m, 1H) ; 2,92 (m, 1H) ; 3,32 (m, 4H) ; 3,80 (m, 2H) ; 3,99 (s, 2H) ; 4,04 (dd, 1H, J = 11,8 et 7,2 Hz) ; 4,37 (m, 2H) ; 6,98 (d, 1H, J = 8,4 Hz) ; 7,39 (d, 1H, J = 2 Hz) ; 7,46 (dd, 1H, J = 8,4 et 2 Hz).

On dissout 0,13 g (0,31 mmol) du composé 95 dans 0,5 ml d'acétonitrile, puis on ajoute 0,028 g (0,31 mmol) d'acide oxalique et 2 ml d'eau. On lyophilise et on obtient 0,15 g (0.29 mmol) de l'oxalate du composé 95 sous la forme d'un solide blanc.
¹H RMN (DMSOd₆) δ : 1,42 (m, 5H) ; 1,84 (m, 2H) ; 2,74 (m, 2H) ; 3,16 (m, 2H) ; 3,35 (m, 6H) ; 3,81 (m, 2H) ; 4,00 (s, 2H) ; 4,11 (dd, 1H, J = 11,6 et 6,8 Hz) ; 4,39 (dd, 1H, J = 11,6 et 2 Hz) ; 4,71 (m, 1H) ; 7,02 (m, 1H) ; 7,50 (m, 1H).
MS m/z 419 (M+1)
HPLC (X-BRIDGE C18, Phase A : 100% H₂O.TFA 0.1%, Phase B : 100% Acétonitrile, gradient B : 0 à 100% sur 6 min, 1,8 ml/min), temps de rétention = 4,35 min, pureté chimique : 99,00%.

### Exemple 96 : (R)-4-(2-(1-(((S)-2,3-dihydrobenzo[b][1,4]dioxin-2-yl)méthyl)pipéridin-4-yl)éthyl)-6-phenylmorpholin-3-one

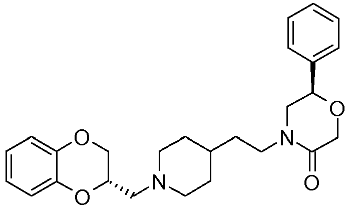

En procédant comme dans l'exemple 1, mais en remplaçant la 3-(2-(pipéridin-4-yl)éthyl)oxazolidin-2-one 3.1 par la 4-(2-(pipéridin-4-yl)éthyl)morpholin-3-one 3.29, on obtient 1 g (2,29 mmol) du composé 96 sous forme d'huile claire, après purification par flash chromatographie sur gel de silice (éluant : dichlorométhane/méthanol = 95/5%).
Rendement : 48%
¹H RMN (CDCl₃) δ : 1,26 (m, 3H) ; 1,51 (m, 2H) ; 1,71 (m, 2H) ; 2,10 (m, 2H) ; 2,54 (m, 1H) ; 2,65 (m, 1H) ; 2,88 (m, 1H) ; 2,98 (m, 1H) ; 3,34 (m, 2H) ; 3,52 (m, 2H) ; 3,96 (dd, 1H, J = 11,6 et 7,8 Hz) ; 4,36 (m, 4H) ; 4,79 (dd, 1H, J = 10,4 et 2,8 Hz) ; 6,85 (m, 4H) ; 7,40 (m, 5H).

On dissout 1 g (2,29 mmol) du composé 96 dans 10 ml de méthanol, puis on ajoute 0,18 g (2,06 mmol) d'acide oxalique en solution dans 5 ml de méthanol. On concentre puis on ajoute un peu d'éther, on triture le milieu et on obtient 1 g (1,90 mmol) de l'oxalate du composé 96 sous la forme d'un solide blanc.
F = 133°C
¹H RMN (DMSOd₆) δ : 1,35 (m, 3H) ; 1,49 (m, 2H) ; 1,84 (m, 2H) ; 2,68 (m, 2H) ; 3,10 (m, 2H) ; 3,38 (m, 6H) ; 3,99 (dd, 1H, J = 11,6 et 6,8 Hz) ; 4,24 (s, 2H) ; 4,29 (dd, 1H, J = 11,4 et 2,4 Hz) ; 4,62 (m, 1H) ; 4,89 (dd, 1H, J = 10,4 et 3,2 Hz) ; 6,88 (m, 4H) ; 7,39 (m, 5H).
HPLC (XBRIDGE C8, acétonitrile/eau/KH₂PO₄ (40/60/6,8G PH 4), 1 ml/min, temps de rétention = 6,53 min, pureté chimique = 90,33%

### Exemple 97 : (S)-3-((4-(2-((R)-5-oxo-2-phenylmorpholino)éthyl)pipéridin-1-yl) méthyl)-2,3-dihydrobenzo[b][1,4]dioxine-6-carbonitrile

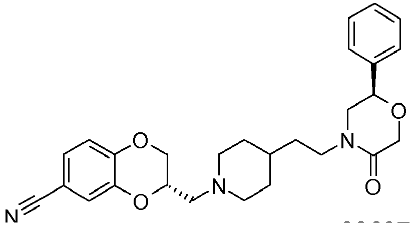

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b][1,4] dioxin-2-yl)méthyl 4-bromobenzenesulfonate 2.11, la 3-(2-(pipéridin-4-yl)éthyl) oxazolidin-2-one 3.1 par la 4-(2-(pipéridin-4-yl)éthyl)morpholin-3-one 3.29, le couple K₂CO₃/KI par le DMAP (1,4 eq) et l'acétonitrile par le diméthylsulfoxide, on obtient 1,13 g (2,45 mmol) du composé 97 sous forme d'huile claire, après purification par flash chromatographie sur gel de silice (éluant : dichlorométhane/méthanol = 95/5%).
Rendement : 53%
¹H RMN (CDCl₃) δ : 1,27 (m, 2H) ; 1,55 (m, 3H) ; 1,73 (m, 2H) ; 2,09 (m, 2H) ; 2,54 (dd, 1H, J = 13,4 et 6,4 Hz) ; 2,65 (dd, 1H, J = 13,2 et 5,6 Hz) ; 2,86 (m, 1H) ; 2,94 (m, 1H) ; 3,35 (m, 2H) ; 3,55 (m, 2H) ; 4,01 (dd, 1H, J = 11,2 et 7,6 Hz) ; 4,29 (m, 1H) ; 4,39 (m, 3H) ; 4,79 (dd, 1H, J = 10,4 et 3,2 Hz) ; 6,90 (m, 1H) ; 7,14 (m, 2H) ; 7,37 (m, 5H).

On dissout 1,13 g (2,45 mmol) du composé 97 dans 10 ml de méthanol, puis on ajoute 0,23 g (2,45 mmol) d'acide oxalique en solution dans 5 ml de méthanol. On concentre puis on ajoute un peu d'éther, on triture le milieu et on obtient 1,1 g (1,99 mmol) de l'oxalate du composé 97 sous la forme d'un solide blanc.
F = 184°C ¹H RMN (DMSOd₆) δ : 1,37 (m, 3H) ; 1,48 (m, 2H) ; 1,84 (m, 2H) ; 2,67 (m, 2H) ; 3,09 (m, 2H) ; 3,38 (m, 6H) ; 4,12 (m, 1H) ; 4,25 (s, 2H) ; 4,40 (m, 1H) ; 4,70 (m, 1H) ; 4,89 (dd, 1H, J = 10,4 et 3,2 Hz) ; 7,09 (m, 1H) ; 7,38 (m, 7H).
MS m/z 462 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1,30 min, pureté chimique : 98,78%.

### Exemple 98 : (S)-3-{4-[4-(2-oxo-oxazolidin-3-yl)-butyl]-pipéridin-1-ylméthyl}-2,3-dihydrobenzo[b][1,4]dioxine-6-carbonitrile

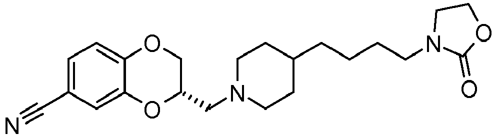

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b][1,4] dioxin-2-yl)méthyl 4-bromobenzenesulfonate 2.11, la 3-(2-(pipéridin-4-yl)éthyl) oxazolidin-2-one 3.1 par la 3- (4-pipéridin-4-yl-butyl)-oxazolidin-2-one 3.34, le couple K₂CO₃/KI par la N,N'-düsopropyléthylamine (1.4 eq), on obtient le composé 98 sous forme d'huile claire, après purification par flash chromatographie sur gel de silice (éluant : dichlorométhane/méthanol = 95/5%).
Rendement : 75%
¹H RMN (CDCl₃) δ : 1,27 (m, 4H) ; 1,50 - 1,66 (m, 7H) ; 2,65 (m, 2H,) ; 2,86 (m, 2H) ; 2,95 (dd, 2H, J = 8 et 6 Hz) ; 3,25 (t, 2H, J = 7 Hz) ; 3,50 (m, 2H) ; 4,02 (dd, 1H, J = 10 et 7 Hz) ; 4,35 (m, 4H) ; 6,89 (d, 1H, J = 8 Hz) ; 7,15 (dd, 1H, J = 8 et 2 Hz) ; 7,16 (d, 1H, J = 2 Hz).

On dissout 0.55 g (1.38 mmol) du composé 98 dans 10 ml d'éthanol, puis on ajoute 0,128 g (1.38 mmol) d'acide oxalique en solution dans 5 ml de méthanol. On concentre puis on ajoute un peu d'éther, on triture le milieu et on obtient 0.4 g (0.77 mmol) de l'oxalate du composé 97 sous la forme d'un solide blanc.
F = 182°C ¹H RMN (DMSOd₆) δ : 1,17 (m, 4H) ; 1,49 (m, 5H) ; 1,82 (m, 2H) ; 3,03 (m, 2H) ; 3,13 (m, 2H) ; 3,35 (m, 5H) ; 3,67 (m, 1H) ; 4.2 (m, 3H) ; 4,46 (dd, 1H, J = 12 et 2,4 Hz) ; 5 (m, 1H) ; 7,1 (d, 1H, J = 7.9 Hz) ; 7,39 (dd, 1H, J = 7.9 et 2 Hz) ; 7,52 (d, 1H, J = 2 Hz).
MS m/z 400 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1,18 min, pureté chimique : 99.7%.

### Exemple 99 : (S)-3-{4-[3-((R)-2-oxo-4-phenyl-oxazolidin-3-yl)propyl]-pipéridin-1-yl méthyl}-2,3-dihydrobenzo[b][1,4]dioxine-6-carbonitrile

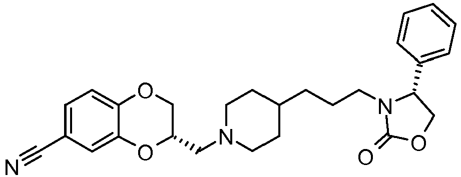

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b][1,4] dioxin-2-yl)méthyl 4-bromobenzenesulfonate 2.11, la 3-(2-(pipéridin-4-yl)éthyl) oxazolidin-2-one 3.1 par la (R)-4-phenyl-3-(3-(pipéridin-4-yl)propyl)-oxazolidin-2-one 3.32, le couple K₂CO₃/KI par la N,N'-diisopropyléthylamine (1.4 eq), on obtient le composé 99 sous forme d'huile claire, après purification par flash chromatographie sur gel de silice (éluant : dichlorométhane/méthanol = 95/5%).
Rendement : 53%
¹H RMN (CDCl₃) δ : 1,16 (m, 5H) ; 1.43 (m, 2H) ; 1.58 (m, 2H) ; 2,05 (m, 2H,) ; 2.55 (dd, 1H) ; 2.65 (dd, 1H) ; 2.7-3 (m, 3H) ; 3,4 (m, 1H,) ; 4-4.15 (m, 2H) ; 4,3 (m, 1Hz) ; 4,35 (dd, 1H, J= 4 et 7 Hz) ; 4.55 (dd, 1H, J = 4 et 7 Hz) ; 4.7 (t, 1H); 6,90 (d, 1H, J = 7 Hz) ; 7,15 (dd, 1H, J = 7 et 2 Hz) ; 7,16 (d, 1H, J = 2 Hz) ; 7.41 (m, 5H).

On dissout 0.17 g (0.37 mmol) du composé 99 dans 10 ml de éthanol, puis on ajoute 0,033 g (0.37 mmol) d'acide oxalique en solution dans 5 ml d'éthanol. On concentre puis on ajoute un peu d'acétone, on triture le milieu et on obtient 0.17 g (0.31 mmol) de l'oxalate du composé 99 sous la forme d'un solide blanc.
F = 196°C
¹H RMN (DMSOd₆) δ : 1,33 (m, 5H) ; 1,71 (m, 4H) ; 2,60 (m, 3H) ; 3,10 (m, 2H) ; 3,12 (m, 1H) ; 3,32 (m, 2H) ; 4,10 (m, 2H) ; 4,39 (m, 1H) ; 4,63 (m, 2H) ; 4,91 (m, 1H) ; 7,20 (2 d, 2H) ; 7,40 (m, 5H).
MS m/z 462 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1.39 min, pureté chimique : 99.7%.

### Exemple 100 :3-(3-{1-[(S)-1-(2,3-dihydrobenzo[b][1,4]dioxin-2-yl)méthyl]-pipéridin-4-yl}-propyl)-oxazolidin-2-one

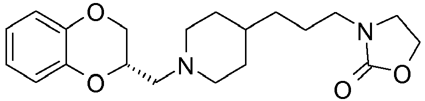

En procédant comme dans l'exemple 1, mais en remplaçant la 3-(2-(pipéridin-4-yl)éthyl)oxazolidin-2-one 3.1 par la 3-(3-(pipéridin-4-yl)propyl)oxazolidin-2-one 3.30, le couple K₂CO₃/KI par la N,N'-diisopropyléthylamine (1.4 eq), on obtient le composé 100 sous forme d'huile claire, après purification par flash chromatographie sur gel de silice (éluant : dichlorométhane/méthanol = 95/5%).
Rendement : 51 %
¹H RMN (CDCl₃) δ : 1,27 (s, 4H) ; 1,48-1.57 (m, 5H) ; 2,1 (m, 2H) ; 2,65 (m, 2H) ; 2,89 (d, 1H, J = 5 Hz) ; 2,99 (m, 1H, J = 5 Hz) ; 3,25 (t, 2H, J = 7.5 Hz) ; 3,55 (t, 2H, J = 8 Hz) ; 3,97 (dd, 1H, J = 7 et 8 Hz) ; 4,35 (m, 4H) ; 6,83 (m, 4H).

On dissout 0.200 g (0.55 mmol) du composé 100 dans 5 ml d'éthanol, puis on ajoute 0,051 g (0.55 mmol) d'acide oxalique en solution dans 5 ml de éthanol. On concentre puis on ajoute un peu d'éther, on triture le milieu et on obtient 0.127 g (0.28 mmol) de l'oxalate du composé 100 sous la forme d'un solide blanc.
F = 129°C ¹H RMN (DMSOd₆) δ : 1.45-1.55 (m, 5H) ; 1,9 (m, 4H) ; 3,11 (m, 2H) ; 3,22 (t, 2H, J = 7.8 Hz) ; 3,31 (m, 1H) ; 3,49 (m, 2H) ; 3,50 (t, 2H, J = 8 Hz) ; 3,7 (m, 1H) ; 4,05 (m, 1H) ; 4,22 (t, 2H, J = 8 Hz) ; 4,32 (m, 1H) ; 4,86 (m, 1H) ; 6,91 (m, 4H) ; 10,3 (s, 2H).
MS m/z 361 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1.01 min, pureté chimique : 99.43%.

### Exemple 101 :4-(3-{1-[(S)-1-(2,3-dihydrobenzo[b][1,4]dioxin-2-yl)méthyl]-pipéridin-4-yl}-propyl)-morpholin-3-one

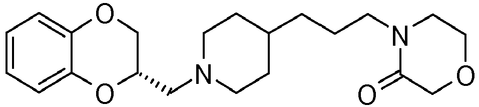

En procédant comme dans l'exemple 1, mais en remplaçant la 3-(2-(pipéridin-4-yl)éthyl)oxazolidin-2-one 3.1 par la 4- (3-pipéridin-4-yl-propyl)-morpholin-3-one 3.33, le couple K₂CO₃/KI par la N,N'-diisopropyléthylamine (1.4 eq), on obtient le composé 101 sous forme d'huile claire, après purification par flash chromatographie sur gel de silice (éluant : dichlorométhane/méthanol = 95/5%).
Rendement : 40%
¹H RMN (CDCl₃) δ : 1,25 (s, 4H) ; 1,50-1.60 (m, 5H) ; 2,1 (m, 2H) ; 2.5-2,65 (m, 2H) ; 2,90 (d, 1H, J = 6 Hz) ; 2,99 (m, 1H, J = 6 Hz) ;3,45 (m, 4H) ; 3,9 (t, 2H, J = 7,6 Hz) ;
4 (m, 1H) ; 4,16 (s, 2H) ; 4,35 (dd, 2H, J = 10 et 4) ; 6.84 (m, 4H)

On dissout 0.160 g (0.427 mmol) du composé 101 dans 5 ml d'éthanol, puis on ajoute 0,038 g (0.427 mmol) d'acide oxalique en solution dans 5 ml d'éthanol. On concentre puis on ajoute un peu d'éther, on triture le milieu et on obtient 0.1 g (0.21 mmol) de l'oxalate du composé 101 sous la forme d'un solide blanc.
F = 139°C
¹H RMN (DMSOd₆) δ : 1.36-1,44 (m, 5H) ; 1,85 (m, 4H) ; 2,63 (m, 2H) ; 3,10 (m, 2H) ; 3,20 (m, 1H) ; 3,33 (m, 5H) ; 3,81 (m, 2H) ; 4,10 (s, 2H) ; 4,12 (dd, 1H, J = 12 et 6 Hz) ; 4,40 (dd, 1H, J = 12 et 6 Hz) ; 4,69 (m, 1H) ; 7,08 (d, 1H, J = 7.5 Hz) ; 7,35-7.48 (m, 4H) ; 10.2 (s, 2H).
MS m/z 375 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 0.97 min, pureté chimique : 99.6%.

### Exemple 102 :(S)-3-{4-[3-(3-oxo-morpholin-4-yl)-propyl]-pipéridin-1-ylméthyl}-2,3-dihydrobenzo[b][1,4]dioxine-6-carbonitrile

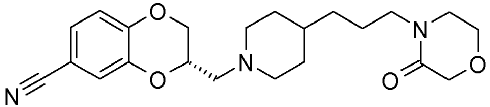

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b][1,4] dioxin-2-yl)méthyl 4-bromobenzenesulfonate 2.11, la 3-(2-(pipéridin-4-yl)éthyl) oxazolidin-2-one 3.1 par la 4- (3-pipéridin-4-yl-propyl)-morpholin-3-one 3.33, le couple K₂CO₃/KI par la N,N'-diisopropyléthylamine (1.4 eq), on obtient le composé 102 sous forme d'huile claire, après purification par flash chromatographie sur gel de silice (éluant : dichlorométhane/méthanol = 95/5%).
Rendement : 66%
¹H RMN (CDCl₃) δ : 1,25 (s, 4H) ; 1,55-1.60 (m, 5H) ; 2,1 (m, 2H) ; 2.5-2,65 (m, 2H) ; 2,85 (m, 2H) ; 3,40 (m, 4H) ; 3,9 (t, 2H, J = 7,6 Hz) ; 4 (m, 1H) ; 4,16 (s, 2H) ; 4,35 (dd, 2H, J = 10 et 4) ; 6,90 (d, 1H, J = 7 Hz) ; 7,15 (dd, 1H, J = 7 et 2 Hz) ; 7,16 (d, 1H, J = 2 Hz).

On dissout 0.27 g (0.67 mmol) du composé 102 dans 5 ml d'éthanol, puis on ajoute 0,061 g (0.67 mmol) d'acide oxalique en solution dans 5 ml d'éthanol. On concentre puis on ajoute un peu d'éther, on triture le milieu et on obtient 0.148 g (0.302 mmol) de l'oxalate du composé 102 sous la forme d'un solide blanc.
F = 168°C
¹H RMN (DMSOd₆) δ : 1.34-1,44 (m, 5H) ; 1,77 (m, 4H) ; 2,63 (m, 2H) ; 3,10 (m, 2H) ; 3,20 (m, 1H) ; 3,33 (m, 5H) ; 3,81 (m, 2H) ; 4,10 (s, 2H) ; 4,12 (dd, 1H, J = 12 et 6 Hz) ; 4,40 (dd, 1H, J = 12 et 2 Hz) ; 4,69 (m, 1H) ; 7,08 (d, 1H, J = 7.5 Hz) ; 7,35 (dd, 1H, J = 7.5 et 2 Hz) ; 7,44 (d, 1H, = 2 Hz) ; 10.5 (s, 2H).
MS m/z 400 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1.98 min, pureté chimique : 99.93%.

### Exemple 103 :3-{3-[1-((S)-7-chloro-2,3-dihydrobenzo[b][1,4]dioxin-2-ylméthyl)-pipéridin-4-yl]-propyl}-oxazolidin-2-one

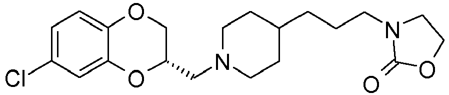

En procédant comme dans l'exemple 1, mais en remplaçant le (R)-2-(bromométhyl)-2,3-dihydrobenzo[b][1,4]dioxine 2.1 par le (R)-2-(bromométhyl)-7-chloro-2,3-dihydrobenzo[b][1,4]dioxine 2.7, la 3-(2-(pipéridin-4-yl)éthyl)oxazolidin-2-one 3.1 par la 3-(3-(pipéridin-4-yl)propyl)oxazolidin-2-one 3.30, le couple K₂CO₃/KI par la N,N'-diisopropyléthylamine (1.4 eq), on obtient le composé 103 sous forme d'huile claire, après purification par flash chromatographie sur gel de silice (éluant : dichlorométhane/méthanol = 95/5%).
Rendement : 60%
¹H RMN (CDCl₃) δ : 1,25 (s, 4H) ; 1,50-1.60 (m, 5H) ; 2,1 (m, 2H) ; 2,65 (m, 2H) ; 2,88 (d, 1H, J = 5 Hz) ; 3 (d, 1H, J = 5 Hz) ; 3,24 (t, 2H, J = 7.5 Hz) ; 3,55 (t, 2H, J = 8 Hz) ; 3,97 (dd, 1H, J = 7 et 8 Hz) ; 4,35 (m, 4H) ; 6,79 (2d, 2H) ; 6.88 (s, 1H).

On dissout 0.17 g (0.43 mmol) du composé 103 dans 5 ml d'éthanol, puis on ajoute 0,04 g (0.43 mmol) d'acide oxalique en solution dans 2 ml d'éthanol. On concentre puis on ajoute un peu d'éther, on triture le milieu et on obtient 0.158 g (0.32 mmol) de l'oxalate du composé 103 sous la forme d'un solide blanc.
F = 149°C ¹H RMN (DMSOd₆) δ : 1,44 (m, 5H) ; 1,89 (m, 4H) ; 3,02 (m, 2H) ; 3,20 (t, 2H) ; 3,38 (m, 3H) ; 3,52 (t, 2H, J = 6.8 Hz) ; 3,63 (m, 1H) ; 4,08 (dd, 1H, J = 12 et 6 Hz) ; 4,21 (t, 2H, J = 7 Hz) ; 4,30 (m, 1H) ; 4,87 (m, 1H) ; 6,92 (1s, 2H) ; 7,1 (1s, 1H) ; 10.2 (1s, 1H).
MS m/z 395 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 0.89 min, pureté chimique : 99.9%.

### Exemple 104 :3-{3-[1-((S)-7-chloro-5-fluoro-2,3-dihydrobenzo[b][1,4]dioxin-2-yl méthyl)-pipéridin-4-yl]-propyl}-oxazolidin-2-one

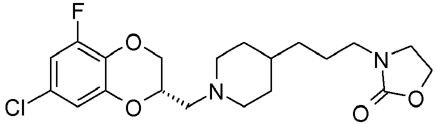

En procédant comme dans l'exemple 1, mais en remplaçant le (R)-2-(bromométhyl)-2,3-dihydrobenzo[b][1,4]dioxine 2.1 par le (R)-(7-chloro-5-fluoro-2,3-dihydrobenzo[b] [1,4]dioxin-2-yl)méthyl 4-bromobenzenesulfonate 2.20, la 3-(2- (pipéridin-4-yl)éthyl) oxazolidin-2-one 3.1 par la 3-(3-(pipéridin-4-yl)propyl)oxazolidin-2-one 3.30, le couple K₂CO₃/KI par la N,N'-diisopropyléthylamine (1.4 eq), on obtient le composé 104 sous forme d'huile claire, après purification par flash chromatographie sur gel de silice (éluant : dichlorométhane/méthanol = 95/5%).
Rendement : 67%
¹H RMN (CDCl₃) δ : 1,26 (s, 4H) ; 1,56-1.65 (m, 5H) ; 2,1 (m, 2H) ; 2,65 (m, 2H) ; 2,75 (m, 1H) ; 2.9 (dd, 2H, J = 8 et 5 Hz) ; 3,25 (t, 2H, J = 7 Hz) ; 3,55 (t, 2H, J = 7 Hz) ; 3,97 (m, 1H) ; 4,35 (m, 4H) ; 6,70 (2d, 2H).

On dissout 0.190 g (0.46 mmol) du composé 104 dans 5 ml d'éthanol, puis on ajoute 0,041 g (0.46 mmol) d'acide oxalique en solution dans 5 ml d'ethanol. On concentre puis on ajoute un peu d'éther, on triture le milieu et on obtient 0.2 g (0.394 mmol) de l'oxalate du composé 104 sous la forme d'un solide blanc.
F = 160°C ¹H RMN (DMSOd₆) δ : 1.30-1.45 (m, 5H) ; 1,92 (m, 4H) ; 3. (m, 2H) ; 3,17 (t, 2H, J = 6 Hz) ; 3,42 (m, 3H) ; 3.49 (t, 2H, J = 8 Hz) ; 3,64 (m, 1H) ; 4,16 (dd, 1H) ; 4,25 (t, 2H, J = 8 Hz) ; 4,40 (m, 1H) ; 4,97 (m, 1H) ; 6,97 (m, 1H) ; 7,12 (m, 1H).
MS m/z 413 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1.03 min, pureté chimique : 99.8%.

### Exemple 105 :3-{3-[1-((S)-5,7difluoro-2,3-dihydro-benzo[b][1,4]dioxin-2-ylméthyl)-pipéridin-4-yl]-propyl}-oxazolidin-2-one

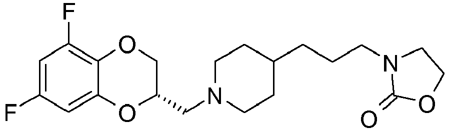

En procédant comme dans l'exemple 1, mais en remplaçant le (R)-2-(bromométhyl)-2,3-dihydrobenzo[b][1,4]dioxine 2.1 par le (R)-(5,7-difluoro-2,3-dihydrobenzo[b][1,4] dioxin-2-yl)méthyl 4-bromobenzenesulfonate 2.19, la 3-(2-(pipéridin-4-yl)éthyl) oxazolidin-2-one 3.1 par la 3-(3-(pipéridin-4-yl)propyl)oxazolidin-2-one 3.30, le couple K₂CO₃/KI par la N,N'-diisopropyléthylamine (1.4 eq), on obtient le composé 105 sous forme d'huile claire, après purification par flash chromatographie sur gel de silice (éluant : dichlorométhane/méthanol = 95/5%).
Rendement : 96%
¹H RMN (CDCl₃) δ : 1,25 (s, 4H) ; 1,55-1.60 (m, 5H) ; 2,1 (m, 2H) ; 2,65 (m, 2H) ; 2,80 (d, 1H, J = 8 Hz) ; 2.95 (d, 1H, J = 8 Hz) ; 3,30 (t, 2H, J = 7 Hz) ; 3,55 (t, 2H, J = 7 Hz) ; 4 (m, 1H) 4,33 (m, 4H) ; 6,45 (2d, 2H).

On dissout 0.270 g (0.68 mmol) du composé 105 dans 5 ml d'éthanol, puis on ajoute 061 g (0.68 mmol) d'acide oxalique en solution dans 5 ml d'éthanol. On concentre puis on ajoute un peu d'éther, on triture le milieu et on obtient 0.19 g (0.39 mmol) de l'oxalate du composé 105 sous la forme d'un solide blanc.
F = 184°C ¹H RMN (DMSOd₆) δ : 1.35-1.47 (m, 5H) ; 1,92 (m, 4H) ; 3,1 (m, 2H) ; 3,20 (m, 2H) ; 3,40 (m, 3H) ; 3,552 (t, 2H J =8 Hz) ; 3,65 (m, 1H) ; 4,13 (dd, 1H, J = 10 et 5 Hz) ; 4,25 (t, 2H, J = 8 Hz) ; 4,38 (m, 1H) ; 4,97 (m, 1H) ; 6,79 (m, 1H) ; 6,95 (m, 1H) ; 10 (s, 1H).
MS m/z 397 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1.49 min, pureté chimique : 99.6%.

### Exemple 106 :3-{3-[1-((S)-7-fluoro-2,3-dihydro-benzo[b][1,4]dioxin-2-ylméthyl)-pipéridin-4-yl]-propyl}-oxazolidin-2-one

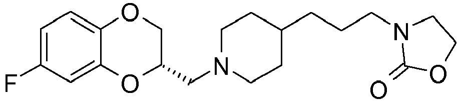

En procédant comme dans l'exemple 1, mais en remplaçant le (R)-2-(bromométhyl)-2,3-dihydrobenzo[b][1,4]dioxine 2.1 par le (R)-(7-fluoro-2,3-dihydrobenzo[b][1,4] dioxin-2-yl)méthyl 4-bromobenzenesulfonate , 2.17 la 3-(2-(pipéridin-4-yl)éthyl) oxazolidin-2-one 3.1 par la 3-(3-(pipéridin-4-yl)propyl)oxazolidin-2-one 3.30, le couple K₂CO₃/KI par la N,N'-diisopropyléthylamine (1.4 eq), on obtient le composé 106 sous forme d'huile claire, après purification par flash chromatographie sur gel de silice (éluant : dichlorométhane/méthanol = 95/5%).
Rendement : 60%
¹H RMN (CDCl₃) δ : 1,24 (s, 4H) ; 1,52-1.60 (m, 5H) ; 2,05 (m, 2H) ; 2,65 (m, 2H) ; 2,88 (d, 1H, J = 5 Hz) ; 3 (d, 1H, J = 5 Hz) ; 3,24 (t, 2H, J = 7.5 Hz) ; 3,55 (t, 2H, J = 8 Hz) ; 3,97 (m, 1H) ; 4,33 (m, 4H) ; 6,55 (1d, 1H, J = 6 Hz) ; 6,77 (1d, 1H, J = 6 Hz) ; 6.80 (s, 1H).

On dissout 0.17 g (0.449 mmol) du composé 106 dans 5 ml d'éthanol, puis on ajoute 0,04 g (0.449 mmol) d'acide oxalique en solution dans 5 ml d'éthanol. On concentre puis on ajoute un peu d'éther, on triture le milieu et on obtient 0.17 g (0.36 mmol) de l'oxalate du composé 106 sous la forme d'un solide blanc.
F = 136°C ¹H RMN (DMSOd₆) δ : 1,45 (m, 5H) ; 1,92 (m, 4H) ; 3,01 (m, 2H) ; 3,20 (m, 2H) ; 3,35 (m, 3H) ; 3,52 (t, 2H,) ; 3,65 (m, 1H) ; 4,05 (dd, 1H, J = 10 et 7 Hz) ; 4,23 (t, 2H, J = 8 Hz) ; 4,25 (m, 1H) ; 4.67 (m, 1H) ; 6,72 (m, 1H) ; 6,85 (m, 1H) ; 6,93 (m, 1H) ; 10,2 (1s, 1H).
MS m/z 379 (M+1)
HPLC (Acquity BEH C8, Phase A : 100% H₂O. HCOONH₄ 0.01M PH4, Phase B : 90% Acétonitrile 10% H2O.HCOONH 0.01M PH4, gradient B : 35 à 60% sur 5 min, 0,75 ml/min), temps de rétention = 1,28 min, pureté chimique : 99.4%.

### Exemple 107 :(S)-3-{4-[3-((S)-4-benzyl-2-oxo-oxazolidin-3-yl)-propyl]-pipéridin-1-yl méthyl}-2,3-dihydrobenzo[b][1,4]dioxine-6-carbonitrile

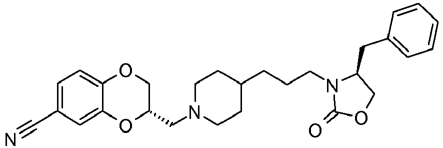

En procédant comme dans l'exemple 1, mais en remplaçant le (R)-2-(bromométhyl)-2,3-dihydrobenzo[b][1,4]dioxine 2.1 par le (R)-(7-cyano-2,3-dihydrobenzo[b][1,4] dioxin-2-yl)méthyl 4-bromobenzenesulfonate 2.11, la 3-(2-(pipéridin-4-yl)éthyl) oxazolidin-2-one 3.1 par la (S)-4-benzyl-3-(3-(pipéridin-4-yl)propyl)oxazolidin-2-one 3.31, le couple K₂CO₃/KI par la N,N'-diisopropyléthylamine (1.4 eq), on obtient le composé 107 sous forme d'huile claire, après purification par flash chromatographie sur gel de silice (éluant : dichlorométhane/méthanol = 95/5%).
Rendement : 43%
¹H RMN (CDCl₃) δ : 1,25 (m, 5H) ; 1.50 (m, 4H) ; 2,1 (m, 2H,) ; 2.55 (dd, 1H) ; 2.65 (dd, 1H) ; 2.7-3 (m, 3H) ; 3,4 (m, 1H) ; 3.47 (m, 2H) ; 4-4.15 (m, 2H) ; 4,3 (m, 1Hz) ; 4,35 (dd, 1H, J= 4 et 7 Hz) ; 4.55 (dd, 1H, J = 4 et 7 Hz) ; 4.7 (t, 1H); 6,91 (d, 1H, J = 7 Hz) ; 7,15 (m, 4H) ; 7,3 (m, 3H) ; 7.41 (m, 5H).

On dissout 0.2 g (0.42 mmol) du composé 107 dans 5 ml d'éthanol, puis on ajoute 0.038 g (0.42 mmol) d'acide oxalique en solution dans 5 ml d'éthanol. On concentre puis on ajoute un peu d'acétone, on triture le milieu et on obtient 0.196 g (0.35 mmol) de l'oxalate du composé 107 sous la forme d'un solide blanc.
F = 160°C
¹H RMN (DMSOd₆) δ : 1,44 (m, 5H) ; 1,92 (m, 4H) ; 2,51 (m, 2H) ; 2,72 (m, 1H) ; 3,08 (m, 4H) ; 3,47 (m, 2H) ; 3,66 (m, 1H) ; 3,97 (m, 1H) ; 4,14 (m, 3H) ; 4,44 (m, 1H) ; 4,97 (m, 1H) ; 7,08 (d, 1H, J = 8 Hz) ; 7,28-7.36 (m, 5H) ; 7,47 (d, 1H, J = 2 Hz) ; 10,2 (1s, 1H).
MS m/z 476 (M+1)
HPLC (Acquity BEH C8, Phase A : 100% H₂O. HCOONH₄ 0.01M PH4, Phase B : 90% Acétonitrile 10% H2O.HCOONH 0.01M PH4, gradient B : 35 à 60% sur 5 min, 0,75 ml/min), temps de rétention = 1,72 min, pureté chimique : 99.94%.

### Exemple 108 :3-{3-[1-((S)-5-chloro-7-fluoro-2,3-dihydro-benzo[b][1,4]dioxin-2-yl méthyl)-pipéridin-4-yl]-propyl}-oxazolidin-2-one

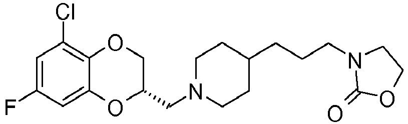

En procédant comme dans l'exemple 1, mais en remplaçant le (R)-2-(bromométhyl)-2,3-dihydrobenzo[b][1,4]dioxine 2.1 par le (R)-(5-chloro-7-fluoro-2,3-dihydrobenzo[b] [1,4]dioxin-2-yl)méthyl 4-bromobenzenesulfonate 2.21, la 3-(2-(pipéridin-4-yl)éthyl) oxazolidin-2-one 3.1 par la 3-(3-(pipéridin-4-yl)propyl)oxazolidin-2-one 3.30, le couple K₂CO₃/KI par la N,N'-diisopropyléthylamine (1.4 eq), on obtient le composé 108 sous forme d'huile claire, après purification par flash chromatographie sur gel de silice (éluant : dichlorométhane/méthanol = 95/5%).
Rendement : 38%
¹H RMN (CDCl₃) δ : 1,24 (m, 4H) ; 1,56-1.67 (m, 5H) ; 2,05 (m, 2H) ; 2,60 (m, 2H) ; 2,75 (m, 1H) ; 2.9 (dd, 2H, J = 8 et 5 Hz) ; 3,25 (t, 2H, J = 7 Hz) ; 3,56 (t, 2H, J = 7 Hz) ; 3,97 (m, 1H) ; 4,35 (m, 4H) ; 6,56 (d, 1H, J = 6 Hz) ; 6.7 (d, 1H, J = 6 Hz).

On dissout 0.12 g (0.29 mmol) du composé 108 dans 5 ml d'éthanol, puis on ajoute 0,026 g (0.29 mmol) d'acide oxalique en solution dans 5 ml d'éthanol. On concentre puis on ajoute un peu d'éther, on triture le milieu et on obtient 0.12 g (0.24 mmol) de l'oxalate du composé 108 sous la forme d'un solide blanc.
F = 108°C ¹H RMN (DMSOd₆) δ : 1.35-1.50 (m, 5H) ; 1,91 (m, 4H) ; 3 (m, 2H) ; 3,20 (t, 2H) ; 3,40 (m, 3H) ; 3,50 (t, 2H, J = 8 Hz) ; 3,60 (m, 1H) ; 4,20 (dd, 1H, J = 12 et 6 Hz) ; 4,25 (t, 2H, J = 8 Hz) ; 4,41 (m, 1H) ; 4,92 (m, 1H) ; 6.88 (d, 1H, J = 5 Hz) ; 7.04 (d, 1H, J = 5 Hz).
MS m/z 413 (M+1)
HPLC (XBridge BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1.08 min, pureté chimique : 98.99%.

### Exemple 109 3-{3-[1-((S)-7-methanesulfonyl-2,3-dihydro-benzo[b][1,4]dioxin-2-yl méthyl)-pipéridin-4-yl]-propyl}-oxazolidin-2-one

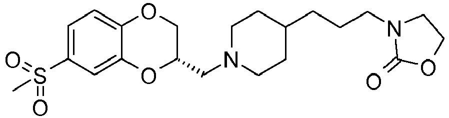

En procédant comme dans l'exemple 1, mais en remplaçant le (R)-2-(bromométhyl)-2,3-dihydrobenzo[b][1,4]dioxine 2.1 par le (R)-(7-(méthylsulfonyl)-2,3-dihydrobenzo [b][1,4]dioxin-2-yl)méthyl 4-bromobenzenesulfonate 2.23, la 3-(2-(pipéridin-4-yl)éthyl)oxazolidin-2-one 3.1 par la 3-(3-(pipéridin-4-yl)propyl)oxazolidin-2-one 3.30, le couple K₂CO₃/KI par la N,N'-diisopropyléthylamine (1.4 eq), on obtient le composé 109 sous forme d'huile claire, après purification par flash chromatographie sur gel de silice (éluant : dichlorométhane/méthanol = 95/5%).
Rendement : 58%
¹H RMN (CDCl₃) δ : 1,26 (s, 4H) ; 1,52-1.60 (m, 5H) ; 2,05 (m, 2H) ; 2,65 (m, 2H) ; 2,88 (d, 1H, J = 5 Hz) ; 3 (d, 1H, J = 5 Hz) ; 3,24 (t, 2H, J = 7.5 Hz) ; 3,55 (t, 2H, J = 8 Hz) ; 3,97 (m, 1H) ; 4,33 (m, 4H) ; 6,54 (1d, 1H, J = 6 Hz) ; 6,75 (1d, 1H, J = 6 Hz) ; 6.77 (s, 1H).

On dissout 0.16 g (0.365 mmol) du composé 109 dans 5 ml d'éthanol, puis on ajoute 0.033 g (0.365 mmol) d'acide oxalique en solution dans 5 ml d'éthanol. On concentre puis on ajoute un peu d'acétone, on triture le milieu et on obtient 0.125 g (1,99 mmol) de l'oxalate du composé 109 sous la forme d'un solide blanc.
F = 127°C ¹H RMN (DMSOd₆) δ : 1,27-1.40 (m, 5H) ; 1,85 (m, 2H) ; 2,70 (m, 2H) ; 3,06 (1s, 2H) ; 3,20 (t, 2H, J = 7.5 Hz) ; 3,30 (m, 2H) ; 3,51 (t, 2H, J = 7 Hz) ; 4,12 (m, 1H) ; 4,22 (m, 2H) ; 4,48 (m, 1H) ; 4,74 (1s, 1H) ; 7,14 (d, 1H, J = 2 Hz) ; 7,45 (m, 2H).
MS m/z 439 (M+1)
HPLC (XBridge BEH C8, Phase A : 100% H₂O. HCOONH₄ 0.01M PH4, Phase B : 90% Acétonitrile 10% H2O.HCOONH 0.01M PH4, gradient B : 35 à 60% sur 5 min, 0,75 ml/min), temps de rétention = 1.44 min, pureté chimique : 99.26%.

### Exemple 110 :(S)-8-fluoro-3-{4-[3-(2-oxo-oxazolidin-3-yl)-propyl]-pipéridin-1-yl méthyl}-2,3-dihydrobenzo[b][1,4]dioxine-6-carbonitrile

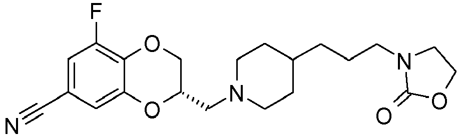

En procédant comme dans l'exemple 1, mais en remplaçant le (R)-2-(bromométhyl)-2,3-dihydrobenzo[b][1,4]dioxine 2.1 par (R)-(7-cyano-5-fluoro-2,3-dihydrobenzo[b] [1,4]dioxin-2-yl) méthyl-4-bromobenzene sulfonate 2.26, la 3-(2-(pipéridin-4-yl)éthyl) oxazolidin-2-one 3.1 par la 3-(3-(pipéridin-4-yl)propyl)oxazolidin-2-one 3.30, le couple K₂CO₃/KI par la N,N'-diisopropyléthylamine (1.4 eq), on obtient le composé 110 sous forme d'huile claire, après purification par flash chromatographie sur gel de silice (éluant : dichlorométhane/méthanol = 95/5%).
Rendement : 47%
¹H RMN (CDCl₃) δ : 1,25 (s, 4H) ; 1,55-1.65 (m, 5H) ; 2,05 (m, 2H) ; 2,60 (m, 2H) ; 2,80 (m, 1H) ; 2.9 (dd, 2H, J = 7 et 5 Hz) ; 3,25 (t, 2H, J = 7 Hz) ; 3,55 (t, 2H, J = 7 Hz) ; 4 (m, 1H) ; 4,30 (m, 4H) ; 6,98 (2d, 2H).

On dissout 0.110 g (0.273 mmol) du composé 110 dans 5 ml d'éthanol, puis on ajoute 0.025 g (0.273 mmol) d'acide oxalique en solution dans 5 ml d'ethanol. On concentre puis on ajoute un peu d'éther, on triture le milieu et on obtient 0.16 g (0.235 mmol) de l'oxalate du composé 110 sous la forme d'un solide blanc.
F = 171°C ¹H RMN (DMSOd₆) δ : 1,20-1.40 (m, 5H) ; 1,80 (m, 2H) ; 2,60 (m, 2H) ; 3,06 (1s, 2H) ; 3,20 (t, 2H, J = 6,8 Hz) ; 3,30 (m, 2H) ; 3,51 (t, 2H, J = 7 Hz) ; 4,12 (m, 1H) ; 4,22 (m, 2H) ; 4,48 (m, 1H) ; 4,75 (1s, 1H) ; 7,37 (d, 1H, J = 2 Hz) ; 7,51 (d, 1H, J = 2 Hz).
MS m/z 404 (M+1)
HPLC (Acquity BEH C8, Phase A : 100% H₂O. HCOONH₄ 0.01M PH4, Phase B : 90% Acétonitrile 10% H2O.HCOONH4 0.01M PH4, gradient B : 35 à 60% sur 5 min, 0,75 ml/min), temps de rétention = 1,12 min, pureté chimique : 97%.

### Partie « benzodioxane »

Les structures de type (2,3-dihydrobenzo[b][1,4]dioxin-2-yl)methanol substituées ou non sur le noyau aromatique et optiquement pures sont préparées suivant deux méthodes, selon que l'on utilise des 2-hydroxybenzaldehydes comme matière première *(*J. Med. Chem. ; 1997 ; 40 ; 4235-4256*:* méthode A) ou des 2-bromophenols (J. Am. Chem. Soc. ; 2001 ; 123 ; 12202-12206 : méthode B). Les méthodes A ou B sont choisies suivant les disponibilités commerciales ou l'accessibilité synthétique des matières premières.

### Méthode A : J. Med. Chem. ; 1997 ; 40 ; 4235-4256

### Intermédiaire 4.1 : (S)-(2,3-dihydrobenzo[b][1,4]dioxin-2-yl)methanol

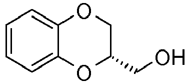

¹H RMN (CDCl₃) δ : 1,94 (t, 1H, J = 6 Hz) ; 3,88 (m, 2H) ; 4,11 (m, 1H) ; 4,30 (m, 2H) ; 6,86 (m, 4H).

### Intermédiaire 4.2 : (R)-(2,3-dihydrobenzo[b][1,4]dioxin-2-yl)methanol

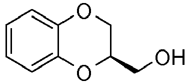

¹H RMN (CDCl₃) δ : cf RMN intermédiaire 4.1

### Intermédiaire 4.3 : (S)-(7-méthyl-2,3-dihydrobenzo[b][1,4]dioxin-2-yl)methanol

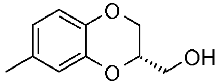

¹H RMN (CDCl₃) δ: 1,94 (t, 1H, J = 6,4 Hz) ; 2,25 (s, 3H) ; 3,86 (m, 2H) ; 4,10 (m, 1H) ; 4,23 (m, 2H) ; 6,65 (dd, 1H, J = 8 et 1,6 Hz) ; 6,71 (d, 1H, J = 1,6 Hz) ; 6,77 (d, 1H, J = 8 Hz).

### Intermédiaire 4.4 : (S)-(7-(trifluorométhyl)-2,3-dihydrobenzo[b][1,4]dioxin-2-yl) methanol

Le 2-hydroxy-5-(trifluorométhyl)benzaldehyde n'est pas commercial, il est synthétisé selon la méthode rapportée dans Bioorg. & Med. Chem. Lett. ; 2006 ; Vol 16 ; 14 ; 3657-3662*.*

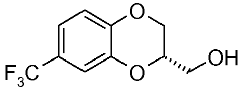

¹H RMN (CDCl₃) δ : 1,88 (t, 1H, J = 6,4 Hz) ; 3,86 (m, 1H) ; 3,94 (m, 1H) ; 4,16 (m, 1H) ; 4,26 (m, 1H) ; 4,36 (m, 1H) ; 6,96 (d, 1H, J = 8,8 Hz) ; 7,12 (dd, 1H, J = 8,8 et 2 Hz) ; 7,17 (d, 1H, J = 2Hz).

### Intermédiaire 4.5 : (S)-(7-chloro-2,3-dihydrobenzo[b][1,4]dioxin-2-yl)methanol

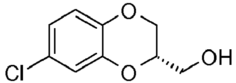

¹H RMN (CDCl₃) δ: 1,94 (t, 1H, J = 6,4 Hz) ; 3,87 (m, 2H) ; 4,11 (m, 1H) ; 4,24 (m, 2H) ; 6,81 (m, 2H) ; 6,90 (m, 1H).

### Intermédiaire 4.6 : (S)-(7-fluoro-2,3-dihydrobenzo[b][1,4]dioxin-2-yl)methanol

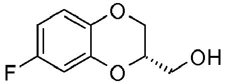

¹H RMN (CDCl₃) δ: 1,93 (t, 1H, J = 6,4 Hz) ; 3,87 (m, 2H) ; 4,08 (dd, 1H, J = 11,8 et 8 Hz) ; 4,27 (m, 2H) ; 6,56 (m, 1H) ; 6,64 (m, 1H) ; 6,81 (m, 1H).

### Intermédiaire 4.7 : (S)-(8-bromo-2,3-dihydrobenzo[b][1,4]dioxin-2-yl)methanol

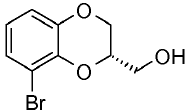

¹H RMN (CDCl₃) δ: 2,14 (t, 1H, J = 6,4 Hz) ; 3,88 (m, 1H) ; 3,97 (m, 1H) ; 4,13 (m, 1H) ; 4,32 (m, 2H) ; 6,73 (m, 1H) ; 6,85 (dd, 1H, J = 8,2 et 1,6 Hz) ; 7,11 (dd, 1H, J = 8 et 1,6 Hz).

### Intermédiaire 4.8 : (S)-(7-bromo-2,3-dihydrobenzo[b][1,4]dioxin-2-yl)methanol

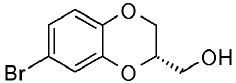

¹H RMN (CDCl₃) δ: 1,93 (t, 1H, J = 6,4 Hz) ; 3,87 (m, 2H) ; 4,09 (dd, 1H, J = 11 et 8 Hz) ; 4,27 (m, 2H) ; 6,75 (d, 1H, J = 8,4 Hz) ; 6,95 (d, 1H, J = 8,4 Hz) ; 7,05 (s, 1H).

### Intermédiaire 4.9 : (S)-(6-bromo-2,3-dihydrobenzo[b][1,4]dioxin-2-yl)methanol

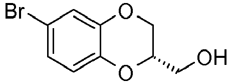

¹H RMN (CDCl₃) δ: 1,91 (t, 1H, J = 6,4 Hz) ; 3,87 (m, 2H) ; 4,10 (dd, 1H, J = 11,2 et 7,6 Hz) ; 4,23 (m, 1H) ; 4,29 (m, 1H) ; 6,77 (d, 1H, J = 8,4 Hz) ; 6,96 (dd, 1H, J = 8,4 et 2,4 Hz) ; 7,03 (d, 1H, J= 2,4 Hz).

### Intermédiaire 4.10 : (S)-3-(hydroxyméthyl)-2,3-dihydrobenzo[b][1,4]dioxine-6-carbonitrile

Produit préparé à partir de l'intermédiaire 4.7, le (S)-(7-bromo-2,3-dihydrobenzo [b][1,4]dioxin-2-yl)methanol, précedemment décrit, selon la méthode rapportée dans Tetrahedron ; 2008 ; 64 ; 10802-10809*.*

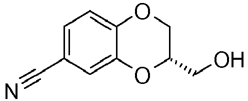

¹H RMN (CDCl₃) δ: 1,88 (t, 1H, J = 6 Hz) ; 3,91 (m, 2H) ; 4,18 (dd, 1H, J = 11,4 et 7,6 Hz) ; 4,26 (m, 1H) ; 4,39 (dd, 1H, J = 11,4 et 2,4 Hz) ; 6,94 (d, 1H, J = 8,4 Hz) ; 7,17 (dd, 1H, J = 8,4 et 2 Hz) ; 7,19 (d, 1H, J = 2 Hz).

### Intermédiaire 4.11 : (S)-3-(hydroxyméthyl)-2,3-dihydrobenzo[b][1,4]dioxine-5-carbonitrile

Produit préparé à partir de l'intermédiaire 4.6, le (S)-(8-bromo-2,3-dihydrobenzo[b][1,4]dioxin-2-yl)methanol, précedemment décrit, selon la méthode rapportée dans Tetrahedron ; 2008 ; 64 ; 10802-10809*.*

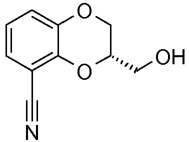

¹H RMN (CDCl₃) δ : 2,25 (t, 1H, J = 6,4 Hz) ; 3,91 (m, 1H) ; 4,00 (m, 1H) ; 4,18 (dd, 1H, J = 12 et 8,4 Hz) ; 4,36 (m, 2H) ; 6,90 (m, 1H) ; 7,10 (dd, 1H, J = 8,4 et 1,6 Hz) ; 7,15 (dd, 1H, J = 7,8 et 1,6 Hz).

### Intermédiaire 4.12 : (S)-(7-(benzyloxy)-2,3-dihydrobenzo[b][1,4]dioxin-2-yl) methanol

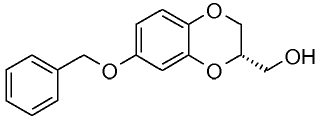

¹H RMN (CDCl₃) δ : 1,89 (t, 1H, J = 6,4 Hz) ; 3,86 (m, 2H) ; 4,06 (m, 1H) ; 4,25 (m, 2H) ; 4,98 (s, 2H) ; 6,50 (dd, 1H, J = 8,8 et 2,8 Hz) ; 6,56 (d, 1H, J = 2,8 Hz) ; 6,79 (d, 1H, J = 8,8 Hz) ; 7,36 (m, 5H).

### Intermédiaire 4.13 : (S)-(6-(benzyloxy)-2,3-dihydrobenzo[b][1,4]dioxin-2-yl) methanol

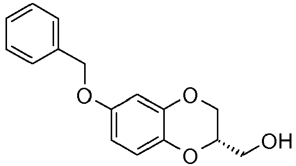

¹H RMN (CDCl₃) δ : 1,90 (t, 1H, J = 6,4 Hz) ; 3,85 (m, 2H) ; 4,09 (m, 1H) ; 4,20 (m, 1H) ; 4,29 (m, 1H) ; 4,98 (s, 2H) ; 6,52 (m, 2H) ; 6,80 (d, 1H, J = 8,8 Hz) ; 7,36 (m, 5H).

### Intermédiaire 4.14 : (S)-(7-(trifluoromethoxy)-2,3-dihydrobenzo[b][1,4]dioxin-2-yl) methanol

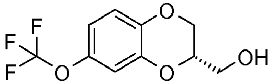

¹H RMN (CDCl₃) δ : 1,88 (t, 1H, J = 6,4 Hz) ; 3,89 (m, 2H) ; 4,11 (dd, 1H, J = 11 et 7,2 Hz) ; 4,26 (m, 1H) ; 4,31 (dd, 1H, J = 11 et 2,4 Hz) ; 6,73 (m, 1H) ; 6,81 (d, 1H, J = 2 Hz) ; 6,87 (d, 1H, J = 8,8 Hz).

### Intermédiaire 4.15 : (S)-(6-bromo-7-methoxy-2,3-dihydrobenzo[b][1,4]dioxin-2-yl) methanol

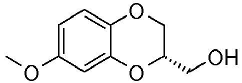

¹H RMN (CDCl₃) δ : 1,93 (t, 1H, J = 6,4 Hz) ; 3,74 (s, 3H) ; 3,86 (m, 2H) ; 4,06 (dd, 1H, J = 11,8 et 8 Hz) ; 4,27 (m, 2H) ; 6,43 (dd, 1H, J = 8,8 et 2,8 Hz) ; 6,49 (d, 1H, J = 2,8 Hz) ; 6,80 (d, 1H, J = 8,8 Hz).

### Méthode B : J. Am. Chem. Soc. ; 2001 ; 123 ; 12202-12206

### Intermédiaire 4.16 : (S)-(5-bromo-2,3-dihydrobenzo[b][1,4]dioxin-2-yl)methanol

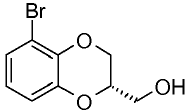

¹H RMN (CDCl₃) δ : 1,87 (t, 1H, J = 6,4 Hz) ; 3,85 (m, 1H) ; 3,94 (m, 1H) ; 4,19 (dd, 1H, J = 11,2 et 8 Hz) ; 4,27 (m, 1H) ; 4,43 (dd, 1H, J = 11,2 et 2 Hz) ; 6,74 (m, 1H) ; 6,87 (dd, 1H, J = 8,4 et 1,6 Hz) ; 7,11 (dd, 1H, J = 8 et 2 Hz).

### Intermédiaire 4.17 : (S)-(8-fluoro-2,3-dihydrobenzo[b][1,4]dioxin-2-yl)methanol

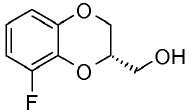

¹H RMN (DMSOd₆) δ : 3,66 (m, 2H) ; 4,05 (dd, 1H, J = 11,6 et 7,2 Hz) ; 4,20 (m, 1H) ; 4,38 (m, 1H) ; 5,12 (t, 1H, J = 6 Hz) ; 6,72 (m, 1H) ; 6,79 (m, 2H).

### Intermédiaire 4.18 : (S)-(6-fluoro-2,3-dihydrobenzo[b][1,4]dioxin-2-yl)methanol

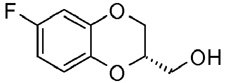

¹H RMN (DMSOd₆) δ : 3,61 (m, 2H) ; 4,00 (dd, 1H, J = 11,2 et 7,6 Hz) ; 4,11 (m, 1H) ; 4,32 (m, 1H) ; 5,07 (t, 1H, J = 6 Hz) ; 6,67 (m, 1H) ; 6,77 (m, 1H) ; 6,87 (m, 1H).

### Intermédiaire 4.19 : (S)-(5,7-difluoro-2,3-dihydrobenzo[b][1,4]dioxin-2-yl)methanol

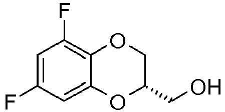

¹H RMN (DMSOd₆) δ : 3,64 (m, 2H) ; 4,03 (dd, 1H, J = 11,4 et 7,2 Hz) ; 4,25 (m, 1H) ; 4,39 (dd, 1H, J = 11,4 et 2,4 Hz) ; 5,13 (t, 1H, J = 5,6 Hz) ; 6,69 (m, 1H) ; 6,85 (m, 1H).

### Intermédiaire 4.20 : (S)-(7-chloro-5-fluoro-2,3-dihydrobenzo[b][1,4]dioxin-2-yl) methanol

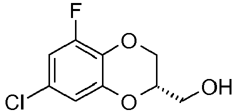

¹H RMN (DMSOd₆) δ : 3,64 (m, 2H) ; 4,07 (dd, 1H, J = 11,6 et 7,2 Hz) ; 4,25 (m, 1H) ; 4,42 (dd, 1H, J = 11,6 et 2,4 Hz) ; 5,14 (t, 1H, J = 5,6 Hz) ; 6,86 (m, 1H) ; 7,01 (m, 1H).

### Intermédiaire 4.21 : (S)-(5-chloro-7-fluoro-2,3-dihydrobenzo[b][1,4]dioxin-2-yl) methanol

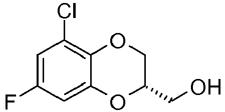

¹H RMN (DMSOd₆) δ : 3,63 (m, 2H) ; 4,07 (dd, 1H, J = 11,4 et 7,6 Hz) ; 2,23 (m, 1H) ; 4,43 (dd, 1H, J = 11,4 et 2,4 Hz) ; 5,14 (t, 1H, J = 5,6 Hz) ; 6,84 (m, 1H) ; 6,98 (m, 1H).

### Intermédiaire 4.22 : (S)-(6,7-difluoro-2,3-dihydrobenzo[b][1,4]dioxin-2-yl)methanol

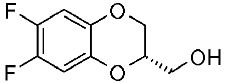

¹H RMN (DMSOd₆) δ : 3,61 (m, 2H) ; 3,49 (dd, 1H, J = 11,4 et 7,2 Hz) ; 4,15 (m, 1H) ; 4,33 (dd, 1H, J = 11,4 et 2,4 Hz) ; 5,09 (t, 1H, J = 5,6 Hz) ; 7,04 (m, 2H).

### Intermédiaire 4.23 : (S)-(7-(méthylsulfonyl)-2,3-dihydrobenzo[b][1,4]dioxin-2-yl) methanol

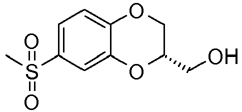

¹H RMN (DMSOd₆) δ : 3,16 (m, 2H) ; 4,12 (dd, 1H, J = 11,4 et 7,6 Hz) ; 4,24 (m, 1H) ; 4,43 (dd, 1H, J = 11,4 et 2 Hz) ; 5,13 (t, 1H, J = 5,6 Hz) ; 7,11 (m, 1H) ; 7,37 (m, 2H).

### Intermédiaire 4.24 : (S)-méthyl 3-(hydroxyméthyl)-2,3-dihydrobenzo[b][1,4]dioxine-6-carboxylate

On utilise l'acide 3-bromo-4-hydroxybenzoique commercial, à partir duquel on prépare l'amine de Weinreb. Cette amine est transformée en ester au niveau de la déprotection du (R)-3-bromo-4-((2,2-diméthyl-1,3-dioxolan-4-yl)methoxy)-N-methoxy-N-méthylbenzamide (cf méthode B) par un traitement en milieu acide.

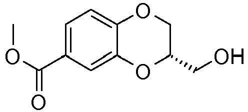

¹H RMN (DMSOd₆) δ : 3,64 (m, 2H) ; 3,80 (s, 3H) ; 4,09 (dd, 1H, J = 11,4 et 7,2 Hz) ; 4,20 (m, 1H) ; 4,41 (dd, 1H, J = 11,4 et 2 Hz) ; 5,10 (t, 1H, J = 5,6 Hz) ; 6,98 (d, 1H, J = 8,4 Hz) ; 7,40 (d, 1H, J = 2 Hz) ; 7,46 (dd, 1H, J = 8,4 et 2 Hz).

### Intermédiaire 4.25 : (S)-2-(hydroxyméthyl)-2,3-dihydrobenzo[b][1,4]dioxine-5-carbonitrile

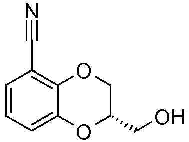

¹H RMN (DMSOd₆) δ : 3,65 (m, 2H) ; 4,17 (dd, 1H, J = 11,4 et 7,6 Hz) ; 4,25 (m, 1H) ; 4,52 (dd, 1H, J = 11,4 et 2 Hz) ; 5,15 (t, 1H, J = 5,6 Hz) ; 6,98 (m, 1H) ; 7,22 (dd, 1H, J = 8 et 1,6 Hz) ; 7,28 (dd, 1H, J = 8 et 1,6 Hz).

### Intermédiaire 4.26 : (R)-3-(hydroxyméthyl)-2,3-dihydrobenzo[b][1,4]dioxine-6-carbonitrile

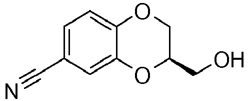

¹H RMN (CDCl₃) δ : cf RMN de l'intermédiaire 4.10

### Intermédiaire 4.27 : (S)-8-fluoro-3-hydroxyméthyl-2,3-dihydrobenzo[b][1,4]dioxine-6-carbonitrile

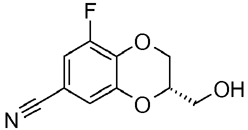

¹H RMN (CDCl₃) δ : 1,89 (s, 1H, OH) ; 3,96 (m, 2H) ; 4,18 (m, 1H) ; 4,28 (m, 1H) ; 4,47 (m, 1H) ; 7.02 (m, 2H)

Les intermédiaires halogénées, mésylates ou brosylates de type 2 sont préparées à partir des intermédiaires (2,3-dihydrobenzo[b][1,4]dioxin-2-yl)methanol précedemment décrits, selon le schéma 2.

### Dérivés bromés : préparation de manière classique NBS/PPh₃/DMF (Eur. J. Med. Chem. ; 2000 ; 35 ; 663-676)

### Intermédiaire 2.1 : (R)-2-(bromométhyl)-2,3-dihydrobenzo[b][1,4]dioxine

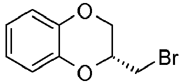

¹H RMN (DMSOd₆) δ : 3,70 (dd, 1H, J = 11 et 6 Hz) ; 3,79 (dd, 1H, J = 11 et 4,4 Hz) ; 4,03 (dd, 1H, J = 11,2 et 6,4 Hz) ; 4,37 (dd, 1H, J = 11,2 et 2,4 Hz) ; 4,42 (m, 1H) ; 6,87 (m, 4H).

### Intermédiaire 2.2 : (S)-2-(bromométhyl)-2,3-dihydrobenzo[b][1,4]dioxine

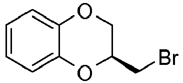

¹H RMN (CDCl₃) δ : cf RMN intermédiaire 2.1

### Intermédiaire 2.3 : (R)-2-(bromométhyl)-7-méthyl-2,3-dihydrobenzo[b][1,4]dioxine

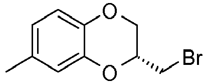

¹H RMN (CDCl₃) δ : 2,25 (s, 3H) ; 3,52 (m, 2H) ; 4,16 (dd, 1H, J = 11,4 et 5,6 Hz) ; 4,31 (dd, 1H, J = 11,4 et 2,4 Hz) ; 4,38 (m, 1H) ; 6,65 (d, 1H, J = 8 Hz) ; 6,72 (s, 1H) ; 6,77 (d, 1H, J = 8 Hz).

### Intermédiaire 2.4 : (R)-8-bromo-2-(bromométhyl)-2,3-dihydrobenzo[b][1,4]dioxine

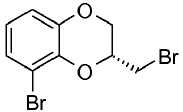

¹H RMN (CDCl₃) δ : 3,52 (m, 1H) ; 3,63 (dd, 1H, J = 10 et 4 Hz) ; 4,23 (dd, 1H, J = 11,8 et 5,2 Hz) ; 4,36 (m, 1H) ; 4,53 (m, 1H) ; 6,74 (m, 1H) ; 6,86 (d, 1H, J = 8 Hz) ; 7,12 (d, 1H, J = 8 Hz).

### Intermédiaire 2.5 : (R)-7-bromo-2-(bromométhyl)-2,3-dihydrobenzo[b][1,4]dioxine

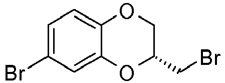

¹H RMN (CDCl₃) δ : 3,52 (m, 2H) ; 4,17 (dd, 1H, J = 11,4 et 6 Hz) ; 4,33 (dd, 1H, J = 11,4 et 2,4 Hz) ; 4,39 (m, 1H) 6,77 (d, 1H, J = 8,6 Hz) ; 6,96 (dd, 1H, J = 8,6 et 2,4 Hz) ; 7,06 (d, 1H, J = 2,4 Hz).

### Intermédiaire 2.6 : (R)-6-bromo-2-(bromométhyl)-2,3-dihydrobenzo[b][1,4]dioxine

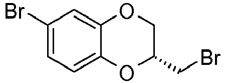

¹H RMN (CDCl₃) δ : 3,52 (m, 2H) ; 4,16 (dd, 1H, J = 11,6 et 6 Hz) ; 4,34 (dd, 1H, J = 11,6 et 2,4 Hz) ; 4,38 (m, 1H) ; 6,77 (d, 1H, J = 8,4 Hz) ; 6,97 (dd, 1H, J = 8,4 et 2,4 Hz) ; 7,04 (d, 1H, J = 2,4 Hz).

### Intermédiaire 2.7 : (R)-2-(bromométhyl)-7-chloro-2,3-dihydrobenzo[b][1,4]dioxine

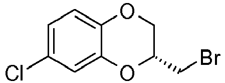

¹H RMN (CDCl₃) δ : 3,52 (m, 2H) ; 4,17 (dd, 1H, J = 11,4 et 6 Hz) ; 4,33 (dd, 1H, J = 11,4 et 2,4 Hz) ; 4,39 (m, 1H) ; 6,81 (s, 2H) ; 6,91 (m, 1H).

### Intermédiaire 2.8 : (R)-2-(bromométhyl)-7-(trifluoromethoxy)-2,3-dihydrobenzo[b] [1,4]dioxine

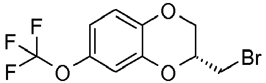

¹H RMN (CDCl₃) δ : ¹H RMN (CDCl₃) δ : 3,53 (m, 2H) ; 4,18 (dd, 1H, J = 11,6 et 6 Hz) ; 4,35 (dd, 1H, J = 11,6 et 2 Hz) ; 4,41 (m, 1H) ; 6,73 (dd, 1H, J = 9 et 2 Hz) ; 6,81 (d, 1H, J = 2 Hz) ; 6,88 (d, 1H, J = 9 Hz).

### Intermédiaire 2.9 : (R)-6-bromo-2-(bromométhyl)-7-methoxy-2,3-dihydrobenzo[b] [1,4]dioxine

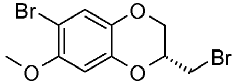

Les conditions de bromation du (S)-(6-bromo-7-methoxy-2,3-dihydrobenzo[b] [1,4]dioxin-2-yl)methanol entraînent aussi la bromation en position 6 du noyau aromatique.
¹H RMN (CDCl₃) δ : 3,53 (m, 2H) ; 3,81 (s, 3H) ; 4,12 (dd, 1H, J = 11,6 et 6 Hz) ; 4,29 (dd, 1H, J = 11,6 et 2 Hz) ; 4,38 (m, 1H) ; 6,51 (s, 1H) ; 7,09 (s, 1H).

### Dérivés brosylates : Préparation de manière classique chlorure de 4-bromobenzene-1-sulfonyl /Et₃N/DMAP/Toluène (WO2004/24731A1, page32)

### Intermédiaire 2.10 : (R)-(7-(trifluorométhyl)-2,3-dihydrobenzo[b][1,4]dioxin-2-yl) méthyl4-bromobenzenesulfonate

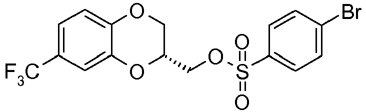

¹H RMN (CDCl₃) δ : 4,10 (dd, 1H, J = 11,8 et 6,8 Hz) ; 4,28 (m, 3H) ; 4,42 (m, 1H) ; 6,93 (d, 1H, J = 8,4 Hz) ; 7,03 (d, 1H, J = 2 Hz) ; 7,11 (dd, 1H, J = 8,4 et 2 Hz) ; 7,75 (m, 4H).

### Intermédiaire 2.11 : (R)-(7-cyano-2,3-dihydrobenzo[b][1,4]dioxin-2-yl)méthyl 4-bromobenzenesulfonate

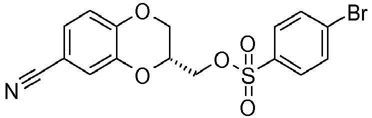

¹H RMN (CDCl₃) δ : 3,50 (dd, 1H, J = 10,8 et 7,2 Hz) ; 3,57 (dd, 1H, J = 10,8 et 4,8 Hz) ; 4,22 (m, 1H) ; 4,37 (m, 2H) ; 6,95 (d, 1H, J = 8,4 Hz) ; 7,18 (dd, 1H, J = 8,4 et 2 Hz) ; 7,21 (d, 1H, J = 2 Hz).

### Intermédiaire 2.12 : (S)-(7-cyano-2,3-dihydrobenzo[b][1,4]dioxin-2-yl)méthyl 4-bromobenzenesulfonate

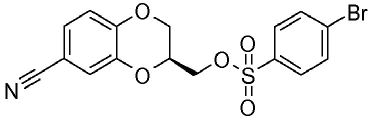

¹H RMN (CDCl₃) δ : idem intermédiaire 2.11

### Intermédiaire 2.13 : (R)-(8-cyano-2,3-dihydrobenzo[b][1,4]dioxin-2-yl)méthyl 4-bromobenzenesulfonate

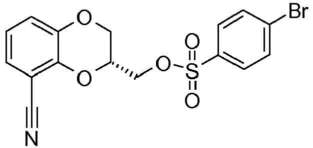

¹H RMN (CDCl₃) δ : 4,14 (dd, 1H, J = 12 et 6,8 Hz) ; 4,32 (m, 3H) ; 4,44 (m, 1H) ; 6,91 (m, 1H) ; 7,09 (dd, 1H, J = 8,2 et 1,6 Hz) ; 7,15 (dd, 1H, J = 7,6 et 1,6 Hz) ; 7,78 (m, 4H).

### Intermédiaire 2.14 : (R)-(5-cyano-2,3-dihydrobenzo[b][1,4]dioxin-2-yl)méthyl 4-bromobenzenesulfonate

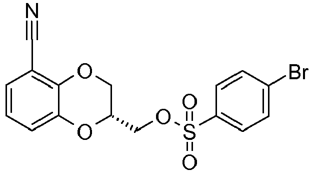

¹H RMN (DMSOd₆) δ : 4,15 (dd, 1H, J = 11,4 et 7,2 Hz) ; 4,31 (dd, 1H, J = 11,6 et 6 Hz) ; 4,47 (m, 2H) ; 4,55 (m, 1H) ; 6,98 (m, 1H) ; 7,11 (dd, 1H, J = 8,2 et 1,6 Hz) ; 7,30 (dd, 1H, J = 7,6 et 1,6 Hz) ; 7,87 (m, 4H).

### Intermédiaire 2.15 : (R)-(5-bromo-2,3-dihydrobenzo[b][1,4]dioxin-2-yl)méthyl 4-bromobenzenesulfonate

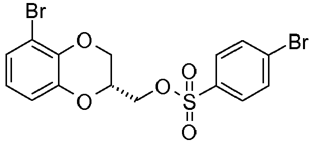

¹H RMN (CDCl₃) δ : 4,13 (dd, 1H, J = 11,6 et 2,4 Hz) ; 4,28 (m, 2H) ; 4,35 (m, 1H) ; 4,42 (m, 1H) ; 6,72 (m, 2H) ; 7,09 (m, 1H) ; 7,71 (m, 2H) ; 7,78 (m, 2H).

### Intermédiaire 2.16 : (R)-(8-fluoro-2,3-dihydrobenzo[b][1,4]dioxin-2-yl)méthyl 4-bromobenzenesulfonate

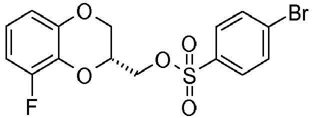

¹H RMN (DMSOd₆) δ : 4,04 (dd, 1H, J = 11,6 et 6,8 Hz) ; 4,29 (dd, 1H, J = 11,6 et 6,4 Hz) ; 4,34 (m, 1H) ; 4,45 (dd, 1H, J = 11,2 et 2,8 Hz) ; 4,55 (m, 1H) ; 6,70 (m ; 1H) ; 6,79 (m, 2H) ; 7,86 (m, 4H).

### Intermédiaire 2.17 : (R)-(7-fluoro-2,3-dihydrobenzo[b][1,4]dioxin-2-yl)méthyl 4-bromobenzenesulfonate

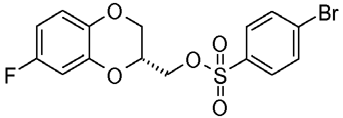

¹H RMN (CDCl₃) δ : 4,03 (dd, 1H, J = 11,6 et 6 Hz) ; 4,21 (dd, 1H, J = 9 et 2,4 Hz) ; 4,24 (m, 2H) ; 4,41 (m, 1H) ; 6,49 (m, 1H) ; 6,55 (m, 1H) ; 6,79 (m, 1H) ; 7,71 (m, 2H) ; 7,77 (m, 2H).

### Intermédiaire 2.18 : (R)-(6-fluoro-2,3-dihydrobenzo[b][1,4]dioxin-2-yl)méthyl 4-bromobenzenesulfonate

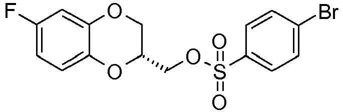

¹H RMN (DMSOd₆) δ : 3,98 (dd, 1H, J = 11,4 et 6,8 Hz) ; 4,26 (dd, 1H, J = 11,4 et 6,4 Hz) ; 4,29 (dd, 1H, J = 11,6 et 1,6 Hz) ; 4,42 (m, 2H) ; 6,67 (m, 1H) ; 6,77 (m, 2H) ; 7,85 (m, 2H) ; 7,89 (m, 2H).

### Intermédiaire 2.19 : (R)-(5,7-difluoro-2,3-dihydrobenzo[b][1,4]dioxin-2-yl)méthyl 4-bromobenzenesulfonate

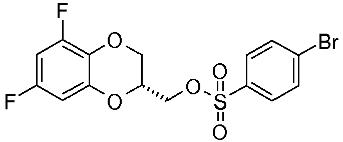

¹H RMN (DMSOd₆) δ : 4,02 (dd, 1H, J = 11,6 et 6,8 Hz) ; 4,29 (dd, 1H, J = 11,6 et 6,4 Hz) ; 4,35 (dd, 1H, J = 11,6 et 2,4 Hz) ; 4,46 (dd, 1H, J = 11,6 et 3,2 Hz) ; 4,58 (m, 1H) ; 6,57 (m, 1H) ; 6,87 (m, 1H) ; 7,88 (m, 4H).

### Intermédiaire 2.20 : (R)-(7-chloro-5-fluoro-2,3-dihydrobenzo[b][1,4]dioxin-2-yl) méthyl 4-bromobenzenesulfonate

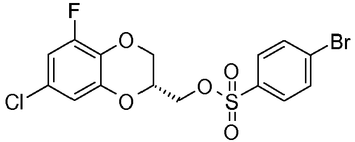

¹H RMN (DMSOd₆) δ : 4,05 (dd, 1H, J = 11,6 et 6,8 Hz) ; 4,29 (dd, 1H, J = 11,4 et 6 Hz) ; 4,38 (dd, 1H, J = 11,6 et 2,4 Hz) ; 4,46 (dd, 1H, J = 11,4 et 2,4 Hz) ; 4,58 (m, 1H) ; 6,72 (m, 1H) ; 7,02 (dd, 1H, J = 10,4 et 2,4 Hz) ; 7,88 (m, 4H).

### Intermédiaire 2.21 : (R)-(5-chloro-7-fluoro-2,3-dihydrobenzo[b][1,4]dioxin-2-yl) méthyl 4-bromobenzenesulfonate

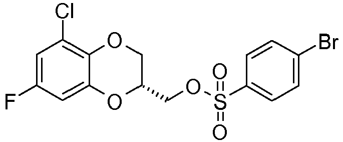

¹H RMN (DMSOd₆) δ : 4,05 (dd, 1H, J = 11,6 et 7,2 Hz) ; 4,28 (dd, 1H, J = 11,6 et 6 Hz) ; 4,39 (dd, 1H, J = 11,6 et 2,8 Hz) ; 4,45 (dd, 1H, J = 11,6 et 2,8 Hz) ; 4,57 (m, 1H) ; 6,73 (dd, 1H, J = 9,6 et 2,8 Hz) ; 7,00 (dd, 1H, J = 8,4 et 2,8 Hz) ; 7,89 (m, 4H).

### Intermédiaire 2.22 : (R)-(6,7-difluoro-2,3-dihydrobenzo[b][1,4]dioxin-2-yl)méthyl 4-bromobenzenesulfonate

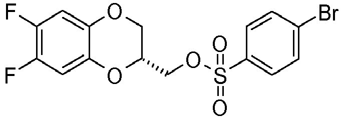

¹H RMN (DMSOd₆) δ : 3,98 (dd, 1H, J = 11,4 et 6,8 Hz) ; 4,25 (dd, 1H, J = 11,4 et 6,4 Hz) ; 4,29 (dd, 1H, J = 11,4 et 2 Hz) ; 4,43 (dd, 1H, J = 11,4 et 2,8 Hz) ; 4,51 (m, 1H) ; 6,93 (dd, 1H, J = 11,2 et 8 Hz) ; 7,04 (dd, 1H, J = 11,2 et 8 Hz) ; 7,87 (m, 4H).

### Intermédiaire 2.23 : (R)-(7-(méthylsulfonyl)-2,3-dihydrobenzo[b][1,4]dioxin-2-yl) méthyl 4-bromobenzenesulfonate

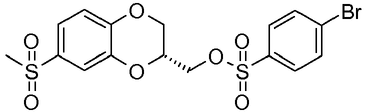

¹H RMN (DMSOd₆) δ : 3,17 (s, 3H) ; 4,07 (dd, 1H, J = 11,6 et 7,2 Hz) ; 4,32 (dd, 1H, J = 11,2 et 6 Hz) ; 4,42 (dd, 1H, J = 11,6 et 2,4 Hz) ; 4,46 (dd, 1H, J = 11,2 et 2,8 Hz) ; 4,53 (m, 1H) ; 7,10 (d, 1H, J = 8,4 Hz) ; 7,33 (d, 1H, J = 2,4 Hz) ; 7,39 (dd, 1H, J = 8,4 et 2,4 Hz) ; 7,89 (m, 4H).

### Intermédiaire 2.24 : (R)-méthyl 3-((((4-bromophenyl)sulfonyl)oxy)méthyl)-2,3-dihydrobenzo[b][1,4]dioxine-6-carboxylate

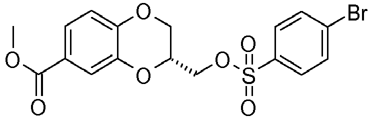

### Intermédiaire 2.25 : (R)-(7-(benzyloxy)-2,3-dihydrobenzo[b][1,4]dioxin-2-yl)méthyl 4-bromobenzenesulfonate

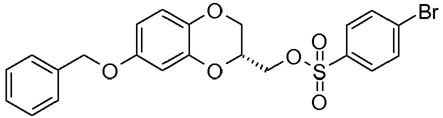

¹H RMN (DMSOd₆) δ : 4,01 (dd, 1H, J = 11,6 et 6 Hz) ; 4,18 (dd, 1H, J = 11,6 et 2 Hz) ; 4,24 (m, 2H) ; 4,40 (m, 1H) ; 4,97 (s, 2H) ; 6,40 (d, 1H, J = 2,8 Hz) ; 6,49 (dd, 1H, J = 8,8 et 2,8 Hz) ; 6,75 (d, 1H, J = 8,8 Hz) ; 7,36 (m, 5H) ; 7,70 (m, 2H) ; 7,77 (m, 2H).

### Intermédiaire 2.26 : (R)-(7-cyano-5-fluoro-2,3-dihydrobenzo[b][1,4]dioxin-2-yl) méthyl-4-bromobenzene sulfonate

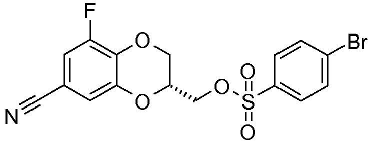

¹H RMN (CDCl3) δ : 4,14 (m, 1H) ; 4,32 (m, 2H) ; 4,46 (m, 1H) ; 4,53 (m, 1H) ; 6,92 (d, 1H, J = 5 Hz) ; 7 (d, 1H, J = 6 Hz) ; 7,75 (m, 4H).

### Dérivés mésylates : Préparation de manière classique avec chlorure de méthanesulfonyl /Et₃N/THF (J. Med. Chem. ; 1995 ; 38 ; 1119-1131)

### Intermédiaire 2.27 : (R)-(6-(benzyloxy)-2,3-dihydrobenzo[b][1,4]dioxin-2-yl)méthyl methanesulfonate

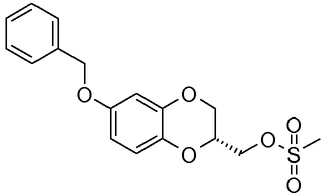

¹H RMN (CDCl₃) δ : 3,07 (s, 3H) ; 4,10 (m, 1H) ; 4,27 (m, 1H) ; 4,38 (m, 3H) ; 5,05 (s, 2H) ; 6,45 (m, 2H) ; 6,80 (m, 1H) ; 7,38 (m, 5H).

### Partie « pipéridine »

### Intermédiaire 3.1 : 3-(2-(pipéridin-4-yl)éthyl)oxazolidin-2-one

### ∘ Etape 1 : Préparation de tert-butyl 4-(2-iodoéthyl)pipéridine-1-carboxylate

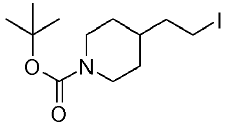

Dans un ballon monocol de 1 l, on met en solution dans 250 ml de dichlorométhane 18,53 g (71 mmol) de triphenylphosphine et 4,83 g (71 mmol) d'imidazole. On ajoute doucement 19,82 g d'iode (78 mmol) et on laisse sous agitation 15 min à température ambiante. On ajoute ensuite goutte à goutte 16,20 g (71 mmol) de tert-butyl 4-(2-hydroxyéthyl)pipéridine-1-carboxylate commercial en solution dans 120 ml de dichlorométhane. On laisse sous agitation à température ambiante toute une nuit. On évapore ensuite le milieu en présence de silice. Le solide obtenu est déposé sur un fritté de silice et on procède à une purification par simple filtration en utilisant comme éluant un mélange n-heptane/AcOEt = 90/10. On récupère 20,91g (62 mmol) du tert-butyl 4-(2-iodoéthyl)pipéridine-1-carboxylate sous forme d'huile très légèrement jaune.
Rendement : 87%
¹H RMN (CDCl₃) δ : 1,10 (m, 2H) ; 1,45 (s, 9H) ; 1,60 (m, 3H) ; 1,78 (m, 2H) ; 2,70 (m, 2H) ; 3,22 (t, 2H, J = 7,2 Hz) ; 4,09 (m, 2H).

### ∘ Etape 2 : Préparation de tert-butyl 4-(2-(2-oxooxazolidin-3-yl)éthyl)pipéridine-1-carboxylate

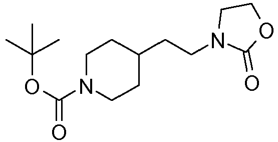

Dans un ballon tricol de 500 ml, on introduit 1,5 g (37,5 mmol) de NaH et 50 ml de diméthylformamide. On ajoute goutte à goutte 3,2 g (37 mmol) de 2-oxazolidinone commerciale en solution dans 30 ml de diméthylformamide. Dès que l'on n'observe plus de dégagement gazeux, on additionne 12,5 g (37 mmol) de tert-butyl 4-(2-iodoéthyl)pipéridine-1-carboxylate en solution dans 50 ml de diméthylformamide. On met ensuite le milieu à chauffer à 70°C pendant une nuit. On concentre ensuite le solvant à la pompe à palette, puis on procède à deux extractions eau/dichlorométhane; la phase organique est séchée sur MgSO₄, filtrée, évaporée et purifiée par flash chromatographie sur gel de silice (éluant : n-heptane/acétone = 60/40). On récupère 8,6 g (29 mmol) du tert-butyl 4-(2-(2-oxooxazolidin-3-yl)éthyl)pipéridine-1-carboxylate sous forme de solide blanc.
Rendement : 78%
¹H RMN (CDCl₃) δ : 1,12 (m, 2H) ; 1,45 (s, 9H) ; 1,49 (m, 3H) ; 1,69 (m, 2H) ; 2,68 (m, 2H) ; 3,32 (t, 2H, J = 7,2 Hz) ; 3,55 (t, 2H, J = 8 Hz) ; 4,08 (m, 2H) ; 4,33 (m, 2H).

### ∘ Etape 3 : Préparation de 3-(2-(pipéridin-4-yl)éthyl)oxazolidin-2-one

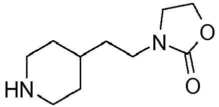

Dans un ballon monocol de 150 ml, on introduit 8,6 g (29 mmol) du tert-butyl 4-(2-(2-oxooxazolidin-3-yl)éthyl)pipéridine-1-carboxylate que l'on solubilise dans 50 ml d'éthanol. On ajoute 20 ml d'une solution HCl 5N dans l'isopropanol (100 mmol). On porte le milieu à 70°C pendant 4h00. On concentre ensuite le milieu, on ajoute 50 ml d'eau, on basifie avec NaOH aq et on extrait avec 2x50 ml de dichlorométhane. La phase organique est séchée sur MgSO₄, filtrée, évaporée et purifiée par flash chromatographie sur gel de silice (éluant : dichlorométhane/méthanol/NH4OH= 90/9/1%). On récupère 5 g (25 mmol) de l'intermédiaire 3.1 sous forme de solide blanc cassé.
Rendement : 87%
¹H RMN (CDCl₃) δ : 1,27 (m, 2H) ; 1,47 (m, 3H) ; 1,78 (m, 2H) ; 2,65 (m, 2H) ; 3,16 (m, 2H) ; 3,31 (t, 2H, J = 3,2 Hz) ; 3,50 (ls, 1H) ; 3,55 (t, 2H, J = 8 Hz) ; 4,33 (t, 2H, J = 8 Hz).

### Intermédiaire 3.2 : (S)-4-isopropyl-3-(2-(pipéridin-4-yl)éthyl)oxazolidin-2-one

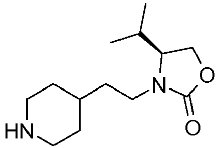

En procédant comme dans l'exemple 3.1 (étapes 2 et 3), mais en remplaçant la 2-oxazolidinone par la (S)-4-isopropyloxazolidin-2-one commerciale, on obtient l'intermédiaire 3.2 sous forme d'huile jaune pâle, après purification par flash chromatographie sur gel de silice (éluant : dichlorométhane/méthanol = 90/10%).
Rendement : 97%
¹H RMN (CDCl₃) δ : 0,87 (d, 3H, J = 6,8 Hz) ; 0,91 (d, 3H, J = 6,8 Hz) ; 1,16 (m, 2H) ; 1,40 (m, 1H) ; 1,48 (m, 2H) ; 1,68 (m, 1H) ; 1,76 (m, 1H) ; 1,95 (m, 1H) ; 2,05 (m, 1H) ; 2,59 (m, 2H) ; 2,98 (m, 1H) ; 3,06 (m, 2H) ; 3,58 (m, 1H) ; 3,74 (m, 1H) ; 4,07 (dd, 1H, J = 9 et 1,6 Hz) ; 4,21 (m, 1H).

### Intermédiaire 3.3 : 6-methoxy-3-(2-(pipéridin-4-yl)éthyl)benzo[d]oxazol-2(3H)-one

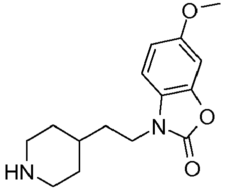

En procédant comme dans l'exemple 3.1 (étapes 2 et 3), mais en remplaçant la 2-oxazolidinone par la 6-methoxybenzo[d]oxazol-2(3H)-one commerciale, on obtient l'intermédiaire 3.3 sous forme de solide crème sans purification.
Rendement : 96%
¹H RMN (CDCl₃) δ : 1,27 (m, 2H) ; 1,46 (m, 1H) ; 1,71 (m, 2H) ; 1,81 (m, 2H) ; 2,34 (ls, 1H) 2,62 (m, 2H) ; 3,14 (m, 2H) ; 3,81 (s, 3H) ; 3,84 (m, 2H) ; 6,73 (dd, 1H, J = 8,8 et 2,4 Hz) ; 6,84 (m, 2H).

### Intermédiaire 3.4 : (S)-4-benzyl-3-(2-(pipéridin-4-yl)éthyl)oxazolidin-2-one

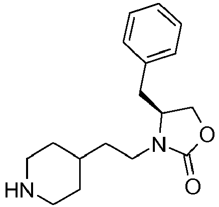

En procédant comme dans l'exemple 3.1 (étapes 2 et 3), mais en remplaçant la 2-oxazolidinone par la (S)-4-benzyloxazolidin-2-one commerciale, on obtient l'intermédiaire 3.4 sous forme de d'huile jaune pâle, après purification par flash chromatographie sur gel de silice (éluant : dichlorométhane/méthanol = 90/10%).
Rendement : 79%
¹H RMN (CDCl₃) δ : 1,22 (m, 2H) ; 1,40 (m, 1H) ; 1,50 (m, 2H) ; 1,73 (m, 2H) ; 2,64 (m, 4H) ; 3,10 (m, 3H) ; 3,57 (m, 1H) ; 4,01 (m, 2H) ; 4,15 (m, 1H) ; 7,16 (m, 1H) ; 7,32 (m, 2H).

### Intermédiaire 3.5 : (R)-4-phenyl-3-(2-(pipéridin-4-yl)éthyl)oxazolidin-2-one

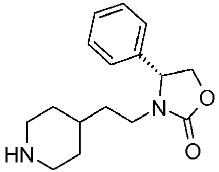

En procédant comme dans l'exemple 3.1 (étapes 2 et 3), mais en remplaçant la 2-oxazolidinone par la (R)-4-phenyloxazolidin-2-one commerciale, on obtient l'intermédiaire 3.5 sous forme de d'huile jaune pâle, après purification par flash chromatographie sur gel de silice (éluant : dichlorométhane/méthanol = 95/5%).
Rendement : 64%
¹H RMN (CDCl₃) δ : 1,05 (m, 2H) ; 1,35 (m, 3H) ; 1,61 (m, 2H) ; 2,53 (m, 2H) ; 2,78 (m, 1H) ; 3,02 (m, 2H) ; 3,47 (m, 1H) ; 4,13 (dd, 1H, J = 8,6 et 6,4 Hz) ; 4,62 (m, 1H) ; 4,76 (dd, 1H, J = 8,8 et 6,8 Hz) ; 7,31 (m, 2H) ; 7,40 (m, 3H).

### Intermédiaire 3.6 : (S)-4-phenyl-3-(2-(pipéridin-4-yl)éthyl)oxazolidin-2-one

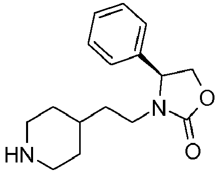

En procédant comme dans l'exemple 3.1 (étapes 2 et 3), mais en remplaçant la 2-oxazolidinone par la (S)-4-phenyloxazolidin-2-one commerciale, on obtient l'intermédiaire 3.6 sous forme de d'huile jaune pâle, après purification par flash chromatographie sur gel de silice (éluant : dichlorométhane/méthanol = 95/5%).
Rendement : 60%
¹H RMN (CDCl₃) δ : idem intermédiaire 3.5.

### Intermédiaire 3.7 : 4-(2-methoxyphenyl)-3-(2-(pipéridin-4-yl)éthyl)oxazolidin-2-one (produit préparé selon Synthetic communication ; 30 ; vol6 ; p-1095-1102 ; 2000)

### ∘ Etape 1 : Préparation de 2-(benzylamino)-2-(2-methoxyphenyl)acetonitrile

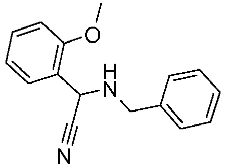

Dans un tricol de 500 ml, on introduit 12,1 g (63 mmol) de Na₂S₂O₅, 110 ml d'eau et 10 ml de méthanol. On ajoute 17,2 g (126 mmol) d'o-anisaldéhyde et en une fois 6,9 g (139 mmol) de NaCN. On met sous agitation vers 28°C pendant 15 min, puis on se place à 0°C et l'on additionne 15 ml (126 mmol) de benzaldéhyde en solution dans 30 ml de méthanol. On agite le milieu à 35°C pendant 8h00. Après retour à température ambiante, on ajoute 50 ml d'eau et on extrait 2 fois avec 100 ml de dichlorométhane. Les phases organiques sont séchées sur MgSO₄, filtrées, évaporées et purifiées par flash chromatographie sur gel de silice (éluant : n-heptane/acétate d'éthyle = 70/30%). On récupère 30 g (119 mmol) du 2-(benzylamino)-2-(2-methoxyphenyl)acetonitrile sous forme d'huile claire.
Rendement : 94% ¹H RMN (CDCl₃) δ : 2,25 (ls, 1H) ; 3,85 (s, 3H) ; 3,92 (d, 1H, J = 13 Hz) ; 4,06 (d, 1H, J = 13 Hz) ; 4,80 (s, 1H) ; 6,95 (m, 2H) ; 7,33 (m, 7H).

### ∘ Etape 2 : Préparation de 2-(benzylamino)-2-(2-methoxyphenyl)acetamide

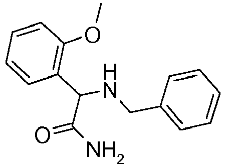

Dans un ballon monocol de 500 ml, on solubilise 30 g (119 mmol) du 2-(benzylamino)-2-(2-methoxyphenyl)acetonitrile obtenu précédemment dans 160 ml de diméthylsulfoxide. On ajoute 8,2 g (59,5 mmol) de K₂CO₃ et on chauffe le milieu à 35°C. On additionne ensuite 19,4 ml (190 mmol) de H₂O₂ à 30%. On laisse sous agitation à 35°C pendant 7h00. Après retour à température ambiante, on ajoute 100 ml d'eau glacée et on extrait 3 fois avec 100 ml d'acétate d'éthyle. Les phases organiques sont rassemblées, lavées 2 fois avec 150 ml d'eau glacée, séchées sur MgSO₄, filtrées, évaporées et purifiées par flash chromatographie sur gel de silice (éluant : n-heptane/acétate d'éthyle = 70/30%). On récupère 23,4 g (86,6 mmol) du 2-(benzylamino)-2-(2-methoxyphenyl)acetamide sous forme de solide blanc.
Rendement : 73%

### ∘ Etape 3 : Préparation de 2-(benzylamino)-2-(2-methoxyphenyl)acetic acid

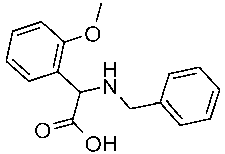

Dans un ballon monocol de 500 ml on solubilise 23,4 g (86,6 mmol) du 2-(benzylamino)-2-(2-methoxyphenyl)acetamide obtenu précédemment dans 156 ml d'IMS (140 ml d'éthanol/16 ml de méthanol) et 35 ml d'eau. On ajoute 5,1 g (129,8 mmol) de soude en pastilles et on porte le milieu à 80°C pendant 30h00. On concentre le milieu, on ajoute 50 ml d'eau et on lave 2 fois avec 100 ml de dichlorométhane. La phase aqueuse est acidifiée à pH 3 avec de l'HCl 10N, concentrée et le résidu huileux est repris dans l'éther. Après trituration, filtration et séchage, on obtient 17,2 g (63,4 mmol) du 2-(benzylamino)-2-(2-methoxyphenyl)acetic acid sous forme de solide crème. Rendement : 73%
¹H RMN (DMSOd₆) δ : 3,76 (s, 3H) ; 4,04 (s, 2H) ; 5,11 (s, 1H) ; 7,03 (m, 1H) ; 7,10 (m, 1H) ; 7,43 (m, 7H).

### ∘ Etape 4 : Préparation de 2-(benzylamino)-2-(2-methoxyphenyl)ethanol

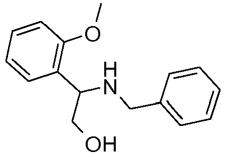

Dans un tricol de 500 ml on introduit 3,6 g (95 mmol) de LiAlH₄ dans 200 ml de tétrahydrofurane. On ajoute à 0°C par portions 17,2 g (63,4 mmol) du 2-(benzylamino)-2-(2-methoxyphenyl)acetic acid obtenu précédemment. On agite ensuite le milieu à 50°C pendant 20h00. Après retour à température ambiante, on refroidit le milieu à 0°C et on ajoute goutte à goutte 18 ml de NaOH à 10%. On filtre les aluminates. Le filtrat est ensuite lavé par 100 ml d'eau et extrait 2 fois avec 100 ml de dichlorométhane. Les phases organiques sont ensuite séchées sur Na₂SO₄, filtrées, évaporées et purifiées par flash chromatographie sur gel de silice (éluant : dichlorométhane/méthanol = 90/10%). On récupère 8,3 g (32,3 mmol) du 2-(benzylamino)-2-(2-methoxyphenyl)ethanol sous forme d'huile claire.
Rendement : 51 %
¹H RMN (CDCl₃) δ : 1,99 (ls, 1H) ; 2,97 (ls, 1H) ; 3,68 (m, 4H) ; 3,82 (s, 3H) ; 4,10 (m, 1H) ; 6,90 (m, 1H) ; 6,97 (m, 1H) ; 7,31 (m, 7H).

### ∘ Etape 5 : Préparation de 2-amino-2-(2-methoxyphenyl)ethanol

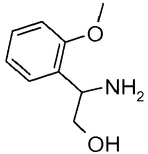

Dans un tricol de 250 ml on solubilise 7 g (27,2 mmol) du 2-(benzylamino)-2-(2-methoxyphenyl)ethanol obtenu précédemment dans 100 ml de méthanol. On ajoute 0,7 g de Pd(OH)₂/C et 8,6 g (136 mmol) de formiate d'ammonium, puis on porte le milieu à 70°C pendant 2h30. Après retour à température ambiante, on filtre le palladium sur célite, on rince avec 20 ml de méthanol, on concentre le filtrat et on purifie par flash chromatographie sur gel de silice (éluant : dichlorométhane/méthanol/NH₄OH = 90/9/1%). On récupère 4,3 g (25,7 mmol) du 2-amino-2-(2-methoxyphenyl)ethanol sous forme d'huile claire.
Rendement : 95%
¹H RMN (CDCl₃) δ : 2,02 (ls, 3H) ; 3,61 (m, 1H) ; 3,74 (dd, 1H, J = 10,3 et 2,4 Hz) ; 3,84 (s, 3H) ; 4,26 (m, 1H) ; 6,88 (m, 1H) ; 6,95 (m, 1H) ; 7,26 (m, 2H).

### ∘ Etape 6 : Préparation de 4-(2-methoxyphenyl)oxazolidin-2-one

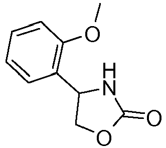

Dans un tricol de 250 ml on solubilise 4,2 g (25 mmol) du 2-amino-2-(2-methoxyphenyl)ethanol obtenu précédemment et 2,5 g (25 mmol) de KHCO₃ dans 40 ml d'eau. Après 10 min d'agitation, on refroidit à 0°C et on ajoute 3,4 g (25 mmol) de K₂CO₃, puis goutte à goutte 17 ml (32,6 mmol) de phosgène à 20% dans le toluène. Le milieu est agité à 0°C pendant 6h00. On observe la formation d'un produit blanc que l'on filtre et que l'on sèche. On obtient 3,6 g (18,6 mmol) du 4-(2-methoxyphenyl)oxazolidin-2-one sous forme de solide blanc.
Rendement : 75%
¹H RMN (CDCl₃) δ : 3,85 (s, 3H) ; 4,18 (dd, 1H, J = 8,8 et 6,4 Hz) ; 4,80 (m, 1H) ; 5,25 (dd, 1H, J = 8,6 et 6,4 Hz) ; 5,46 (ls, 1H) ; 6,90 (m, 1H) ; 7,01 (m, 1H) ; 7,32 (m, 2H).

### ∘ Etape 7 : Préparation de tert-butyl 4-(2-(4-(2-methoxyphenyl)-2-oxooxazolidin-3-yl)éthyl)pipéridine-1-carboxylate

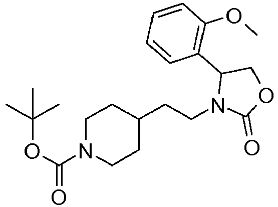

En procédant comme dans l'exemple 3.1 (étape 2), mais en remplaçant la 2-oxazolidinone par la 4-(2-methoxyphenyl)oxazolidin-2-one obtenue précédemment, on obtient le tert-butyl 4-(2-(4-(2-methoxyphenyl)-2-oxooxazolidin-3-yl)éthyl)pipéridine-1-carboxylate sous forme d'huile jaune pâle, après purification par flash chromatographie sur gel de silice (éluant : n-heptane/acétate d'éthyle =70/30%).
Rendement : 57%
¹H RMN (CDCl₃) δ : 1,03 (m, 2H) ; 1,40 (m, 3H) ; 1,44 (s, 9H) ; 1,61 (m, 2H) ; 2,63 (m, 2H) ; 2,79 (m, 1H) ; 3,54 (m, 1H) ; 3,85 (s, 3H) ; 4,02 (m, 2H) ; 4,11 (dd, 1H, J = 8,4 et 5,6 Hz) ; 4,59 (m, 1H) ; 5,15 (dd, 1H, J = 9 et 5,6 Hz) ; 6,93 (m, 1H) ; 6,99 (m, 1H) ; 7,18 (m, 1H) ; 7,34 (m, 1H).

### ∘ Etape 8 : Préparation de 4-(2-methoxyphenyl)-3-(2-(pipéridin-4-yl)éthyl) oxazolidin-2-one

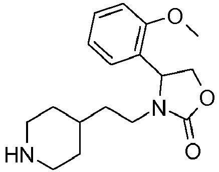

En procédant comme dans l'exemple 3.1 (étape 3) mais en remplaçant le de tert-butyl 4-(2-(2-oxooxazolidin-3-yl)éthyl)pipéridine-1-carboxylate par le de tert-butyl 4-(2-(4-(2-methoxyphenyl)-2-oxooxazolidin-3-yl)éthyl)pipéridine-1-carboxylate, on obtient l'intermédiaire 3.7 sous forme d'huile jaune pâle, après purification par flash chromatographie sur gel de silice (éluant : dichlorométhane/méthanol = 90/10%).
Rendement : 96%
¹H RMN (CDCl₃) δ : 1,18 (m, 2H) ; 1,39 (m,2H) ; 1,69 (m, 2H) ; 2,63 (m, 4H) ; 3,13 (m, 2H) ; 3,53 (m, 1H) ; 3,84 (s, 3H) ; 4,11 (dd, 1H, J = 8,4 et 5,6 Hz) ; 4,59 (m, 1H) ; 5,15 (dd, 1H, J = 9 et 5,6 Hz) ; 6,93 (m, 1H) ; 6,99 (m, 1H) ; 7,18 (m, 1H) ; 7,33 (m, 1H).

### Intermédiaire 3.8 : 4-(4-methoxyphenyl)-3-(2-(pipéridin-4-yl)éthyl)oxazolidin-2-one

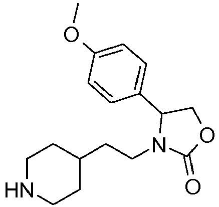

En procédant comme dans l'exemple 3.7, mais en remplaçant l'o-anisaldéhyde par le p-anisaldéhyde commercial, on obtient l'intermédiaire 3.8 sous forme de d'huile jaune pâle, après purification par flash chromatographie sur gel de silice (éluant : dichlorométhane/méthanol = 90/10%).
Rendement : 80%
¹H RMN (CDCl₃) δ : 1,15 (m,2H) ; 1,38 (m, 2H) ; 1,61 (m, 2H) ; 2,57 (m, 2H) ; 2,82 (m, 2H) ; 3,07 (m, 2H) ; 3,44 (m, 1H) ; 3,83 (s, 3H) ; 4,10 (m, 1H) ; 4,59 (m, 1H) ; 4,71 (m, 1H) ; 6,93 (d, 2H, J = 8,1 Hz) ; 7,21 (d, 2H, J = 8,1 Hz).

### Intermédiaire 3.9 :(S)-4-(4-methoxybenzyl)-3-(2-(pipéridin-4-yl)éthyl)oxazolidin-2-one

### ∘ Etape 1 : Préparation de (S)-2-amino-3-(4-methoxyphenyl)propan-1-ol

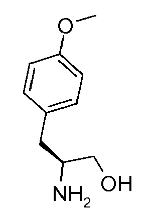

Dans un ballon tricol de 100 ml, on solubilise 0,5 g (2,6 mmol) de (S)-2-amino-3-(4-methoxyphenyl)propanoic acid dans 5 ml de tétrahydrofurane. On refroidit à 0°C et l'on ajoute goutte à goutte 12,8 ml (12,8 mmol) d'une solution 1M de BH₃ dans le tétrahydrofurane. On chauffe ensuite le milieu à 70°C pendant 4h00. On refroidit à nouveau à 0°C et l'on additionne 30 ml d'une solution d'HCl 1N. On lave le milieu réactionnel avec 30 ml de dichlorométhane. La phase aqueuse est ensuite basifiée par 30 ml d'une solution de NaOH 1N et extraite 2 fois avec 100 ml de dichlorométhane. Ces phases organiques sont ensuite séchées sur Na₂SO₄, filtrées et évaporées. On récupère 0,3 g (1,7 mmol) du (S)-2-amino-3-(4-methoxyphenyl)propan-1-ol sous forme de solide blanc.
Rendement : 65%
¹H RMN (CDCl₃) δ : 1,59 (ls, 3H) ; 2,47 (dd, 1H, J = 13,6 et 8,4 Hz) ; 2,73 (dd, 1H, J = 13,6 et 5,2 Hz) ; 3,07 (m, 1H) ; 3,36 (dd, 1H, J = 10,4 et 7,2 Hz) ; 3,63 (dd, 1H, J = 10,4 et 7,6 Hz) ; 3,80 (s, 3H) ; 6,85 (d, 2H, J = 8,8 Hz) ; 7,11 (d, 2H, J = 8,8 Hz).
∘ Etape 2 : Préparation de (S)-4-(4-methoxybenzyl)oxazolidin-2-one

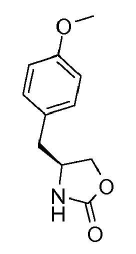

En procédant comme dans l'exemple 3.7 (étape 6), mais en remplaçant le (S)2-amino-2-(2-methoxyphenyl)ethanol par le (S)-2-amino-3-(4-methoxyphenyl)propan-1-ol, on obtient le (S)-4-(4-methoxybenzyl)oxazolidin-2-one sous forme d'huile jaune pâle après purification par flash chromatographie sur gel de silice (éluant : dichlorométhane/méthanol = 95/5%).
Rendement : 87%
¹H RMN (CDCl₃) δ : 2,81 (m, 2H) ; 3,80 (s, 3H) ; 4,04 (m, 1H) ; 4,14 (dd, 1H, J = 8,8 et 5,6 Hz) ; 4,45 (m, 1H) ; 6,87 (d, 2H, J = 8,8 Hz) ; 7,09 (d, 2H, J = 8,8 Hz).

### ∘ Etape 3 : Préparation de (S)-tert-butyl 4-(2-(4-(4-methoxybenzyl)-2-oxooxazolidin-3-yl)éthyl)pipéridine-1-carboxylate

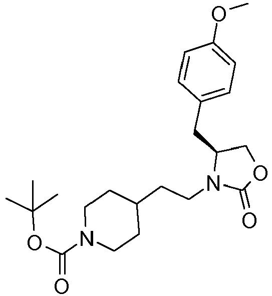

En procédant comme dans l'exemple 3.1 (étape 2), mais en remplaçant la 2-oxazolidinone par la (S)-4-(4-methoxybenzyl)oxazolidin-2-one, on obtient le (S)-tert-butyl 4-(2-(4-(4-methoxybenzyl)-2-oxooxazolidin-3-yl)éthyl)pipéridine-1-carboxylate sous forme d'huile jaune pâle après purification par flash chromatographie sur gel de silice (éluant : n-heptane/acétate d'éthyle = 70/30%).
Rendement : 71%
¹H RMN (CDCl₃) δ : 1,11 (m, 2H) ; 1,45 (s, 9H) ; 1,46 (m, 3H) ; 1,68 (m, 2H) ; 2,63 (m, 3H) ; 3,07 (m, 2H) ; 3,56 (m, 1H) ; 3,80 (s, 3H) ; 3,98 (m, 2H) ; 4,14 (m, 3H) ; 6,86 (d, 2H, J = 8,4 Hz) ; 7,07 (d, 2H, J = 8,4 Hz).

### ∘ Etape 4 : Préparation de (S)-4-(4-methoxybenzyl)-3-(2-(pipéridin-4-yl) éthyl)oxazolidin-2-one

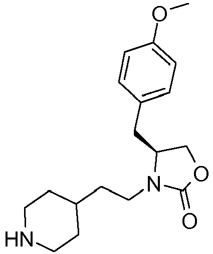

En procédant comme dans l'exemple 3.1 (étape 3), mais en remplaçant le tert-butyl 4-(2-(2-oxooxazolidin-3-yl)éthyl)pipéridine-1-carboxylate par le (S)-tert-butyl 4-(2-(4-(4-methoxybenzyl)-2-oxooxazolidin-3-yl)éthyl)pipéridine-1-carboxylate, on obtient l'intermédiaire 3.9 sous forme d'huile jaune pâle sans purification.
Rendement : 84%
¹H RMN (CDCl₃) δ : 1,27 (m, 2H) ; 1,42 (m, 1H) ; 1,51 (m, 2H) ; 1,76 (m, 2H) ; 2,63 (m, 4H) ; 3,10 (m, 3H) ; 3,56 (m, 1H) ; 3,80 (s, 3H) ; 3,98 (m, 2H) ; 4,18 (m, 1H) ; 6,86 (d, 2H, J = 8,4 Hz) ; 7,07 (d, 2H, J = 8,4 Hz).

### Intermédiaire 3.10 : (S)-4-(4-(benzyloxy)benzyl)-3-(2-(pipéridin-4-yl)éthyl) oxazolidin-2-one

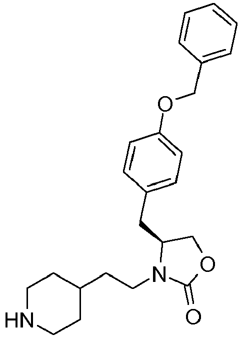

En procédant comme dans l'exemple 3.9, mais en remplaçant le (S)-2-amino-3-(4-methoxyphenyl)propanoic acid par le (S)-2-amino-3-(4-(benzyloxy)phenyl)propanoic acid, on obtient l'intermédiaire 3.10 sous forme d'huile jaune pâle sans purification. Rendement : 81 %
¹H RMN (CDCl₃) δ : 1,25 (m, 2H) ; 1,41 (m, 1H) ; 1,49 (m, 2H) ; 1,75 (m, 2H) ; 2,62 (m, 4H) ; 3,08 (m, 3H) ; 3,56 (m, 1H) ; 3,98 (m, 2H) ; 2,67 (m, 1H) ; 5,05 (s, 2H) ; 6,94 (d, 2H, J = 8,4 Hz) ; 7,07 (d, 2H, J = 8,4 Hz) ; 7,38 (m, 5H).

### Intermédiaire 3.11 : (S)-4-(3,4-dimethoxybenzyl)-3-(2-(pipéridin-4-yl)éthyl) oxazolidin-2-one

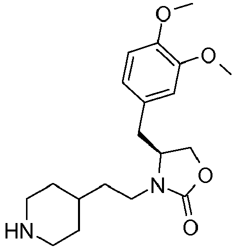

En procédant comme dans l'exemple 3.9, mais en remplaçant le (S)-2-amino-3-(4-methoxyphenyl)propanoic acid par le (S)-2-amino-3-(3,4-dimethoxyphenyl)propanoic acid, on obtient l'intermédiaire 3.11 sous forme d'huile jaune pâle sans purification. Rendement : 94%
¹H RMN (CDCl₃) δ : 1,22 (m, 2H) ; 1,42 (m, 1H) ; 1,52 (m, 2H) ; 1,73 (m, 2H) ; 2,36 (ls, 1H) ; 2,62 (m, 3H) ; 3,07 (m, 4H) ; 3,57 (m, 1H) ; 3,87 (s, 3H) ; 3,88 (s, 3H) ; 3,99 (m, 2H) ; 4,17 (m, 1H) ; 6,64 (d, 1H, J = 2 Hz) ; 6,70 (dd, 1H, J = 8 et 2 Hz) ; 6,82 (d, 1H, J = 8 Hz).

### Intermédiaire 3.12 : (S)-4-(4-fluorobenzyl)-3-(2-(pipéridin-4-yl)éthyl)oxazolidin-2-one

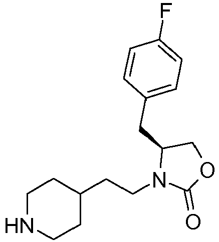

En procédant comme dans l'exemple 3.9, mais en remplaçant le (S)-2-amino-3-(4-methoxyphenyl)propanoic acid par le (S)-2-amino-3-(4-fluorophenyl)propanoic acid, on obtient l'intermédiaire 3.12 sous forme d'huile incolore après purification par flash chromatographie sur gel de silice (éluant : dichlorométhane/méthanol/NH₄OH = 90/9/1%).
Rendement : 80%
¹H RMN (CDCl₃) δ : 1,20 (m, 2H) ; 1,39 (m, 1H) ; 1,50 (m, 2H) ; 1,72 (m, 2H) ; 1,92 (ls, 1H) ; 2,63 (m, 3H) ; 3,08 (m, 4H) ; 3,58 (m, 1H) ; 3,98 (m, 2H) ; 4,17 (m, 1H) ; 7,03 (m, 2H) ; 7,13 (m, 2H).

### Intermédiaire 3.13 : (R)-4-(phénoxyméthyl)-3-(2-(pipéridin-4-yl)éthyl)oxazolidin-2-one

### ∘ Etape 1 : Préparation de (S)-méthyl 2-oxooxazolidine-4-carboxylate

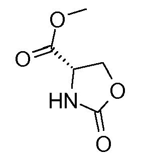

En procédant comme dans l'exemple 3.7 (étape 6), mais en remplaçant le 2-amino-2-(2-methoxyphenyl)ethanol par le chlorhydrate du (S)-méthyl 2-amino-3-hydroxypropanoate, on obtient le (S)-méthyl 2-oxooxazolidine-4-carboxylate sous forme d'huile incolore, après purification par flash chromatographie sur gel de silice (éluant : dichlorométhane/méthanol/NH₄OH = 90/9/1%).
Rendement : 88%
¹H RMN (DMSOd₆) δ : 3,71 (s, 3H) ; 4,34 (m, 1H) ; 4,48 (m, 2H) ; 8,24 (s, 1H).

### ∘ Etape 2 : Préparation de (R)-4-(hydroxyméthyl)oxazolidin-2-one

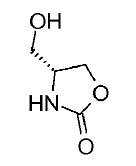

Dans un ballon tricol de 250 ml, on introduit 2,2 g (59,95 mmol) de NaBH₄ dans 20 ml d'éthanol. On ajoute goutte à goutte à 0°C, 5,8 g (39,96 mmol) du (S)-méthyl 2-oxooxazolidine-4-carboxylate obtenu précedemment en solution dans 80 ml de méthanol. Après 3h00 d'agitation à 0°C, on laisse revenir à température ambiante puis on traite par 15 ml d'une solution saturée de NH₄Cl. On filtre le précipité blanc qui se forme. Le filtrat est concentré pour donner 4,5 g (38,43 mmol) du (R)-4-(hydroxyméthyl)oxazolidin-2-one sous la forme d'un solide blanc.
Rendement : 96%
¹H RMN (D₂O) δ : 3,60 (dd, 1H, J = 12 et 4 Hz) ; 3,68 (dd, 1H, J = 12 et 3,6 Hz) ; 4,07 (m, 1H) ; 4,30 (m, 1H) ; 4,57 (m, 1H).

### ∘ Etape 3 : Préparation de (S)-(2-oxooxazolidin-4-yl)méthyl 4-méthylbenzenesulfonate

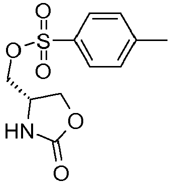

On solubilise 4 g (34,16 mmol) du (R)-4-(hydroxyméthyl)oxazolidin-2-one précedemment obtenu dans 40 ml de dichlorométhane. On refroidit à 0°C et l'on ajoute 7,1 g (37,57 mmol) de chlorure de tosyle et 5,2 ml (37,57 mmol) de triéthylamine. On laisse sous agitation à température ambiante pendant 20h00, puis on concentre à sec. On fait ensuite deux extractions eau/acétate d'éthyle. Les phases organiques sont ensuite séchées sur MgSO₄, filtrées, concentrées et on obtient 4,3 g (15,85 mmol) du (S)-(2-oxooxazolidin-4-yl)méthyl 4-méthylbenzenesulfonate sous la forme d'un solide blanc, après purification par flash chromatographie sur gel de silice (éluant : acétate d'éthyle = 100%).
Rendement : 47%
¹H RMN (CDCl₃) δ : 2,47 (s, 3H) ; 4,07 (m, 4H) ; 4,48 (m, 1H) ; 5,51 (s, 1H) ; 7,39 (d, 2H, J = 8 Hz) ; 7,80 (d, 2H, J = 8 Hz).

### ∘ Etape 4 : Préparation de (R)-4-(phénoxyméthyl)oxazolidin-2-one

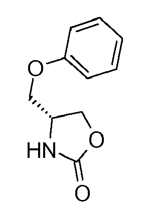

On solubilise 4,3 g (15,85 mmol) du (S)-(2-oxooxazolidin-4-yl)méthyl 4-méthylbenzenesulfonate précédemment décrit dans 40 ml d'acétonitrile. On ajoute 7,9 g (24,32 mmol) de Cs₂CO₃ et 1,6 g (17,84 mmol) de phénol, puis on met le milieu à chauffer vers 80°C pendant 2h00. Après retour à température ambiante, on filtre le Cs₂CO₃, et on concentre le milieu. On fait deux extractions eau/dichlorométhane. Les phases organiques sont séchées sur MgSO₄, filtrées, évaporées et après trituration du résidu dans l'éther diéthylique on obtient 1,9 g (9,83 mmol) du (R)-4-(phénoxyméthyl)oxazolidin-2-one sous la forme d'un solide blanc.
Rendement : 65%
¹H RMN (CDCl₃) δ : 4,04 (m, 2H) ; 4,28 (m, 2H) ; 4,59 (m, 1H) ; 5,65 (s, 1H) ; 6,88 (m, 2H) ; 7,00 (m, 1H) ; 7,30 (m, 2H).

### ∘ Etape 5 : Préparation de (R)-tert-butyl 4-(2-(2-oxo-4-(phénoxyméthyl) oxazolidin-3-yl)éthyl)pipéridine-1-carboxylate

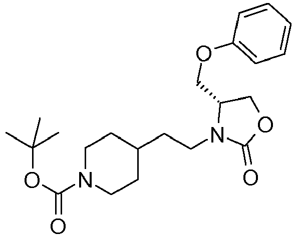

En procédant comme dans l'exemple 3.1 (étape 2), mais en remplaçant la 2-oxazolidinone commerciale par la (R)-4-(phénoxyméthyl)oxazolidin-2-one précédemment décrite, on obtient le (R)-tert-butyl 4-(2-(2-oxo-4-(phénoxyméthyl) oxazolidin-3-yl)éthyl)pipéridine-1-carboxylate sous forme d'un solide blanc, après purification par flash chromatographie sur gel de silice (éluant : dichlorométhane/ acétate d'éthyle = 70/30%).
Rendement : 65%
¹H RMN (CDCl₃) δ : 1,17 (m, 2H) ; 1,45 (s, 9H) ; 1,53 (m, 3H) ; 1,68 (m, 2H) ; 2,67 (m, 2H) ; 3,24 (m, 1H) ; 3,55 (m, 1H) ; 4,13 (m, 6H) ; 4,46 (m, 1H) ; 6,88 (m, 2H) ; 7,01 (m, 1H) ; 7,31 (m, 2H).

### ∘ Etape 6 : Préparation de (R)-4-(phénoxyméthyl)-3-(2-(pipéridin-4-yl)éthyl) oxazolidin-2-one

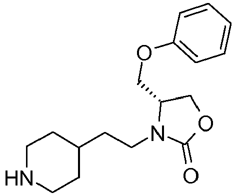

En procédant comme dans l'exemple 3.1 (étape 3), mais en remplaçant le tert-butyl 4-(2-(2-oxooxazolidin-3-yl)éthyl)pipéridine-1-carboxylate par le (R)-tert-butyl4-(2-(2-oxo-4-(phénoxyméthyl)oxazolidin-3-yl)éthyl)pipéridine-1-carboxylate précédemment obenu, on obtient le (R)-4-(phénoxyméthyl)-3-(2-(pipéridin-4-yl)éthyl)oxazolidin-2-one sous forme d'un solide blanc, après traitement et évaporation.
Rendement : 89%
¹H RMN (CDCl₃) δ : 1,13 (m, 2H) ; 1,40 (m, 1H) ; 1,53 (m, 2H) ; 1,68 (m, 2H) ; 1,87 (ls, 1H) ; 2,57 (m, 2H) ; 3,06 (m, 2H) ; 3,21 (m, 1H) ; 3,55 (m, 1H) ; 4,04 (m, 2H) ; 4,19 (m, 2H) ;
4,45 (m, 1H) ; 6,89 (m, 2H) ; 7,01 (m, 1H) ; 7,31 (m, 2H).

### Intermédiaire 3.14 : (S)-4-(phénoxyméthyl)-3-(2-(pipéridin-4-yl)éthyl)oxazolidin-2-one

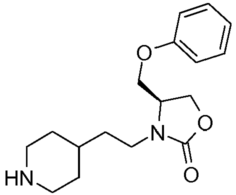

En procédant comme dans l'exemple 3.13, mais en remplaçant le (S)-méthyl 2-amino-3-hydroxypropanoate par le (R)-méthyl 2-amino-3-hydroxypropanoate, on obtient l'intermédiaire 3.14 sous la forme d'un solide blanc.
Rendement : 82%
¹H RMN (CDCl₃) δ : idem intermédiare 3.13

### Intermédiaire 3.15 : (R)-4-((4-fluorophénoxy)méthyl)-3-(2-(pipéridin-4-yl)éthyl) oxazolidin-2-one

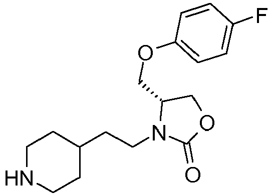

En procédant comme dans l'exemple 3.13 (étape 4), mais en remplaçant le phenol par le 4-fluoro phénol, on obtient l'intermédiaire 3.15 sous la forme d'une huile claire.
Rendement : 79%
¹H RMN (CDCl₃) δ : 1,17 (m, 2H) ; 1,41 (m, 1H) ; 1,50 (m, 2H) ; 1,71 (m, 2H) ; 1,87 (ls, 1H) ; 2,59 (m, 2H) ; 3,07 (m, 2H) ; 3,20 (m, 1H) ; 3,55 (m, 1H) ; 4,00 (d, 2H, J = 4 Hz) ; 4,18 (m, 2H) ; 4,45 (m, 1H) ; 6,83 (m, 2H) ; 7,00 (m, 2H).

### Intermédiaire 3.16 : (R)-4-((3-fluorophénoxy)méthyl)-3-(2-(pipéridin-4-yl)éthyl) oxazidin-2-one

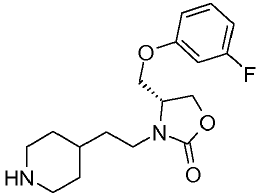

En procédant comme dans l'exemple 3.13 (étape 4), mais en remplaçant le phenol par le 3-fluoro phénol, on obtient l'intermédiaire 3.16 sous la forme d'un solide blanc. Rendement : 100%
¹H RMN (CDCl₃) δ : 1,20 (m, 2H) ; 1,43 (m, 1H) ; 1,52 (m, 2H) ; 1,71 (m, 2H) ; 2,38 (ls, 1H) ; 2,59 (m, 2H) ; 3,08 (m, 2H) ; 3,20 (m, 1H) ; 3,56 (m, 1H) ; 4,03 (d, 2H, J = 4,4 Hz) ; 4,19 (m, 2H) ; 4,46 (m, 1H) ; 6,61 (m, 1H) ; 6,71 (m, 2H) ; 7,26 (m, 1H).

### Intermédiaire 3.17 : (R)-4-((3,4-dimethoxyphénoxy)méthyl)-3-(2-(pipéridin-4-yl) éthyl)oxazolidin-2-one

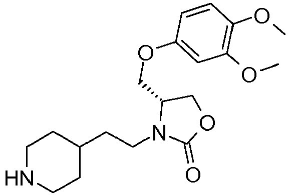

En procédant comme dans l'exemple 3.13 (étape 4), mais en remplaçant le phenol par le 3,4-dimethoxy phenol, on obtient l'intermédiaire 3.17 sous la forme d'une huile claire. Rendement : 100%
¹H RMN (CDCl₃) δ : 1,24-1,92 (m, 7H) ; 2,68 (m, 4H) ; 3,22 (m, 2H) ; 3,55 (m, 1H) ; 3,84 (s, 3H) ; 3,86 (s, 3H) ; 3,99 (m, 2H) ; 4,13 (m, 1H) ; 4,23 (m, 1H) ; 4,45 (m, 1H) ; 6,36 (dd, 1H, J = 8,8 et 2,8 Hz) ; 6,49 (d, 1H, J = 2,8 Hz) ; 6,78 (d, 1H, J = 8,8 Hz).

### Intermédiaire 3.18 : (R)-4-((3-(benzyloxy)phénoxy)méthyl)-3-(2-(pipéridin-4-yl) éthyl)oxazolidin-2-one

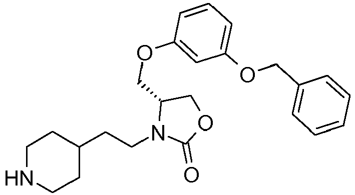

En procédant comme dans l'exemple 3.13 (étape 4), mais en remplaçant le phenol par le 3-(benzyloxy)phenol, lui-même préparé à partir du resorcinol selon une procédure décrite dans J. O.C. vol 62, N°10, 1997, p 3062-75*,* on obtient l'intermédiaire 3.18 sous la forme d'un solide crème.
Rendement : 45%
¹H RMN (CDCl₃) δ : 1,15 (m, 2H) ; 1,40 (m, 1H) ; 1,49 (m, 2H) ; 1,70 (m, 2H) ; 1,91 (ls, 1H) ; 2,58 (m, 2H) ; 3,06 (m, 2H) ; 3,19 (m, 1H) ; 3,54 (m, 1H) ; 3,98 (m, 2H) ; 4,13 (m, 1H) ; 4,20 (m, 1H) ; 4,43 (m, 1H) ; 5,02 (s, 2H) ; 6,81 (m, 2H) ; 6,91 (m, 2H) ; 7,37 (m, 5H).

### Intermédiaire 3.19 : (R)-4-((4-(benzyloxy)phénoxy)méthyl)-3-(2-(pipéridin-4-yl) éthyl)oxazolidin-2-one

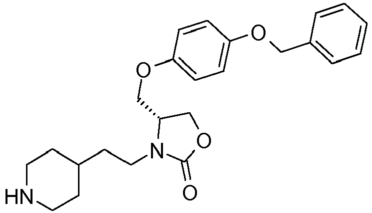

En procédant comme dans l'exemple 3.13 (étape 4), mais en remplaçant le phenol par le 4-(benzyloxy)phenol, on obtient l'intermédiaire 3.19 sous la forme d'un solide crème. Rendement : 60%
¹H RMN (CDCl₃) δ : 1,20 (m, 2H) ; 1,43 (m, 1H) ; 1,50 (m, 2H) ; 1,73 (m, 2H) ; 2,43 (ls, 1H) ; 2,60 (m, 2H) ; 3,10 (m, 2H) ; 3,19 (m, 1H) ; 3,54 (m, 1H) ; 4,01 (d, 2H, J = 4,4 Hz) ; 4,17 (m, 2H) ; 4,44 (m, 1H) ; 5,04 (s, 2H) ; 6,50 (m, 2H) ; 6,64 (m, 1H) ; 7,20 (m, 1H) ; 7,38 (m, 5H) ;

### Intermédiaire 3.20 : (R)-4-((2,6-diméthylphénoxy)méthyl)-3-(2-(pipéridin-4-yl)éthyl) oxazolidin-2-one

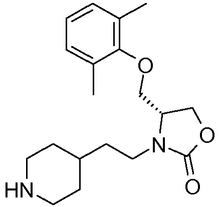

En procédant comme dans l'exemple 3.13 (étape 4),, mais en remplaçant le phenol par le 2,6-diméthylphenol, on obtient l'intermédiaire 3.20 sous la forme d'une huile claire. Rendement : 98%
¹H RMN (CDCl₃) δ : 1,22 (m, 2H) ; 1,44 (m, 1H) ; 1,55 (m, 2H) ; 1,75 (m, 2H) ; 2,11 (ls, 1H) ; 2,27 (s, 6H) ; 2,62 (m, 2H) ; 3,12 (m, 2H) ; 3,26 (m, 1H) ; 3,64 (m, 1H) ; 3,80 (m, 1H) ; 3,93 (m, 1H) ; 4,18 (m, 1H) ; 4,31 (m, 1H) ; 4,50 (m, 1H) ; 6,96 (m, 1H) ; 7,02 (m, 2H).

### Intermédiaire 3.21 : (R)-4-(hydroxyméthyl)-3-(2-(pipéridin-4-yl)éthyl)oxazolidin-2-one

### ∘ Etape 1 : Préparation de (S)-tert-butyl 4-(2-((3-hydroxy-1-methoxy-1-oxopropan-2-yl)amino)éthyl)pipéridine-1-carboxylate

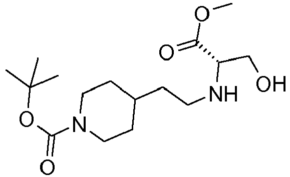

Dans un ballon de 250 ml on introduit 5,33 g (34,3 mmol) du chlorhydrate du (S)-méthyl 2-amino-3-hydroxypropanoate, 100 ml de dichlorométhane et 4,78 ml (34,3 mmol) de triéthylamine. On laisse sous agitation 15 min, puis on additionne 7,79 g (34,3 mmol) de tert-butyl 4-(2-oxoéthyl)pipéridine-1-carboxylate (préparé selon la méthode rapportée dans Journal of Medicinal Chemistry, 2005, Vol. 48, No. 6, p 2100-2107) en solution dans 10 ml de dichlorométhane et 1,25 g de MgSO₄ anhydre. Après une nuit à température ambiante, on filtre, on concentre le milieu, puis on ajoute 70 ml de méthanol et 1,85 g (34.3 mmol) de KBH₄ à 0°C. Au bout de 4h00, on ajoute 100 ml d'acétate d'éthyle, on lave avec 100 ml d'eau. Après 3 extractions à l'acétate d'éthyle, les phases organiques sont rassemblées, séchées sur Na₂SO₄, filtrées et évaporées. On obtient 7,38 g (22,33 mmol) du (S)-tert-butyl 4-(2-((3-hydroxy-1-methoxy-1-oxopropan-2-yl)amino)éthyl)pipéridine-1-carboxylate sous forme d'huile orange, après purification par flash chromatographie sur gel de silice (gradient : dichlorométhane = 100% à dichlorométhane/(méthanol : NH₄OH 9 : 1) = 95/5% sur 30 min).
Rendement : 67%
¹H RMN (CDCl₃) δ : 1,11 (m, 2H) ; 1,45 (s, 9H) ; 1,48 (m, 3H) ; 1,63 (m, 2H) ; 2,00 (lm, 2H) ; 2,53 (m, 1H) ; 2,71 (m, 3H) ; 3,36 (dd, 1H, J = 6,6 et 4,4 Hz) ; 3,57 (dd, 1H, J = 10,6 et 6,8 Hz) ; 3,76 (s, 3H) ; 3,9 (m, 1H) ; 4,07 (m, 2H).

### ∘ Etape 2 : Préparation de (S)-méthyl 3-(2-(1-(tert-butoxycarbonyl)pipéridin-4-yl)éthyl)-2-oxooxazolidine-4-carboxylate

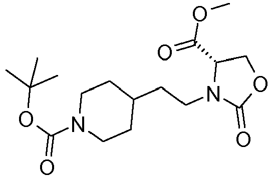

Dans un ballon de 250 ml on solubilise 7,38 g (22,33 mmol) du (S)-tert-butyl 4-(2-((3-hydroxy-1-methoxy-1-oxopropan-2-yl)amino)éthyl)pipéridine-1-carboxylate précédemment décrit dans 80 ml de tétrahydrofurane. On ajoute 7,24 g (44,7 mmol) de 1,1'-carbonyldiimidazole et on porte le milieu à 70°C pendant 8h00. On ajoute ensuite 100 ml d'eau et on extrait 2 fois avec 100 ml d'éther diéthylique. Les phases organiques sont rassemblées, séchées sur Na2SO4, filtrées, évaporées et on obtient 7 g (19,64 mmol) du (S)-méthyl 3-(2-(1-(tert-butoxycarbonyl)pipéridin-4-yl)éthyl)-2-oxooxazolidine-4-carboxylate sous la forme d'huile jaune, après purification sur gel de silice (gradient : dichlorométhane = 100% à dichlorométhane/acétate d'éthyle = 60/40% sur 30 min).
Rendement : 88%
¹H RMN (CDCl₃) δ : 1,12 (m, 2H) ; 1,45 (s, 9H) ; 1,46 (m, 3H) ; 1,69 (m, 2H) ; 2,67 (m, 2H) ; 3,18 (m, 1H) ; 3,62 (m, 1H) ; 3,82 (s, 3H) ; 4,09 (m, 2H) ; 4,34 (m, 2H) ; 4,47 (m, 1H).

### ∘ Etape 3 : Préparation de (R)-tert-butyl 4-(2-(4-(hydroxyméthyl)-2-oxooxazolidin-3-yl)éthyl)pipéridine-1-carboxylate

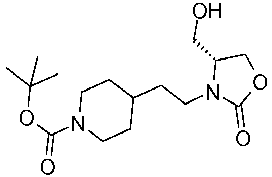

On solubilise 1 g (2,81 mmol) du (S)-méthyl 3-(2-(1-(tert-butoxycarbonyl)pipéridin-4-yl)éthyl)-2-oxooxazolidine-4-carboxylate décrit précédemment dans 10 ml d'éthanol. On se place à 0°C et l'on ajoute très doucement par portions 0,16 g (4,21 mmol) de NaBH₄, en faisant attention à ce que la température ne dépasse pas 5°C. On laisse à 0°C pendant 1h00, puis on laisse revenir doucement à température ambiante. On remet ensuite à 0°C pour rajouter une solution aqueuse saturée de NH₄Cl, puis on ajoute de l'eau et on extrait 3 fois à l'acétate d'éthyle. Les phases organiques sont rassemblées, séchées sur Na₂SO₄, et on obtient 0,9 g (2,74 mmol) du (R)-tert-butyl 4-(2-(4-(hydroxyméthyl)-2-oxooxazolidin-3-yl)éthyl)pipéridine-1-carboxylate, après purification sur gel de silice (gradient : n-heptane = 100% à acétate d'éthyle = 100% sur 30 min).
Rendement : 98%
¹H RMN (CDCl₃) δ : 1,12 (m, 2H) ; 1,44 (m, 1H) ; 1,45 (s, 9H) ; 1,52 (m, 2H) ; 1,68 (m, 2H) ; 2,07 (m, 1H) ; 2,68 (m, 2H) ; 3,17 (m, 1H) ; 3,52 (m, 1H) ; 3,70 (m, 1H) ; 3,79 (m, 1H) ; 3,88 (m, 1H) ; 4,08 (m, 2H) ; 4,20 (m, 1H) ; 4,36 (m, 1H).

### ∘ Etape 4 : Préparation de (R)-4-(hydroxyméthyl)-3-(2-(pipéridin-4-yl)éthyl) oxazolidin-2-one

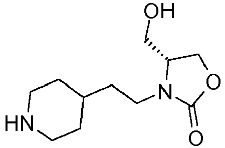

On solubilise 0,75 g (2,30 mmol) dans 10 ml d'éthanol et on ajoute 2 ml (10 mmol) d'une solution d'HCl 5N dans l'isopropanol. On met le milieu à chauffer à 70°C pendant 5h00, puis on concentre le milieu, on basifie et on extrait au dichlorométhane. La phase organique est séchée sur Na2SO4, filtrée, évaporée et on récupère 0,37 g (1,61 mmol) d'huile claire après purification sur gel de silice (éluant : dichlorométhane/méthanol/NH₄OH = 90/9/1%).
Rendement : 70%
¹H RMN (CD₃OD) δ : 1,40 (m, 2H) ; 1,63 (m, 3H) ; 2,02 (m, 2H) ; 2,97 (m, 2H) ; 3,25 (m, 1H) ; 3,37 (m, 2H) ; 3,50 (m, 1H) ; 3,57 (m, 1H) ; 3,75 (m, 1H) ; 3,94 (m, 1H) ; 4,19 (m, 1H) ; 4,39 (m, 1H).

### Intermédiaire 3.22 : (S)-4-(hydroxyméthyl)-3-(2-(pipéridin-4-yl)éthyl)oxazolidin-2-one

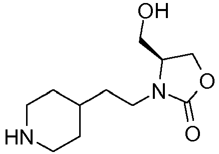

En procédant comme dans l'exemple 3.21, mais en remplaçant le chlorhydrate du (S)-méthyl 2-amino-3-hydroxypropanoate par le chlorhydrate du (R)-méthyl 2-amino-3-hydroxypropanoate, on obtient l'intermédiaire 3.22 sous la forme d'un solide blanc. Rendement : 90%
¹H RMN (CDCl₃) δ : idem intermédiaire 3.21
Intermédiaire 3.23 : (R)-4-(hydroxyméthyl)-5,5-diméthyl-3-(2-(pipéridin-4-yl)éthyl) oxazolidin-2-one

### ∘ Etape 1 : Préparation de (R)-méthyl 2-((tert-butoxycarbonyl)amino)-3-((tert-butyldiméthylsilyl)oxy)propanoate

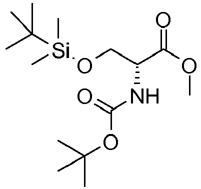

Dans un ballon de 250 ml, on solubilise 10 g (45,6 mmol) de (R)-méthyl 2-((tert-butoxycarbonyl)amino)-3-hydroxypropanoate dans 50 ml de diméthylformamide. On refroidit le milieu à 0°C puis on ajoute 4,66 g (68,4 mmol) d'imidazole et 7,56 g (50,2 mmol) de tert-butylchlorodiméthylsilane. Après retour à température ambiante, on laisse sous agitation pendant 3h00. On concentre ensuite le milieu puis on fait 2 extractions eau/acétate d'éthyle. Les phases organiques sont rassemblées, séchées sur Na₂SO₄ et on obtient 14,5 g (43,48 mmol) de (R)-méthyl 2-((tert-butoxycarbonyl)amino)-3-((tert-butyldiméthylsilyl)oxy)propanoate sous la forme d'huile incolore, après purification par flash chromatographie sur gel de silice (gradient : n-heptane = 100% à n-heptane/acétate d'éthyle = 50/50% sur 30 min).
Rendement : 95%
¹H RMN (CDCl₃) δ : 0,02 (s, 3H) ; 0,04 (s, 3H) ; 0,86 (s, 9H) ; 1,46 (s, 9H) ; 3,74 (s, 3H) ; 3,82 (dd, 1H, J = 10 et 3,2 Hz) ; 4,04 (dd, 1H, J = 10 et 2,4 Hz) ; 4,35 (m, 1H) ; 5,33 (m, 1H).

### ∘ Etape 2 : Préparation de (R)-tert-butyl (1-((tert-butyldiméthylsilyl)oxy)-3-hydroxy-3-méthylbutan-2-yl)carbamate

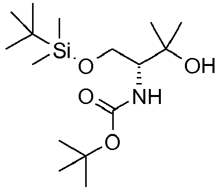

Dans un ballon de 500 ml balayé à l'azote, on solubilise 7 g (20,99 mmol) du (R)-méthyl 2-((tert-butoxycarbonyl)amino)-3-((tert-butyldiméthylsilyl)oxy)propanoate précédemment décrit dans 200 ml de tétrahydrofurane. On rajoute goutte à goutte à 0°C 25 ml (75 mmol) d'une solution 3M dans l'éther de méthyl magnesium bromide. On laisse sous agitation 24h00 à température ambiante. On ajoute une solution saturée de NH₄Cl à 0°C et on extrait 3 fois à l'acétate d'éthyle. Les phases organiques sont rassemblées, séchées sur Na₂SO₄, filtrées, évaporées et on obtient 4,5 g (13,49 mmol) de (R)-tert-butyl (1-((tert-butyldiméthylsilyl)oxy)-3-hydroxy-3-méthylbutan-2-yl) carbamate sous la forme d'huile jaune pâle, après purification par flash chromatographie sur gel de silice (gradient : n-heptane = 100% à n-heptane/acétate d'éthyle = 50/50% sur 30 min).
Rendement : 64%
¹H RMN (CDCl₃) δ : 0,08 (s, 6H) ; 0,90 (s, 9H) ; 1,19 (s, 3H) ; 1,34 (s, 3H) ; 1,45 (s, 9H) ; 3,39 (m, 1H) ; 3,60 (s, 1H) ; 3,85 (dd, 1H, J = 10,6 et 2 Hz) ; 4,06 (dd, 1H, J = 10,6 et 2,4 Hz) ; 5,36 (m, 1H).

### ∘ Etape 3 : Préparation de (R)-4-(((tert-butyldiméthylsilyl)oxy)méthyl)-5,5-diméthyloxazolidin-2-one

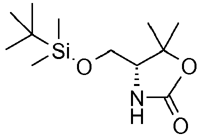

Dans un ballon de 500 ml contenant 50 ml de tétrahydrofurane, on introduit 0,65 g (16,19 mmol) de NaH à 60% dans l'huile; on additionne ensuite goutte à goutte 4,5 g (13,49 mmol) du (R)-tert-butyl(1-((tert-butyldiméthylsilyl)oxy)-3-hydroxy-3-méthylbutan-2-yl)carbamate, précédemment préparé, en solution dans 100 ml de tétrahydrofurane. On porte à reflux pendant 3h00. Après retour à température ambiante, le milieu est versé sur une solution saturée de NH₄Cl et extrait 3 fois avec de l'acétate d'éthyle. Les phases organiques sont séchées sur Na₂SO₄, filtrées, évaporées et on obtient 2,9 g (11,18 mmol) du R)-4-(((tert-butyldiméthylsilyl)oxy)méthyl)-5,5-diméthyloxazolidin-2-one sous la forme d'un solide blanc, après purification par flash chromatographie sur gel de silice (gradient : n-heptane = 100% à n-heptane/acétate d'éthyle = 70/30% sur 30 min).
Rendement : 83%
¹H RMN (CDCl₃) δ : 0,07 (s, 6H) ; 0,88 (s, 9H) ; 1,38 (s, 3H) ; 1,49 (s, 3H) ; 3,60 (m, 3H) ; 5,41 (s, 1H).

### ∘ Etape 4 : Préparation de (R)-tert-butyl 4-(2-(4-(((tert-butyldiméthylsilyl) oxy)méthyl)-5,5-diméthyl-2-oxooxazolidin-3-yl)éthyl)pipéridine-1-carboxylate

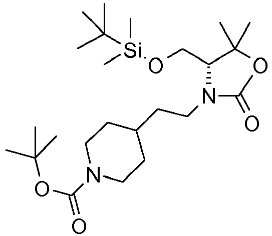

En procédant comme dans l'exemple 3.1 (étape 2), mais en remplaçant la 2-oxazolidinone commerciale par la (R)-4-(((tert-butyldiméthylsilyl)oxy)méthyl)-5,5-diméthyloxazolidin-2-one précédemment préparée, on obtient le de (R)-tert-butyl 4-(2-(4-(((tert-butyldiméthylsilyl)oxy)méthyl)-5,5-diméthyl-2-oxooxazolidin-3-yl)éthyl)
pipéridine-1-carboxylate sous la forme d'une huile jaune pâle.
Rendement : 92%
¹H RMN (CDCl₃) δ : 0,07 (s, 3H) ; 0,08 (s, 3H) ; 0,89 (s, 9H) ; 1,11 (m, 2H) ; 1,40 (s, 3H) ; 1,43 (s, 3H) ; 1,47 (m, 3H) ; 1,68 (m, 2H) ; 2,67 (m, 2H) ; 3,07 (m, 1H) ; 3,37 (m, 1H) ; 3,54 (m, 1H) ; 3,70 (m, 2H) ; 4,07 (m, 2H).

### ∘ Etape 5 : Préparation de (R)-4-(hydroxyméthyl)-5,5-diméthyl-3-(2-(pipéridin-4-yl)éthyl)oxazolidin-2-one

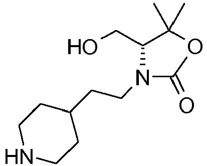

En procédant comme dans l'exemple 3.1 (étape 3), mais en remplaçant le de tert-butyl 4-(2-(2-oxooxazolidin-3-yl)éthyl)pipéridine-1-carboxylate par le (R)-tert-butyl 4-(2-(4-(((tert-butyldiméthylsilyl)oxy)méthyl)-5,5-diméthyl-2-oxooxazolidin-3-yl)éthyl) pipéridine-1-carboxylate précédemment préparé, on obtient le de (R)-tert-butyl 4-(2-(4-(((tert-butyldiméthylsilyl)oxy)méthyl)-5,5-diméthyl-2-oxooxazolidin-3-yl)éthyl) pipéridine-1-carboxylate sous la forme d'une huile jaune pâle.
Rendement : 49%
¹H RMN (CDCl₃) δ : 1,17 (m, 2H) ; 1,43 (s, 3H) ; 1,45 (s, 3H) ; 1,50 (m, 3H) ; 1,73 (m, 2H) ; 2,33 (ls, 2H) ; 2,59 (m, 2H) ; 3,06 (m, 2H) ; 3,15 (m, 1H) ; 3,44 (m, 1H) ; 3,53 (m, 1H) ; 3,78 (d, 2H, J = 5,2 Hz).

### Intermédiaire 3.24 : (S)-4-(2-hydroxypropan-2-yl)-3-(2-(pipéridin-4-yl)éthyl) oxazolidin-2-one

### ∘ Etape 1 : Préparation de (S)-tert-butyl 4-(2-(4-(2-hydroxypropan-2-yl)-2-oxooxazolidin-3-yl)éthyl)pipéridine-1-carboxylate

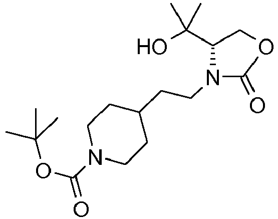

On solubilise 1,5 g (4,21 mmol) du (S)-méthyl 3-(2-(1-(tert-butoxycarbonyl)pipéridin-4-yl)éthyl)-2-oxooxazolidine-4-carboxylate (décrit pour l'intermédiaire 3.21, étape 2) dans 40 ml de tétrahydrofurane. On refroidit le milieu à 0°C et l'on ajoute goutte à goutte 3,4 ml (10,10 mmol) d'une solution 3M dans l'éther de méthyl magnesium bromide. On laisse doucement revenir à température ambiante, puis on laisse 5h00 sous agitation à cette température. On ajoute une solution saturée de NH₄Cl, puis on extrait 3 fois avec de l'acétate d'éthyle. Les phases organiques sont rassemblées, séchées sur Na₂SO₄, filtrées et évaporées. On obtient 1,1 g (3,08 mmol) du (S)-tert-butyl 4-(2-(4-(2-hydroxypropan-2-yl)-2-oxooxazolidin-3-yl)éthyl)pipéridine-1-carboxylate sous la forme d'huile incolore, après purification par flash chromatographie sur gel de silice (gradient : n-heptane = 100% à n-heptane/acétate d'éthyle = 80/20% sur 30 min).
Rendement : 73%
¹H RMN (CDCl₃) δ : 1,14 (m, 2H) ; 1,18 (s, 3H) ; 1,22 (s, 3H) ; 1,38 (m, 1H) ; 1,45 (s, 9H) ; 1,66 (m, 4H) ; 2,59 (m, 1H) ; 2,68 (m, 2H) ; 3,43 (m, 1H) ; 3,64 (m, 1H) ; 3,73 (m, 1H) ; 4,05 (m, 3H) ; 4,25 (m, 1H).

### ∘ Etape 2 : Préparation de (S)-4-(2-hydroxypropan-2-yl)-3-(2-(pipéridin-4-yl)éthyl)oxazolidin-2-one

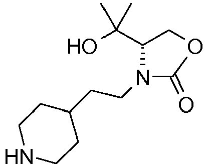

En procédant comme dans l'exemple 3.1 (étape 3), mais en remplaçant le tert-butyl 4-(2-(2-oxooxazolidin-3-yl)éthyl)pipéridine-1-carboxylate par le (S)-tert-butyl 4-(2-(4-(2-hydroxypropan-2-yl)-2-oxooxazolidin-3-yl)éthyl)pipéridine-1-carboxylate précédemment préparé, on obtient le (S)-4-(2-hydroxypropan-2-yl)-3-(2-(pipéridin-4-yl)éthyl)oxazolidin-2-one sous la forme d'un solide jaune.
Rendement : 94%
¹H RMN (CDCl₃) δ : 1,22 (s, 3H) ; 1,24 (m, 2H) ; 1,25 (s, 3H) ; 1,41 (m, 1H) ; 1,57 (m, 2H) ; 1,76 (m, 2H) ; 1,88 (ls, 2H) ; 2,62 (m, 2H) ; 3,11 (m, 2H) ; 3,39 (m, 1H) ; 3,65 (m, 1H) ; 3,74 (m, 1H) ; 4,00 (m, 1H) ; 4,27 (m, 1H).

### Intermédiaire 3.25 : (4R)-4-(((tert-butyldiméthylsilyl)oxy)méthyl)-5-phenyl-3-(2-(pipéridin-4-yl)éthyl)oxazolidin-2-one

### ∘ Etape 1 : Préparation de tert-butyl 4-(2-((4R)-4-(((tert-butyldiméthylsilyl) oxy)méthyl)-2-oxo-5-phenyloxazolidin-3-yl)éthyl)pipéridine-1-carboxylate

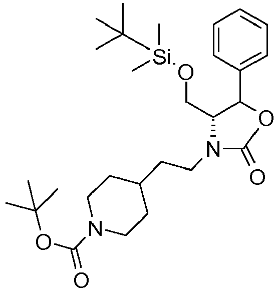

En procédant comme dans l'exemple 3.1 (étape 2), mais en remplaçant la 2-oxazolidinone commerciale par la (4R)-4-(((tert-butyldiméthylsilyl)oxy)méthyl)-5-phenyloxazolidin-2-one, synthétisée selon J. Org. Chem., 63, N°24, 1998, p 8837-8842*,* on obtient le tert-butyl 4-(2-((4R)-4-(((tert-butyldiméthylsilyl)oxy)méthyl)-2-oxo-5-phenyloxazolidin-3-yl)éthyl)pipéridine-1-carboxylate sous la forme d'une huile incolore. L'isomère majoritaire est le tert-butyl 4-(2-((4R,5R)-4-(((tert-butyldiméthylsilyl)oxy)méthyl)-2-oxo-5-phenyloxazolidin-3yl)éthyl)pipéridine-1-carboxylate si l'on se réfère à la synthèse utilisée pour préparer l'oxazolidinone ; toutefois ce produit doit également contenir un peu de l'autre isomère tert-butyl 4-(2-((4R,5 S)-4-(((tert-butyldiméthylsilyl)oxy)méthyl)-2-oxo-5-phenyloxazo lidin-3-yl)éthyl) pipéridine-1-carboxylate.
Rendement : 78%
¹H RMN (CDCl₃) δ : 0,10 (s, 3H) ; 0,11 (s, 3H) ; 0,92 (s, 9H) ; 1,06 (m, 2H) ; 1,30 (m, 1H) ; 1,44 (s, 9H) ; 1,46 (m, 2H) ; 1,54 (m, 1H) ; 1,70 (m, 1H) ; 2,62 (m, 2H) ; 3,13 (m, 1H) ; 3,58 (m, 2H) ; 3,77 (d, 2H, J = 4 Hz) ; 4,05 (lm, 2H) ; 5,24 (d, 1H, J = 4,8 Hz) ; 7,37 (m, 5H).

### ° Etape 2 : Préparation de tert-butyl 4-(2-((4R)-4-(hydroxyméthyl)-2-oxo-5-phenyloxazolidin-3-yl)éthyl)pipéridine-1-carboxylate

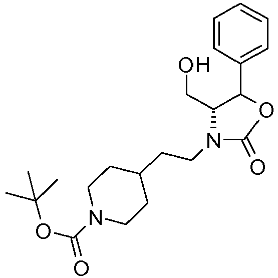

On solubilise 2,4 g (4,63 mmol) du tert-butyl 4-(2-((4R)-4-(((tert-butyldiméthylsilyl)oxy)méthyl)-2-oxo-5-phenyloxazolidin-3-yl)éthyl)pipéridine-1-carboxylate précédemment préparé dans 25 ml de tétrahydrofurane. On ajoute goutte à goutte à température ambiante 6,9 ml (6,9 mmol) d'une solution de tetrabutyl ammonium fluoride 1M dans le tétrahydrofurane et on laisse sous agitation à température ambiante pendant 3h00. On concentre le milieu, puis on fait un lavage avec une solution aqueuse à 10% de NaHCO₃ et on extrait 3 fois avec le l'acétate d'éthyle. Les phases organiques sont séchées sur Na₂SO₄, filtrées, évaporées et on obtient 1,7 g (4,2 mmol) de tert-butyl 4-(2-((4R)-4-(hydroxyméthyl)-2-oxo-5-phenyloxazolidin-3-yl)éthyl)pipéridine-1-carboxylate sous la forme d'huile incolore après purification par flash chromatographie sur gel de silice (gradient : n-heptane = 100% à n-heptane/acétate d'éthyle = 80/20% sur 30 min).
Rendement : 91%
¹H RMN (CDCl₃) δ : 1,08 (m, 2H) ; 1,35 (m, 1H) ; 1,44 (s, 9H) ; 1,48 (m, 2H) ; 1,58 (m, 1H) ; 1,59 (1s, 2H) ; 1,71 (m, 1H) ; 2,63 (m, 2H) ; 3,18 (m, 1H) ; 3,58 (m, 1H) ; 3,65 (m, 1H) ; 3,79 (m, 1H) ; 3,88 (m, 1H) ; 4,05 (m, 2H) ; 5,34 (d, 1H, J = 5,6 Hz) ; 7,38 (m, 5H).

### ° Etape 3 : Préparation de (4R)-4-(hydroxyméthyl)-5-phenyl-3-(2-(pipéridin-4-yl)éthyl)oxazolidin-2-one

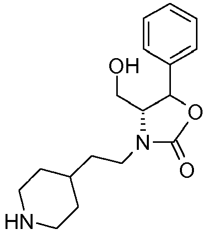

En procédant comme dans l'exemple 3.1 (étape 3), mais en remplaçant le tert-butyl 4-(2-(2-oxooxazolidin-3-yl)éthyl)pipéridine-1-carboxylate par le tert-butyl 4-(2-((4R)-4-(hydroxyméthyl)-2-oxo-5-phenyloxazolidin-3-yl)éthyl)pipéridine-1-carboxylate précédemment préparé, on obtient le (4R)-4-(hydroxyméthyl)-5-phenyl-3-(2-(pipéridin-4-yl)éthyl)oxazolidin-2-one sous la forme d'un solide jaune pâle.
Rendement : 91 %
¹H RMN (CDCl₃) δ : 1,20 (m, 2H) ; 1,37 (m, 1H) ; 1,49 (m, 2H) ; 1,64 (m, 1H) ; 1,77 (m, 1H) ; 2,10 (lm, 2H) ; 2,58 (m, 2H) ; 3,09 (m, 2H) ; 3,19 (m, 1H) ; 3,57 (m, 1H) ; 3,65 (m, 1H) ; 3,79 (dd, 1H, J = 11,6 et 7,6 Hz) ; 3,88 (dd, 1H, J = 11,6 et 4 Hz) ; 5,34 (d, 1H, J = 5,6 Hz) ; 7,34 (m, 5H).

### Intermédiaire 3.26 : (S)-4-((R ou S)-hydroxy(phenyl)méthyl)-3-(2-(pipéridin-4-yl) éthyl)oxazolidin-2-one

### ° Etape 1 : Préparation de (S)-tert-butyl 4-(2-(4-formyl-2-oxooxazolidin-3-yl)éthyl)pipéridine-1-carboxylate

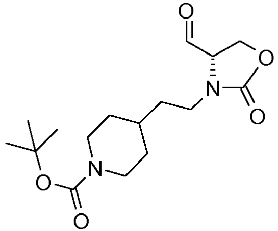

Dans un ballon de 250 ml balayé à l'azote, on solubilise 2,5 g de (S)-méthyl 3-(2-(1-(tert-butoxycarbonyl)pipéridin-4-yl)éthyl)-2-oxooxazolidine-4-carboxylate (intermédiaire 3.21, étape 2) dans 100 ml de dichlorométhane. On refroidit à -78°C et l'on ajoute goutte à goutte 17,54 ml (17,54 mmol) d'une solution de DIBAL 1M dans le dichlorométhane. Après 3h00 à -78°C, on ajoute du méthanol et une solution saturée de sel de rochelle. On enlève le bain froid et on laisse 1h00 sous agitation avant d'extraire 3 fois au dichlorométhane. Les phases organiques sont ensuite séchées sur Na₂SO₄, filtrées et concentrées. L'aldéhyde ainsi obtenu est utilisé brut pour l'étape suivante.

### ° Etape 2 : Préparation de tert-butyl 4-(2-((S)-4-((R ou S)-hydroxy (phenyl)méthyl)-2-oxooxazolidin-3-yl)éthyl)pipéridine-1-carboxylate

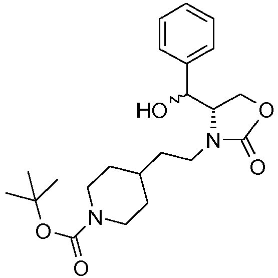

Dans un ballon de 150 ml balayé à l'azote, on solubilise 2,28 g (produit brut) de (S)-tert-butyl 4-(2-(4-formyl-2-oxooxazolidin-3-yl)éthyl)pipéridine-1-carboxylate, précédemment préparé dans 25 ml de tétrahydrofurane. On refroidit à 0°C, puis on additionne goutte à goutte 5,12 ml (15,37 mmol) d'une solution de bromure de phényl magnésium 3M dans l'éther diéthylique. On laisse 1h30 sous agitation à 0°C, puis on laisse remonter à température ambiante une nuit. On rajoute à 0°C 30 ml d'une solution saturée de NH₄Cl et on extrait 3 fois à l'acétate d'éthyle. Les phases organiques sont ensuite séchées sur Na₂SO₄, filtrées, évaporées et on obtient 1,8 g (4,45 mmol) de tert-butyl -(2-((4S)-4-(hydroxy(phenyl)méthyl)-2-oxooxazolidin-3-yl)éthyl) pipéridine-1-carboxylate sous la forme d'huile jaune pâle, après purification par flash chromatographie sur gel de silice (gradient : n-heptane = 100% à acétate d'éthyle = 100% sur 30 min)
Rendement : 64%

Les deux isomères formés sont séparables sur phase inverse : HPLC (XBRIDGE C8, acétonitrile/eau/KH₂PO₄ (60/40/6,8G PH 4), 1 ml/min), temps de rétention intermédiaire 3.26 (étape 2) = 5,34 min, temps de rétention intermédiaire 3.27 (étape 2) = 5,73 min.
¹H RMN (CDCl₃) δ : 1,12 (m, 2H) ; 1,39 (m, 1H) ; 1,44 (s, 9H) ; 1,63 (m, 4H) ; 2,47 (1s, 1H) ; 2,66 (m, 2H) ; 3,41 (m, 1H) ; 3,61 (m, 1H) ; 3,86 (m, 1H) ; 3,96 (m, 1H) ; 4,06 (m, 3H) ; 4,74 (d, 1H, J = 6,8 Hz) ; 7,37 (m, 5H).

### ° Etape 3 : Préparation de (S)-4-((R ou S)-hydroxy(phenyl)méthyl)-3-(2-(pipéridin-4-yl)éthyl)oxazolidin-2-one

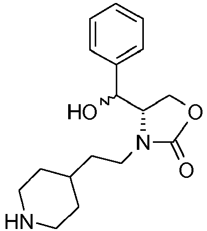

En procédant comme dans l'exemple 3.1 (étape 3), mais en remplaçant le tert-butyl 4-(2-(2-oxooxazolidin-3-yl)éthyl)pipéridine-1-carboxylate par le tert-butyl 4-(2-((S)-4-((R ou S)-hydroxy(phenyl)méthyl)-2-oxooxazolidin-3-yl)éthyl)pipéridine-1-carboxylate précédemment préparé, on obtient le (S)-4-((R ou S)-hydroxy(phenyl)méthyl)-3-(2-(pipéridin-4-yl)éthyl)oxazolidin-2-one sous la forme d'une huile jaune pâle.
Rendement : 90%
¹H RMN (CDCl₃) δ : 1,17 (m, 2H) ; 1,35 (m, 1H) ; 1,57 (m, 1H) ; 1,66 (m, 3H) ; 2,51 (m, 2H) ; 2,93 (m, 2H) ; 3,42 (m, 1H) ; 3,59 (m, 1H) ; 3,84 (m, 1H) ; 3,93 (m, 1H) ; 4,05 (m, 1H) ; 4,69 (d, 1H, J = 7,2 Hz) ; 7,36 (m, 5H).

### Intermédiaire 3.27 : (S)-4-((S ou R)-hydroxy(phenyl)méthyl)-3-(2-(pipéridin-4yl) éthyl)oxazolin-2-one

### ° Etape 2 : Préparation de tert-butyl 4-(2-((S)-4-((S ou R)-hydroxy (phenyl)méthyl)-2-oxooxazolidin-3-yl)éthyl)pipéridine-1-carboxylate

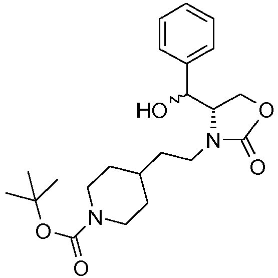

Ce produit est obtenu par séparation sur phase inverse (intermédiaire 3.26, étape 2)
¹H RMN (CDCl₃) δ : 1,09 (m, 2H) ; 1,39 (m, 1H) ; 1,45 (s, 9H) ; 1,47 (m, 2H) ; 1,64 (m, 2H) ; 2,63 (m, 3H) ; 2,95 (m, 1H) ; 3,54 (m, 1H) ; 3,98 (m, 1H) ; 4,09 (m, 3H) ; 4,38 (m, 1H) ; 4,93 (d, 1H, J = 3,2 Hz) ; 7,37 (m, 5H). 1,08 (m, 2H) ; 1,33 (m, 1H) ;

### ° Etape 3 : Préparation de (S)-4-((S ou R)-hydroxy(phenyl)méthyl)-3-(2-(pipéridin-4yl)éthyl)oxazolin-2-one

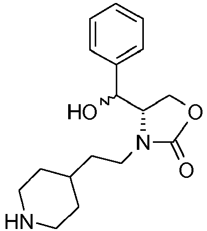

En procédant comme dans l'exemple 3.1 (étape 3), mais en remplaçant le tert-butyl 4-(2-(2-oxooxazolidin-3-yl)éthyl)pipéridine-1-carboxylate par le tert-butyl 4-(2-((S)-4-((S ou R)-hydroxy(phenyl)méthyl)-2-oxooxazolidin-3-yl)éthyl)pipéridine-1-carboxylate précédemment préparé, on obtient le (S)-4-((S ou R)-hydroxy(phenyl)méthyl)-3-(2-(pipéridin-4-yl)éthyl)oxazolidin-2-one sous la forme d'une huile jaune pâle.
Rendement : 90%
¹H RMN (CDCl₃) δ : 1,08 (m, 2H) ; 1,33 (m, 1H) ; 1,47 (m, 2H) ; 1,64 (m, 2H) ; 2,51 (m, 3H) ; 2,95 (m, 3H) ; 3,54 (m, 1H) ; 3,96 (m, 1H) ; 4,03 (m, 1H) ; 4,37 (m, 1H) ; 4,92 (d, 1H, J = 3,6 Hz) ; 7,35 (m, 5H).

### Intermédiaire 3.28 : 4-(2-(pipéridin-4-yl)éthyl)morpholin-3-one

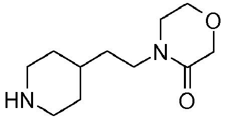

En procédant comme dans l'exemple 3.1 (étapes 2 et 3), mais en remplaçant la 2-oxazolidinone commerciale par la morpholin-3-one, on obtient la 4-(2-(pipéridin-4-yl)éthyl)morpholin-3-one sous la forme d'une huile claire.
¹H RMN (CDCl₃) δ : 1,18 (m, 2H) ; 1,40 (m, 1H) ; 1,49 (m, 2H) ; 1,73 (m, 2H) ; 2,21 (s, 1H) ; 2,59 (m, 2H) ; 3,08 (m, 2H) ; 3,35 (m, 2H) ; 3,45 (m, 2H) ; 3,88 (m, 2H) ; 4,16 (s, 2H).

### Intermédiaire 3.29 : (R)-6-phenyl-4-(2-(pipéridin-4-yl)éthyl)morpholin-3-one

### ° Etape 1 : Préparation de tert-butyl 4-(2-(1,3-dioxoisoindolin-2-yl)éthyl) pipéridine-1-carboxylate

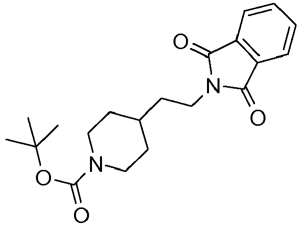

Dans un ballon de 250 ml, on solubilise 5 g (21,80 mmol) de tert-butyl 4-(2-hydroxyéthyl)pipéridine-1-carboxylate dans 80 ml de tétrahydrofurane. On ajoute 5,75 g 21,92 mmol) de Ph₃P et 3,25 g (22,08 mmol) de phtalimide. On refroidit le milieu à 0°C et l'on ajoute goutte à goutte 4,30 ml (21,84 mmol) de DIAD. On laisse ensuite revenir à température ambiante, puis on laisse sous agitation 24h00. On concentre le milieu et on obtient 5,5 g (15,34 mmol) de tert-butyl 4-(2-(1,3-dioxoisoindolin-2-yl)éthyl)pipéridine-1-carboxylate sous la forme d'un solide blanc, après purification par flash chromatographie sur gel de silice (éluant : n-heptane/acétatone = 80/20%).
Rendement : 70%
¹H RMN (DMSOd₆) δ : 0,98 (m, 2H) ; 1,38 (s, 9H) ; 1,40 (1s, 1H) ; 1,52 (m, 2H) ; 1,70 (m, 2H) ; 2,65 (m, 2H) ; 3,60 (m, 2H) ; 3,90 (m, 2H) ; 7,85 (m, 4H).

### ° Etape 2 : Préparation de tert-butyl 4-(2-aminoéthyl)pipéridine-1-carboxylate

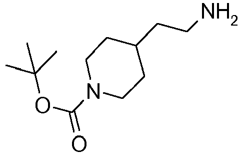

On solubilise 5,5 g (15,34 mmol) du tert-butyl 4-(2-(1,3-dioxoisoindolin-2-yl)éthyl) pipéridine-1-carboxylate préparé précédemment dans 40 ml d'éthanol amine et on laisse sous agitation à température ambiante pendant 5h30. On verse ensuite dans 150 ml d'eau et on extrait 2 fois avec 200 ml de dichlorométhane. Les phases organiques sont séchées sur MgSO₄, filtrées, évaporées et on obtient 2,74 g (11,95 mmol) de tert-butyl 4-(2-aminoéthyl)pipéridine-1-carboxylate sous la forme d'une huile claire, après purification par flash chromatographie sur gel de silice (éluant : dichlorométhane/ méthanol/NH4OH = 90/9/1%).
Rendement : 78%
¹H RMN (CDCl₃) δ : 1,10 (m, 2H) ; 1,40 (m, 2H) ; 1,45 (s, 9H) ; 1,49 (1s ; 3H) ; 1,64 (m, 2H) ; 3,58 (m, 4H) ; 4,07 (m, 2H).

### ° Etape 3 : Préparation de (R)-tert-butyl 4-(2-((2-hydroxy-2-phenyléthyl) amino)éthyl)pipéridine-1-carboxylate

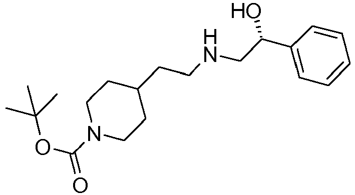

Dans un ballon de 100 ml, on introduit 2,74 g (11,95 mmol) du tert-butyl 4-(2-aminoéthyl)pipéridine-1-carboxylate précédemment préparé, on ajoute 0,68 ml (5,94 mmol) de (R)-2-phenyloxirane et 5 ml de tétrahydrofurane. On chauffe à 70°C pendant 40h00. On reprend ensuite le milieu avec 50 ml d'eau et on extrait 2 fois avec 100 ml de dichlorométhane. Les phases organiques sont ensuite séchées sur MgSO₄, filtrées et on obtient 1,17 g (3,36 mmol) de (R)-tert-butyl 4-(2-((2-hydroxy-2-phenyléthyl) amino)éthyl)pipéridine-1-carboxylate sous la forme une huile crème qui tend à se solidifier, après purification par flash chromatographie sur gel de silice (éluant : dichlorométhane/méthanol = 90/10%).
Rendement : 57%
¹H RMN (CDCl₃) δ : 1,10 (m, 2H) ; 1,43 (m, 3H) ; 1,45 (s, 9H) ; 1,63 (m, 2H) ; 2,68 (m, 5H) ; 2,89 (m, 1H) ; 4,06 (m, 2H) ; 4,71 (dd, 1H, J = 8,9 et 3,4 Hz) ; 7,28 (m, 1H) ; 7,35 (m, 4H).

### ° Etape 4 : Préparation de (R)-tert-butyl 4-(2-(5-oxo-2-phenylmorpholino) éthyl)pipéridine-1-carboxylate

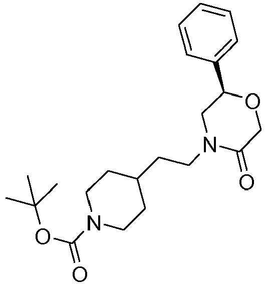

Dans un ballon de 100 ml, on solubilise 1,17 g (3,36 mmol) du (R)-tert-butyl 4-(2-((2-hydroxy-2-phenyléthyl)amino)éthyl)pipéridine-1-carboxylate précédemment préparé dans 5 ml de tétrahydrofurane et 3 ml d'eau. On ajoute 2 ml d'une solution aqueuse de NaOH à 30% et 0,1 g (0,32 mmol) de benzyl tributyl ammonium chloride. On refroidit ensuite le milieu à 0°C et on ajoute goutte à goutte 0,53 ml (6,66 mmol) de 2-chloroacetyl chloride et une pointe de spatule de KI. On laisse sous agitation à température ambiante pendant 24 h00. On ajoute ensuite 50 ml d'eau et on extrait 2 fois avec du dichlorométhane. Les phases organiques sont séchées sur MgSO₄, filtrées, évaporées et on obtient 1,15 g (2,96 mmol) de (R)-tert-butyl 4-(2-(5-oxo-2-phenylmorpholino)éthyl)pipéridine-1-carboxylate sous la forme d'huile claire, après purification par flash chromatographie sur gel de silice (éluant : dichlorométhane/ acétate d'éthyle = 70/30%).
Rendement : 88%
¹H RMN (CDCl₃) δ : 1,12 (m, 2H) ; 1,45 (1s, 10H) ; 1,52 (m, 2H) ; 1,71 (m, 2H) ; 2,68 (m, 2H) ; 3,36 (m, 2H) ; 3,55 (m, 2H) ; 4,07 (m, 2H) ; 4,31 (d, 1H, J = 16,6 Hz) ; 4,43 (d, 1H, J = 16,6 Hz) ; 4,79 (dd, 1H, J = 10,4 et 2,4 Hz) ; 7,36 (m, 5H).

### ° Etape 5 : Préparation de (R)-6-phenyl-4-(2-(pipéridin-4-yl)éthyl) morpholin-3-one

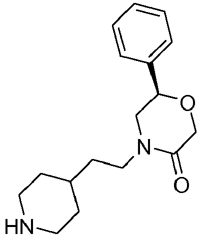

En procédant comme dans l'exemple 3.1 (étape 3), mais en remplaçant le tert-butyl 4-(2-(2-oxooxazolidin-3-yl)éthyl)pipéridine-1-carboxylate par le (R)-tert-butyl 4-(2-(5-oxo-2-phenylmorpholino)éthyl)pipéridine-1-carboxylate précédemment préparé, on obtient le (R)-6-phenyl-4-(2-(pipéridin-4-yl)éthyl)morpholin-3-one sous la forme d'une huile claire.
Rendement : 93%
¹H RMN (CDCl₃) δ : 1,18 (m, 2H) ; 1,40 (m, 1H) ; 1,53 (m, 2H) ; 1,72 (m, 2H) ; 2,23 (1s, 1H) ; 2,60 (m, 2H) ; 3,09 (m, 2H) ; 3,36 (m, 2H) ; 3,55 (m, 2H) ; 4,31 (d, 1H, J = 16,6 Hz) ; 4,43 (d, 1H, J = 16,6 Hz) ; 4,79 (dd, 1H, J = 10,4 et 2,6 Hz) ; 7,37 (m, 5H).

### Intermédiaire 3.30 : 3-(3-(pipéridin-4-yl)propyl)oxazolidin-2-one

### ° Etape 1 : tert-butyl 4-(3-hydroxypropyl)pipéridine-1-carboxylate

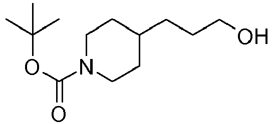

Dans un ballon de 500 ml balayé à l'azote, on solubilise 5 g (17,53 mmol) de tert-butyl 4-(3-methoxy-3-oxopropyl)pipéridine-1-carboxylate (préparé selon la méthode rapportée dans J. Med. Chem. 1995, 38, p 3332-3341 en prenant la tert-butyl 4-(hydroxyméthyl)pipéridine-1-carboxylate commercial comme produit de départ) dans 100 ml de dichlorométhane. On refroidit le milieu à -78°C et l'on ajoute goutte à goutte 52,6 ml (52,6 mmol) d'une solution de DIBAL-H 1M dans le dichlorométhane. On agite 2h00 à 0°C. On refroidit à -78°C et l'on ajoute une solution aqueuse saturée desel de rochelle. Après retour à température ambiante, on extrait ; la phase organique est séchée sur Na₂SO₄, filtrée, évaporée et on obtient 4,5 g d'huile brute utilisée tel quel dans l'étape suivante.

### ° Etape 1 : tert-butyl 4-(3-iodopropyl)pipéridine-1-carboxylate

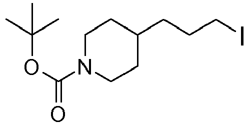

En procédant comme dans l'exemple 3.1 (étape 1), mais en remplaçant le tert-butyl 4-(2-hydroxyéthyl)pipéridine-1-carboxylate commercial par le tert-butyl 4-(3-hydroxypropyl)pipéridine-1-carboxylate précédemment préparé, on obtient le tert-butyl 4-(3-iodopropyl)pipéridine-1-carboxylate sous la forme d'huile incolore.
Rendement : 85%
¹H RMN (CDCl₃) δ : 1,10 (m, 2H) ; 1,35 (m, 3H) ; 1,45 (s, 9H) ; 1,64 (m, 2H) ; 1,85 (m, 2H) ; 2,67 (m, 2H) ; 3,17 (t, 2H, J = 6,8 Hz) ; 4,06 (m, 2H).

### ° Etape 2 : Préparation tert-butyl 4-(3-(2-oxooxazolidin-3-yl)propyl) pipéridine-1-carboxylate

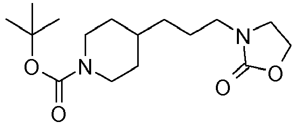

En procédant comme dans l'exemple 3.1 (étape 2), mais en remplaçant le tert-butyl 4-(2-iodoéthyl)pipéridine-1-carboxylate par le tert-butyl 4-(3-iodopropyl)pipéridine-1-carboxylate précédemment préparé, on obtient le tert-butyl 4-(3-(2-oxooxazolidin-3-yl)propyl)pipéridine-1-carboxylate sous la forme de solide blanc.
Rendement : 81 %
¹H RMN (CDCl₃) δ : 1,08 (m, 2H) ; 1,26 (m, 2H) ; 1,39 (m, 1H) ; 1,45 (s, 9H) ; 1,60 (m, 4H) ; 2,67 (m, 2H) ; 3,25 (t, 2H, J = 7,6 Hz) ; 3,55 (m, 2H) ; 4,07 (m, 2H) ; 4,32 (m, 2H).

### ° Etape 3 : Préparation de 3-(3-(pipéridin-4-yl)propyl)oxazolidin-2-one

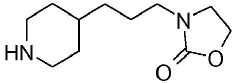

En procédant comme dans l'exemple 3.1 (étape 3), mais en remplaçant le tert-butyl 4-(2-(2-oxooxazolidin-3-yl)éthyl)pipéridine-1-carboxylate par le tert-butyl 4-(3-(2-oxooxazolidin-3-yl)propyl)pipéridine-1-carboxylate précédemment préparé, on obtient le 3-(3-(pipéridin-4-yl)propyl)oxazolidin-2-one sous la forme d'huile claire.
Rendement : 90%
¹H RMN (CDCl₃) δ : 1,25 (m, 4H) ; 1,40 (m, 1H) ; 1,56 (m, 2H) ; 1,71 (m, 2H) ; 2,63 (m, 2H) ; 2,68 (1s, 1H) ; 3,14 (m, 2H) ; 3,25 (m, 2H) ; 3,56 (m, 2H) ; 4,33 (m, 2H).

### Intermédiaire 3.31 : (S)-4-benzyl-3-(3-(pipéridin-4-yl)propyl)oxazolidin-2-one

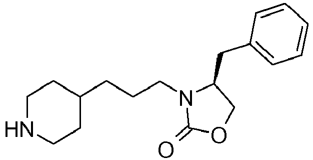

En procédant comme dans l'exemple 3.1, mais en remplaçant la 2-oxazolidinone commerciale par la (S)-4-benzyloxazolidin-2-one commerciale, on obtient la (S)-4-benzyl-3-(3-(pipéridin-4-yl)propyl)oxazolidin-2-one sous la forme d'huile jaune pâle. Le produit est utilisé brut.

### Intermédiaire 3.32 : (R)-4-phenyl-3-(3-(pipéridin-4-yl)propyl)-oxazolidin-2-one

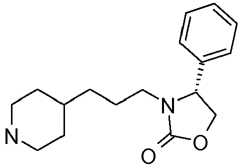

En procédant comme dans l'exemple 3.1, mais en remplaçant la 2-oxazolidinone commerciale par la (R)-(-)-4-phenyl-oxazolidin-2-one commerciale, on obtient la (R)-4-phenyl-3-(3-(pipéridin-4-yl)propyl)oxazolidin-2-one sous la forme d'huile claire. Le produit est utilisé brut.

### Intermédiaire 3.33 : 4- (3-pipéridin-4-yl-propyl)-morpholin-3-one

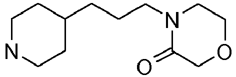

En procédant comme dans l'exemple 3.1, mais en remplaçant la 2-oxazolidinone commerciale par la morpholin-3-one commerciale, on obtient la 4- (3-pipéridin-4-yl-propyl)-morpholin-3-one sous la forme d'huile jaune clair. Le produit est utilisé brut.

### Intermédiaire 3.34 : 3- (4-pipéridin-4-yl-butyl)-oxazolidin-2-one

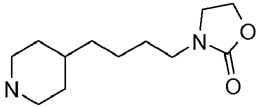

En procédant comme dans l'exemple 3.1 où on utilise la 2-oxazolidinone commerciale, mais en remplaçant dans l'étape 1 la tert-butyl-4-(2-iodoéthyl)pipéridine-1-carboxylate par la tert-butyl-4-(4-iodobutyl)pipéridine-1-carboxylate que l'on prépare par réduction à LiAlH4 de l'ester commercial comme décrit dans WO2008/130718.

| Ex | structure | intermédiaires | schéma réactionnel |
|---|---|---|---|
| 1 | 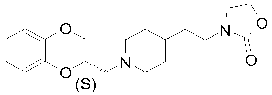 | 4.1/2.1 | schéma 2 |
| | | 3.1 | schéma 3 |
| 2 | 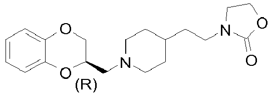 | 4.2/2.2 | schéma 2 |
| | | 3.1 | schéma 3 |
| 3 | 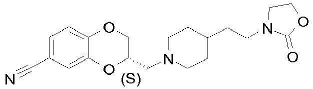 | 4.10/2.11 | schéma 2 |
| | | 3.1 | schéma 3 |
| 4 | 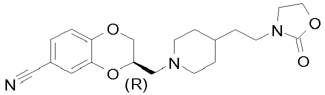 | 4.26/2.12 | schéma 2 |
| | | 3.1 | schéma 3 |
| 5 | 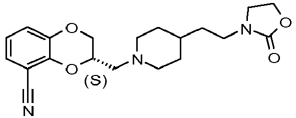 | 4.11/2.13 | schéma 2 |
| | | 3.1 | schéma 3 |
| 6 | 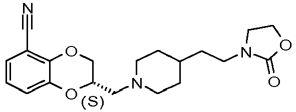 | 4.25/2.14 | schéma 2 |
| | | 3.1 | schéma 3 |
| 7 | 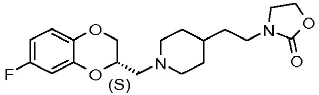 | 4.6/2.17 | schéma 2 |
| | | 3.1 | schéma 3 |
| 8 | 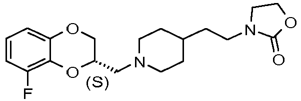 | 4.17/2.16 | schéma 2 |
| | | 3.1 | schéma 3 |
| 9 | 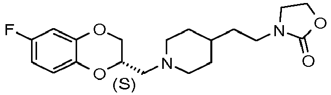 | 4.18/2.18 | schéma 2 |
| | | 3.1 | schéma 3 |
| 10 | 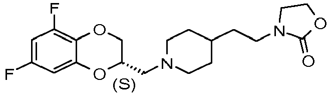 | 4.19/2.19 | schéma 2 |
| | | 3.1 | schéma 3 |
| 11 | 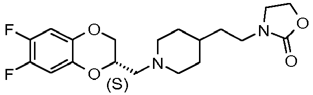 | 4.22/2.22 | schéma 2 |
| | | 3.1 | schéma 3 |
| 12 | 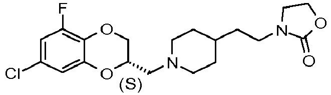 | 4.20/2.20 | schéma 2 |
| | | 3.1 | schéma 3 |
| 13 | 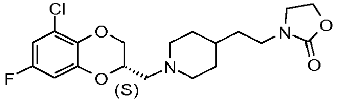 | 4.21/2.21 | schéma 2 |
| | | 3.1 | schéma 3 |
| 14 | 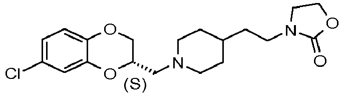 | 4.5/2.7 | schéma 2 |
| | | 3.1 | schéma 3 |
| 15 | 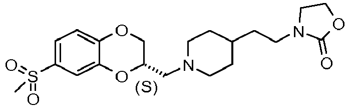 | 4.23/2.23 | schéma 2 |
| | | 3.1 | schéma 3 |
| 16 | 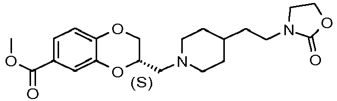 | 4.24/2.24 | schéma 2 |
| | | 3.1 | schéma 3 |
| 17 | 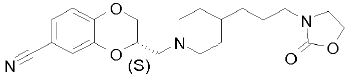 | 4.10/2.11 | schéma 2 |
| | | 3.30 | schémas 6 et 4 |
| 18 | 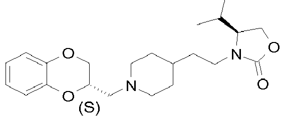 | 4.1/2.1 | schéma 2 |
| | | 3.2 | schéma 3 |
| 19 | 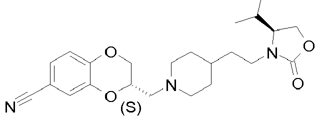 | 4.10/2.11 | schéma 2 |
| | | 3.2 | schéma 3 |
| 20 | 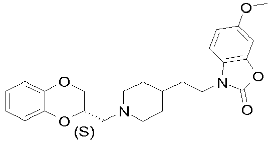 | 4.1/2.1 | schéma 2 |
| | | 3 | schéma 3 |
| 21 | 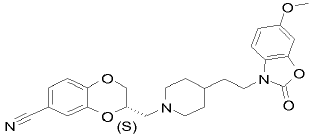 | 4.10/2.11 | schéma 2 |
| | | 3.3 | schéma 3 |
| 22 | 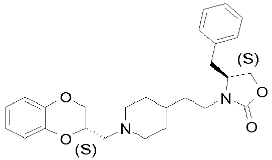 | 4.1/2.1 | schéma 2 |
| | | 3.4 | schéma 3 |
| 23 | 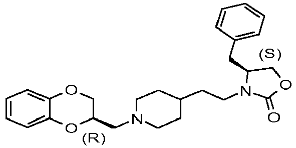 | 4.2/2.2 | schéma 2 |
| | | 3.4 | schéma 3 |
| 24 | 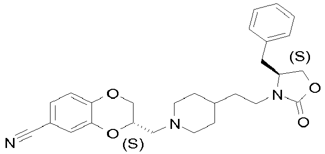 | 4.10/2.11 | schéma 2 |
| | | 3.4 | schéma 3 |
| 25 | 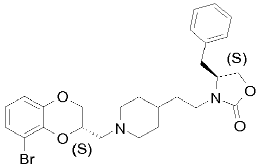 | 4.7/2.4 | schéma 2 |
| | | 3.4 | schéma 3 |
| 26 | 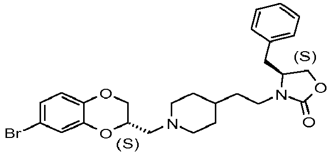 | 4.8/2.5 | schéma 2 |
| | | 3.4 | schéma 3 |
| 27 | 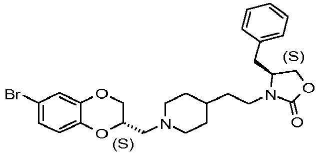 | 4.9/2.6 | schéma 2 |
| | | 3.4 | schéma 3 |
| 28 | 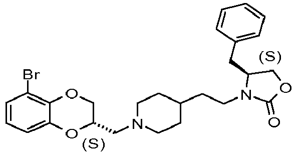 | 4.16/2.15 | schéma 2 |
| | | 3.4 | schéma 3 |
| 29 | 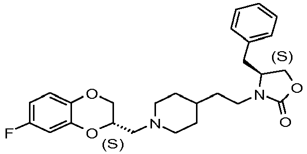 | 4.6/2.17 | schéma 2 |
| | | 3.4 | schéma 3 |
| 30 | 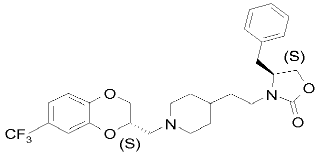 | 4.4/2.10 | schéma 2 |
| | | 3.4 | schéma 3 |
| 31 | 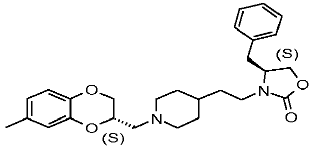 | 4.3/2.3 | schéma 2 |
| | | 3.4 | schéma 3 |
| 32 | 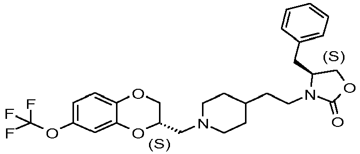 | 4.14/2.8 | schéma 2 |
| | | 3.4 | schéma 3 |
| 33 | 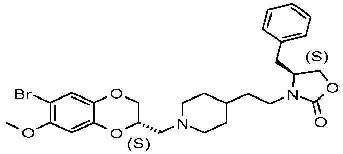 | 4.15/2.9 | schéma 2 |
| | | 3.4 | schéma 3 |
| 34 | 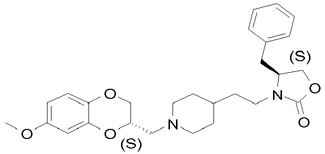 | 4.15/2.9 | schéma 2 |
| | | 3.4 | schéma 3 |
| 35 | 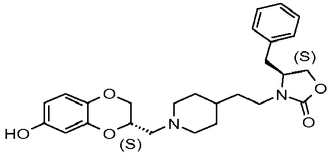 | 4.12/2.25 | schéma 2 |
| | | 3.4 | schéma 3 |
| 36 | 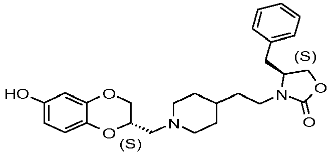 | 4.13/2.27 | schéma 2 |
| | | 3.4 | schéma 3 |
| 37 | 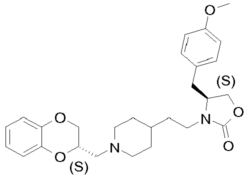 | 4.1/2.1 | schéma 2 |
| | | 3.9 | schémas 9 et 3 |
| 38 | 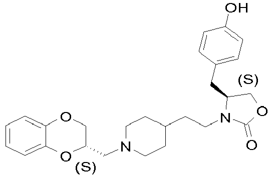 | 4.1/2.1 | schéma 2 |
| | | 3.10 | schémas 9 et 3 |
| 39 | 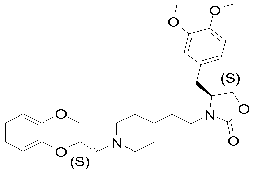 | 4.1/2.1 | schéma 2 |
| | | 3.11 | schémas 9 et 3 |
| 40 | 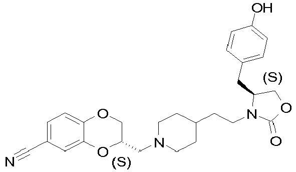 | 4.10/2.11 | schéma 2 |
| | | 3.10 | schémas 9 et 3 |
| 41 | 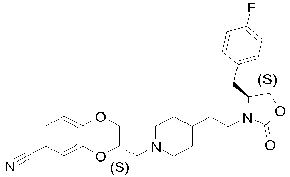 | 4.10/2.11 | schéma 2 |
| | | 3.12 | schémas 9 et 3 |
| 42 | 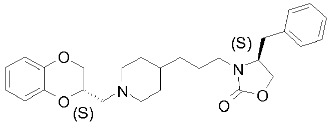 | 4.1/2.1 | schéma 2 |
| | | 3.31 | schéma 3 |
| 43 | 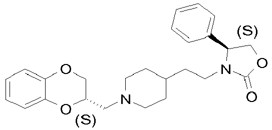 | 4.1/2.1 | schéma 2 |
| | | 3.6 | schéma 3 |
| 44 | 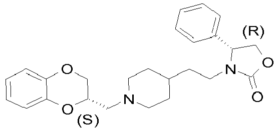 | 4.1/2.1 | schéma 2 |
| | | 3.5 | schéma 3 |
| 45 | 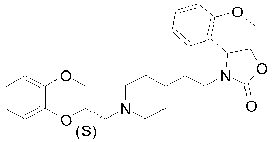 | 4.1/2.1 | schéma 2 |
| | | 3.7 | schémas 8 et 3 |
| 46 | 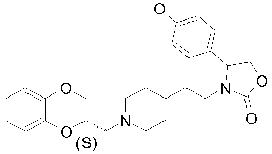 | 4.1/2.1 | schéma 2 |
| | | 3.8 | schémas 8 et 3 |
| 47 | 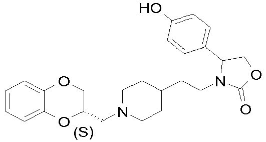 | 4.1/2.1 | schéma 2 |
| | | 3.8 | schémas 8 et 3 |
| 48 | 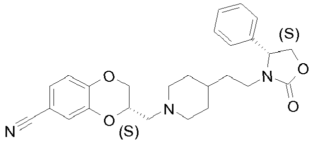 | 4.10/2.11 | schéma 2 |
| | | 3.5 | schéma 3 |
| 49 | 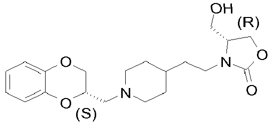 | 4.1/2.1 | schéma 2 |
| | | 3.21 | schémas 5 et 7 |
| 50 | 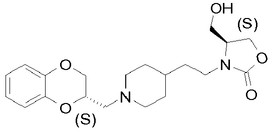 | 4.1/2.1 | schéma 2 |
| | | 3.22 | schémas 5 et 7 |
| 51 | 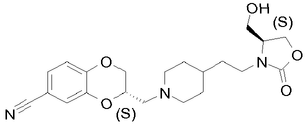 | 4.10/2.11 | schéma 2 |
| | | 3.22 | schémas 5 et 7 |
| 52 | 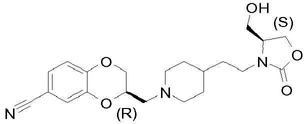 | 4.26/2.12 | schéma 2 |
| | | 3.22 | schémas 5 et 7 |
| 53 | 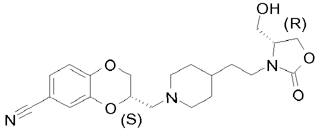 | 4.10/2.11 | schéma 2 |
| | | 3.21 | schémas 5 et 7 |
| 54 | 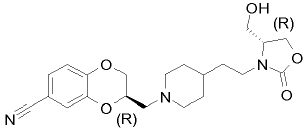 | 4.26/2.12 | schéma 2 |
| | | 3.21 | schémas 5 et 7 |
| 55 | 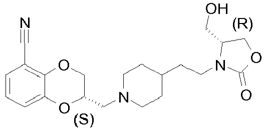 | 4.25/2.14 | schéma 2 |
| | | 3.21 | schémas 5 et 7 |
| 56 | 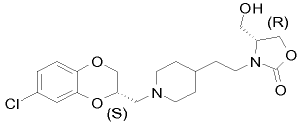 | 4.5/2.7 | schéma 2 |
| | | 3.21 | schémas 5 et 7 |
| 57 | 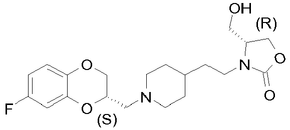 | 4.6/2.17 | schéma 2 |
| | | 3.21 | schémas 5 et 7 |
| 58 | 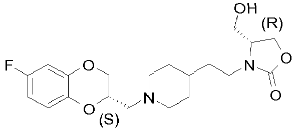 | 4.18/2.18 | schéma 2 |
| | | 3.21 | schémas 5 et 7 |
| 59 | 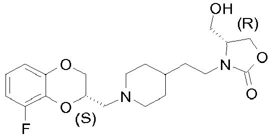 | 4.17/2.16 | schéma 2 |
| | | 3.21 | schémas 5 et 7 |
| 60 | 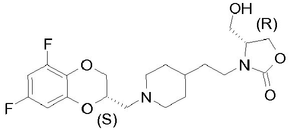 | 4.19/2.19 | schéma 2 |
| | | 3.21 | schémas 5 et 7 |
| 61 | 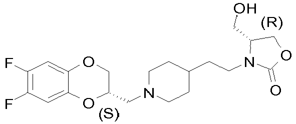 | 4.22/2.22 | schéma 2 |
| | | 3.21 | schémas 5 et 7 |
| 62 | 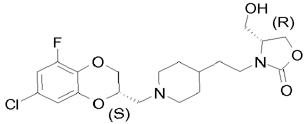 | 4.20/2.20 | schéma 2 |
| | | 3.21 | schémas 5 et 7 |
| 63 | 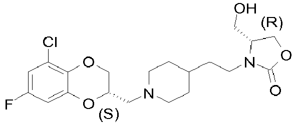 | 4.21/2.21 | schéma 2 |
| | | 3.21 | schémas 5 et 7 |
| 64 | 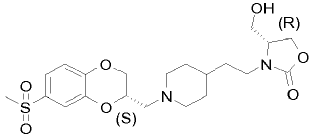 | 4.23/2.23 | schéma 2 |
| | | 3.21 | schémas 5 et 7 |
| 65 | 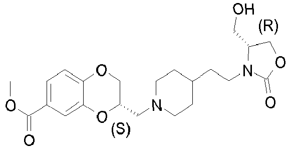 | 4.24/2.24 | schéma 2 |
| | | 3.21 | schémas 5 et 7 |
| 66 | 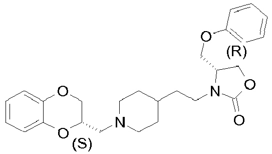 | 4.1/2.1 | schéma 2 |
| | | 3.13 | schémas 10 et 3 |
| 67 | 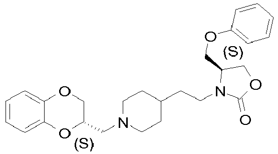 | 4.1/2.1 | schéma 2 |
| | | 3.15 | schémas 10 et 3 |
| 68 | 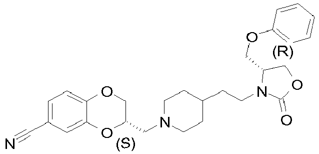 | 4.10/2.11 | schéma 2 |
| | | 3.13 | schémas 10 et 3 |
| 69 | 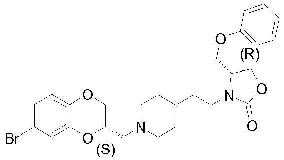 | 4.8/2.5 | schéma 2 |
| | | 3.13 | schémas 10 et 3 |
| 70 | 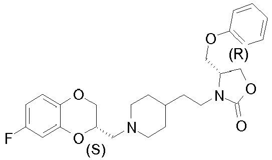 | 4.6/2.17 | schéma 2 |
| | | 3.13 | schémas 10 et 3 |
| 71 | 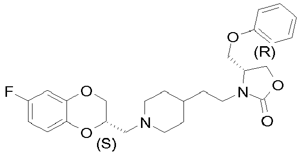 | 4.18/2.18 | schéma 2 |
| | | 3.13 | schémas 10 et 3 |
| 72 | 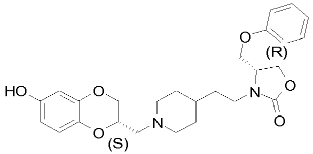 | 4.13/2.27 | schéma 2 |
| | | 3.13 | schémas 10 et 3 |
| 73 | 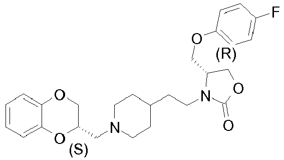 | 4.1/2.1 | schéma 2 |
| | | 3.15 | schémas 10 et 3 |
| 74 | 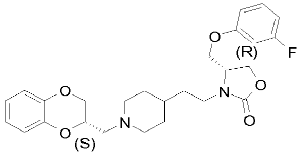 | 4.1/2.1 | schéma 2 |
| | | 3.16 | schémas 10 et 3 |
| 75 | 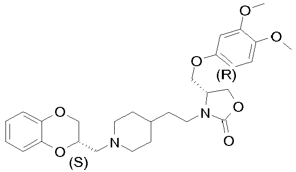 | 4.1/2.1 | schéma 2 |
| | | 3.17 | schémas 10 et 3 |
| 76 | 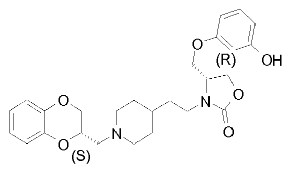 | 4.1/2.1 | schéma 2 |
| | | 3.18 | schémas 10 et 3 |
| 77 | 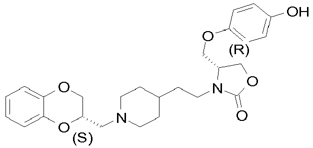 | 4.1/2.1 | schéma 2 |
| | | 3.19 | schémas 10 et 3 |
| 78 | 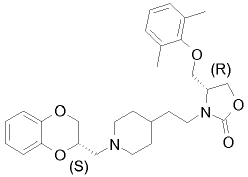 | 4.1/2.1 | schéma 2 |
| | | 3.20 | schémas 10 et 3 |
| 79 | 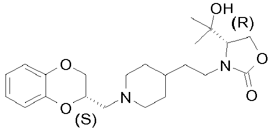 | 4.1/2.1 | schéma 2 |
| | | 3.24 | schémas 5 et 7 |
| 80 | 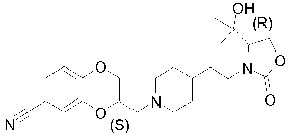 | 4.10/2.11 | schéma 2 |
| | | 3.24 | schémas 5 et 7 |
| 81 | 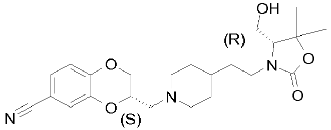 | 4.10/2.11 | schéma 2 |
| | | 3.23 | schémas 11 et 3 |
| 82 | 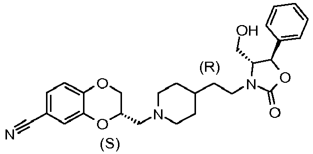 | 4.10/2.11 | schéma 2 |
| | | 3.25 | schémas 11 et 3 |
| 83 | 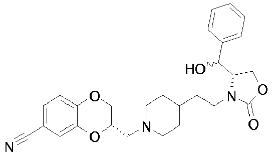 | 4.10/2.11 | schéma 2 |
| | | 3.26 | schémas 5 et 7 |
| 84 | 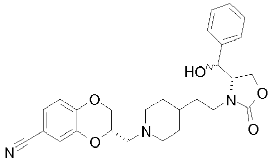 | 4.10/2.11 | schéma 2 |
| | | 3.27 | schémas 5 et 7 |
| 85 | 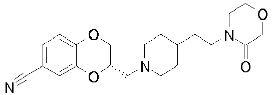 | 4.10/2.11 | schéma 2 |
| | | 3.27 | schéma 3 |
| 86 | 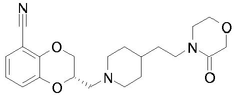 | 4.25/2.14 | schéma 2 |
| | | 3.27 | schéma 3 |
| 87 | 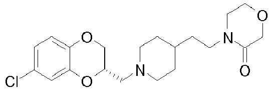 | 4.5/2.7 | schéma 2 |
| | | 3.27 | schéma 3 |
| 88 | 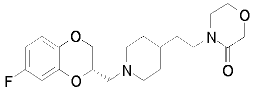 | 4.6/2.17 | schéma 2 |
| | | 3.27 | schéma 3 |
| 89 | 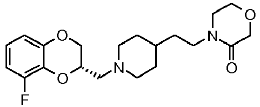 | 4.17/2.16 | schéma 2 |
| | | 3.27 | schéma 3 |
| 90 | 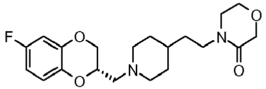 | 4.18/2.18 | schéma 2 |
| | | 3.28 | schéma 3 |
| 91 | 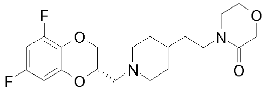 | 4.19/2.19 | schéma 2 |
| | | 3.28 | schéma 3 |
| 92 | 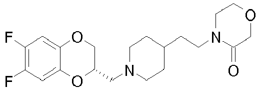 | 4.22/2.22 | schéma 2 |
| | | 3.28 | schéma 3 |
| 93 | 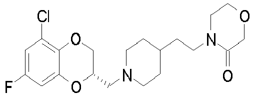 | 4.21/2.21 | schéma 2 |
| | | 3.28 | schéma 3 |
| 94 | 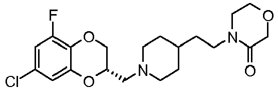 | 4.20/2.20 | schéma 2 |
| | | 3.28 | schéma 3 |
| 95 | 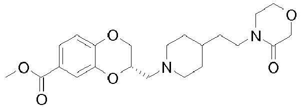 | 4.24/2.24 | schéma 2 |
| | | 3.28 | schéma 3 |
| 96 | 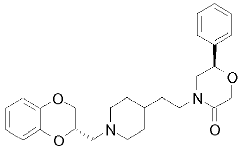 | 4.1/2.1 | schéma 2 |
| | | 3.29 | schémas 6 et 4 |
| 97 | 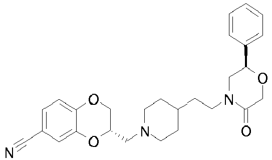 | 4.10/2.11 | schéma 2 |
| | | 3.29 | schémas 6 et 4 |
| 98 | 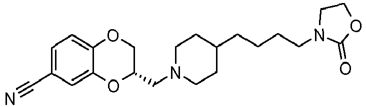 | 4.10/2.11 | schéma 2 |
| | | 3.34 | schéma 3 |
| 99 | 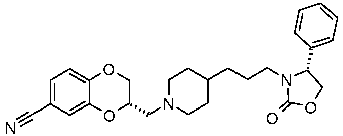 | 4.10/2.11 | schéma 2 |
| | | 3.32 | schéma 3 |
| 100 | 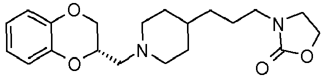 | 4.1/2.10 | schéma 2 |
| | | 3.30 | schéma 3 |
| 101 | 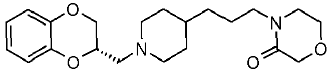 | 4.1/2.10 | schéma 2 |
| | | 3.33 | schéma 3 |
| 102 | 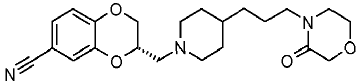 | 4.10/2.11 | schéma 2 |
| | | 3.33 | schéma 3 |
| 103 | 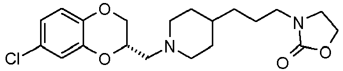 | 4.10/2.11 | schéma 2 |
| | | 3.29 | schéma 3 |
| 104 | 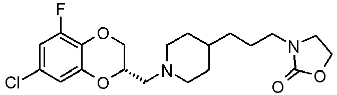 | 4.20/2.20 | schéma 2 |
| | | 3.30 | schéma 3 |
| 105 | 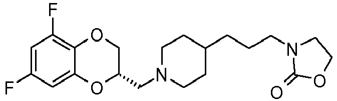 | 4.19/2.19 | schéma 2 |
| | | 3.30 | schéma 3 |
| 106 | 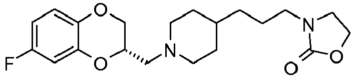 | 4.6/2.17 | schéma 2 |
| | | 3.30 | schéma 3 |
| 107 | 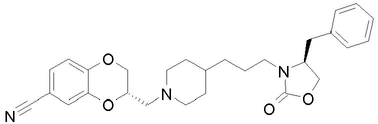 | 4.10/2.11 | schéma 2 |
| | | 3.31 | schéma 3 |
| 108 | 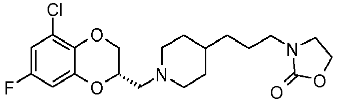 | 4.21/2.21 | schéma 2 |
| | | 3.30 | schéma 3 |
| 109 | 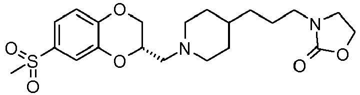 | 4.23/2.23 | schéma 2 |
| | | 3.30 | schéma 3 |
| 110 | 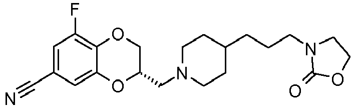 | 4.27/2.26 | schéma 2 |
| | | 3.30 | schéma 3 |

L'invention a également pour objet une composition pharmaceutique comprenant au moins un composé de formule générale (I) ou un de sels pharmaceutiquement acceptables, et un excipient pharmaceutiquement acceptable.

En tenant compte de la modulation sélective des transmissions de la dopamine et de la noradrénaline que les récepteurs D2, D3 et alpha2C exercent dans le cerveau, les composés de l'invention conviennent à diverses applications thérapeutiques. Les composés de l'invention peuvent donc être employés pour la préparation de compositions et des médicaments pharmaceutiques pour le traitement de maladies, affections ou troubles neurologiques ou psychiatriques impliquant les récepteurs D2, D3, et alpha2C, tels que la schizophrénie, le trouble bipolaire, les troubles envahissants du développement, la dépression, ainsi que les déficits cognitifs associés à divers affections neurologiques.

L'invention concerne également une méthode de traitement d'affections, maladies, ou troubles neurologiques ou psychiatriques, qui consiste à administrer à un patient qui requiert un traitement, un composé de formule 1 en quantité thérapeutiquement efficace. Elle concerne en outre les composés de formule 1 pour leur utilisation en tant que médicament et l'utilisation d'un composé de formule 1 pour la fabrication d'un médicament pour le traitement d'une maladie ou un trouble neurologique ou psychiatrique ou le dysfonctionnement de la fonction érectile ou la dépendance aux drogues et aux substances addictives.

L'invention concerne les composés de formule générale 1 pour la fabrication d'un médicament pour le traitement de la schizophrénie, le trouble bipolaire, les troubles envahissants du développement ou troubles autistiques, la dépression majeure et les troubles cognitifs liés à l'âge ou associés aux maladies neurologiques telles que la maladie de Parkinson, la maladie d'Alzheimer, et l'épilepsie.

Les composés de formule 1 selon l'invention peuvent être administrés par voie orale, systémique, parentérale, nasale ou rectale. En particulier le composé peut être administré par la voie orale dans une formulation appropriée. Les dosages des composés de formule 1 dans les compositions de l'invention peuvent être ajustés afin d'obtenir une quantité de substance active qui est efficace pour obtenir la réponse thérapeutique désirée pour une composition particulière à la méthode d'administration. Le niveau choisi de dosage dépend donc de l'effet thérapeutique désiré, de la voie de l'administration, de la durée désirée du traitement et d'autres facteurs.

Les composés de formule 1 ont été évalués *in vitro* comme ligands des récepteurs dopaminergiques et noradrénergiques et modulateurs de l'activité de ces récepteurs selon l'invention sur des cellules exprimant les récepteurs recombinant humain. Les constantes d'inhibition (Kᵢ) ont été mesurées pour les récepteurs dopaminergiques D2 et D3, et pour les récepteurs adrénergiques alpha2A, alpha2B et alpha2C, selon les méthodes décrites (Newman-Tancredi A, Assié MB, Martel JC, Cosi C, Slot LB, Palmier C, Rauly-Lestienne I, Colpaert F, Vacher B, Cussac D. F15063, a potential antipsychotic with D2/D3 antagonist, 5-HT 1A agonist and D4 partial agonist properties. I. In vitro receptor affinity and efficacy profile. Br J Pharmacol., 2007, 151:237-252). L'activité intrinsèque des produits pour le récepteur D2 a été mesurée (Bruins Slot LA, De Vries L, Newman-Tancredi A, Cussac D. Differential profile of antipsychotics at serotonin 5-HT1A and dopamine D2S receptors coupled to extracellular signal-regulated kinase. Eur J Pharmacol, 2006, 534 : 63-70 Les résultats obtenus pour certains de ces composés sont présentés dans le tableau suivant.

Des composés de formule 1 ont été évalués *in vivo* dans le test d'hyperactivité induite par le MK-801 chez la souris (Leriche L. et al., Neuropharmacology 2003, 45, 174).

Les résultats invitro et in vivo figurent à titre illustratif dans le tableau suivant :

| Exemple N° | pKi | | | | | A.I.* | ED₅₀ (mg/kg)*** |
|---|---|---|---|---|---|---|---|
| | D2 | D3 | α2A | α2B | α2C | | |
| 7 | 7.84 | 8.19 | 8.06 | 8.71 | 9.07 | 36 | 0.15 |
| 10 | 8.34 | 8.81 | 8.34 | 9.09 | 9.27 | 6 | 0.13 |
| 13 | 9.02 | 9.22 | 8.15 | 8.59 | 9.02 | 16 | 0.04 |
| 26 | 9.05 | 9.35 | 7.46 | 8.02 | 8.69 | 8 | 0.9 |
| 31 | 8.93 | 9.28 | 8.29 | 8.53 | 9.00 | 0 | 2.1 |
| 32 | 8.9 | 9.45 | 7.82 | 7.74 | 8.18 | ND | 2.4 |
| 36 | 8.67 | 8.89 | 8.22 | 8.93 | 9.25 | ND | 3.4 |
| 62 | 8.8 | 9.46 | 7.51 | 7.98 | 8.79 | 0 | 2.1 |
| 63 | 8.51 | 8.88 | 7.27 | 8.08 | 8.57 | 5 | 0.2 |
| 69 | 9.62 | 9.38 | 6.7 | 6.73 | 8.06 | 13 | 0.6 |
| 85 | 7.72 | 8.91 | 7.24 | 7.09 | 8.17 | ND** | 0.5 |
| 91 | 7.82 | 8.48 | 8.31 | 8.81 | 9.36 | 24 | 0.7 |
| 94 | 8.78 | 9.46 | 8.27 | 8.35 | 9.21 | 0 | 0.19 |

| | | | | | | | |
|---|---|---|---|---|---|---|---|
| * A.I. : Activité intrinsèque sur le récepteur D2 in vitro en pourcentage de celle de la dopamine ; ** ND : non déterminé ;*** ED₅₀ dans le test de l'hyperactivité induite par le MK-801. | | | | | | | |

L'Exemple 31 a été évalué chez le rat dans un test de mémoire, le test d'évitement passif (Chopin P, Briley M. Effects of four non-cholinergic cognitive enhancers in comparison with tacrine and galanthamine on scopolamine-induced amnesia in rats, Psychopharmacology (Berl), 1992;106:26-30). Ce test consiste à faire apprendre à un rat à ne plus entrer dans un compartiment sombre (sa place de préférence naturelle) où il a précédemment reçu un choc électrique (0.8 mA). La réticence de l'animal (le laps du temps, ou la « latence », pour entrer dans le compartiment sombre), mesuré 48 h après son entraînement, est considéré comme une indice de son mémoire explicite-déclarative. La latence augmente fortement lorsqu'un produit amnésiant comme la scopolamine est administré juste après avoir reçu le premier choc. Un produit aux propriétés promnésiques s'oppose aux effets de la scopolamine. L'exemple 31, administré aux doses de 0.16, 0.63, 2.5 et 10 mg/kg, s'est opposé à l'effet de la scopolamine : les latences exprimées en secondes étaient de 24 ± 12, 60 ± 21 et 78 ± 22, respectivement après traitement par le véhicule et par l'exemple 1 aux doses de 0.63 et 2.5 mg/kg (P < 0.05 pour les 2 doses de l'exemple 1 vs. véhicule par le test de Mann-Whitney).

La dose quotidienne totale des composés utiles selon cette invention administrée dans les doses simples ou divisées peut être dans les quantités, par exemple, de environ de 0.001 à environ 100 mg/kg de poids corporel quotidien.

Le niveau de dose spécifique pour n'importe quel patient particulier dépendra d'une variété de facteurs comprenant le poids corporel, la santé générale, le sexe, le régime, la période et la voie de l'administration, les taux d'absorption et de résorption intestinale et d'excrétion, la combinaison avec d'autres médicaments et la sévérité de l'affection particulière étant traitée.

## Revendications

1. Un composé de formule générale 1 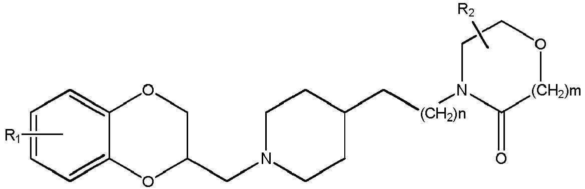 dans laquelle :
- R1 représente un ou plusieurs substituant(s) identique(s) ou différent(s) sur le noyau benzénique, chacun représentant indépendamment un atome d'hydrogène ou un halogène, ou un groupe alkyle en C₁₋₄, ou un groupe alkoxy en C₁₋₄,ou un groupe hydroxyalkyle en C₁₋₄, ou un groupe alkylcarbonyle, ou un groupe alkoxycarbonyle, ou un groupe OH, ou un groupe SO2R avec R alkyle, ou un groupe CN, ou un groupe CF3,
ou un groupe OCF3 ;
- n = 1, 2 ou 3 ;
- R2 représente un ou plusieurs substituant(s) identique(s) ou différent(s) sur les noyaux oxazolidinone ou morpholinone, chacun représentant indépendamment :
un atome d'hydrogène, ou un groupe alkyle en C₁₋₄, ou un groupe alkoxy en C₁₋₄, ou un groupe hydroxyalkyle en C₁₋₄,ou un groupe alkylcarbonyle, ou un groupe alkoxycarbonyle, ou un groupe alkoxyphenyl - éventuellement substitué par un ou plusieurs substituant(s) identique(s) ou différent(s), chacun représentant indépendamment un groupe alkoxy en C₁₋₄, ou un groupe alkyl en C₁₋₄ ou un halogène, ou un hydroxy- ou un groupe alkoxybenzyle - éventuellement substitué par un ou plusieurs substituant(s) identique(s) ou différent(s), chacun représentant indépendamment un groupe alkoxy en C₁₋₄, ou un groupe alkyl en C₁₋₄ ou un halogène, ou un hydroxy- ou un groupe hydroxybenzyle, ou un groupe benzyle - éventuellement substitué par un ou plusieurs substituant(s) identique(s) ou différent(s), chacun représentant indépendamment un groupe alkoxy en C₁₋₄, ou un groupe alkyl en C₁₋₄ ou un halogène, ou un hydroxy - ou un groupe phényle - éventuellement substitué par un ou plusieurs substituant(s) identique(s) ou différent(s), chacun représentant indépendamment un groupe alkoxy en C₁₋₄, ou un groupe alkyl en C₁₋₄ ou un halogène, ou un hydroxy - ou alors R2 constitue un cycle fusionné avec le groupe oxazolidinone ou morpholinone qui le porte, constitué par un benzène substitué ou non par un alkoxy en C₁₋₄, ou un hydroxy, ou un halogène ou un cyano ;
- m = 0 ou 1.

2. Composé selon la revendication 1, **caractérisé en ce que** n = 1 et m = 0.

3. Composé selon la revendication 1, **caractérisé en ce que** n = 1 et m = 1.

4. Composé selon la revendication 1, **caractérisé en ce que** n = 2 et m = 0.

5. Composé selon la revendication 1, **caractérisé en ce que** n = 2 et m = 1.

6. Composé selon la revendication 1, **caractérisé en ce que** n = 3 et m = 0.

7. Composé selon la revendication 1, **caractérisé en ce que** n = 3 et m = 1.

8. Composé selon l'une des revendications 1 à 6, **caractérisé en ce qu'**il est sélectionné parmi le groupe de composés :

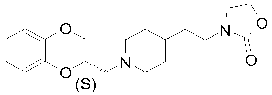

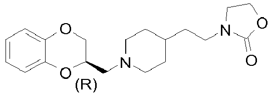

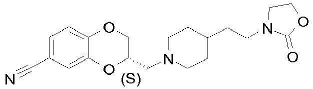

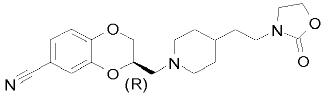

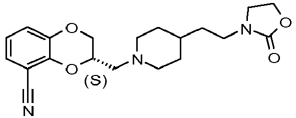

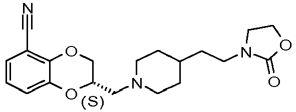

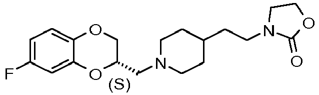

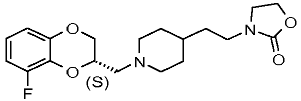

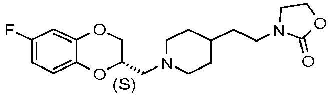

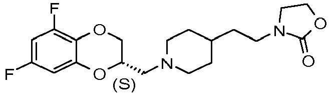

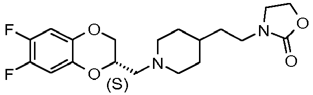

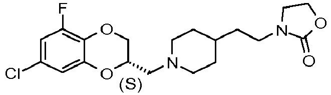

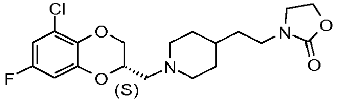

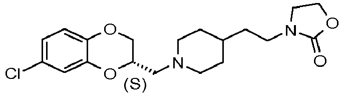

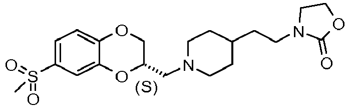

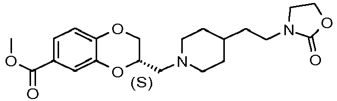

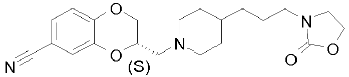

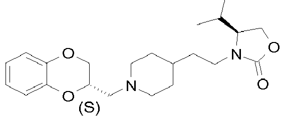

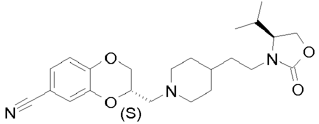

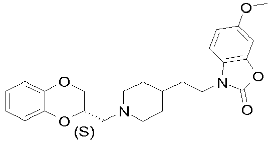

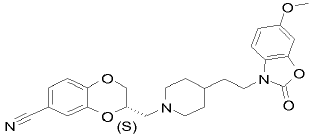

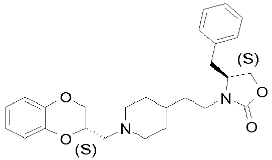

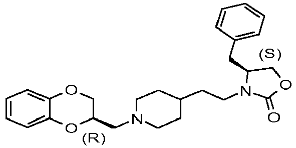

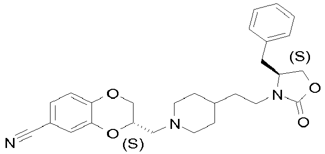

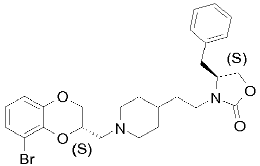

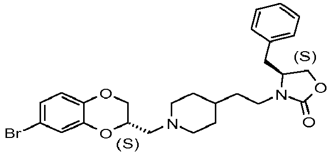

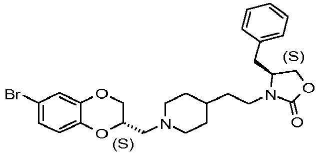

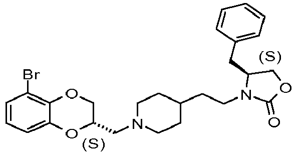

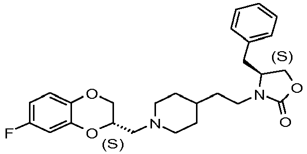

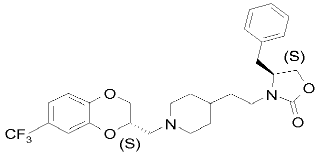

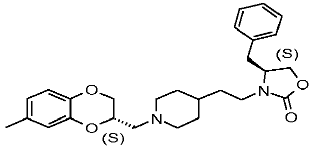

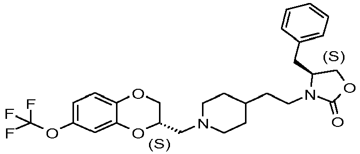

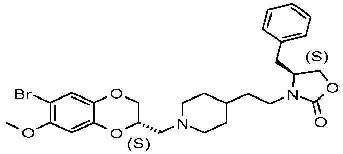

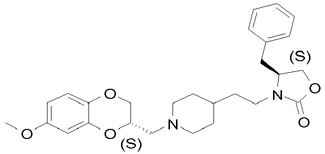

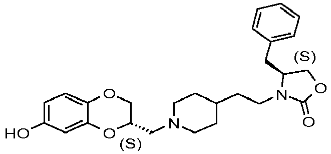

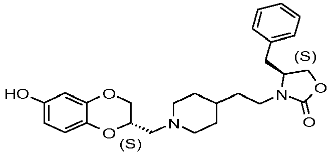

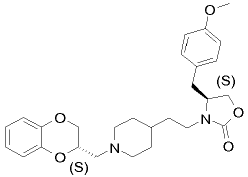

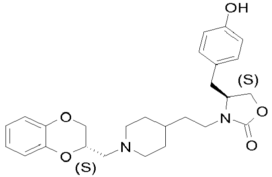

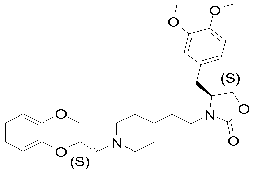

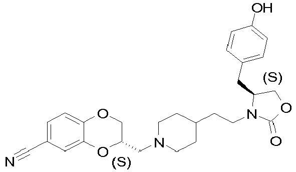

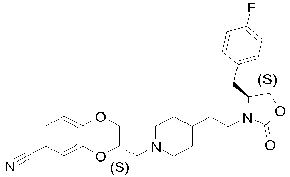

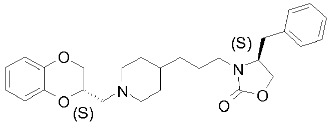

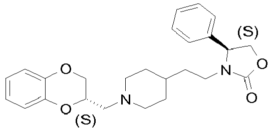

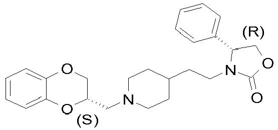

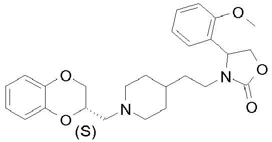

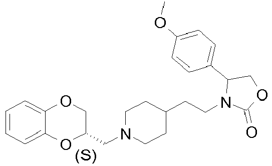

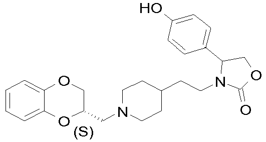

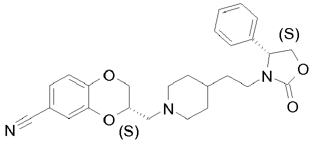

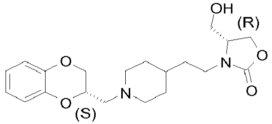

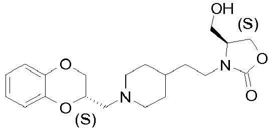

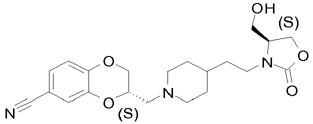

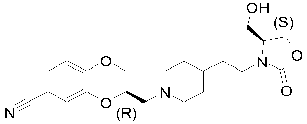

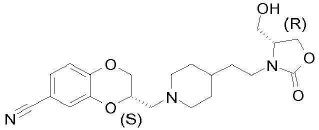

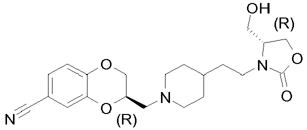

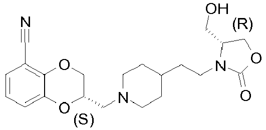

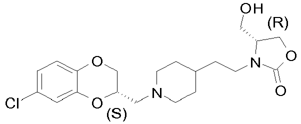

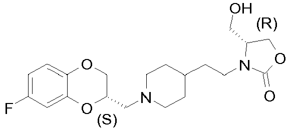

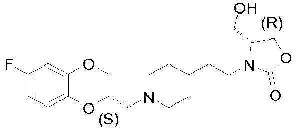

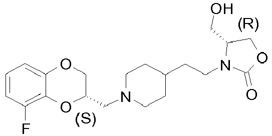

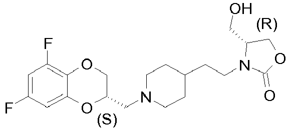

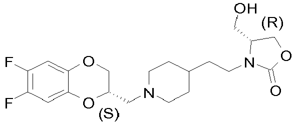

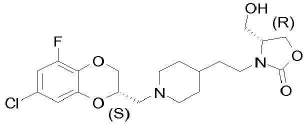

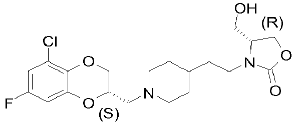

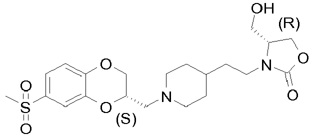

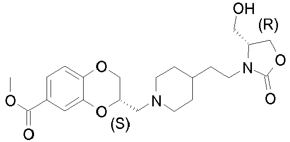

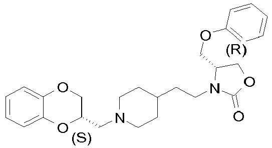

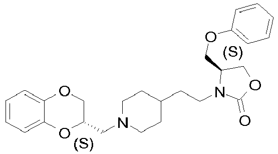

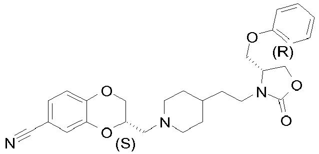

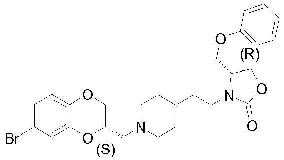

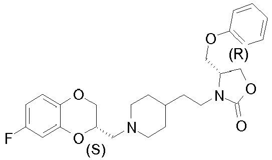

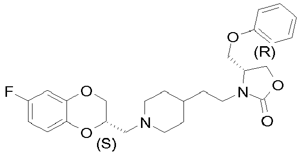

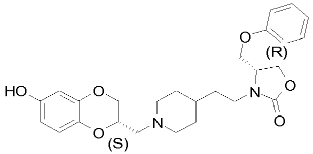

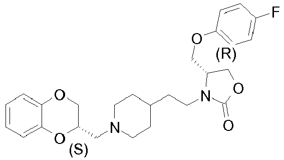

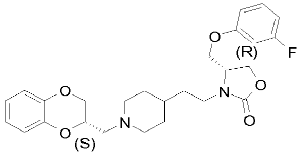

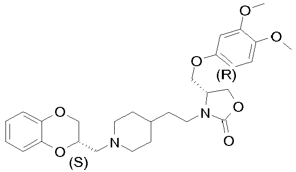

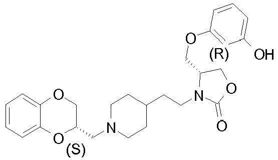

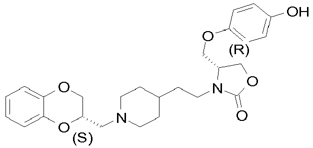

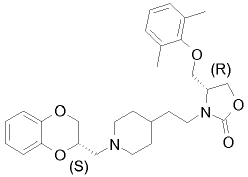

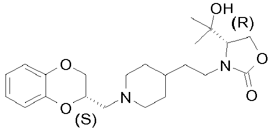

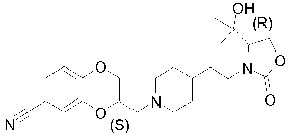

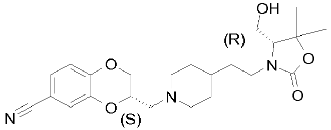

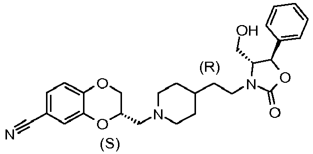

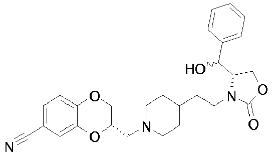

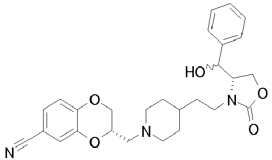

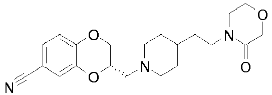

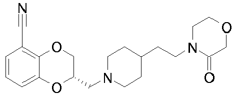

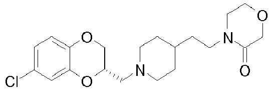

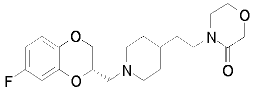

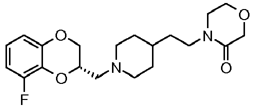

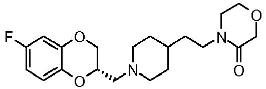

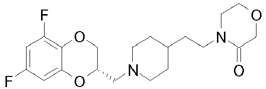

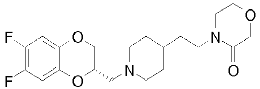

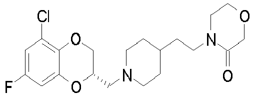

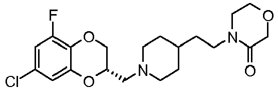

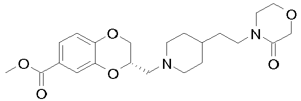

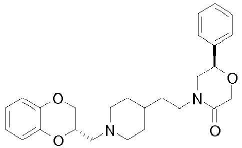

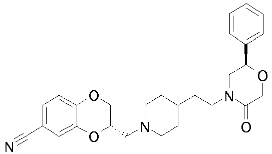

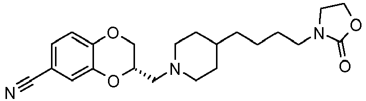

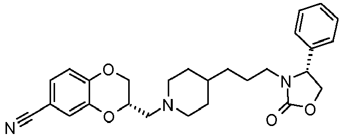

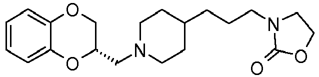

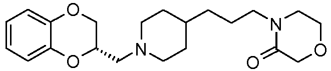

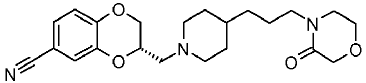

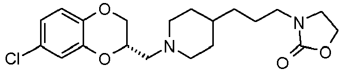

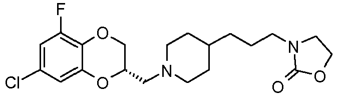

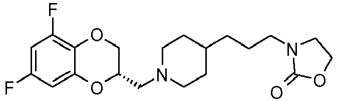

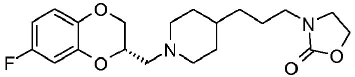

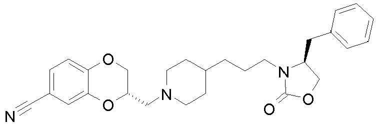

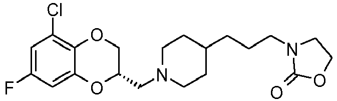

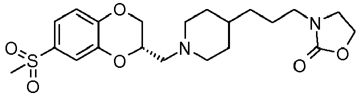

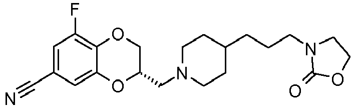

9. Formes stéréoisomériques des composés selon l'une des revendications 1 à 8 et leurs sels.

10. Composition pharmaceutique comprenant au moins un composé selon l'une des revendications 1 à 9 ou un de ses sels pharmaceutiquement acceptables et un excipient pharmaceutiquement acceptable.

11. Composé selon l'une des revendications 1 à 9 pour son utilisation en tant que médicament.

12. Composé selon la revendication 11 pour son utilisation en tant que médicament pour le traitement de la schizophrénie, les troubles bipolaires, les troubles envahissants du développement et la dépression.

13. Composé selon la revendication 11 pour son utilisation en tant que médicament pour le traitement des troubles cognitifs associés à l'âge, le trouble du déficit de l'attention avec hyperactivité, une maladie neurodégénérative les accidents vasculaires cérébraux et leurs conséquences, l'épilepsie, les troubles de l'humeur, les troubles d'anxiété et des maladies mentales propagées par le stress.

## Patentansprüche

1. Verbindung der allgemeinen Formel 1 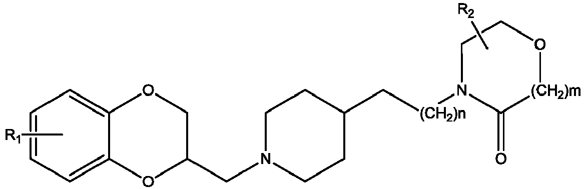 in welcher:
- R₁ einen oder mehrere identische oder verschiedene Substituenten auf dem Benzolring darstellt, wobei jeder unabhängig ein Wasserstoffatom oder ein Halogen oder eine C₁₋₄-Alkylgruppe oder eine C₁₋₄-Alkoxygruppe oder eine C₁₋₄-Hydroxyalkylgroppe oder eine Alkylcarbonylgruppe oder eine Alkoxycarbonylgruppe oder eine OH-Gruppe oder eine SO₂R-Grupp mit R als Alkyl oder eine CN-Gruppe oder eine CF₃-Gruppe oder eine OCF₃-Gruppe darstellt;
- n = 1, 2 oder 3;
- R₂ einen oder mehrere identische oder verschiedene Substituenten auf dem Oxazolidinon- oder Morpholinonring darstellt, wobei jeder unabhängig darstellt:
ein Wasserstoffatom oder eine C₁₋₄-Alkylgruppe oder eine C₁₋₄-Alkoxygruppe oder eine C₁₋₄-Hydroxyalkylgruppe oder eine Alkylcarbonylgruppe oder eine Alkoxycarbonylgruppe oder eine Alkoxyphenylgruppe - eventuell substituiert durch einen oder mehrere identische oder unterschiedliche Substituenten, wobei jeder unabhängig eine C₁₋₄-Alkoxygruppe oder eine C₁₋₄-Alkylgruppe oder ein Halogen oder ein Hydroxy-oder eine Alkoxybenzylgruppe darstellt - eventuell substituiert durch einen oder mehrere identische oder unterschiedliche Substituenten, wobei jeder unabhängig eine C₁₋₄-Alkoxygruppe oder eine C₁₋₄-Alkylgruppe oder ein Halogen oder ein Hydroxy- oder eine Hydroxybenzylgruppe oder eine Benzylgruppe darstellt - eventuell substituiert durch einen oder mehrere identische oder unterschiedliche Substituenten, wobei jeder unabhängig eine C₁₋₄-Alkoxygruppe oder eine C₁₋₄-Alkylgruppe oder ein Halogen oder ein Hydroxy- oder eine Phenylgruppe darstellt - eventuell substituiert durch einen oder mehrere identische oder unterschiedliche Substituenten, wobei jeder unabhängig eine C₁₋₄-Alkoxygruppe oder eine C₁₋₄-Alkylgruppe oder ein Halogen oder ein Hydroxy- darstellt - oder R₂ einen Ring darstellt, der mit der Oxazolidinon- oder Morpholinongruppe verschmolzen ist, der es trägt, gebildet von einem Benzol, substituiert oder nicht durch ein C₁₋₄-Alkoxy oder ein Hydroxy oder ein Halogen oder ein Cyano;
- m = 0 oder 1.

2. Verbindung nach Anspruch 1, **dadurch gekennzeichnet, dass** n = 1 und m = 0 ist.

3. Verbindung nach Anspruch 1, **dadurch gekennzeichnet, dass** n = 1 und m = 1 ist.

4. Verbindung nach Anspruch 1, **dadurch gekennzeichnet, dass** n = 2 und m = 0 ist.

5. Verbindung nach Anspruch 1, **dadurch gekennzeichnet, dass** n = 2 und m = 1 ist.

6. Verbindung nach Anspruch 1, **dadurch gekennzeichnet, dass** n = 3 und m = 0 ist.

7. Verbindung nach Anspruch 1, **dadurch gekennzeichnet, dass** n = 3 und m = 1 ist.

8. Verbindung nach einem der Ansprüche 1 bis 6, **dadurch gekennzeichnet, dass** sie aus der Gruppe von Verbindungen ausgewählt ist:

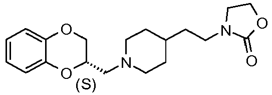

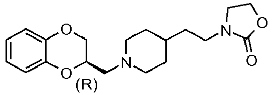

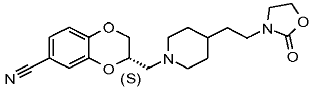

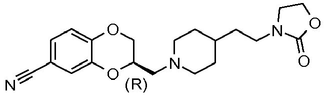

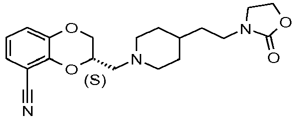

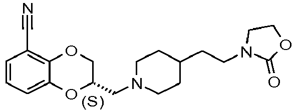

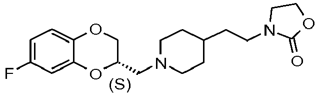

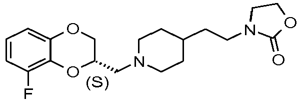

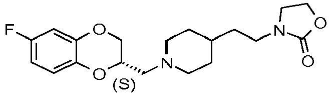

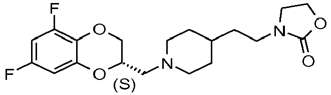

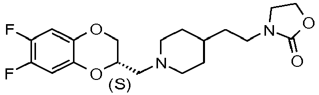

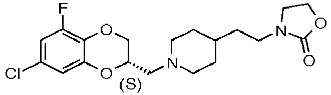

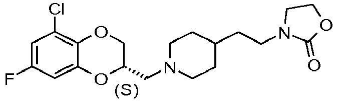

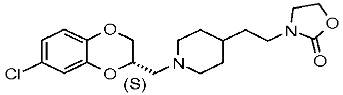

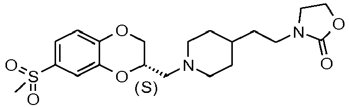

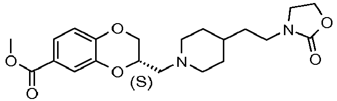

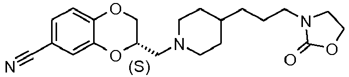

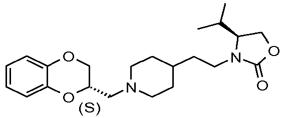

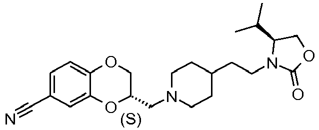

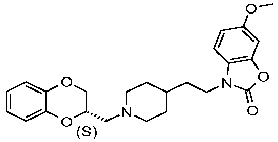

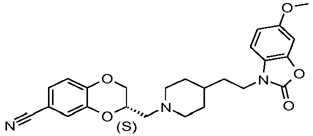

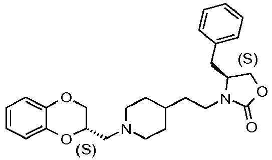

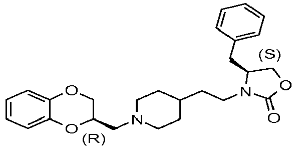

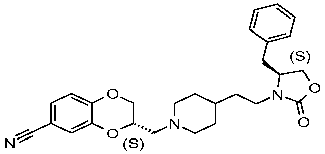

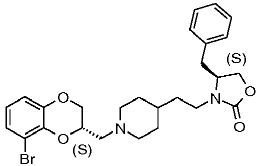

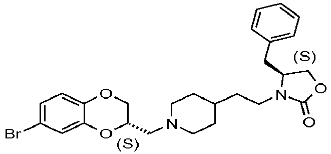

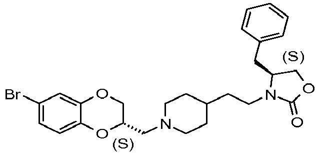

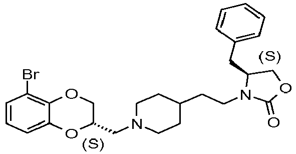

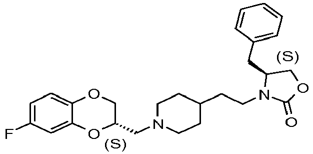

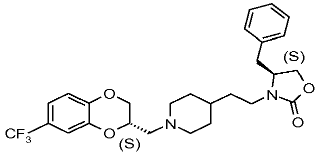

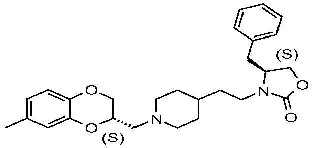

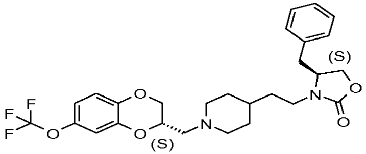

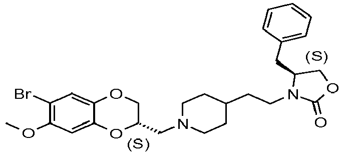

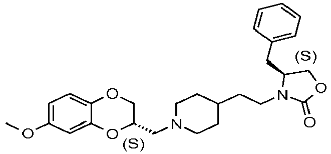

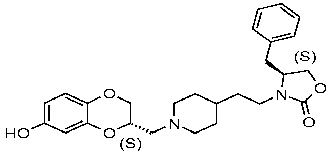

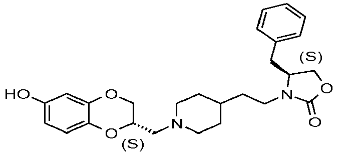

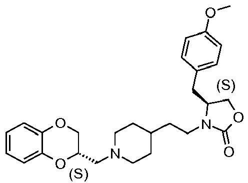

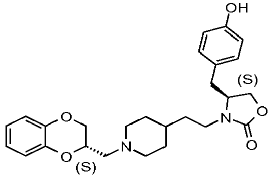

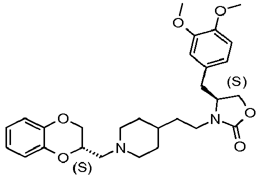

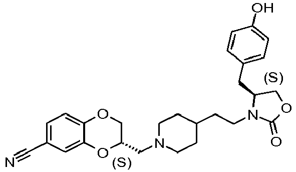

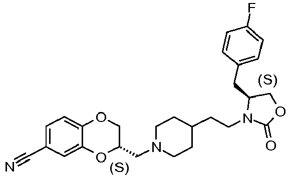

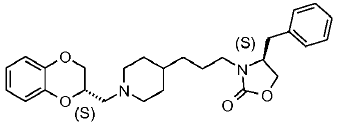

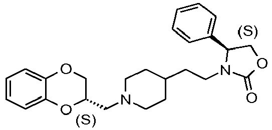

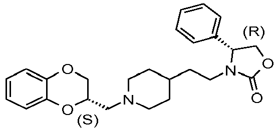

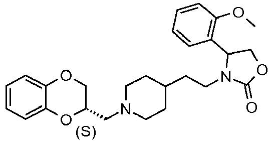

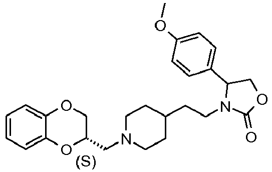

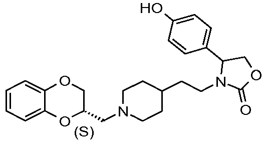

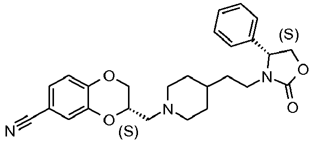

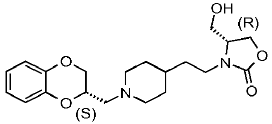

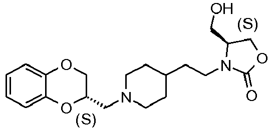

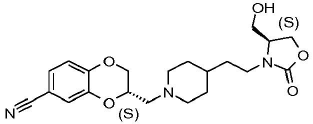

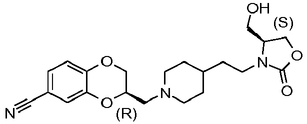

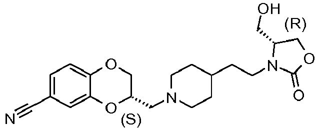

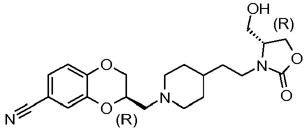

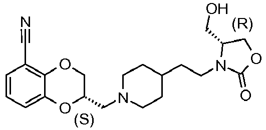

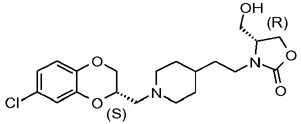

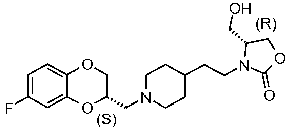

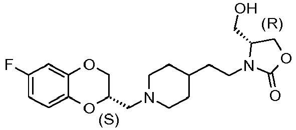

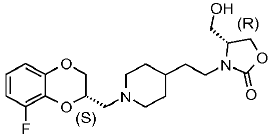

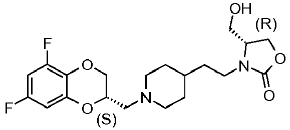

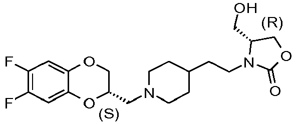

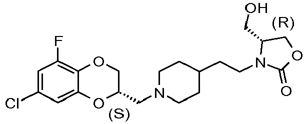

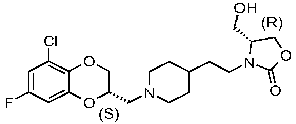

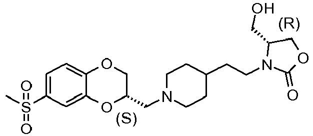

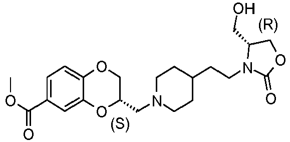

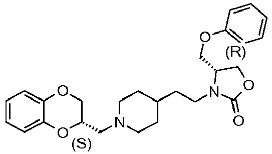

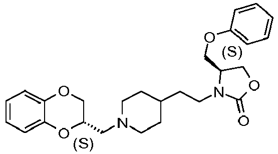

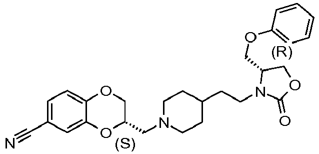

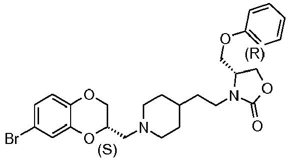

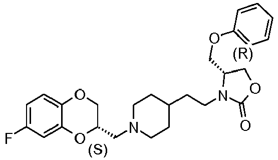

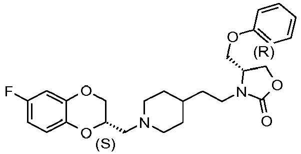

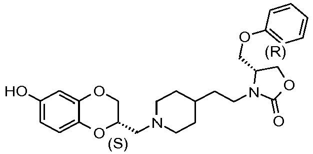

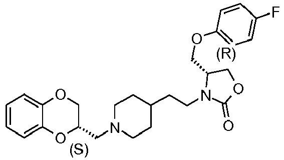

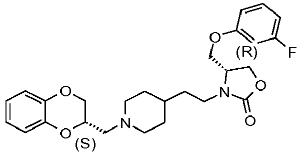

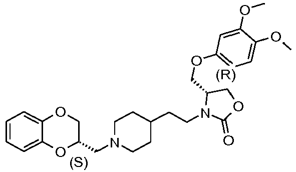

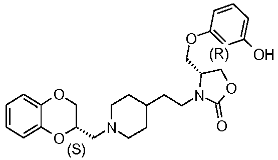

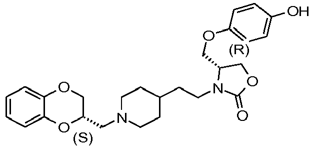

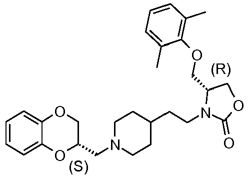

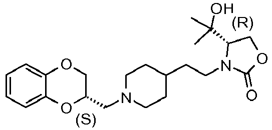

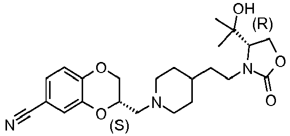

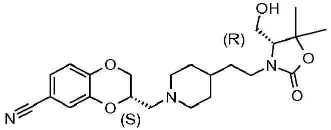

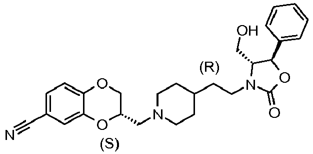

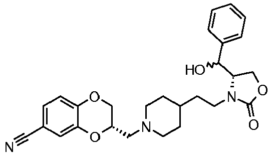

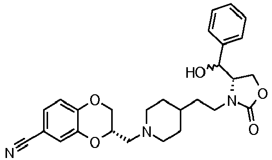

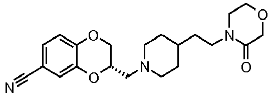

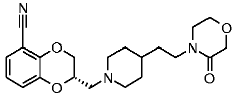

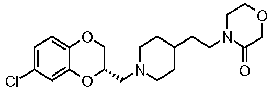

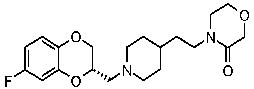

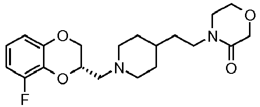

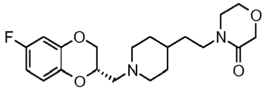

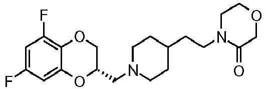

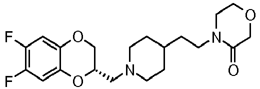

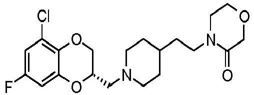

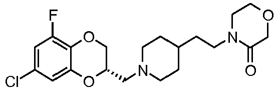

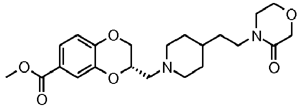

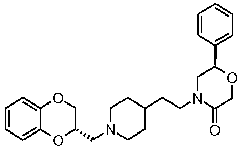

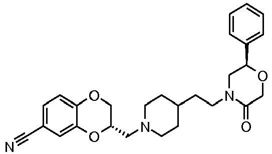

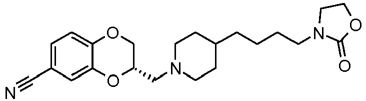

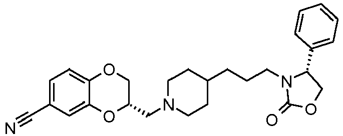

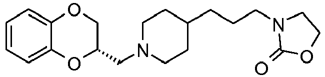

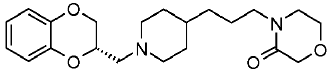

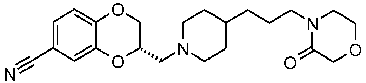

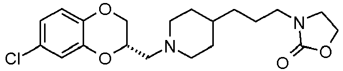

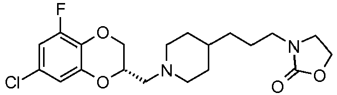

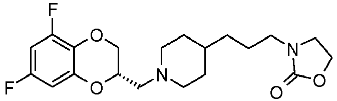

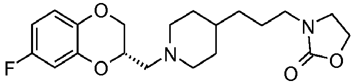

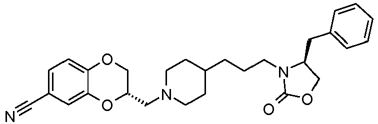

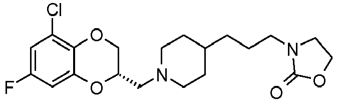

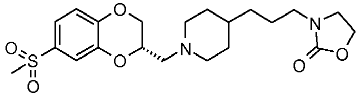

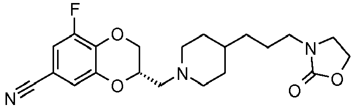

9. Stereoisomere Formen der Verbindungen nach einem der Ansprüche 1 bis 8 und ihre Salze.

10. Pharmazeutische Zusammensetzung, umfassend mindestens eine Verbindung nach einem der Ansprüche 1 bis 9 oder eines ihrer pharmazeutisch akzeptablen Salze und einen pharmazeutisch akzeptablen Hilfsstoff.

11. Verbindung nach einem der Ansprüche 1 bis 9 für ihre Verwendung als Arzneimittel.

12. Verbindung nach Anspruch 11 für ihre Verwendung als Arzneimittel zur Behandlung der Schizophrenie, der bipolaren Störungen, der tiefgreifenden Entwicklungsstörungen und der Depression.

13. Verbindung nach Anspruch 11 für die Verwendung als Arzneimittel zur Behandlung der altersbedingten kognitiven Störungen, der Aufmerksamkeitsdefizitstörung mit Hyperaktivität, einer neurodegenerativen Krankheit, der Schlaganfälle und ihrer Folgen, der Epilepsie, der Stimmungsschwankungen, der Angststörungen und der stressbedingten mentalen Krankheiten.

## Claims

1. A compound of general formula 1 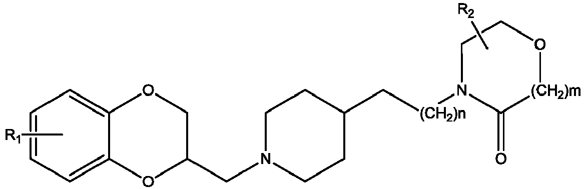 in which:
- R₁ is one or more identical or different substituent(s) on the benzene ring, each independently being a hydrogen or a halogen atom, or a C₁₋₄ alkyl group, or a C₁₋₄ alkoxy group, or a C₁₋₄ hydroxyalkyl group, or an alkylcarbonyl group, or an alkoxycarbonyl group, or an OH group, or an SO₂R group with R being an alkyl, or a CN group, or a CF₃ group, or an OCF₃ group;
- n = 1, 2 or 3;
- R₂ is one or more identical or different substituent(s) on the oxazolidinone or morpholinone rings, each independently being:
a hydrogen atom, or a C₁₋₄ alkyl group, or a C₁₋₄ alkoxy group, or a C₁₋₄ hydroxyalkyl group, or an alkylcarbonyl group, or an alkoxycarbonyl group, or an alkoxyphenyl group - optionally substituted with one or more identical or different substituent(s), each independently being a C₁₋₄ alkoxy group, or a C₁₋₄ alkyl group or a halogen, or a hydroxy - or an alkoxybenzyl group - optionally substituted with one or more identical or different substituent(s), each independently being a C₁₋₄ alkoxy group, or a C₁₋₄ alkyl group or a halogen, or a hydroxy - or a hydroxybenzyl group, or a benzyl group - optionally substituted with one or more identical or different substituent(s), each independently being a C₁₋₄ alkoxy group, or a C₁₋₄ alkyl group or a halogen, or a hydroxy - or a phenyl group - optionally substituted with one or more identical or different substituent(s), each independently being a C₁₋₄ alkoxy group, or a C₁₋₄ alkyl group or a halogen, or a hydroxy - or else R₂ is a ring fused with the oxazolidinone or morpholinone group that bears it, constituted by a benzene substituted or not with a C₁₋₄ alkoxy, or a hydroxy, or a halogen or a cyano;
- m = 0 or 1.

2. The compound according to claim 1, **characterized in that** n = 1 and m = 0.

3. The compound according to claim 1, **characterized in that** n = 1 and m = 1.

4. The compound according to claim 1, **characterized in that** n = 2 and m = 0.

5. The compound according to claim 1, **characterized in that** n = 2 and m = 1.

6. The compound according to claim 1, **characterized in that** n = 3 and m = 0.

7. The compound according to claim 1, **characterized in that** n = 3 and m = 1.

8. The compound according to one of claims 1 to 6, **characterized in that** it is selected from the group of compounds:

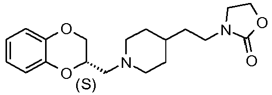

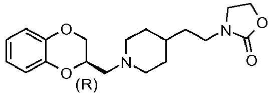

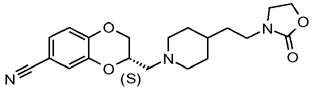

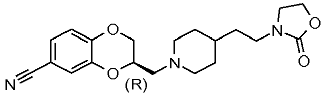

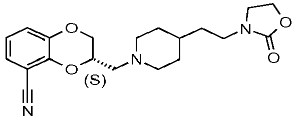

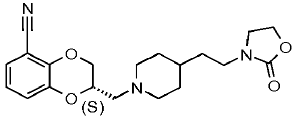

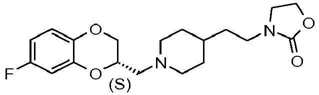

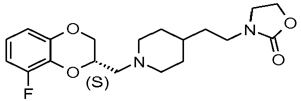

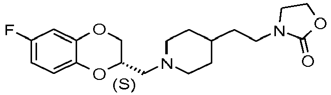

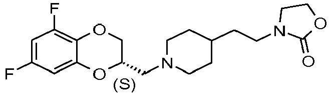

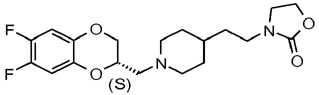

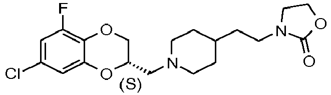

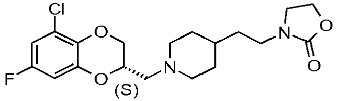

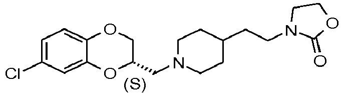

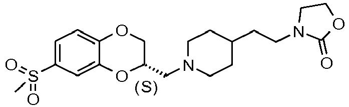

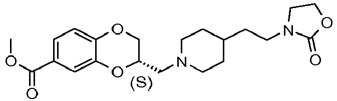

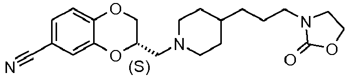

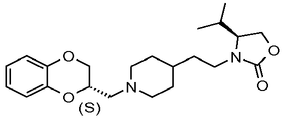

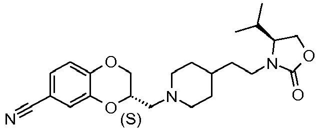

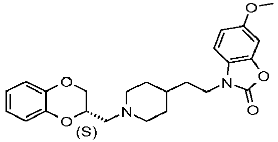

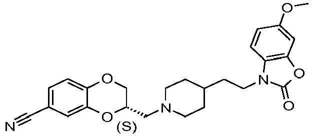

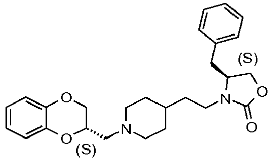

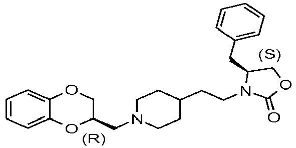

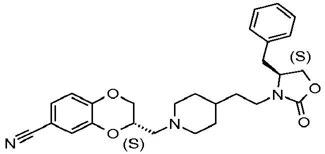

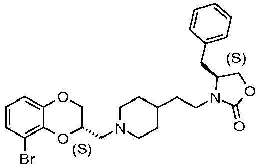

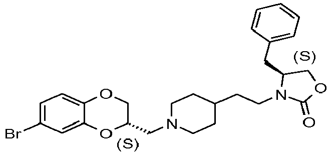

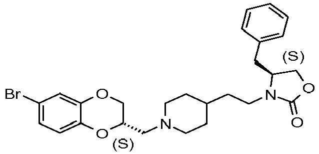

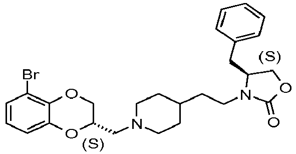

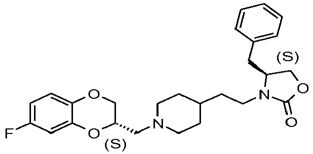

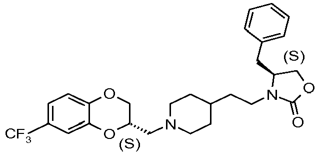

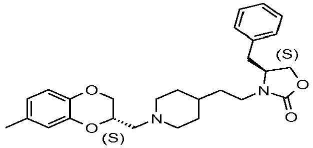

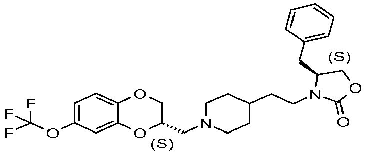

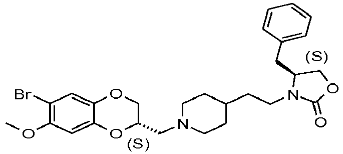

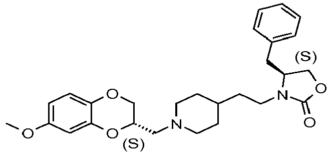

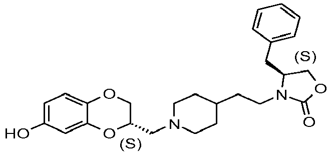

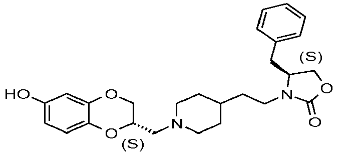

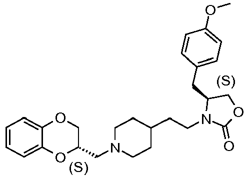

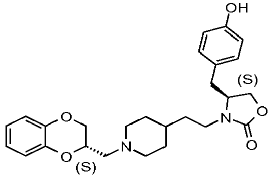

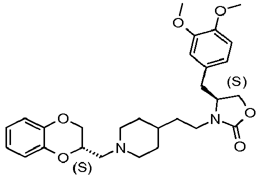

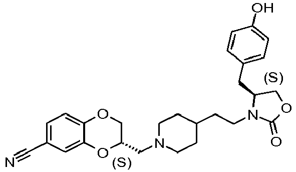

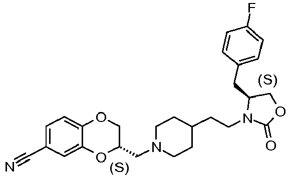

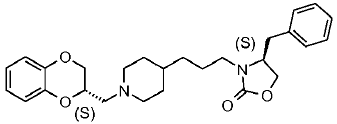

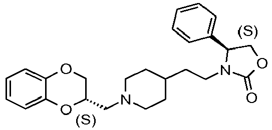

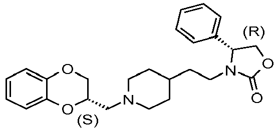

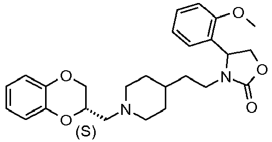

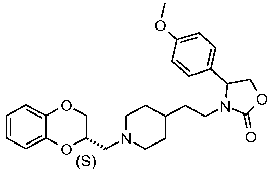

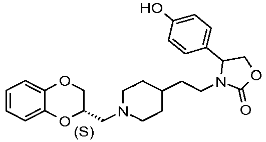

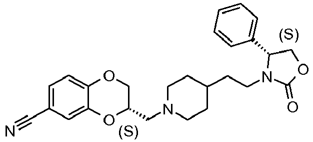

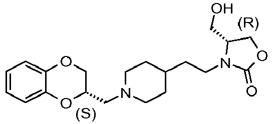

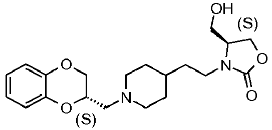

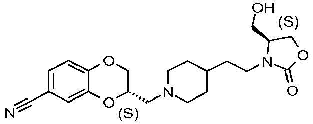

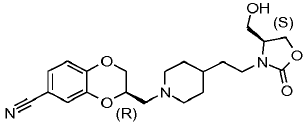

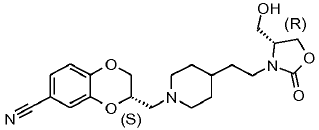

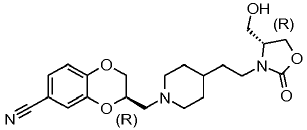

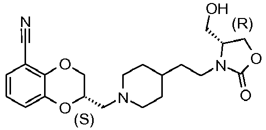

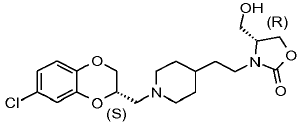

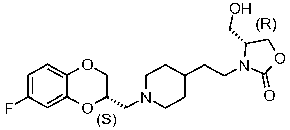

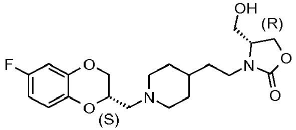

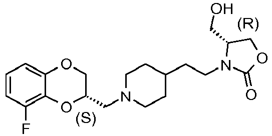

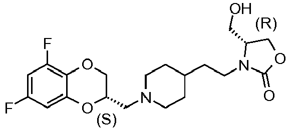

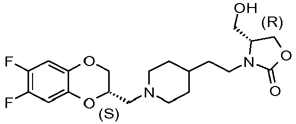

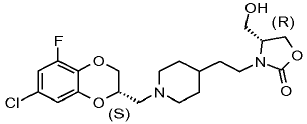

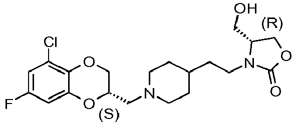

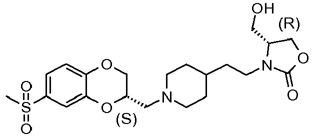

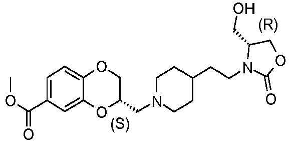

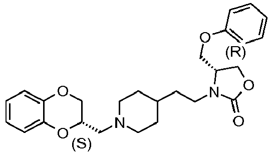

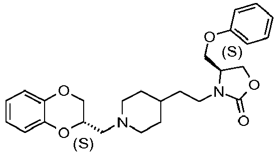

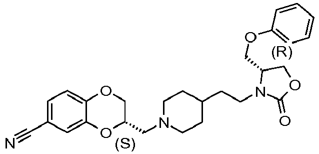

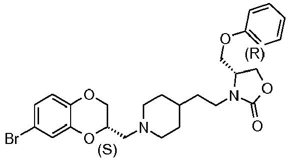

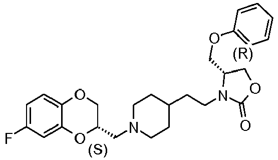

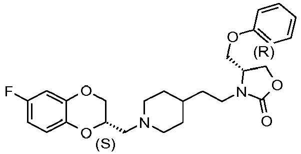

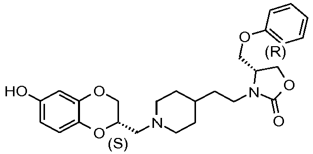

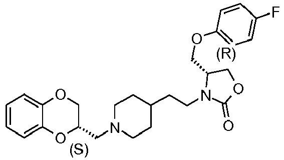

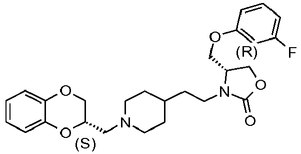

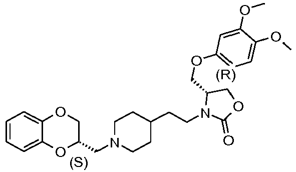

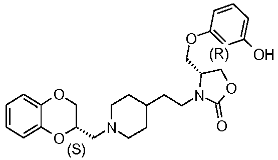

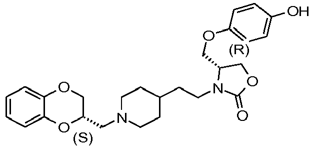

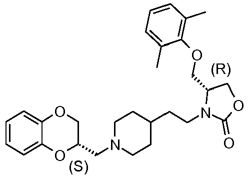

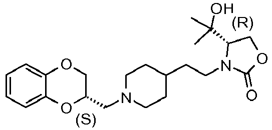

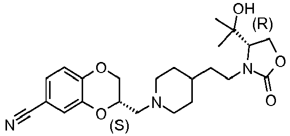

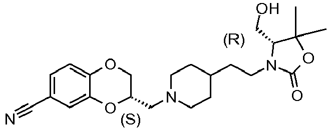

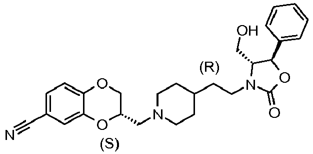

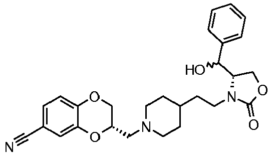

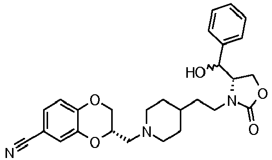

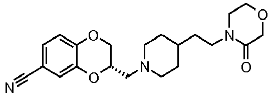

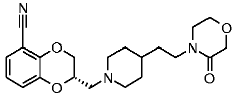

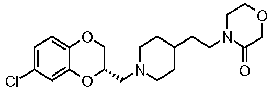

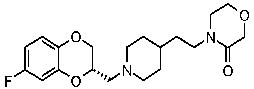

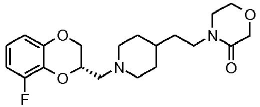

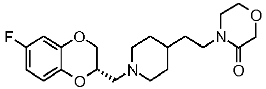

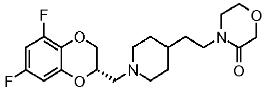

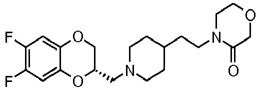

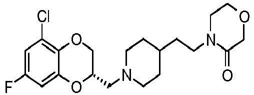

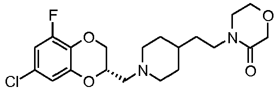

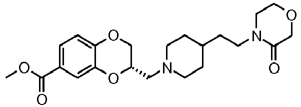

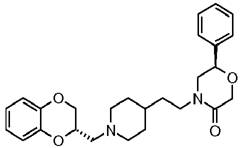

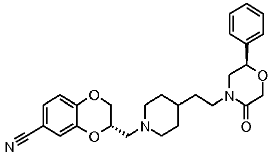

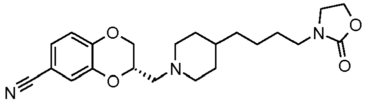

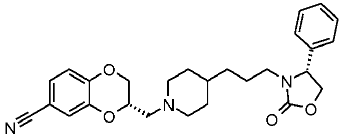

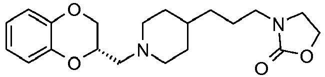

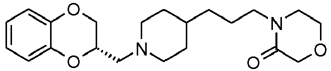

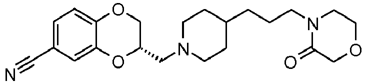

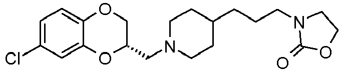

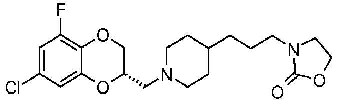

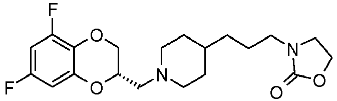

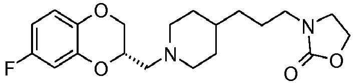

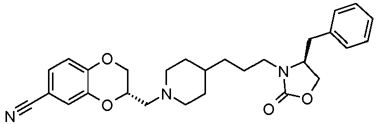

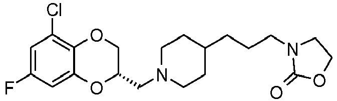

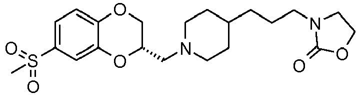

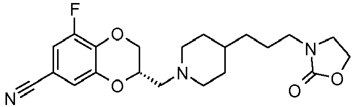

9. Stereoisomeric forms of the compounds according to one of claims 1 to 8 and salts thereof.

10. A pharmaceutical composition comprising at least one compound according to one of claims 1 to 9 or a pharmaceutically acceptable salt thereof and a pharmaceutically acceptable excipient.

11. The compound according to one of claims 1 to 9 for use as a drug.

12. The compound according to claim 11 for use as a medication for the treatment of schizophrenia, bipolar disorders, pervasive developmental disorders and depression.

13. The compound according to claim 11 for use as a medication for the treatment of age-associated cognitive disorders, attention-deficit/hyperactivity disorder, neurodegenerative disease, cerebrovascular accidents and the consequences thereof, epilepsy, mood disorders, anxiety disorders and stress-mediated mental illness.
